# EUROPEAN PATENT SPECIFICATION

(11) **EP 1 786 905 B1**
(45) Date of publication and mention of the grant of the patent: **03.08.2011**
(21) Application number: 05774823.8
(22) Date of filing: 18.08.2005
(51) Int. Cl.: C12N 15/11, C07H 21/02, A61P 35/04, A61K 31/7105, A61K 48/00

(54) **SMALL INTERFERING RNA MOLECULES AGAINST RIBONUCLEOTIDE REDUCTASE AND USES THEREOF**
KLEINE INTERFERIERENDE RNA-MOLEKÜLE GEGEN RIBONUKLEOTIDREDUKTASE UND IHRE VERWENDUNGEN
MOLECULES DE PETIT ARN INTERFERANT DIRIGEES CONTRE LA RIBONUCLEOTIDE REDUCTASE ET SES UTILISATIONS

(30) Priority: 18.08.2004 US 602824 P
(43) Date of publication of application: 23.05.2007
(73) Proprietor: Lorus Therapeutics Inc., Toronto, Ontario M9W 4Z7 (CA)
(72) Inventor: YOUNG, Aiping, H., North York, Ontario M2R 1P3 (CA); WRIGHT, Jim, A., Oakville, Ontario L6L 5Z3 (CA)
(74) Representative: Dempster, Robert Charles
(86) International application number: PCT/CA2005/001258
(87) International publication number: WO 2006/017932

(56) References cited:
- WO-A-03/070918
- WO-A1-00/47733
- WO-A2-98/05769
- DUXBURY M S ET AL: "Retrovirally mediated RNA interference targeting the M2 subunit of ribonucleotide reductase: A novel therapeutic strategy in pancreatic cancer" SURGERY, C.V. MOSBY CO., ST. LOUIS, US, vol. 136, no. 2, 1 August 2004 (2004-08-01), pages 261-269, XP004541864 ISSN: 0039-6060
- PING LIN Z. ET AL: 'Stable suppression of the R2 subunit of ribonucleotide reductase by R2-targeted short interference RNA sensitizes p53(-/-) HCT-116 colon cancer cells to DNA-damaging agents and ribonucleotide reductase inhibitors' JOURNAL OF BIOLOGICAL CHEMISTRY vol. 279, no. 26, 25 June 2004, pages 27030 - 27038, XP002407218
- DATABASE GENBANK [Online] STRAUSBERG R.L. ET AL: 'Generation and initial analysis of more than 15,000 full-length human and mouse cDNA sequences', XP008097186 Database accession no. (BC01886) & NATIONAL CENTER FOR BIOTECHNOLOGY INFORMATION 2005,
- DUXBURY M.S. ET AL: 'RNA interference targeting the M2 subunit of ribonucleotide reductase enhances pancreatic adenocarcinoma chemosensitivity to gemcitabine' ONCOGENE vol. 23, no. 8, February 2004, pages 1539 - 1548, XP002407219

### SEQUENCE LISTING

<110> GeneSense Technologies Inc.
   Aiping Young
<120> Small Interfering RNA Molecules Against Ribonucleotide Reductase And Uses Thereof
<130> 683-132PCT
<140> N/A
   <141> 2005-08-18
<150> US 60/602,824
   <151> 2004-08-18
<160> 436
<170> FastSEQ for Windows Version 4.0
<210> 1
   <211> 21
   <212> DNA
   <213> Artificial Sequence
<220>
   <223> siRNA target sequence in human ribonucleotide reductase R1 subunit mRNA starting at position 82
<400> 1
   aagaaagtgc tgtctggctc c 21
<210> 2
   <211> 21
   <212> DNA
   <213> Artificial Sequence
<220>
   <223> siRNA target sequence in human ribonucleotide reductase R1 subunit mRNA starting at position 85
<400> 2
   aaagtgctgt ctggctccaa c 21
<210> 3
   <211> 21
   <212> DNA
   <213> Artificial Sequence
<220>
   <223> siRNA target sequence in human ribonucleotide reductase R1 subunit mRNA starting at position 86
<400> 3
   aagtgctgtc tggctccaac t 21
<210> 4
   <211> 21
   <212> DNA
   <213> Artificial Sequence
<220>
   <223> siRNA target sequence in human ribonucleotide reductase R1 subunit mRNA starting at position 135
<400> 4
   aacctaaccc ttcccactct g 21
<210> 5
   <211> 21
   <212> DNA
   <213> Artificial Sequence
<220>
   <223> siRNA target sequence in human ribonucleotide reductase R1 subunit mRNA starting at position 261
<400> 5
   aagaacgagt catgtttgac a 21
<210> 6
   <211> 21
   <212> DNA
   <213> Artificial Sequence
<220>
   <223> siRNA target sequence in human ribonucleotide reductase R1 subunit mRNA starting at position 295
<400> 6
   aatccagaag ctttgttatg g 21
<210> 7
   <211> 21
   <212> DNA
   <213> Artificial Sequence
<220>
   <223> siRNA target sequence in human ribonucleotide reductase R1 subunit mRNA starting at position 356
<400> 7
   aaagtaatcc aaggcttgta c 21
<210> 8
   <211> 21
   <212> DNA
   <213> Artificial Sequence
<220>
   <223> siRNA target sequence in human ribonucleotide reductase R1 subunit mRNA starting at position 357
<400> 8
   aagtaatcca aggcttgtac a 21
<210> 9
   <211> 21
   <212> DNA
   <213> Artificial Sequence
<220>
   <223> siRNA target sequence in human ribonucleotide reductase R1 subunit mRNA starting at position 361
<400> 9
   aatccaaggc ttgtacagtg g 21
<210> 10
   <211> 21
   <212> DNA
   <213> Artificial Sequence
<220>
   <223> siRNA target sequence in human ribonucleotide reductase R1 subunit mRNA starting at position 396
<400> 10
   aactagatac tttggctgct g 21
<210> 11
   <211> 21
   <212> DNA
   <213> Artificial Sequence
<220>
   <223> siRNA target sequence in human ribonucleotide reductase R1 subunit mRNA starting at position 417
<400> 11
   aaacagctgc aaccttgact a 21
<210> 12
   <211> 21
   <212> DNA
   <213> Artificial Sequence
<220>
   <223> siRNA target sequence in human ribonucleotide reductase R1 subunit mRNA starting at position 418
<400> 12
   aacagctgca accttgacta c 21
<210> 13
   <211> 21
   <212> DNA
   <213> Artificial Sequence
<220>
   <223> siRNA target sequence in human ribonucleotide reductase R1 subunit mRNA starting at position 427
<400> 13
   aaccttgact actaagcacc c 21
<210> 14
   <211> 21
   <212> DNA
   <213> Artificial Sequence
<220>
   <223> siRNA target sequence in human ribonucleotide reductase R1 subunit mRNA starting at position 440
<400> 14
   aagcaccctg actatgctat c 21
<210> 15
   <211> 21
   <212> DNA
   <213> Artificial Sequence
<220>
   <223> siRNA target sequence in human ribonucleotide reductase R1 subunit mRNA starting at position 503
<400> 15
   aagaaagtgt tcagtgatgt g 21
<210> 16
   <211> 21
   <212> DNA
   <213> Artificial Sequence
<220>
   <223> siRNA target sequence in human ribonucleotide reductase R1 subunit mRNA starting at position 506
<400> 16
   aaagtgttca gtgatgtgat g 21
<210> 17
   <211> 21
   <212> DNA
   <213> Artificial Sequence
<220>
   <223> siRNA target sequence in human ribonucleotide reductase R1 subunit mRNA starting at position 507
<400> 17
   aagtgttcag tgatgtgatg g 21
<210> 18
   <211> 21
   <212> DNA
   <213> Artificial Sequence
<220>
   <223> siRNA target sequence in human ribonucleotide reductase R1 subunit mRNA starting at position 548
<400> 18
   aatccacata atggcaaaca c 21
<210> 19
   <211> 21
   <212> DNA
   <213> Artificial Sequence
<220>
   <223> siRNA target sequence in human ribonucleotide reductase R1 subunit mRNA starting at position 557
<400> 19
   aatggcaaac actctcccat g 21
<210> 20
   <211> 21
   <212> DNA
   <213> Artificial Sequence
<220>
   <223> siRNA target sequence in human ribonucleotide reductase R1 subunit mRNA starting at position 589
<400> 20
   aacattggat attgttctgg c 21
<210> 21
   <211> 21
   <212> DNA
   <213> Artificial Sequence
<220>
   <223> siRNA target sequence in human ribonucleotide reductase R1 subunit mRNA starting at position 614
<400> 21
   aaagatcgcc tgaattctgc t 21
<210> 22
   <211> 21.
   <212> DNA
   <213> Artificial Sequence
<220>
   <223> siRNA target sequence in human ribonucleotide reductase R1 subunit mRNA starting at position 615
<400> 22
   aagatcgcct gaattctgct a 21
<210> 23
   <211> 21
   <212> DNA
   <213> Artificial Sequence
<220>
   <223> siRNA target sequence in human ribonucleotide reductase R1 subunit mRNA starting at position 662
<400> 23
   aattacttcg gctttaagac g 21
<210> 24
   <211> 21
   <212> DNA
   <213> Artificial Sequence
<220>
   <223> siRNA target sequence in human ribonucleotide reductase R1 subunit mRNA starting at position 677
<400> 24
   aagacgctag agcggtctta t 21
<210> 25
   <211> 21
   <212> DNA
   <213> Artificial Sequence
<220>
   <223> siRNA target sequence in human ribonucleotide reductase R1 subunit mRNA starting at position 704
<400> 25
   aagatcaatg gaaaagtggc t 21
<210> 26
   <211> 21
   <212> DNA
   <213> Artificial Sequence
<220>
   <223> siRNA target sequence in human ribonucleotide reductase R1 subunit mRNA starting at position 710
<400> 26
   aatggaaaag tggctgaaag a 21
<210> 27
   <211> 21
   <212> DNA
   <213> Artificial Sequence
<220>
   <223> siRNA target sequence in human ribonucleotide reductase R1 subunit mRNA starting at position 715
<400> 27
   aaaagtggct gaaagaccac a 21
<210> 28
   <211> 21
   <212> DNA
   <213> Artificial Sequence
<220>
   <223> siRNA target sequence in human ribonucleotide reductase R1 subunit mRNA starting at position 715
<400> 28
   aaagtggctg aaagaccaca a 21
<210> 29
   <211> 21
   <212> DNA
   <213> Artificial Sequence
<220>
   <223> siRNA target sequence in human ribonucleotide reductase R1 subunit mRNA starting at position 717
<400> 29
   aagtggctga aagaccacaa c 21
<210> 30
   <211> 21
   <212> DNA
   <213> Artificial Sequence
<220>
   <223> siRNA target sequence in human ribonucleotide reductase R1 subunit mRNA starting at position 770
<400> 30
   aaagaagaca ttgatgcagc a 21
<210> 31
   <211> 21
   <212> DNA
   <213> Artificial Sequence
<220>
   <223> siRNA target sequence in human ribonucleotide reductase R1 subunit mRNA starting at position 771
<400> 31
   aagaagacat tgatgcagca a 21
<210> 32
   <211> 21
   <212> DNA
   <213> Artificial Sequence
<220>
   <223> siRNA target sequence in human ribonucleotide reductase R1 subunit mRNA starting at position 774
<400> 32
   aagacattga tgcagcaatt g 21
<210> 33
   <211> 21
   <212> DNA
   <213> Artificial Sequence
<220>
   <223> siRNA target sequence in human ribonucleotide reductase R1 subunit mRNA starting at position 803
<400> 33
   aatcttcttt ctgagaggtg g 21

<210> 34
   <211> 21
   <212> DNA
   <213> Artificial Sequence
<220>
   <223> siRNA target sequence in human ribonucleotide reductase R1 subunit mRNA starting at position 863
<400> 34
   aaccgcccac aactttctag c 21
<210> 35
   <211> 21
   <212> DNA
   <213> Artificial Sequence
<220>
   <223> siRNA target sequence in human ribonucleotide reductase R1 subunit mRNA starting at position 902
<400> 35 '
   aaagatgaca gcattgaagg c 21
<210> 36
   <211> 21
   <212> DNA
   <213> Artificial Sequence
<220>
   <223> siRNA target sequence in human ribonucleotide reductase R1 subunit.mRNA starting at position 903
<400> 36
   aagatgacag cattgaaggc a 21
<210> 37
   <211> 21
   <212> DNA
   <213> Artificial Sequence
<220>
   <223> siRNA target sequence in human ribonucleotide reductase R1 subunit mRNA starting at position 959
<400> 37
   aagtctgctg gaggaattgg t 21
<210> 38
   <211> 21
   <212> DNA
   <213> Artificial Sequence
<220>
   <223> siRNA target sequence in human ribonucleotide reductase R1 subunit mRNA starting at position 973
<400> 38
   aattggtgtt gctgtgagtt g 21
<210> 39
   <211> 21
   <212> DNA
   <213> Artificial Sequence
<220>
   <223> siRNA target sequence in human ribonucleotide reductase R1 subunit mRNA starting at position 1028
<400> 39
   aatggcaatt ccaatggcct t 21
<210> 40
   <211> 21
   <212> DNA
   <213> Artificial Sequence
<220>
   <223> siRNA target sequence in human ribonucleotide reductase R1 subunit mRNA starting at position 1034
<400> 40
   aattccaatg gccttgtacc g 21
<210> 41
   <211> 21
   <212> DNA
   <213> Artificial Sequence
<220>
   <223> siRNA target sequence in human ribonucleotide reductase R1 subunit mRNA starting at position 1040
<400> 41
   aatggccttg taccgatgct g 21
<210> 42
   <211> 21
   <212> DNA
   <213> Artificial Sequence
<220>
   <223> siRNA target sequence in human ribonucleotide reductase R1 subunit mRNA starting at position 1070
<400> 42
   aacaacacag ctagatatgt g 21
<210> 43
   <211> 21
   <212> DNA
   <213> Artificial Sequence
<220>
   <223> siRNA target sequence in human ribonucleotide reductase R1 subunit mRNA starting at position 1073
<400> 43
   aacacagcta gatatgtgga t 21
<210> 44
   <211> 21
   <212> DNA
   <213> Artificial Sequence
<220>
   <223> siRNA target sequence in human ribonucleotide reductase R1 subunit mRNA starting at position 1174
<400> 44
   aaagaagaac acaggaaagg a 21
<210> 45
   <211> 21
   <212> DNA
   <213> Artificial Sequence
<220>
   <223> siRNA target sequence in human ribonucleotide reductase R1 subunit mRNA starting at position 1175
<400> 45
   aagaagaaca caggaaagga a 21
<210> 46
   <211> 21
   <212> DNA
   <213> Artificial Sequence
<220>
   <223> siRNA target sequence in human ribonucleotide reductase R1 subunit mRNA starting at position 1178
<400> 46
   aagaacacag gaaaggaaga g 21
<210> 47
   <211> 21
   <212> DNA
   <213> Artificial Sequence
<220>
   <223> siRNA target sequence in human ribonucleotide reductase R1 subunit mRNA starting at position 1181
<400> 47
   aacacaggaa aggaagagca g 21
<210> 48
   <211> 21
   <212> DNA
   <213> Artificial Sequence
<220>
   <223> siRNA target sequence in human ribonucleotide reductase R1 subunit mRNA starting at position 1250
<400> 48
   aaacgagtgg agactaatca g 21
<210> 49
   <211> 21
   <212> DNA
   <213> Artificial Sequence
<220>
   <223> siRNA target sequence in human ribonucleotide reductase R1 subunit mRNA starting at position 1251
<400> 49
   aacgagtgga gactaatcag g 21
<210> 50
   <211> 21
   <212> DNA
   <213> Artificial Sequence
<220>
   <223> siRNA target sequence in human ribonucleotide reductase R1 subunit mRNA starting at position 1265
<400> 50
   aatcaggact ggtctttgat g 21
<210> 51
   <211> 21
   <212> DNA
   <213> Artificial Sequence
<220>
   <223> siRNA target sequence in human ribonucleotide reductase R1 subunit mRNA starting at position 1291
<400> 51
   aaatgagtgt cctggtctgg a 21
<210> 52
   <211> 21
   <212> DNA
   <213> Artificial Sequence
<220>
   <223> siRNA target sequence in human ribonucleotide reductase R1 subunit mRNA starting at position 1292
<400> 52
   aatgagtgtc ctggtctgga t 21
<210> 53
   <211> 21
   <212> DNA
   <213> Artificial Sequence
<220>
   <223> siRNA target sequence in human ribonucleotide reductase R1 subunit mRNA starting at position 1348
<400> 53
   aagttatgag aaacaaggtc g 21
<210> 54
   <211> 21
   <212> DNA
   <213> Artificial Sequence
<220>
   <223> siRNA target sequence in human ribonucleotide reductase R1 subunit mRNA starting at position 1358
<400> 54
   aaacaaggtc gtgtccgcaa a 21
<210> 55
   <211> 21
   <212> DNA
   <213> Artificial Sequence
<220>
   <223> siRNA target sequence in human ribonucleotide reductase R1 subunit mRNA starting at position 1359
<400> 55
   aacaaggtcg tgtccgcaaa g 21
<210> 56
   <211> 21
   <212> DNA
   <213> Artificial Sequence
<220>
   <223> siRNA target sequence in human ribonucleotide reductase R1 subunit mRNA starting at position 1362
<400> 56
   aaggtcgtgt ccgcaaagtt g 21
<210> 57
   <211> 21
   <212> DNA
   <213> Artificial Sequence
<220>
   <223> siRNA target sequence in human ribonucleotide reductase R1 subunit mRNA starting at position 1376
<400> 57
   aaagttgtaa aagctcagca g 21
<210> 58
   <211> 21
   <212> DNA
   <213> Artificial Sequence
<220>
   <223> siRNA target sequence in human ribonucleotide reductase R1 subunit mRNA starting at position 1377
<400> 58
   aagttgtaaa agctcagcag c 21
<210> 59
   <211> 21
   <212> DNA
   <213> Artificial Sequence
<220>
   <223> siRNA target sequence in human ribonucleotide reductase R1 subunit mRNA starting at position 1385
<400> 59
   aaagctcagc agctttggta t 21
<210> 60
   <211> 21
   <212> DNA
   <213> Artificial Sequence
<220>
   <223> siRNA target sequence in human ribonucleotide reductase R1 subunit mRNA starting at position 1386
<400> 60
   aagctcagca gctttggtat g 21
<210> 61
   <211> 21
   <212> DNA
   <213> Artificial Sequence
<220>
   <223> siRNA target sequence in human ribonucleotide reductase R1 subunit mRNA starting at position 1466
<400> 61
   aatcgaaaga gcaaccagca g 21
<210> 62
   <211> 21
   <212> DNA
   <213> Artificial Sequence
<220>
   <223> siRNA target sequence in human ribonucleotide reductase R1 subunit mRNA starting at position 1471
<400> 62
   aaagagcaac cagcagaacc t 21
<210> 63
   <211> 21
   <212> DNA
   <213> Artificial Sequence
<220>
   <223> siRNA target sequence in human ribonucleotide reductase R1 subunit mRNA starting at position 1472
<400> 63
   aagagcaacc agcagaacct g 21
<210> 64
   <211> 21
   <212> DNA
   <213> Artificial Sequence
<220>
   <223> siRNA target sequence in human ribonucleotide reductase R1 subunit mRNA starting at position 1487
<400> 64
   aacctgggaa ccatcaaatg c 21
<210> 65
   <211> 21
   <212> DNA
   <213> Artificial Sequence
<220>
   <223> siRNA target sequence in human ribonucleotide reductase R1 subunit mRNA starting at position 1495
<400> 65
   aaccatcaaa tgcagcaacc t 21
<210> 66
   <211> 21
   <212> DNA
   <213> Artificial Sequence
<220>
   <223> siRNA target sequence in human ribonucleotide reductase R1 subunit mRNA starting at position 1502
<400> 66
   aaatgcagca acctgtgcac a 21
<210> 67
   <211> 21
   <212> DNA
   <213> Artificial Sequence
<220>
   <223> siRNA target sequence in human ribonucleotide reductase R1 subunit mRNA starting at position 1503
<400> 67
   aatgcagcaa cctgtgcaca g 21
<210> 68
   <211> 21
   <212> DNA
   <213> Artificial Sequence
<220>
   <223> siRNA target sequence in human ribonucleotide reductase R1 subunit mRNA starting at position 1511
<400> 68
   aacctgtgca cagaaatagt g 21
<210> 69
   <211> 21
   <212> DNA
   <213> Artificial Sequence
<220>
   <223> siRNA target sequence in human ribonucleotide reductase R1 subunit mRNA starting at position 1524
<400> 69
   aaatagtgga gtacaccagc a 21
<210> 70
   <211> 21
   <212> DNA
   <213> Artificial Sequence
<220>
   <223> siRNA target sequence in human ribonucleotide reductase R1 subunit mRNA starting at position 1525
<400> 70
   aatagtggag tacaccagca a 21
<210> 71
   <211> 21
   <212> DNA
   <213> Artificial Sequence
<220>
   <223> siRNA target sequence in human ribonucleotide reductase R1 subunit mRNA starting at position 1544
<400> 71
   aaagatgagg ttgctgtttg t 21
<210> 72
   <211> 21
   <212> DNA
   <213> Artificial Sequence
<220>
   <223> siRNA target sequence in human ribonucleotide reductase R1 subunit mRNA starting at position 1545
<400> 72
   aagatgaggt tgctgtttgt a 21
<210> 73 ,
   <211> 21
   <212> DNA
   <213> Artificial Sequence
<220>
   <223> siRNA target sequence in human ribonucleotide reductase R1 subunit mRNA starting at position 1622
<400> 73
   aagaagttgg ctgaagtcac t 21
<210> 74
   <211> 21
   <212> DNA
   <213> Artificial Sequence
<220>
   <223> siRNA target sequence in human ribonucleotide reductase R1 subunit mRNA starting at position 1625
<400> 74
   aagttggctg aagtcactaa a 21
<210> 75
   <211> 21
   <212> DNA
   <213> Artificial Sequence
<220>
   <223> siRNA target sequence in human ribonucleotide reductase R1 subunit mRNA starting at position 1635
<400> 75
   aagtcactaa agtcgttgtc c 21
<210> 76
   <211> 21
   <212> DNA
   <213> Artificial Sequence
<220>
   <223> siRNA target sequence in human ribonucleotide reductase R1 subunit mRNA starting at position 1643
<400> 76
   aaagtcgttg tccgaaactt g 21
<210> 77
   <211> 21
   <212> DNA
   <213> Artificial Sequence

<220>
   <223> siRNA target sequence in human ribonucleotide reductase R1 subunit mRNA starting at position 1644
<400> 77
   aagtcgttgt ccgaaacttg a 21
<210> 78
   <211> 21
   <212> DNA
   <213> Artificial Sequence
<220>
   <223> siRNA target sequence in human ribonucleotide reductase R1 subunit mRNA starting at position 1681
<400> 78
   aaactactat cctgtaccag a 21
<210> 79
   <211> 21
   <212> DNA
   <213> Artificial Sequence
<220>
   <223> siRNA target sequence in human ribonucleotide reductase R1 subunit mRNA starting at position 1682
<400> 79
   aactactatc ctgtaccaga g 21
<210> 80
   <211> 21
   <212> DNA
   <213> Artificial Sequence
<220>
   <223> siRNA target sequence in human ribonucleotide reductase R1 subunit mRNA starting at position 1749
<400> 80
   aaggtctggc agatgctttt a 21
<210> 81
   <211> 21
   <212> DNA
   <213> Artificial Sequence
<220>
   <223> siRNA target sequence in human ribonucleotide reductase R1 subunit mRNA starting at position 1800
<400> 81
   aagcccagtt actgaataag c 21
<210> 82
   <211> 21
   <212> DNA
   <213> Artificial Sequence
<220>
   <223> siRNA target sequence in human ribonucleotide reductase R1 subunit mRNA starting at position 1893
<400> 82
   aaacctatga gggctctcca g 21
<210> 83
   <211> 21
   <212> DNA
   <213> Artificial Sequence
<220>
   <223> siRNA target sequence in human ribonucleotide reductase R1 subunit mRNA starting at position 1894
<400> 83
   aacctatgag ggctctccag t 21
<210> 84
   <211> 21
   <212> DNA
   <213> Artificial Sequence
<220>
   <223> siRNA target sequence in human ribonucleotide reductase R1 subunit mRNA starting at position 1946
<400> 84
   aatgttactc ctacagacct a 21
<210> 85
   <211> 21
   <212> DNA
   <213> Artificial Sequence
<220>
   <223> siRNA target sequence in human ribonucleotide reductase R1 subunit mRNA starting at position 1976
<400> 85
   aaggttctca aggagaagat t 21
<210> 86
   <211> 21
   <212> DNA
   <213> Artificial Sequence
<220>
   <223> siRNA target sequence in human ribonucleotide reductase R1 subunit mRNA starting at position 2072
<400> 86
   aatgagtcca ttgaacctta c 21
<210> 87
   <211> 21
   <212> DNA
   <213> Artificial Sequence
<220>
   <223> siRNA target sequence in human ribonucleotide reductase R1 subunit mRNA starting at position 2085
<400> 87
   aaccttacac cagcaacatc t 21
<210> 88
   <211> 21
   <212> DNA
   <213> Artificial Sequence
<220>
   <223> siRNA target sequence in human ribonucleotide reductase R1 subunit mRNA starting at position 2099
<400> 88
   aacatctata ctcgcagagt c 21
<210> 89
   <211> 21
   <212> DNA
   <213> Artificial Sequence
<220>
   <223> siRNA target sequence in human ribonucleotide reductase R1 subunit mRNA starting at position 2219
<400> 89
   aatggctcta ttcagagcat a 21
<210> 90
   <211> 21
   <212> DNA
   <213> Artificial Sequence
<220>
   <223> siRNA target sequence in human ribonucleotide reductase R1 subunit mRNA starting at position 2244
<400> 90
   aaattcctga tgacctgaag c 21
<210> 91
   <211> 21
   <212> DNA
   <213> Artificial Sequence
<220>
   <223> siRNA target sequence in human ribonucleotide reductase R1 subunit mRNA starting at position 2245
<400> 91
   aattcctgat gacctgaagc a 21
<210> 92
   <211> 21
   <212> DNA
   <213> Artificial Sequence
<220>
   <223> siRNA target sequence in human ribonucleotide reductase R1 subunit mRNA starting at position 2273
<400> 92
   aaaactgtgt gggaaatctc t 21
<210> 93
   <211> 21
   <212> DNA
   <213> Artificial Sequence
<220>
   <223> siRNA target sequence in human ribonucleotide reductase R1 subunit mRNA starting at position 2274
<400> 93
   aaactgtgtg ggaaatctct c 21
<210> 94
   <211> 21
   <212> DNA
   <213> Artificial Sequence
<220>
   <223> siRNA target sequence in human ribonucleotide reductase R1 subunit mRNA starting at position 2275
<400> 94
   aactgtgtgg gaaatctctc a 21
<210> 95
   <211> 21
   <212> DNA
   <213> Artificial Sequence
<220>
   <223> siRNA target sequence in human ribonucleotide reductase R1 subunit mRNA starting at position 2297
<400> 95
   aaaactgttc tcaagatggc a 21
<210> 96
   <211> 21
   <212> DNA
   <213> Artificial Sequence
<220>
   <223> siRNA target sequence in human ribonucleotide reductase R1 subunit mRNA starting at position 2298
<400> 96
   aaactgttct caagatggca g 21
<210> 97
   <211> 21
   <212> DNA
   <213> Artificial Sequence
<220>
   <223> siRNA target sequence in human ribonucleotide reductase R1 subunit mRNA starting at position 2299
<400> 97
   aactgttctc aagatggcag c 21
<210> 98
   <211> 21
   <212> DNA
   <213> Artificial Sequence
<220>
   <223> siRNA target sequence in human ribonucleotide reductase R1 subunit mRNA starting at position 2309
<400> 98
   aagatggcag ctgagagagg t 21
<210> 99
   <211> 21
   <212> DNA
   <213> Artificial Sequence
<220>
   <223> siRNA target sequence in human ribonucleotide reductase R1 subunit mRNA starting at position 2343
<400> 99
   aaagccaatc tttgaacatc c 21
<210> 100
   <211> 21
   <212> DNA
   <213> Artificial Sequence
<220>
   <223> siRNA target sequence in human ribonucleotide reductase R1 subunit mRNA starting at position 2344
<400> 100
   aagccaatct ttgaacatcc a 21
<210> 101
   <211> 21
   <212> DNA
   <213> Artificial Sequence
<220>
   <223> siRNA target sequence in human ribonucleotide reductase R1 subunit mRNA starting at position 2357
<400> 101
   aacatccaca ttgctgagcc t 21
<210> 102
   <211> 21
   <212> DNA
   <213> Artificial Sequence
<220>
   <223> siRNA target sequence in human ribonucleotide reductase R1 subunit mRNA starting at position 2378
<400> 102
   aactatggca aactcactag t 21
<210> 103
   <211> 21
   <212> DNA
   <213> Artificial Sequence
<220>
   <223> siRNA target sequence in human ribonucleotide reductase R1 subunit mRNA starting at position 2387
<400> 103
   aaactcacta gtatgcactt c 21
<210> 104
   <211> 21
   <212> DNA
   <213> Artificial Sequence
<220>
   <223> siRNA target sequence in human ribonucleotide reductase R1 subunit mRNA starting at position 2388
<400> 104
   aactcactag tatgcacttc t 21
<210> 105
   <211> 21
   <212> DNA
   <213> Artificial Sequence
<220>
   <223> siRNA target sequence in human ribonucleotide reductase R1 subunit mRNA starting at position 2417
<400> 105
   aagcagggtt tgaagactgg g 21
<210> 106
   <211> 21
   <212> DNA
   <213> Artificial Sequence
<220>
   <223> siRNA target sequence in human ribonucleotide reductase R1 subunit mRNA starting at position 2449
<400> 106
   aaggacaaga ccagcagcta a 21
<210> 107
   <211> 21
   <212> DNA
   <213> Artificial Sequence
<220>
   <223> siRNA target sequence in human ribonucleotide reductase R1 subunit mRNA starting at position 2455
<400> 107
   aagaccagca gctaatccaa t 21
<210> 108
   <211> 21
   <212> DNA
   <213> Artificial Sequence
<220>
   <223> siRNA target sequence in human ribonucleotide reductase R1 subunit mRNA starting at position 2468
<400> 108
   aatccaatcc agttcactct a 21
<210> 109
   <211> 21
   <212> DNA
   <213> Artificial Sequence
<220>
   <223> siRNA target sequence in human ribonucleotide reductase R1 subunit mRNA starting at position 2524
<400> 109
   aaaagaggaa gaagagaagg a 21
<210> 110
   <211> 21
   <212> DNA
   <213> Artificial Sequence
<220>
   <223> siRNA target sequence in human ribonucleotide reductase R1 subunit mRNA starting at position 2525
<400> 110
   aaagaggaag aagagaagga g 21
<210> 111
   <211> 21
   <212> DNA
   <213> Artificial Sequence
<220>
   <223> siRNA target sequence in human ribonucleotide reductase R1 subunit mRNA starting at position 2526
<400> 111
   aagaggaaga agagaaggag a 21
<210> 112
   <211> 21
   <212> DNA
   <213> Artificial Sequence
<220>
   <223> siRNA target sequence in human ribonucleotide reductase R1 subunit mRNA starting at position 2532
<400> 112
   aagaagagaa ggagaggaac a 21
<210> 113
   <211> 21
   <212> DNA
   <213> Artificial Sequence
<220>
   <223> siRNA target sequence in human ribonucleotide reductase R1 subunit mRNA starting at position 2535
<400> 113
   aagagaagga gaggaacaca g 21
<210> 114
   <211> 21
   <212> DNA
   <213> Artificial Sequence
<220>
   <223> siRNA target sequence in human ribonucleotide reductase R1 subunit mRNA starting at position 2589
<400> 114
   aatgtctgat gtgtggatcc t 21
<210> 115
   <211> 21
   <212> DNA
   <213> Artificial Sequence
<220>
   <223> siRNA target sequence in human ribonucleotide reductase R1 subunit mRNA starting at position 2614
<400> 115
   aaagacttgg aagagaccag c 21
<210> 116
   <211> 21
   <212> DNA
   <213> Artificial Sequence
<220>
   <223> siRNA target sequence in human ribonucleotide reductase
   R1 subunit mRNA starting at position 2615
<400> 116
   aagacttgga agagaccagc a 21
<210> 117
   <211> 21
   <212> DNA
   <213> Artificial Sequence
<220>
   <223> siRNA target sequence in human ribonucleotide reductase R1 subunit mRNA starting at position 2624
<400> 117
   aagagaccag catgtcttca g 21
<210> 118
   <211> 21
   <212> DNA
   <213> Artificial Sequence
<220>
   <223> siRNA target sequence in human ribonucleotide reductase R1 subunit mRNA starting at position 2699
<400> 118
   aaggctttgc tggaccctgt t 21
<210> 119
   <211> 21
   <212> DNA
   <213> Artificial Sequence
<220>
   <223> siRNA target sequence in human ribonucleotide reductase R1 subunit mRNA starting at position 2880
<400> 119
   aagtcatctt gcatacaggg a 21
<210> 120
   <211> 21
   <212> DNA
   <213> Artificial Sequence
<220>
   <223> siRNA target sequence in human ribonucleotide reductase R1 subunit mRNA starting at position 2907

<400> 120
   aagtaaggtt tcatcaccca t 21
<210> 121
   <211> 21
   <212> DNA
   <213> Artificial Sequence
<220>
   <223> siRNA target sequence in human ribonucleotide reductase R1 subunit mRNA starting at position 2911
<400> 121
   aaggtttcat cacccattta g 21
<210> 122
   <211> 21
   <212> DNA
   <213> Artificial Sequence
<220>
   <223> siRNA target sequence in human ribonucleotide reductase R1 subunit mRNA starting at position 3008
<400> 122
   aactgagtga taactcatga g 21
<210> 123
   <211> 21
   <212> DNA
   <213> Artificial Sequence
<220>
   <223> siRNA target sequence in human ribonucleotide reductase R1 subunit mRNA starting at position 3019
<400> 123
   aactcatgag aagtactgat a 21
<210> 124
   <211> 21
   <212> DNA
   <213> Artificial Sequence
<220>
   <223> siRNA target sequence in human ribonucleotide reductase R2 subunit mRNA starting at position 477
<400> 124
   aagaaggcag aggcttcctt t 21
<210> 125
   <211> 21
   <212> DNA
   <213> Artificial Sequence
<220>
   <223> siRNA target sequence in human ribonucleotide reductase R2 subunit mRNA starting at position 480
<400> 125
   aaggcagagg cttccttttg g 21
<210> 126
   <211> 21
   <212> DNA
   <213> Artificial Sequence
<220>
   <223> siRNA target sequence in human ribonucleotide reductase R2 subunit mRNA starting at position 525
<400> 126
   aaggacattc agcactggga a 21
<210> 127
   <211> 21
   <212> DNA
   <213> Artificial Sequence
<220>
   <223> siRNA target sequence in human ribonucleotide reductase R2 subunit mRNA starting at position 544
<400> 127
   aatccctgaa acccgaggag a 21
<210> 128
   <211> 21
   <212> DNA
   <213> Artificial Sequence
<220>
   <223> siRNA target sequence in human ribonucleotide reductase R2 subunit mRNA starting at position 552
<400> 128
   aaacccgagg agagatattt t 21
<210> 129
   <211> 21
   <212> DNA
   <213> Artificial Sequence
<220>
   <223> siRNA target sequence in human ribonucleotide reductase R2 subunit mRNA starting at position 553
<400> 129
   aacccgagga gagatatttt a 21
<210> 130
   <211> 21
   <212> DNA
   <213> Artificial Sequence
<220>
   <223> siRNA target sequence in human ribonucleotide reductase R2 subunit mRNA starting at position 602
<400> 130
   aagcgatggc atagtaaatg a 21
<210> 131
   <211> 21
   <212> DNA
   <213> Artificial Sequence
<220>
   <223> siRNA target sequence in human ribonucleotide reductase R2 subunit mRNA starting at position 617
<400> 131
   aaatgaaaac ttggtggagc g 21
<210> 132
   <211> 21
   <212> DNA
   <213> Artificial Sequence
<220>
   <223> siRNA target sequence in human ribonucleotide reductase R2 subunit mRNA starting at position 618
<400> 132
   aatgaaaact tggtggagcg a 21
<210> 133
   <211> 21
   <212> DNA
   <213> Artificial Sequence
<220>
   <223> siRNA target sequence in human ribonucleotide reductase R2 subunit mRNA starting at position 622
<400> 133
   aaaacttggt ggagcgattt a 21
<210> 134
   <211> 21
   <212> DNA
   <213> Artificial Sequence
<220>
   <223> siRNA target sequence in human ribonucleotide reductase R2 subunit mRNA starting at position 623
<400> 134
   aaacttggtg gagcgattta g 21
<210> 135
   <211> 21
   <212> DNA
   <213> Artificial Sequence
<220>
   <223> siRNA target sequence in human ribonucleotide reductase R2 subunit mRNA starting at position 624
<400> 135
   aacttggtgg agcgatttag c 21
<210> 136
   <211> 21
   <212> DNA
   <213> Artificial Sequence
<220>
   <223> siRNA target sequence in human ribonucleotide reductase R2 subunit mRNA starting at position 649
<400> 136
   aagttcagat tacagaagcc c 21
<210> 137
   <211> 21
   <212> DNA
   <213> Artificial Sequence
<220>
   <223> siRNA target sequence in human ribonucleotide reductase R2 subunit mRNA starting at position 664
<400> 137
   aagcccgctg tttctatggc t 21
<210> 138
   <211> 21
   <212> DNA
   <213> Artificial Sequence
<220>
   <223> siRNA target sequence in human ribonucleotide reductase R2 subunit mRNA starting at position 746
<400> 138
   aaaagatccc aaagaaaggg a 21
<210> 139
   <211> 21
   <212> DNA
   <213> Artificial Sequence
<220>
   <223> siRNA target sequence in human ribonucleotide reductase R2 subunit mRNA starting at position 747
<400> 139
   aaagatccca aagaaaggga a 21
<210> 140
   <211> 21
   <212> DNA
   <213> Artificial Sequence
<220>
   <223> siRNA target sequence in human ribonucleotide reductase R2 subunit mRNA starting at position 748
<400> 140
   aagatcccaa agaaagggaa t 21
<210> 141
   <211> 21
   <212> DNA
   <213> Artificial Sequence
<220>
   <223> siRNA target sequence in human ribonucleotide reductase R2 subunit mRNA starting at position 761
<400> 141
   aagggaattt ctcttcaatg c 21
<210> 142
   <211> 21
   <212> DNA
   <213> Artificial Sequence
<220>
   <223> siRNA target sequence in human ribonucleotide reductase R2 subunit mRNA starting at position 777
<400> 142
   aatgccattg aaacgatgcc t 21
<210> 143
   <211> 21
   <212> DNA
   <213> Artificial Sequence
<220>
   <223> siRNA target sequence in human ribonucleotide reductase R2 subunit mRNA starting at position 787
<400> 143
   aaacgatgcc ttgtgtcaag a 21
<210> 144
   <211> 21
   <212> DNA
   <213> Artificial Sequence
<220>
   <223> siRNA target sequence in human ribonucleotide reductase R2 subunit mRNA starting at position 788
<400> 144
   aacgatgcct tgtgtcaaga a 21
<210> 145
   <211> 21
   <212> DNA
   <213> Artificial Sequence
<220>
   <223> siRNA target sequence in human ribonucleotide reductase R2 subunit mRNA starting at position 843
<400> 145
   aaagaggcta cctatggtga a 21
<210> 146
   <211> 21
   <212> DNA
   <213> Artificial Sequence
<220>
   <223> siRNA target sequence in human ribonucleotide reductase R2 subunit mRNA starting at position 844
<400> 146
   aagaggctac ctatggtgaa c 21
<210> 147
   <211> 21
   <212> DNA
   <213> Artificial Sequence
<220>
   <223> siRNA target sequence in human ribonucleotide reductase R2 subunit mRNA starting at position 862
<400> 147
   aacgtgttgt agcctttgct g 21
<210> 148
   <211> 21
   <212> DNA
   <213> Artificial Sequence
<220>
   <223> siRNA target sequence in human ribonucleotide reductase R2 subunit mRNA starting at position 889
<400> 148
   aaggcatttt cttttccggt t 21
<210> 149
   <211> 21
   <212> DNA
   <213> Artificial Sequence
<220>
   <223> siRNA target sequence in human ribonucleotide reductase R2 subunit mRNA starting at position 933
<400> 149
   aagaaacgag gactgatgcc t 21
<210> 150
   <211> 21
   <212> DNA
   <213> Artificial Sequence
<220>
   <223> siRNA target sequence in human ribonucleotide reductase R2 subunit mRNA starting at position 973
<400> 150
   aacttattag cagagatgag g 21
<210> 151
   <211> 21
   <212> DNA
   <213> Artificial Sequence
<220>
   <223> siRNA target sequence in human ribonucleotide reductase R2 subunit mRNA starting at position 1026
<400> 151
   aaacacctgg tacacaaacc a 21
<210> 152
   <211> 21
   <212> DNA
   <213> Artificial Sequence
<220>
   <223> siRNA target sequence in human ribonucleotide reductase R2 subunit mRNA starting at position 1027
<400> 152
   aacacctggt acacaaacca t 21
<210> 153
   <211> 21
   <212> DNA
   <213> Artificial Sequence
<220>
   <223> siRNA target sequence in human ribonucleotide reductase R2 subunit mRNA starting at position 1041
<400> 153
   aaaccatcgg aggagagagt a 21
<210> 154
   <211> 21
   <212> DNA
   <213> Artificial Sequence
<220>
   <223> siRNA target sequence in human ribonucleotide reductase R2 subunit mRNA starting at position 1042
<400> 154
   aaccatcgga ggagagagta a 21
<210> 155
   <211> 21
   <212> DNA
   <213> Artificial Sequence
<220>
   <223> siRNA target sequence in human ribonucleotide reductase R2 subunit mRNA starting at position 1077
<400> 155
   aatgctgttc ggatagaaca g 21
<210> 156
   <211> 21
   <212> DNA
   <213> Artificial Sequence
<220>
   <223> siRNA target sequence in human ribonucleotide reductase R2 subunit mRNA starting at position 1093
<400> 156
   aacaggagtt cctcactgag g 21
<210> 157
   <211> 21
   <212> DNA
   <213> Artificial Sequence
<220>
   <223> siRNA target sequence in human ribonucleotide reductase R2 subunit mRNA starting at position 1125
<400> 157
   aagctcattg ggatgaattg c 21
<210> 158
   <211> 21
   <212> DNA
   <213> Artificial Sequence
<220>
   <223> siRNA target sequence in human ribonucleotide reductase R2 subunit mRNA starting at position 1159
<400> 158
   aatacattga gtttgtggca g 21
<210> 159
   <211> 21
   <212> DNA
   <213> Artificial Sequence
<220>
   <223> siRNA target sequence in human ribonucleotide reductase R2 subunit mRNA starting at position 1195
<400> 159
   aactgggttt tagcaaggtt t 21
<210> 160
   <211> 21
   <212> DNA
   <213> Artificial Sequence
<220>
   <223> siRNA target sequence in human ribonucleotide reductase R2 subunit mRNA starting at position 1209
<400> 160
   aaggttttca gagtagagaa c 21
<210> 161
   <211> 21
   <212> DNA
   <213> Artificial Sequence
<220>
   <223> siRNA target sequence in human ribonucleotide reductase R2 subunit mRNA starting at position 1227
<400> 161
   aacccatttg actttatgga g 21
<210> 162
   <211> 21
   <212> DNA
   <213> Artificial Sequence
<220>
   <223> siRNA target sequence in human ribonucleotide reductase R2 subunit mRNA starting at position 1284
<400> 162
   aagagagtag gcgagtatca g 21

<210> 163
   <211> 21
   <212> DNA
   <213> Artificial Sequence
<220>
   <223> siRNA target sequence in human ribonucleotide reductase R2 subunit mRNA starting at position 1363
<400> 163
   aaatgaactg aagatgtgcc c 21
<210> 164
   <211> 21
   <212> DNA
   <213> Artificial Sequence
<220>
   <223> siRNA target sequence in human ribonucleotide reductase R2 subunit mRNA starting at position 1364
<400> 164
   aatgaactga agatgtgccc t 21
<210> 165
   <211> 21
   <212> DNA
   <213> Artificial Sequence
<220>
   <223> siRNA target sequence in human ribonucleotide reductase R2 subunit mRNA starting at position 1368
<400> 165
   aactgaagat gtgcccttac t 21
<210> 166
   <211> 21
   <212> DNA
   <213> Artificial Sequence
<220>
   <223> siRNA target sequence in human ribonucleotide reductase R2 subunit mRNA starting at position 1373
<400> 166
   aagatgtgcc cttacttggc t 21
<210> 167
   <211> 21
   <212> DNA
   <213> Artificial Sequence
<220>
   <223> siRNA target sequence in human ribonucleotide reductase R2 subunit mRNA starting at position 1419
<400> 167
   aaaatcagct gaagtgttac c 21
<210> 168
   <211> 21
   <212> DNA
   <213> Artificial Sequence
<220>
   <223> siRNA target sequence in human ribonucleotide reductase R2 subunit mRNA starting at position 1420
<400> 168
   aaatcagctg aagtgttacc a 21
<210> 169
   <211> 21
   <212> DNA
   <213> Artificial Sequence
<220>
   <223> siRNA target sequence in human ribonucleotide reductase R2 subunit mRNA starting at position 1421
<400> 169
   aatcagctga agtgttacca a 21
<210> 170
   <211> 21
   <212> DNA
   <213> Artificial Sequence
<220>
   <223> siRNA target sequence in human ribonucleotide reductase R2 subunit mRNA starting at position 1430
<400> 170
   aagtgttacc aactagccac a 21
<210> 171
   <211> 21
   <212> DNA
   <213> Artificial Sequence
<220>
   <223> siRNA target sequence in human ribonucleotide reductase R2 subunit mRNA starting at position 1440
<400> 171
   aactagccac accatgaatt g 21
<210> 172
   <211> 21
   <212> DNA
   <213> Artificial Sequence
<220>
   <223>.siRNA target sequence in human ribonucleotide reductase R2 subunit mRNA starting at position 1488
<400> 172
   aaaactgtgt agctacctca c 21
<210> 173
   <211> 21
   <212> DNA
   <213> Artificial Sequence
<220>
   <223> siRNA target sequence in human ribonucleotide reductase R2 subunit mRNA starting at position 1489
<400> 173
   aaactgtgta gctacctcac a 21
<210> 174
   <211> 21
   <212> DNA
   <213> Artificial Sequence
<220>
   <223> siRNA target sequence in human ribonucleotide reductase R2. subunit mRNA starting at position 1490
<400> 174
   aactgtgtag ctacctcaca a 21
<210> 175
   <211> 21
   <212> DNA
   <213> Artificial Sequence
<220>
   <223> siRNA target sequence in human ribonucleotide reductase R2 subunit mRNA starting at position 1509
<400> 175
   aaccagtcct gtctgtttat a 21
<210> 176
   <211> 21
   <212> DNA
   <213> Artificial Sequence
<220>
   <223> siRNA target sequence in human ribonucleotides reductase R2 subunit mRNA starting at position 1592
<400> 176
   aatggcagtc ttggctttaa a 21
<210> 177
   <211> 21
   <212> DNA
   <213> Artificial Sequence
<220>
   <223> siRNA target sequence in human ribonucleotide reductase R2 subunit mRNA starting at position 1653
<400> 177
   aaacagtcct ttaaccagca c 21
<210> 178
   <211> 21
   <212> DNA
   <213> Artificial Sequence
<220>
   <223> siRNA target sequence in human ribonucleotide Reductase R2 subunit mRNA starting at position 1654
<400> 178
   aacagtcctt taaccagcac a 21
<210> 179
   <211> 21
   <212> DNA
   <213> Artificial Sequence
<220>
   <223> siRNA target sequence in human ribonucleotide reductase R2 subunit mRNA starting at position 1665
<400> 179
   aaccageaca gccagttaaa a 21
<210> 180
   <211> 21
   <212> DNA
   <213> Artificial Sequence
<220>
   <223> siRNA target sequence in human ribonucleotide reductase R2 subunit mRNA starting at position 1682
<400> 180
   aaaagatgca gcctcactgc t 21
<210> 181
   <211> 21
   <212> DNA
   <213> Artificial Sequence
<220>
   <223> siRNA target sequence in human ribonucleotide reductase R2 subunit mRNA starting at position 1683
<400> 181
   aaagatgcag cctcactgct t 21
<210> 182
   <211> 21
   <212> DNA
   <213> Artificial Sequence
<220>
   <223> siRNA target sequence in human ribonucleotide reductase R2 subunit mRNA starting at position 1684
<400> 182
   aagatgcagc ctcactgctt c 21
<210> 183
   <211> 21
   <212> DNA
   <213> Artificial Sequence
<220>
   <223> siRNA target sequence in human ribonucleotide reductase R2 subunit mRNA starting at position 1734
<400> 183
   aaacctggca ctttacaaac a 21
<210> 184
   <211> 21
   <212> DNA
   <213> Artificial Sequence
<220>
   <223> siRNA target sequence in human ribonucleotide reductase R2 subunit mRNA starting at position 1735
<400> 184
   aacctggcac tttacaaaca a 21
<210> 185
   <211> 21
   <212> DNA
   <213> Artificial Sequence
<220>
   <223> siRNA target sequence in human ribonucleotide reductase R2 subunit mRNA starting at position 1759
<400> 185
   aacattgttt tgtactcacg g 21
<210> 186
   <211> 21
   <212> DNA
   <213> Artificial Sequence
<220>
   <223> siRNA target sequence in human ribonucleotide reductase R2 subunit mRNA starting at position 1816
<400> 186
   aaatacattc tcctgaccac t 21
<210> 187
   <211> 21
   <212> DNA
   <213> Artificial Sequence
<220>
   <223> siRNA target sequence in human ribonucleotide reductase R2 subunit mRNA starting at position 1817
<400> 187
   aatacattct cctgaccact a 21
<210> 188
   <211> 21
   <212> DNA
   <213> Artificial Sequence
<220>
   <223> siRNA target sequence in human ribonucleotide reductase R2 subunit mRNA starting at position 1837
<400> 188
   aatgggagcc aattcacaat t 21
<210> 189
   <211> 21
   <212> DNA
   <213> Artificial Sequence
<220>
   <223> siRNA target sequence in human ribonucleotide reductase R2 subunit mRNA starting at position 1881
<400> 189
   aacttgtgta gactaagcat 21
<210> 190
   <211> 21
   <212> DNA
   <213> Artificial Sequence
<220>
   <223> siRNA target sequence in human ribonucleotide reductase R2 subunit mRNA starting at position 1942
<400>
   aaccaacttt aaagtcagtc c 21
<210> 191
   <211> 21
   <212> DNA
   <213> Artificial Sequence
<220>
   <223> siRNA target sequence in human ribonucleotide reductase R2 subunit mRNA starting at position 1946
<400> 191
   aactttaaag tcagtcctgt g 21
<210> 192
   <211> 21
   <212> DNA
   <213> Artificial Sequence
<220>
   <223> siRNA target sequence in human ribonucleotide reductase R2 subunit mRNA starting at position 1952
<400> 192
   aaagtcagtc ctgtgtatac c 21
<210> 193
   <211> 21
   <212> DNA
   <213> Artificial Sequence
<220>
   <223> siRNA target sequence in human ribonucleotide reductase R2 subunit mRNA starting at position 1953
<400> 193
   aagtcagtcc tgtgtatacc t 21
<210> 194
   <211> 21
   <212> DNA
   <213> Artificial Sequence
<220>
   <223> siRNA target sequence in human ribonucleotide reductase R2 subunit mRNA starting at position 2081
<400> 194
   aaaggaatct ctcagggcaa g 21
<210> 195
   <211> 21
   <212> DNA
   <213> Artificial Sequence
<220>
   <223> siRNA target sequence in human ribonucleotide reductase R2 subunit mRNA starting at position 2082
<400> 195
   aaggaatctc tcagggcaag g 21
<210> 196
   <211> 21
   <212> DNA
   <213> Artificial Sequence
<220>
   <223> siRNA target sequence in human ribonucleotide reductase R2 subunit mRNA starting at position 2086
<400> 196
   aatctctcag ggcaaggagc t 21
<210> 197
   <211> 21
   <212> DNA
   <213> Artificial
<220>
   <223> siRNA target sequence in human ribonucleotide reductase R2 subunit mRNA starting at position 2198

<400> 197
   aacgtctggt tgatgagaaa a 21
<210> 198
   <211> 21
   <212> DNA
   <213> Artificial Sequence
<220>
   <223> siRNA target sequence in human ribonucleotide reductase R2 subunit mRNA starting at position 2227
<400> 198
   aagagttttc atatgtggga g 21
<210> 199
   <211> 21
   <212> DNA
   <213> Artificial Sequence
<220>
   <223> siRNA target sequence in human ribonucleotide reductase R2 subunit mRNA starting at position 2288
<400> 199
   aatgatccac ctaagatctt g 21
<210> 200
   <211> 21
   <212> DNA
   <213> Artificial Sequence
<220>
   <223> siRNA target sequence in human ribonucleotide reductase R2 subunit mRNA starting at position 2317
<400> 200
   aagtggtgaa atcaactaga g 21
<210> 201
   <211> 21
   <212> DNA
   <213> Artificial Sequence
<220>
   <223> siRNA target sequence in human ribonucleotide reductase R2 subunit mRNA starting at position 2325
<400> 201
   aaatcaacta gaggtggttc c 21
<210> 202
   <211> 21
   <212> DNA
   <213> Artificial Sequence
<220>
   <223> siRNA target sequence in human ribonucleotide reductase R2 subunit mRNA starting at position 2326
<400> 202
   aatcaactag aggtggttcc t 21
<210> 203
   <211> 21
   <212> DNA
   <213> Artificial Sequence
<220>
   <223> siRNA target sequence in human ribonucleotide reductase R2 subunit mRNA starting at position 2330
<400> 203
   aactagaggt ggttcctaca a 21
<210> 204
<220>
   <223> Blank sequence
<400> 204
   000
<210> 205
   <211> 20
   <212> DNA
   <213> Artificial Sequence
<220>
   <223> Antisense oligonucleotide (AS-I-35-20) against human ribonucleotide reductase R1 subunit
<400> 205
   gttccagcca gacagcactt 20
<210> 206
   <211> 20
   <212> DNA
   <213> Artificial Sequence
<220>
   <223> Antisense oligonucleotide (AS-I-37-20) against human ribonucleotide reductase R1 subunit
<400> 206
   gagttccagc cagacagcac 20
<210> 207
   <211> 20
   <212> DNA
   <213> Artificial Sequence
<220>
   <223> Antisense oligonucleotide (AS-I-85-20) against human ribonucleotide reductase R1 subunit
<400> 207
   cagagtggga agggttaggt 20
<210> 208
   <211> 20
   <212> DNA
   <213> Artificial Sequence
<220>
   <223> Antisense oligonucleotide (AS-I-91-20) against human ribonucleotide reductase R1 subunit
<400> 208
   aggtgacaga gtgggaaggg 20
<210> 209
   <211> 20
   <212> DNA
   <213> Artificial Sequence
<220>
   <223> Antisense oligonucleotide (AS-I-129-20) against human ribonucleotide reductase R1 subunit
<400> 209
   gactggactg cggctctaaa 20
<210> 210
   <211> 20
   <212> DNA
   <213> Artificial Sequence
<220>
   <223> Antisense oligonucleotide (AS-I-203-20) against human ribonucleotide reductase R1 subunit
<400> 210
   atgactcgtt cttggcggcc 20
<210> 211
   <211> 20
   <212> DNA
   <213> Artificial Sequence
<220>
   <223> Antisense oligonucleotide (AS-I-239-20) against human ribonucleotide reductase R1 subunit
<400> 211
   caaagcttct ggattcgaga 20
<210> 212
   <211> 20
   <212> DNA
   <213> Artificial Sequence
<220>
   <223> Antisense oligonucleotide (AS-I-287-20) against human ribonucleotide reductase R1 subunit
<400> 212
   ttcatggtga tctgagcagg 20
<210> 213
   <211> 20
   <212> DNA
   <213> Artificial Sequence
<220>
   <223> Antisense oligonucleotide (AS-I-300-20) against human ribonucleotide reductase R1 subunit
<400> 213
   gccttggatt actttcatgg 20
<210> 214
   <211> 20
   <212> DNA
   <213> Artificial Sequence
<220>
   <223> Antisense oligonucleotide (AS-I-348-20) against human ribonucleotide reductase R1 subunit
<400> 214
   ttcagcagcc aaagtatcta 20
<210> 215
   <211> 20
   <212> DNA
   <213> Artificial Sequence
<220>
   <223> Antisense oligonucleotide (AS-I-395-20) against human ribonucleotide reductase R1 subunit
<400> 215
   gccaggatag catagtcagg 20
<210> 216
   <211> 20
   <212> DNA
   <213> Artificial Sequence
<220>
<223> Antisense oligonucleotide (AS-I-439-20) against human ribonucleotide reductase R1 subunit
<400> 216
   ctttctttgt ttctttgtgc 20
<210> 217
   <211> 20
   <212> DNA
   <213> Artificial Sequence
<220>
   <223> Antisense oligonucleotide (AS-I-504-20) against human ribonucleotide reductase R1 subunit
<400> 217
   gggagagtgt ttgccattat 20
<210> 218
   <211> 20
   <212> DNA
   <213> Artificial Sequence
<220>
   <223> Antisense oligonucleotide (AS-I-520-20) against human ribonucleotide reductase R1 subunit
<400> 218
   ttgacttggc caccatggga 20
<210> 219
   <211> 20
   <212> DNA
   <213> Artificial Sequence
<220>
   <223> Antisense oligonucleotide (AS-I-540-20) Against human ribonucleotide reductase R1 subunit
<400> 219
   ggccagaaca atatccaatg 20
<210> 220
   <211> 20
   <212> DNA
   <213> Artificial Sequence
<220>
   <223> Antisense oligonucleotide (AS-I-556-20) against human ribonucleotide reductase R1 subunit
<400> 220
   tcaggcgatc tttattggcc 20
<210> 221
   <211> 20
   <212> DNA
   <213> Artificial Sequence
<220>
   <223> Antisense oligonucleotide (AS-1-635-20) against human ribonucleotide reductase R1 subunit
<400> 221
   ttcaacaaat aagaccgctc 20
<210> 222
   <211> 20
   <212> DNA
   <213> Artificial Sequence
<220>
   <223> Antisense oligonucleotide (AS-I-658-20) against human ribonucleotide reductase R1 subunit
<400> 222
   tttcagccac ttttccattg 20
<210> 223
   <211> 20
   <212> DNA
   <213> Artificial Sequence
<220>
   <223> Antisense oligonucleotide (AS-I-662-20) against human ribonucleotide reductase R1 subunit
<400> 223
   ggtctttcag ccacttttcc 20
<210> 224
   <211> 20
   <212> DNA
   <213> Artificial Sequence
<220>
   <223> Antisense oligonucleotide (AS-I-782-20) against human ribonucleotide reductase R1 subunit
<400> 224
   ttgaagagag tgggcgaagc 20
<210> 225
   <211> 20
   <212> DNA
   <213> Artificial Sequence
<220>
   <223> Antisense oligonucleotide (AS-I-786-20) against human ribonucleotide reductase R1 subunit
<400> 225
   agcattgaag agagtgggcg 20
<210> 226
   <211> 20
   <212> DNA
   <213> Artificial Sequence
<220>
   <223> Antisense oligonucleotide (AS-I-809-20) against human ribonucleotide reductase R1 subunit
<400> 226
   gaaagttgcg ggcggttggt 20
<210> 227
   <211> 20
   <212> DNA
   <213> Artificial Sequence
<220>
   <223> Antisense oligonucleotide (AS-I-893-20) against human ribonucleotide reductase R1 subunit
<400> 227
   gctgtcatct ttcatactca 20
<210> 228
   <211> 20
   <212> DNA
   <213> Artificial Sequence
<220>
   <223> Antisense oligonucleotide (AS-1-908-20) against human ribonucleotide reductase R1 subunit
<400> 228
   ccaattcctc cagcagactt 20
<210> 229
   <211> 20
   <212> DNA
   <213> Artificial Sequence
<220>
   <223> Antisense oligonucleotide (AS-I-923-20) against human ribonucleotide reductase R1 subunit
<400> 229
   caactcacag caacaccaat 20
<210> 230
   <211> 20
   <212> DNA
   <213> Artificial Sequence
<220>
   <223> Antisense oligonucleotide (AS-I-932-20) against human ribonucleotide reductase R1 subunit
<400> 230
   gcccgaatac aactcacagc 20
<210> 231
   <211> 20
   <212> DNA
   <213> Artificial Sequences
<220>
   <223> Antisense oligonucleotide (AS-I-967-20) against human ribonucleotide reductase R1 subunit
<400> 231
   aattgccatt agtcccagca 20
<210> 232
   <211> 20
   <212> DNA
   <213> Artificial Sequence
<220>
   <223> Antisense oligonucleotide (AS-I-1051-20) against human ribonucleotide reductase R1 subunit
<400> 232
   atgccccagg acgcttgttc 20
<210> 233
   <211> 20
   <212> DNA
   <213> Artificial Sequence
<220>
   <223> Antisense oligonucleotide (AS-I-1074-20) against human ribonucleotide reductase R1 subunit
<400> 233
   ccaaggctcc aggtaaatag 20
<210> 234
   <211> 20
   <212> DNA
   <213> Artificial Sequence
<220>
   <223> Antisense oligonucleotide (AS-I-1134-20) against human ribonucleotide reductase R1 subunit

<400> 234
   acgctgctct tcctttcctg 20
<210> 235
   <211> 20
   <212> DNA
   <213> Artificial Sequence
<220>
   <223> Antisense oligonucleotide (AS-I-1162-20) against human ribonucleotide reductase R1 subunit
<400> 235
   tccaaagagc aaagaaaaga 20
<210> 236
   <211> 20
   <212> DNA
   <213> Artificial Sequence
<220>
   <223> Antisense oligonucleotide (AS-I-1258-20) against human ribonucleotide reductase R1 subunit
<400> 236
   cctctcccca aacctcatcc 20
<210> 237
   <211> 20
   <212> DNA
   <213> Artificial Sequence
<220>
   <223> Antisense oligonucleotide (AS-I-1311-20) against human ribonucleotide reductase R1 subunit
<400> 237
   aactttgcgg acacgacctt 20
<210> 238
   <211> 20
   <212> DNA
   <213> Artificial Sequence
<220>
   <223> Antisense oligonucleotide (AS-I-1370-20) against human ribonucleotide reductase R1 subunit
<400> 238
   ggggtgcctg tttccgtctg 20
<210> 239
   <211> 20
   <212> DNA
   <213> Artificial Sequence.
<220>
   <223> Antisense oligonucleotide (AS-I-1418-20) against human ribonucleotide reductase R1 subunit
<400> 239
   ttctgctggt tgctctttcg 20
<210> 240
   <211> 20
   <212> DNA
   <213> Artificial Sequence
<220>
   <223> Antisense oligonucleotide (AS-I-1421-20) against human ribonucleotide reductase R1 subunit
<400> 240
   aggttctgct ggttgctctt 20
<210> 241
   <211> 20
   <212> DNA
   <213> Artificial Sequence
<220>
   <223> Antisense oligonucleotide (AS-I-1513-20) against human ribonucleotide reductase R1 subunit
<400> 241
   gggccaggga agccaaatta 20
<210> 242
   <211> 20
   <212> DNA
   <213> Artificial Sequence
<220>
   <223> Antisense oligonucleotide (AS-I-1662-20) against human ribonucleotide reductase R1 subunit
<400> 242
   ggggcgatgg .cgtttatttg 20
<210> 243
   <211> 20
   <212> DNA
   <213> Artificial Sequence
<220>
   <223> Antisense oligonucleotide (AS-I-1666-20) against human ribonucleotide reductase R1 subunit
<400> 243
   caatggggcg atggcgttta 20
<210> 244
   <211> 20
   <212> DNA
   <213> Artificial Sequence
<220>
   <223> Antisense oligonucleotide (AS-I-1785-20) against human ribonucleotide reductase R1 subunit
<400> 244
   ttccagagca ccataataaa 20
<210> 245
   <211> 20
   <212> DNA
   <213> Artificial Sequence
<220>
   <223> Antisense oligonucleotide (AS-I-1818-20) against human ribonucleotide reductase R1 subunit
<400> 245
   tgggccctgc tccttggcaa 20
<210> 246
   <211> 20
   <212> DNA
   <213> Artificial Sequence
<220>
   <223> Antisense oligonucleotide (AS-I-1970-20) against human ribonucleotide reductase R1 subunit
<400> 246
   ggcatcgggg caataagtaa 20
<210> 247
   <211> 20
   <212> DNA
   <213> Artificial Sequence
<220>
   <223> Antisense oligonucleotide (AS-I-1976-20) against human ribonucleotide reductase R1 subunit
<400> 247
   gctgtaggca tcggggcaat 20
<210> 248
   <211> 20
   <212> DNA
   <213> Artificial Sequence
<220>
   <223> Antisense oligonucleotide (AS-I-2119-20) against human ribonucleotide reductase R1 subunit
<400> 248
   catgccatag gccccgctcg 20
<210> 249
   <211> 20
   <212> DNA
   <213> Artificial Sequence
<220>
   <223> Antisense oligonucleotide (AS-I-2198-20) against human ribonucleotide reductase R1 subunit
<400> 249
   agttgcttca ggtcatcagg 20
<210> 250
   <211> 20
   <212> DNA
   <213> Artificial Sequence
<220>
   <223> Antisense oligonucleotide (AS-I-2251-20) against human ribonucleotide reductase subunit
<400> 250
   cagctgccat cttgagaaca 20
<210> 251
   <211> 20
   <212> DNA
   <213> Artificial Sequence
<220>
   <223> Antisense oligonucleotide (AS-I-2304-20) against human ribonucleotide reductase R1 subunit
<400> 251
   ctcagcaatg tggatgttca 20
<210> 252
   <211> 20
   <212> DNA
   <213> Artificial Sequence
<220>
   <223> Antisense oligonucleotide (AS-I-2364-20) against human ribonucleotide reductase R1 subunit
<400> 252
   agtcttcaaa ccctgcttcc 20
<210> 253
   <211> 20
   <212> DNA
   <213> Artificial Sequence
<220>
   <223> Antisense oligonucleotide (AS-I-2370-20) against human ribonucleotide reductase R1 subunit
<400> 253
   catcccagtc ttcaaaccct 20
<210> 254
   <211> 20
   <212> DNA
   <213> Artificial Sequence
<220>
   <223> Antisense oligonucleotide (AS-1-2414-20) against human ribonucleotide reductase R1 subunit
<400> 254
   gtgaactgga ttggattagc 20
<210> 255
   <211> 20
   <212> DNA
   <213> Artificial Sequence
<220>
   <223> Antisense oligonucleotide (AS-I-2491-20) against human ribonucleotide reductase R1 subunit
<400> 255
   tggctgctgt gttcctctcc 20
<210> 256
   <211> 20
   <212> DNA
   <213> Artificial Sequence
<220>
   <223> Antisense oligonucleotide (AS-I-2556-20) against human ribonucleotide reductase R1 subunit
<400> 256
   cttccaagtc tttcctcagg 20
<210> 257
   <211> 20
   <212> DNA
   <213> Artificial Sequence
<220>
   <223> Antisense oligonucleotide (AS-I-2629-20) against human ribonucleotide reductase R1 subunit
<400> 257
   taccacctca agcaaaccca 20
<210> 258
   <211> 20
   <212> DNA
   <213> Artificial Sequence
<220>
   <223> Antisense oligonucleotide (AS-I-2650-20) against human ribonucleotide reductase R1 subunit
<400> 258
   caacagggtc cagcaaagcc 20
<210> 259
   <211> 20
   <212> DNA
   <213> Artificial Sequence
<220>
   <223> Antisense oligonucleotide (AS-I-2769-20) against human ribonucleotide reductase R1 subunit
<400> 259
   tccgtttttt ttttcttttt 20
<210> 260
   <211> 20
   <212> DNA
   <213> Artificial Sequence
<220>
   <223> Antisense oligonucleotide (AS-I-2863-20) against human ribonucleotide reductase R1 subunit
<400> 260
   tgctaaatgg gtgatgaaac 20
<210> 261
   <211> 20
   <212> DNA
   <213> Artificial Sequence.
<220>
   <223> Antisense oligonucleotide (AS-I-2922-20) against human ribonucleotide reductase R1 subunit
<400> 261
   cccaccagtc aaagcagtaa 20
<210> 262
   <211> 20
   <212> DNA
   <213> Artificial Sequence
<220>
   <223> Antisense oligonucleotide (AS-I-2594-20) against human ribonucleotide reductase R1 subunit
<400> 262
   ctcaagaagt agtttggcta 20
<210> 263
   <211> 20
   <212> DNA
   <213> Artificial Sequence
<220>
   <223> Antisense oligonucleotide (AS-I-3-20) against human ribonucleotide reductase R1 subunit
<400> 263
   aggcgcaaca atccaaatcc 20
<210> 264
   <211> 20
   <212> DNA
   <213> Artificial Sequence
<220>
   <223> Antisense oligonucleotide (AS-I-19-20) against human ribonucleotide reductase R1 subunit
<400> 264
   actttcttca gagcagaggc 20
<210> 265
   <211> 20
   <212> DNA
   <213> Artificial Sequence
<220>
   <223> Antisense oligonucleotide (AS-I-55-20) against human ribonucleotide reductase R1 subunit
<400> 265
   gctcagggga aagaactgga 20
<210> 266
   <211> 20
   <212> DNA
   <213> Artificial Sequence
<220>
   <223> Antisense oligonucleotide (AS-I-73-20) against human ribonucleotide reductase R1 subunit
<400> 266
   ggttaggttc caggcgttgc 20
<210> 267
   <211> 20
   <212> DNA
   <213> Artificial Sequence
<220>
   <223> Antisense oligonucleotide (AS-I-158-20) against human ribonucleotide reductase R1 subunit
<400> 267
   gctagtggct gaggctctga 20
<210> 268
   <211> 20
   <212> DNA
   <213> Artificial Sequence
<220>
   <223> Antisense oligonucleotide (AS-I-329-20) against human ribonucleotide reductase R1 subunit
<400> 268
   agttccactg tggtgacccc 20
<210> 269
   <211> 20
   <212> DNA
   <213> Artificial Sequence
<220>
   <223> Antisense oligonucleotide (AS-I-378-20) against human ribonucleotide reductase R1 subunit
<400> 269
   agggtgctta gtagtcaagg 20
<210> 270
   <211> 20
   <212> DNA
   <213> Artificial Sequence
<220>
   <223> Antisense oligonucleotide (AS-I-420-20) against human ribonucleotide reductase R1 subunit
<400> 270
   caagttagag acagcgatcc 20
<210> 271
   <211> 20
   <212> DNA
   <213> Artificial Sequences
<220>
   <223> Antisense oligonucleotide (AS-I-492-20) against human ribonucleotide reductase R1 subunit

<400> 271
   gccattatgt ggatttatgt 20
<210> 272
   <211> 20
   <212> DNA
   <213> Artificial Sequence
<220>
   <223> Antisense oligonucleotide (AS-I-578-20) against human ribonucleotide reductase R1 subunit
<400> 272
   cggtcataga taatagcaga 20
<210> 273
   <211> 20
   <212> DNA
   <213> Artificial Sequence
<220>
   <223> Antisense oligonucleotide (AS-I-603-20) against human ribonucleotide reductase R1 subunit
<400> 273
   gccgaagtaa ttgtaagaga 20
<210> 274
   <211> 20
   <212> DNA
   <213> Artificial Sequence
<220>
   <223> Antisense oligonucleotide (AS-I-618-20) against human ribonucleotide reductase R1 subunit
<400> 274
   ctctagcgtc ttaaagccga 20
<210> 275
   <211> 20
   <212> DNA
   <213> Artificial Sequence
<220>
   <223> Antisense oligonucleotide (AS-I-720-20) against human ribonucleotide reductase R1 subunit
<400> 275
   tgctgcatca atgtcttctt 20
<210> 276
   <211> 20
   <212> DNA
   <213> Artificial Sequence
<220>
   <223> Antisense oligonucleotide (AS-I-758-20) against human ribonucleotide reductase R1 subunit
<400> 276
   gtaaaccacc tctcagaaag 20
<210> 277
   <211> 20
   <212> DNA
   <213> Artificial Sequence
<220>
   <223> Antisense oligonucleotide (AS-I-808-20) against human ribonucleotide reductase R1 subunit
<400> 277
   aaagttgcgg gcggttggta 20
<210> 278
   <211> 20
   <212> DNA
   <213> Artificial Sequence
<220>
   <223> Antisense oligonucleotide (AS-I-863-20) against human ribonucleotide reductase R1 subunit
<400> 278
   gtgtcataaa tgccttcaat 20
<210> 279
   <211> 20
   <212> DNA
   <213> Artificial Sequence
<220>
   <223> Antisense oligonucleotide (AS-I-941-20) against human ribonucleotide reductase R1 subunit
<400> 279
   ctgccagtag cccgaataca 20
<210> 280
   <211> 20
   <212> DNA
   <213> Artificial Sequence
<220>
   <223> Antisense oligonucleotide (AS-I-996-20) against human ribonucleotide reductase R1 subunit
<400> 280
   tactctcagc atcggtacaa 20
<210> 281
   <211> 20
   <212> DNA
   <213> Artificial Sequence
<220>
   <223> Antisense oligonucleotide (AS-I-1057-20) against human ribonucleotide reductase R1 subunit
<400> 281
   tagcaaatgc cccaggacgc 20
<210> 282
   <211> 20
   <212> DNA
   <213> Artificial Sequence
<220>
   <223> Antisense oligonucleotide (AS-I-1083-20) against human ribonucleotide reductase R1 subunit
<400> 282
   gtctaaatgc caaggctcca 20
<210> 283
   <211> 20
   <212> DNA
   <213> Artificial Sequence
<220>
   <223> Antisense oligonucleotide (AS-I-1135-20) against human ribonucleotide reductase R1 subunit
<400> 283
   cacgctgctc ttcctttcct 20
<210> 284
   <211> 20
   <212> DNA
   <213> Artificial Sequence
<220>
   <223> Antisense oligonucleotide (AS-I-1235-20) against human ribonucleotide reductase R1 subunit
<400> 284
   ccaggacact catttggaca 20
<210> 285
   <211> 20
   <212> DNA
   <213> Artificial Sequence
<220>
   <223> Antisense oligonucleotide (AS-I-1298-20) against human ribonucleotide reductase R1 subunit
<400> 285
   cgaccttgtt tctcataact 20
<210> 286
   <211> 20
   <212> DNA
   <213> Artificial Sequence
<220>
   <223> Antisense oligonucleotide (AS-I-1319-20) against human ribonucleotide reductase R1 subunit
<400> 286
   gcttttacaa ctttgcggac 20
<210> 287
   <211> 20
   <212> DNA
   <213> Artificial Sequence
<220>
   <223> Antisense oligonucleotide (AS-I-1351-20) against human ribonucleotide reductase R1 subunit
<400> 287
   gagactcaat gatggcatac 20
<210> 288
   <211> 20
   <212> DNA
   <213> Artificial Sequence
<220>
   <223> Antisense oligonucleotide (AS-I-1441-20) against human ribonucleotide reductase R1 subunit
<400> 288
   tgctgcattt gatggttccc 20
<210> 289
   <211> 20
   <212> DNA
   <213> Artificial Sequence
<220>
   <223> Antisense oligonucleotide (AS-I-1483-20) against human ribonucleotide reductase R1 subunit
<400> 289
   cctcatcttt gctggtgtac 20
<210> 290
   <211> 20
   <212> DNA
   <213> Artificial Sequence
<220>
   <223> Antisense oligonucleotide (AS-I-1570-20) against human ribonucleotide reductase R1 subunit
<400> 290
   tgacttcagc caacttctta 20
<210> 291
   <211> 20
   <212> DNA
   <213> Artificial Sequence
<220>
   <223> Antisense oligonucleotide (AS-I-1599-20) against human ribonucleotide reductase R1 subunit
<400> 291
   tttattcaag tttcggacaa 20
<210> 292
   <211> 20
   <212> DNA
   <213> Artificial Sequence
<220>
   <223> Antisense oligonucleotide (AS-I-1636-20) against human ribonucleotide reductase R1 subunit
<400> 292
   atgcctctgg tacaggatag 20
<210> 293
   <211> 20
   <212> DNA
   <213> Artificial Sequence
<220>
   <223> Antisense oligonucleotide (AS-I-1661-20) against human ribonucleotide reductase R1 subunit
<400> 293
   gggcgatggc gtttatttga 20
<210> 294
   <211> 20
   <212> DNA
   <213> Artificial Sequence
<220>
   <223> Antisense oligonucleotide (AS-I-1685-20) against human ribonucleotide reductase R1 subunit
<400> 294
   agaccttgta ccccaattcc 20
<210> 295
   <211> 20
   <212> DNA
   <213> Artificial Sequence
<220>
   <223> Antisense oligonucleotide (AS-I-1704-20) against human ribonucleotide reductase R1 subunit
<400> 295
   caggataaaa gcatctgcca 20
<210> 296
   <211> 20
   <212> DNA
   <213> Artificial Sequence
<220>
   <223> Antisense oligonucleotide (AS-I-1721-20) against human ribonucleotide reductase R1 subunit
<400> 296
   tcaaaagggt atctcatcag 20
<210> 297
   <211> 20
   <212> DNA
   <213> Artificial Sequence
<220>
   <223> Antisense oligonucleotide (AS-I-1839-20) against human ribonucleotide reductase R1 subunit
<400> 297
   agagccctca taggtttcgt 20
<210> 298
   <211> 20
   <212> DNA
   <213> Artificial Sequence
<220>
   <223> Antisense oligonucleotide (AS-I-1840-20) against human ribonucleotide reductase R1 subunit
<400> 298
   gagagccctc ataggtttcg 20
<210> 299
   <211> 20
   <212> DNA
   <213> Artificial Sequence
<220>
   <223> Antisense oligonucleotide (AS-I-1900-20) against human ribonucleotide reductase R1 subunit
<400> 299
   cccataggtc tgtaggagta 20
<210> 300
   <211> 20
   <212> DNA
   <213> Artificial Sequence
<220>
   <223> Antisense oligonucleotide (as-I-2004-20) against human ribonucleotide reductase R1 subunit
<400> 300
   attattcccc aggatctgag 20
<210> 301
   <211> 20
   <212> DNA
   <213> Artificial Sequence
<220>
   <223> Antisense oligonucleotide (AS-I-2034-20) against human ribonucleotide reductase R1 subunit
<400> 301
   gatgttgctg gtgtaaggtt 20
<210> 302
   <211> 20
   <212> DNA
   <213> Artificial Sequence
<220>
   <223> Antisense oligonucleotide (AS-I-2060-20) against human ribonucleotide reductase R1 subunit
<400> 302
   tctcctgaca agactctgcg 20
<210> 303
   <211> 20
   <212> DNA
   <213> Artificial Sequence
<220>
   <223> Antisense oligonucleotide (AS-I-2220-20) against human ribonucleotide reductase R1 subunit
<400> 303
   gatttcccac acagttttat 20
<210> 304
   <211> 20
   <212> DNA
   <213> Artificial Sequence
<220>
   <223> Antisense oligonucleotide (AS-1-2324-20) against human ribonucleotide reductase R1 subunit
<400> 304
   gtgagtttgc catagttagg 20
<210> 305
   <211> 20
   <212> DNA
   <213> Artificial Sequence
<220>
   <223> Antisense oligonucleotide (AS-I-2358-20) against human ribonucleotide reductase R1 subunit
<400> 305
   caaaccctgc ttccagccgt 20
<210> 306
   <211> 20
   <212> DNA
   <213> Artificial Sequence
<220>
   <223> Antisense oligonucleotide (AS-I-2390-20) against human ribonucleotide reductase R1 subunit
<400> 306
   ggtctcgtcc ttaaataata 20
<210> 307
   <211> 20
   <212> DNA
   <213> Artificial Sequence
<220>
   <223> Antisense oligonucleotide (AS-I₋2584-20) against human ribonucleotide reductase R1 subunit
<400> 307
   agtttggcta ctgaagacat 20
<210> 308
   <211> 20
   <212> DNA
   <213> Artificial Sequence
<220>
   <223> Antisense oligonucleotide (AS-I-2669-20) against human ribonucleotide reductase R1 subunit

<400> 308
   caattactcc ttttgcctgc 20
<210> 309
   <211> 20
   <212> DNA
   <213> Artificial Sequence
<220>
   <223> Antisense oligonucleotide (AS-I-2831-20) against human ribonucleotide reductase R1 subunit
<400> 309
   tccctgtatg caagatgact 20
<210> 310
   <211> 20
   <212> DNA
   <213> Artificial Sequence
<220>
   <223> Antisense oligonucleotide (AS-I-2924-20) against human ribonucleotide reductase R1 subunit
<400> 310
   cccaccagtc aaagcagtaa 20
<210> 311
   <211> 20
   <212> DNA
   <213> Artificial Sequence
<220>
   <223> Antisense oligonucleotide (AS-I-2986-20) against human ribonucleotide reductase R1 subunit
<400> 311
   ccagataaag gtcctatcag 20
<210> 312
   <211> 20
   <212> DNA
   <213> Artificial Sequence
<220>
   <223> Antisense oligonucleotide (AS-II-6-20) against human ribonucleotide reductase R2 subunit
<400> 312
   acccttccca ttggctgcgc 20
<210> 313
   <211> 20
   <212> DNA
   <213> Artificial Sequence
<220>
   <223> Partially phosphorothioated antisense oligonucleotide (AS-II-13-20) against human ribonucleotide reductase R2 subunit
   <221> misc_difference
   <222> (1)...(2)
   <223> Partially phosphorothioated antisense oligonucleotide
   <221> misc_difference
   <222> (3)...(4)
   <223> Partially phosphorothioated antisense oligonucleotide
   <221> misc_difference
   <222> (7)...(8)
   <223> Partially phosphorothioated antisense oligonucleotide
   <221> misc_difference
   <222> (10)...(11)
   <223> Partially phosphorothoated antisense oligonucleotide
   <221> misc_difference
   <222> (14)...(15)
   <223> Partially phosphorothoated antisense oligonucleotide
   <221> misc_difference
   <222> (16)...(17)
   <223> Partially phosphorothoated antisense oligonucleotide
   <221> misc_difference
   <222> (19)...(20)
   <223> Partially phosphorothoated antisense oligonucleotide
<400> 313
   gcctccgacc cttcccattg 20
<210> 314
   <211> 20
   <212> DNA
   <213> Artificial Sequence
<220>
   <223> Antisense oligonucleotide (AS-II-14-20) against human ribonucleotide reductase R2 subunit
<400> 314
   tgcctccgac ccttcccatt 20
<210> 315
   <211> 18
   <212> DNA
   <213> Artificial Sequence
<220>
   <223> Antisense oligonucleotide (AS-II-16-18) against human ribonucleotide reductase R2 subunit
<400> 315
   tgcctccgac ccttccca 18
<210> 316
   <211> 20
   <212> DNA
   <213> Artificial Sequence
<220>
   <223> Partially phosphorothioated antisense oligonucleotide (AS-II-75-20) against human ribonucleotide reductase R2 subunit
<220>
   <223> Partially phosphorothioated antisense oligonucleotide (AS-II-75-20) against human ribonucleotide reductase R2 subunit
   <221> misc_difference
   <222> (1)...(2)
   <223> Phosphorothioate internucleotide linkage
   <221> misc_difference
   <222> (4)...(5)
   <223> Phosphorothioate internucleotide linkage
   <221> misc_difference
   <222> (7)...(8)
   <223> Phosphorothioate internucleotide linkage
   <221> misc difference
   <222> (10)...(11)
   <223> Phosphorothioate internucleotide linkage
   <221> misc_difference
   <222> (13)...(14)
   <223> Phosphorothioate internucleotide linkage
   <221> misc_difference
   <222> (16)...(17)
   <223> Phosphorothioate internucleotide linkage
   <221> misc_difference
   <222> (19)...(20)
   <223> Phosphorothioate internucleotide linkage
<400> 316
   cgcgcgctcc cggcccttcc 20
<210> 317
   <211> 20
   <212> DNA
   <213> Artificial Sequence
<220>
   <223> Antisense oligonucleotide (AS-II-75-20) against human ribonucleotide reductase R2 subunit
<400> 317
   cgcgcgctcc cggcccttcc 20
<210> 318
   <211> 14
   <212> DNA
   <213> Artificial Sequence
<220>
   <223> Antisense oligonucleotide (AS-II-79-14) against human ribonucleotide reductase R2 subunit
<400> 318
   cgcgctcccg gccc 14
<210> 319
   <211> 20
   <212> DNA
   <213> Artificial Sequence
<220>
   <223> Partially phosphorothioated antisense oligonucleotide (AS-II-109-20) against human ribonucleotide reductase R2 subunit
   <221> misc_difference
   <222> (1) ... (2)
   <223> Phosphorothioate internucleotide linkage
   <221> misc_difference
   <222> (4)...(5)
   <223> Phosphorothioate internucleotide linkage
   <221> misc_difference
   <222> (8)...(9)
   <223> Phosphorothioate internucleotide linkage
   <221> misc_difference
   <222> (11)...(12)
   <223> Phosphorothioate internucleotide linkage
   <221> misc_difference
   <222> (14)...(15)
   <223> Phosphorothioate internucleotide linkage
   <221> misc_difference
   <222> (17)...(18)
   <223> Phosphorothioate internucleotide linkage
   <221> misc_difference
   <222> (19)...(20)
   <223> Phosphorothioate internucleotide linkage
<400> 319
   cccctcactc cagcagcctt 20
<210> 320
   <211> 20
   <212> DNA
   <213> Artificial Sequence
<220>
   <223> Antisense oligonucleotide (AS-II-110-20) against human ribonucleotide reductase R2 subunit
<400> 320
   acccctcact ccagcagcct 20
<210> 321
   <211> 20
   <212> DNA
   <213> Artificial Sequence
<220>
   <223> Antisense oligonucleotide (AS-II-114-20) against human ribonucleotide reductase R2 subunit
<400> 321
   ggcgacccct cactccagca 20
<210> 322
   <211> 12
   <212> DNA
   <213> Artificial Sequence
<220>
   <223> Antisense oligonucleotide (AS-II-127-12) against human ribonucleotide reductase R2 subunit
<400> 322
   gcacgggcga cc 12
<210> 323
   <211> 20
   <212> DNA
   <213> Artificial Sequence
<220>
   <223> Antisense oligonucleotide (AS-II-130-20) against human ribonucleotide reductase R2 subunit
<400> 323
   tgggacaggg tgcacgggcg 20
<210> 324
   <211> 20
   <212> DNA
   <213> Artificial Sequence
<220>
   <223> Antisense oligonucleotide (AS-II-134-20) against human ribonucleotide reductase R2 subunit
<400> 324
   gacggctggg acagggtgca 20
<210> 325
   <211> 20
   <212> DNA
   <213> Artificial Sequence
<220>
   <223> Antisense oligonucleotide (AS-II-151-20) against human ribonucleotide reductase R2 subunit
<400> 325
   gagcagccag gacaggacgg 20
<210> 326
   <211> 20
   <212> DNA
   <213> Artificial Sequence
<220>
   <223> Partially phosphorothioated antisense oligonucleotide (AS-II-163-20) against human ribonucleotide reductase R2 subunit
   <221> misc_difference
   <222> (1)...(2)
   <223> Phosphorothioate internucleotide linkage
   <221> misc_difference
   <222> (3)...(4)
   <223> Phosphorothioate internucleotide linkage
   <221> misc_difference
   <222> (6)...(7)
   <223> Phosphorothioate internucleotide linkage
   <221> misc_difference
   <222> (9)...(10)
   <223> Phosphorothioate internucleotide linkage
   <221> misc_difference
   <222> (12)...(13)
   <223> Phosphorothioate internucleotide linkage
   <221> misc_difference
   <222> (15)...(16)
   <223> Phosphorothioate internucleotide linkage
   <221> misc_difference
   <222> (19)... (20)
   <223> Phosphorothioate internucleotide linkage
<400> 326
   gcgaagcaga gcgagcagcc 20
<210> 327
   <211> 20
   <212> DNA
   <213> Artificial Sequence
<220>
   <223> Antisense oligonucleotide (AS-II-166-20) against human ribonucleotide reductase R2 subunit
<400> 327
   gcagcgaagc agagcgagca 20
<210> 328
   <211> 20
   <212> DNA
   <213> Artificial Sequence
<220>
   <223> Antisense oligonucleotide (AS-II-185-20) against human ribonucleotide reductase R2 subunit
<400> 328
   gggagagcat agtggaggcg 20
<210> 329
   <211> 20
   <212> DNA
   <213> Artificial Sequence
<220>
   <223> Antisense oligonucleotide (AS-II-189-20) against human ribonucleotide reductase R2 subunit
<400> 329
   cggagggaga gcatagtgga 20
<210> 330
   <211> 20
   <212> DNA
   <213> Artificial Sequence
<220>
   <223> Antisense oligonucleotide (AS-II-201-20) against human ribonucleotide reductase R2 subunit
<400> 330
   gcgagcggga cacggaggga 20
<210> 331
   <211> 20
   <212> DNA
   <213> Artificial Sequence
<220>
   <223> Antisense oligonucleotide (AS-II-217-20) against human ribonucleotide reductase R2 subunit
<400> 331
   cgggtccgtg atgggcgcga 20
<210> 332
   <211> 20
   <212> DNA
   <213> Artificial Sequence
<220>
   <223> Antisense oligonucleotide (AS-II-225-20) against human ribonucleotide reductase R2 subunit
<400> 332
   agctgctgcg ggtccgtgat 20
<210> 333
   <211> 14
   <212> DNA
   <213> Artificial Sequence
<220>
   <223> Antisense oligonucleotide (AS-II-253-14) against human ribonucleotide reductase R2 subunit
<400> 333
   ccccttcagc ggcg 14
<210> 334
   <211> 20
   <212> DNA
   <213> Artificial Sequence
<220>
   <223> Antisense oligonucleotide (AS-II-280-20) against human ribonucleotide reductase R2 subunit
<400> 334
   cggcggcgtg ttctccttgt 20
<210> 335
   <211> 12
   <212> DNA
   <213> Artificial Sequence
<220>
   <223> Antisense oligonucleotide (AS-II-288-12) against human ribonucleotide reductase R2 subunit
<400> 335
   cggcggcgtg tt 12
<210> 336
   <211> 20
   <212> DNA
   <213> Artificial Sequence
<220>
   <223> Antisense oligonucleotide (AS-II-323-20) against human ribonucleotide reductase R2 subunit

<400> 336
   tcctcgcggt cttgctggcc 20
<210> 337
   <211> 20
   <212> DNA
   <213> Artificial Sequence
<220>
   <223> Antisense oligonucleotide (AS-II-344-20) against human ribonucleotide reductase R2 subunit
<400> 337
   ccgtgggctc ctggaagatc 20
<210> 338
   <211> 20
   <212> DNA
   <213> Artificial Sequence
<220>
   <223> Antisense oligonucleotide (AS-II-362-20) against human ribonucleotide reductase R2 subunit
<400> 338
   ctgctttagt tttcggctcc 20
<210> 339
   <211> 17
   <212> DNA
   <213> Artificial Sequence
<220>
   <223> Antisense oligonucleotide (AS-II-391-17) against human ribonucleotide reductase R2 subunit
<400> 339
   cggctcatcc tccacgc 17
<210> 340
   <211> 20
   <212> DNA
   <213> Artificial Sequence
<220>
   <223> Antisense oligonucleotide (AS-II-404-20) against human ribonucleotide reductase R2 subunit
<400> 340
   ggttttctct cagcagcggc 20
<210> 341
   <211> 20
   <212> DNA
   <213> Artificial Sequence
<220>
   <223> Antisense oligonucleotide (AS-II-412-20) against human ribonucleotide reductase R2 subunit
<400> 341
   gcggcggggg ttttctctca 20
<210> 342
   <211> 20
   <212> DNA
   <213> Artificial Sequence
<220>
   <223> Antisense oligonucleotide (AS-II-414-20) against human ribonucleotide reductase R2 subunit
<400> 342
   aagcggcggg ggttttctct 20
<210> 343
   <211> 20
   <212> DNA
   <213> Artificial Sequence
<220>
   <223> Antisense oligonucleotide (AS-II-425-20) against human ribonucleotide reductase R2 subunit
<400> 343
   ggaagatgac aaagcggcgg 20
<210> 344
   <211> 20
   <212> DNA
   <213> Artificial Sequence
<220>
   <223> Antisense oligonucleotide (AS-II-439-20) against human ribonucleotide reductase R2 subunit
<400> 344
   atggtactcg atggggaaga 20
<210> 345
   <211> 20
   <212> DNA
   <213> Artificial Sequence
<220>
   <223> Antisense oligonucleotide (AS-II-472-20) against human ribonucleotide reductase R2 subunit
<400> 345
   agcctctgcc ttcttataca 20
<210> 346
   <211> 20
   <212> DNA
   <213> Artificial Sequence
<220>
   <223> Antisense oligonucleotide (AS-II-494-20) against human ribonucleotide reductase R2 subunit
<400> 346
   cctcctcggc ggtccaaaag 20
<210> 347
   <211> 16
   <212> DNA
   <213> Artificial Sequence
<220>
   <223> Antisense oligonucleotide (AS-II-496-16) against human ribonucleotide reductase R2 subunit
<400> 347
   tcctcggcgg tccaaa 16
<210> 348
   <211> 20
   <212> DNA
   <213> Artificial Sequence
<220>
   <223> Antisense oligonucleotide (AS-II-549-20) against human ribonucleotide reductase R2 subunit
<400> 348
   tatctctcct cgggtttcag 20
<210> 349
   <211> 20
   <212> DNA
   <213> Artificial Sequence
<220>
   <223> Antisense oligonucleotide (AS-II-579-20) against human ribonucleotide reductase R2 subunit
<400> 349
   gcaaagaaag ccagaacatg 20
<210> 350
   <211> 20
   <212> DNA
   <213> Artificial Sequence
<220>
   <223> Antisense oligonucleotide (AS-II-619-20) against human ribonucleotide reductase R2 subunit
<400> 350
   tcgctccacc aagttttcat 20
<210> 351
   <211> 20
   <212> DNA
   <213> Artificial Sequence
<220>
   <223> Antisense oligonucleotide (AS-II-626-20) against human ribonucleotide reductase R2 subunit
<400> 351
   ggctaaatcg ctccaccaag 20
<210> 352
   <211> 20
   <212> DNA
   <213> Artificial Sequence
<220>
   <223> Antisense oligonucleotide (AS-II-634-20) against human ribonucleotide reductase R2 subunit
<400> 352
   aacttcttgg ctaaatcgct 20
<210> 353
   <211> 20
   <212> DNA
   <213> Artificial Sequence
<220>
   <223> Antisense oligonucleotide (AS-II-667-20) against human ribonucleotide reductase R2 subunit
<400> 353
   gaagccatag aaacagcggg 20
<210> 354
   <211> 20
   <212> DNA
   <213> Artificial Sequence
<220>
   <223> Antisense oligonucleotide (AS-II-784-20) against human ribonucleotide reductase R2 subunit
<400> 354
   gacacaaggc atcgtttcaa 20
<210> 355
   <211> 20
   <212> DNA
   <213> Artificial Sequence
<220>
   <223> Antisense oligonucleotide (AS-II-798-20) against human ribonucleotide reductase R2 subunit
<400> 355
   tctgccttct tcttgacaca 20
<210> 356
   <211> 20
   <212> DNA
   <213> Artificial Sequence
<220> .
   <223> Antisense oligonucleotide (AS-II-816-20) against human ribonucleotide reductase R2 subunit
<400> 356
   atccagcgca aggcccagtc 20
<210> 357
   <211> 20
   <212> DNA
   <213> Artificial Sequence
<220>
   <223> Antisense oligonucleotide (AS-II-861-20) against human ribonucleotide reductase R2 subunit
<400> 357
   gcaaaggcta caacacgttc 20
<210> 358
   <211> 20
   <212> DNA
   <213> Artificial Sequence
<220>
   <223> Antisense oligonucleotide (AS-II-890-20) against human ribonucleotide reductase R2 subunit
<400> 358
   aaccggaaaa gaaaatgcct 20
<210> 359
   <211> 20
   <212> DNA
   <213> Artificial Sequence
<220>
   <223> Antisense oligonucleotide (AS-II-909-20) against human ribonucleotide reductase R2 subunit
<400> 359
   cagaatatcg acgcaaaaga 20
<210> 360
   <211> 20
   <212> DNA
   <213> Artificial Sequence
<220>
   <223> Antisense oligonucleotide (AS-II-933-20) against human ribonucleotide reductase R2 subunit
<400> 360
   ggcatcagtc ctcgtttctt 20
<210> 361
   <211> 20
   <212> DNA
   <213> Artificial Sequence
<220>
   <223> Antisense oligonucleotide (AS-II-981-20) against human ribonucleotide reductase R2 subunit
<400> 361
   tgtaaaccct catctctgct 20
<210> 362
   <211> 20
   <212> DNA
   <213> Artificial Sequence
<220>
   <223> Antisense oligonucleotide (AS-II-1001-20) against human ribonucleotide reductase R2 subunit
<400> 362
   tcaggcaagc aaaatcacag 20
<210> 363
   <211> 20
   <212> DNA
   <213> Artificial Sequence
<220>
   <223> Antisense oligonucleotide (AS-II-1006-20) against human ribonucleotide reductase R2 subunit
<400> 363
   gaacatcagg caagcaaaat 20
<210> 364
   <211> 20
   <212> DNA
   <213> Artificial Sequence
<220>
   <223> Antisense oligonucleotide (AS-II-1023-20) against human ribonucleotide reductase R2 subunit
<400> 364
   ttgtgtacca ggtgtttgaa 20
<210> 365
   <211> 20
   <212> DNA
   <213> Artificial Sequence
<220>
   <223> Antisense oligonucleotide (AS-II-1040-20) against human ribonucleotide reductase R2 subunit
<400> 365
   ctctctcctc cgatggtttg 20
<210> 366
   <211> 20
   <212> DNA
   <213> Artificial Sequence
<220>
   <223> Antisense oligonucleotide (AS-II-1048-20) against human ribonucleotide reductase R2 subunit
<400> 366
   ttctcttact ctctcctccg 20
<210> 367
   <211> 20
   <212> DNA
   <213> Artificial Sequence
<220>
   <223> Antisense oligonucleotide (AS-II-1144-20) against human ribonucleotide reductase R2 subunit
<400> 367
   gtattgcttc attagagtgc 20
<210> 368
   <211> 20
   <212> DNA
   <213> Artificial Sequence
<220>
   <223> Antisense oligonucleotide (AS-II-1182-20) against human ribonucleotide reductase R2 subunit
<400> 368
   cccagttcca gcataagtct 20
<210> 369
   <211> 20
   <212> DNA
   <213> Artificial Sequence
<220>
   <223> Antisense oligonucleotide (AS-II-1197-20) against human ribonucleotide reductase.R2 subunit
<400> 369
   aaaaccttgc taaaacccag 20
<210> 370
   <211> 20
   <212> DNA
   <213> Artificial Sequence
<220>
   <223> Antisense oligonucleotide (AS-II-1217-20) against human ribonucleotide reductase R2 subunit
<400> 370
   caaatgggtt ctctactctg 20
<210> 371
   <211> 20
   <212> DNA
   <213> Artificial Sequence
<220>
   <223> Antisense oligonucleotide (AS-II-1224-20) against human ribonucleotide reductase R2 subunit
<400> 371
   ataaagtcaa atgggttctc 20
<210> 372
   <211> 20
   <212> DNA
   <213> Artificial Sequence
<220>
   <223> Antisense oligonucleotide (AS-II-1254-20) against human ribonucleotide reductase R2 subunit
<400> 372
   ttagtctttc cttccagtga 20
<210> 373
   <211> 20
   <212> DNA
   <213> Artificial Sequence
<220>
   <223> Antisense oligonucleotide (AS-II-1278-20) against human ribonucleotide reductase R2 subunit
<400> 373
   tcgcctactc tcttctcaaa 20

<210> 374
   <211> 20
   <212> DNA
   <213> Artificial Sequence
<220>
   <223> Antisense oligonucleotide (AS-II-1288-20) against human ribonucleotide reductase R2 subunit
<400> 374
   cctctgatac tcgcctactc 20
<210> 375
   <211> 20
   <212> DNA
   <213> Artificial Sequence
<220>
   <223> Antisense oligonucleotide (AS-II-1302-20) against human ribonucleotide reductase R2 subunit
<400> 375
   gacatcactc ccatcctctg 20
<210> 376
   <211> 20
   <212> DNA
   <213> Artificial Sequence
<220>
   <223> Antisense oligonucleotide (AS-II-1335-20) against human ribonucleotide reductase R2 subunit
<400> 376
   gcatccaagg taaaagaatt 20
<210> 377
   <211> 20
   <212> DNA
   <213> Artificial Sequence
<220>
   <223> Antisense oligonucleotide (AS-II-1338-20) against human ribonucleotide reductase R2 subunit
<400> 377
   tcagcatcca aggtaaaaga 20
<210> 378
   <211> 20
   <212> DNA
   <213> Artificial Sequence
<220>
   <223> Antisense oligonucleotide (AS-II-1342-20) against human ribonucleotide reductase R2 subunit
<400> 378
   gaagtcagca tccaaggtaa 20
<210> 379
   <211> 20
   <212> DNA
   <213> Artificial Sequence
<220>
   <223> Antisense oligonucleotide (AS-II-1345-20) against human ribonucleotide reductase R2 subunit
<400> 379
   ttagaagtca gcatccaagg 20
<210> 380
   <211> 20
   <212> DNA
   <213> Artificial Sequence
<220>
   <223> Antisense oligonucleotide (AS-II-1362-20) against human ribonucleotide reductase R2 subunit
<400> 380
   gcacatcttc agttcattta 20
<210> 381
   <211> 20
   <212> DNA
   <213> Artificial Sequence
<220>
   <223> Antisense oligonucleotide (AS-II-1364-20) against human ribonucleotide reductase R2 subunit
<400> 381
   gggcacatct tcagttcatt 20
<210> 382
   <211> 20
   <212> DNA
   <213> Artificial Sequence
<220>
   <223> Antisense oligonucleotide (AS-II-1381-20) against human ribonucleotide reductase R2 subunit
<400> 382
   aaaaatcagc caagtaaggg 20
<210> 383
   <211> 20
   <212> DNA
   <213> Artificial Sequence
<220>
   <223> Antisense oligonucleotide (AS-II-1390-20) against human ribonucleotide reductase R2 subunit
<400> 383
   atggaaaaaa aaaatcagcc 20
<210> 384
   <211> 20
   <212> DNA
   <213> Artificial Sequence
<220>
   <223> Antisense oligonucleotide (AS-II-1438-20) against human ribonucleotide reductase R2 subunit
<400> 384
   ttcatggtgt ggctagttgg 20
<210> 385
   <211> 20
   <212> DNA
   <213> Artificial Sequence
<220>
   <223> Antisense oligonucleotide (AS-II-1499-20) against human ribonucleotide reductase R2 subunit
<400> 385
   aggactggtt gtgaggtagc 20
<210> 386
   <211> 20
   <212> DNA
   <213> Artificial Sequence
<220>
   <223> Antisense oligonucleotide (AS-II-1517-20) against human ribonucleotide reductase R2 subunit
<400> 386
   ccagcactat aaacagacag 20
<210> 387
   <211> 20
   <212> DNA
   <213> Artificial Sequence
<220>
   <223> Antisense oligonucleotide (AS-II-1538-20) against human ribonucleotide reductase R2 subunit
<400> 387
   ttctggcaaa aggtgatact 20
<210> 388
   <211> 20
   <212> DNA
   <213> Artificial Sequence
<220>
   <223> Antisense oligonucleotide (AS-II-1560-20) against human ribonucleotide reductase R2 subunit
<400> 388
   gtaagtcaca gccagccagg 20
<210> 389
   <211> 20
   <212> DNA
   <213> Artificial Sequence
<220>
   <223> Antisense oligonucleotide (AS-II-1581-20) against human ribonucleotide reductase R2 subunit
<400> 389
   actgccattg tcactgctat 20
<210> 390
   <211> 20
   <212> DNA
   <213> Artificial Sequence
<220>
   <223> Antisense oligonucleotide (AS-II-1659-20) against human ribonucleotide reductase R2 subunit
<400> 390
   tggctgtgct ggttaaagga 20
<210> 391
   <211> 20
   <212> DNA
   <213> Artificial Sequence
<220>
   <223> Antisense oligonucleotide (AS-II-1666-20) against human ribonucleotide reductase R2 subunit
<400> 391
   ttttaactgg ctgtgctggt 20
<210> 392
   <211> 20
   <212> DNA
   <213> Artificial Sequence
<220>
   <223> Antisense oligonucleotide (AS-II-1700-20) against human ribonucleotide reductase R2 subunit
<400> 392
   attaaaatct gcgttgaagc 20
<210> 393
   <211> 20
   <212> DNA
   <213> Artificial Sequence
<220>
   <223> Antisense oligonucleotide (AS-II-1768-20) against human ribonucleotide reductase R2 subunit
<400> 393
   tatcgccgcc gtgagtacaa 20
<210> 394
   <211> 20
   <212> DNA
   <213> Artificial Sequence
<220>
   <223> Antisense oligonucleotide (AS-11-1773-20) against human ribonucleotide reductase R2 subunit
<400> 394
   gctattatcg ccgccgtgag 20
<210> 395
   <211> 12
   <212> DNA
   <213> Artificial Sequence
<220>
   <223> Antisense oligonucleotide (AS-II-1775-12) against human ribonucleotide reductase R2 subunit
<400> 395
   atcgccgccg tg 12
<210> 396
   <211> 20
   <212> DNA
   <213> Artificial Sequence
<220>
   <223> Antisense oligonucleotide (AS-II-1790-20) against human ribonucleotide reductase R2 subunit
<400> 396
   gaaaccaaat aaatcaagct 20
<210> 397
   <211> 20
   <212> DNA
   <213> Artificial Sequence
<220>
   <223> Antisense oligonucleotide (AS-II-1819-20) against human ribonucleotide reductase R2 subunit
<400> 397
   ttagtggtca ggagaatgta 20
<210> 398
   <211> 20
   <212> DNA
   <213> Artificial Sequence
<220>
   <223> Antisense oligonucleotide (AS-II-1976-20) against human ribonucleotide reductase R2 subunit
<400> 398
   tggcaccaac tgactaatat 20
<210> 399
   <211> 20
   <212> DNA
   <213> Artificial Sequence
<220>
   <223> Antisense oligonucleotide (AS-II-1989-20) against human ribonucleotide reductase R2 subunit
<400> 399
   cctgtcttct atctggcacc 20
<210> 400
   <211> 20
   <212> DNA
   <213> Artificial Sequence
<220>
   <223> Antisense oligonucleotide (AS-II-2009-20) against human ribonucleotide reductase R2 subunit
<400> 400
   gccacaggat aaaaacacaa 20
<210> 401
   <211> 20
   <212> DNA
   <213> Artificial Sequence
<220>
   <223> Antisense oligonucleotide (AS-II-2026-20) against human ribonucleotide reductase R2 subunit
<400> 401
   cccaggacac tacacaagcc 20
<210> 402
   <211> 20
   <212> DNA
   <213> Artificial Sequence
<220>
   <223> Antisense oligonucleotide (AS-II-2044-20) against human ribonucleotide reductase R2 subunit
<400> 402
   tcagaggggg cagagaatcc 20
<210> 403
   <211> 20
   <212> DNA
   <213> Artificial Sequence
<220>
   <223> Antisense oligonucleotide (AS-II-2067-20) against human ribonucleotide reductase R2 subunit
<400> 403
   tcctttatcc cacaacactc 20
<210> 404
   <211> 20
   <212> DNA
   <213> Artificial Sequence
<220>
   <223> Antisense oligonucleotide (AS-II-2083-20) against human ribonucleotide reductase R2 subunit
<400> 404
   ccttgccctg agagattcct 20
<210> 405
   <211> 20
   <212> DNA
   <213> Artificial Sequence
<220>
   <223> Partially phosphorothioated antisense oligonucleotide (AS-II-2083-20) against human ribonucleotide reductase R2 subunit
   <221> misc_difference
   <222> (1) ... (2)
   <223> Phosphorothioate internucleotide linkage
   <221> misc_difference
   <222> (3)...(4)
   <223> Phosphorothioate internucleotide linkage
   <221> misc_difference
   <222> (5)...(6)
   <223> Phosphorothioate internucleotide linkage
   <221> misc_difference
   <222> (7)...(8)
   <223> Phosphorothioate internucleotide linkage
   <221> misc_difference
   <222> (9)...(10)
   <223> Phosphorothioate internucleotide linkage
   <221> misc_difference
   <222> (11)...(12)
   <223> Phosphorothioate internucleotide linkage
   <221> misc_difference
   <222> (13)...(14)
   <223> Phosphorothioate internucleotide linkage
   <221> misc_difference
   <222> (15)... (16)
   <223> Phosphorothioate internucleotide linkage
   <221> misc_difference
   <222> (17)...(18)
   <223> Phosphorothioate internucleotide linkage
   <221> misc_difference
   <222> (19)...(20)
   <223> Phosphorothioate internucleotide linkage
<400> 405
   ccttgccctg agagattcct 20
<210> 406
   <211> 20
   <212> DNA
   <213> Artificial Sequence
<220>
   <223> Antisense oligonucleotide (AS-II-2128-20) against human ribonucleotide reductase R2 subunit
<400> 406
   ggcccagatc acccctaaat 20
<210> 407
   <211> 20
   <212> DNA
   <213> Artificial Sequence
<220>
   <223> Antisense oligonucleotide (AS-II-2151-20) against human ribonucleotide reductase R2 subunit
<400> 407
   aaacggcttc tcacacatat 20
<210> 408
   <211> 20
   <212> DNA
   <213> Artificial Sequence

<220>
   <223> Antisense oligonucleotide (AS-II-2164-20) against human ribonucleotide reductase R2 subunit
<400> 408
   gagaaataaa atgaaacggc 20
<210> 409
   <211> 20
   <212> DNA
   <213> qArtificial Sequence
<220>
   <223> Antisense oligonucleotide (AS-II-2182-20) against human ribonucleotide reductase R2 subunit
<400> 409
   cgttgaggaa aatacagtga 20
<210> 410
   <211> 20
   <212> DNA
   <213> Artificial Sequence
<220>
   <223> Antisense oligonucleotide (AS-II-2229A-20) against human ribonucleotide reductase R2 subunit
<400> 410
   gctcccacat atgaaaactc 20
<210> 411
   <211> 20
   <212> DNA
   <213> Artificial Sequence
<220>
   <223> Antisense oligonucleotide (AS-II-2372-20) against human ribonucleotide reductase R2 subunit
<400> 411
   cacacaacct acttacacca 20
<210> 412
   <211> 22
   <212> DNA
   <213> Artificial Sequence
<220>
   <223> siRNA target sequence in human ribonucleotide reductase R2 subunit mRNA
<400> 412
   aacttggtgg agcgatttag cc 22
<210> 413
   <211> 21
   <212> DNA
   <213> Artificial Sequence
<220>
   <223> Scrambled control oligonucleotide
<400> 413
   aactaggtac cacacgagag c 21
<210> 414
   <211> 21
   <212> DNA
   <213> Artificial Sequence
<220>
   <223> Scrambled control oligonucleotide
<400> 414
   aatgctagtg tcgtagtcag c 21
<210> 415
   <211> 22
   <212> DNA
   <213> Artificial Sequence
<220>
   <223> Scrambled control oligonucleotide
<400> 415
   aatgctagtg tcgtagtgag cc 22
<210> 416
   <211> 21
   <212> DNA
   <213> Artificial Sequence
<220>
   <223> Scrambled control oligonucleotide
<400> 416
   aatcgagtac tgagactact g 21
<210> 417
   <211> 21
   <212> DNA
   <213> Artificial Sequence
<220>
   <223> Scrambled control oligonucleotide
<400> 417
   aatgcaggac tgagactact g 21
<210> 418
   <211> 21
   <212> DNA
   <213> Artificial Sequence
<220>
   <223> Scrambled control oligonucleotide
<400> 418
   aactagcgta cagatgagag c 21
<210> 419
   <211> 21
   <212> DNA
   <213> Artificial Sequence
<220>
   <223> Scrambled control oligonucleotide
<400> 419
   aactagggta gacgatgaga g 21
<210> 420
   <211> 3117
   <212> RNA
   <213> Homo sapiens
<220>
   <221> mRNA
   <222> (1)...(3117)
   <223> mRNA sequence for the human ribonucleotide reductase R1 subunit
<400> 420
   attcgaaccc cgtcgcgccc ctttgtgcgt cacgggtggc gggcgcggga aggggatttg 60
   gattgttgcg cctctgctct gaagaaagtg ctgtctggct ccaactccag ttctttcccc 120
   tgagcagcgc ctggaaccta acccttccca ctctgtcacc ttctcgatcc cgccggcgct 180
   ttagagccgc agtccagtct tggatccttc agagcctcag ccactagctg cgatgcatgt 240
   gatcaagcga gatggccgcc aagaacgagt catgtttgac aaaattacat ctcgaatcca 300
   gaagctttgt tatggactca atatggattt tgttgatcct gctcagatca ccatgaaagt 360
   aatccaaggc ttgtacagtg gggtcaccac agtggaacta gatactttgg ctgctgaaac 420
   agctgcaacc ttgactacta agcaccctga ctatgctatc ctggcagcca ggatcgctgt 480
   ctctaacttg cacaaagaaa caaagaaagt gttcagtgat gtgatggaag acctctataa 540
   ctacataaat ccacataatg gcaaacactc tcccatggtg gccaagtcaa cattggatat 600
   tgttctggcc aataaagatc gcctgaattc tgctattatc tatgaccgag atttctctta 660
   caattacttc ggctttaaga cgctagagcg gtcttatttg ttgaagatca atggaaaagt 720
   ggctgaaaga ccacaacata tgttgatgag agtatctgtt gggatccaca aagaagacat 780
   tgatgcagca attgaaacat ataatcttct ttctgagagg tggtttactc atgcttcgcc 840
   cactctcttc aatgctggta ccaaccgccc acaactttct agctgttttc ttctgagtat 900
   gaaagatgac agcattgaag gcatttatga cactctaaag caatgtgcat tgatttctaa 960
   gtctgctgga ggaattggtg ttgctgtgag ttgtattcgg gctactggca gctacattgc 1020
   tgggactaat ggcaattcca atggccttgt accgatgctg agagtatata acaacacagc 1080
   tagatatgtg gatcaaggtg ggaacaagcg tcctggggca tttgctattt acctggagcc 1140
   ttggcattta gacatctttg aattccttga tttaaagaag aacacaggaa aggaagagca 1200
   gcgtgccaga gatcttttct ttgctctttg gattccggat ctcttcatga aacgagtgga 1260
   gactaatcag gactggtctt tgatgtgtcc aaatgagtgt cctggtctgg atgaggtttg 1320
   gggagaggaa tttgagaaac tatatgcaag ttatgagaaa caaggtcgtg tccgcaaagt 1380
   tgtaaaagct cagcagcttt ggtatgccat cattgagtct cagacggaaa caggcacccc 1440
   gtatatgctc tacaaagatt cctgtaatcg aaagagcaac cagcagaacc tgggaaccat 1500
   caaatgcagc aacctgtgca cagaaatagt ggagtacacc agcaaagatg aggttgctgt 1560
   ttgtaatttg gcttccctgg ccctgaatat gtatgtcaca tcagaacaca catacgactt 1620
   taagaagttg gctgaagtca ctaaagtcgt tgtccgaaac ttgaataaaa ttattgatat 1680
   aaactactat cctgtaccag aggcatgcct atcaaataaa cgccatcgcc ccattggaat 1740
   tggggtacaa ggtctggcag atgcttttat cctgatgaga tacccttttg agagtgcaga 1800
   agcccagtta ctgaataagc agatctttga aactatttat tatggtgctc tggaagccag 1860
   ctgtgacctt gccaaggagc agggcccata cgaaacctat gagggctctc cagttagcaa 1920
   aggaattctt cagtatgata tgtggaatgt tactcctaca gacctatggg actggaaggt 1980
   tctcaaggag aagattgcaa agtatggtat aagaaacagt ttacttattg ccccgatgcc 2040
   tacagcttcc actgctcaga tcctggggaa taatgagtcc attgaacctt acaccagcaa 2100
   catctatact cgcagagtct tgtcaggaga atttcagatt gtaaatcctc acttattgaa 2160
   agatcttacc gagcggggcc tatggcatga agagatgaaa aaccagatta ttgcatgcaa 2220
   tggctctatt cagagcatac cagaaattcc tgatgacctg aagcaacttt ataaaactgt 2280
   gtgggaaatc tctcagaaaa ctgttctcaa gatggcagct gagagaggtg ctttcattga 2340
   tcaaagccaa tctttgaaca tccacattgc tgagcctaac tatggcaaac tcactagtat 2400
   gcacttctac ggctggaagc agggtttgaa gactgggatg tattatttaa ggacaagacc 2460
   agcagctaat ccaatccagt tcactctaaa taaggagaag ctaaaagata aagaaaaggt 2520
   atcaaaagag gaagaagaga aggagaggaa cacagcagcc atggtgtgct ctttggagaa 2580
   tagagatgaa tgtctgatgt gtggatcctg aggaaagact tggaagagac cagcatgtct 2640
   tcagtagcca aactacttct tgagcataga taggtatagt gggtttgctt gaggtggtaa 2700
   ggctttgctg gaccctgttg caggcaaaag gagtaattga tttaaagtac tgttaatgat 2760
   gttaatgatt tttttttaaa ctcatatatt gggattttca ccaaaataat gcttttgaaa 2820
   aaaagaaaaa aaaaacggat atattgagaa tcaaagtaga agttttagga atgcaaaata 2880
   agtcatcttg catacaggga gtggttaagt aaggtttcat cacccattta gcatgctttt 2940
   ctgaagactt cagttttgtt aaggagattt agttttactg ctttgactgg tgggtctcta 3000
   gaagcaaaac tgagtgataa ctcatgagaa gtactgatag gacctttatc tggatatggt 3060
   cctataggtt attctgaaat aaagataaac atttctaagt gaaaaaaaaa aaaaaaa    3117
<210> 421
   <211> 2500
   <212> RNA
   <213> Homo sapiens
<220>
   <221> mRNA
   <222> (1)...(2500)
   <223> mRNA sequence for the human ribonucleotide reductase R2 subunit
<400> 421
   cccaggcgca gccaatggga agggtcggag gcatggcaca gccaatggga agggccgggg 60
   caccaaagcc aatgggaagg gccgggagcg cgcggcgcgg gagatttaaa ggctgctgga 120
   gtgaggggtc gcccgtgcac cctgtcccag ccgtcctgtc ctggctgctc gctctgcttc 180
   gctgcgcctc cactatgctc tccctccgtg tcccgctcgc gcccatcacg gacccgcagc 240
   agctgcagct ctcgccgctg aaggggctca gcttggtcga caaggagaac acgccgccgg 300
   ccctgagcgg gacccgcgtc ctggccagca agaccgcgag gaggatcttc caggagccca 360
   cggagccgaa aactaaagca gctgcccccg gcgtggagga tgagccgctg ctgagagaaa 420
   acccccgccg ctttgtcatc ttccccatcg agtaccatga tatctggcag atgtataaga 480
   aggcagaggc ttccttttgg accgccgagg aggttgacct ctccaaggac attcagcact 540
   gggaatccct gaaacccgag gagagatatt ttatatccca tgttctggct ttctttgcag 600
   caagcgatgg catagtaaat gaaaacttgg tggagcgatt tagccaagaa gttcagatta 660
   cagaagcccg ctgtttctat ggcttccaaa ttgccatgga aaacatacat tctgaaatgt 720
   atagtcttct tattgacact tacataaaag atcccaaaga aagggaattt ctcttcaatg 780
   ccattgaaac gatgccttgt gtcaagaaga aggcagactg ggccttgcgc tggattgggg 840
   acaaagaggc tacctatggt gaacgtgttg tagcctttgc tgcagtggaa ggcattttct 900
   tttccggttc ttttgcgtcg atattctggc tcaagaaacg aggactgatg cctggcctca 960
   cattttctaa tgaacttatt agcagagatg agggtttaca ctgtgatttt gcttgcctga 1020
   tgttcaaaca cctggtacac aaaccatcgg aggagagagt aagagaaata attatcaatg 1080
   ctgttcggat agaacaggag ttcctcactg aggccttgcc tgtgaagctc attgggatga 1140
   attgcactct aatgaagcaa tacattgagt ttgtggcaga cagacttatg ctggaactgg 1200
   gttttagcaa ggttttcaga gtagagaacc catttgactt tatggagaat atttcactgg 1260
   aaggaaagac taacttcttt gagaagagag taggcgagta tcagaggatg ggagtgatgt 1320
   caagtccaac agagaattct tttaccttgg atgctgactt ctaaatgaac tgaagatgtg 1380
   cccttacttg gctgattttt tttttccatc tcataagaaa aatcagctga agtgttacca 1440
   actagccaca ccatgaattg tccgtaatgt tcattaacag catctttaaa actgtgtagc 1500
   tacctcacaa ccagtcctgt ctgtttatag tgctggtagt atcacctttt gccagaaggc 1560
   ctggctggct gtgacttacc atagcagtga caatggcagt cttggcttta aagtgagggg 1620
   tgacccttta gtgagcttag cacagcggga ttaaacagtc ctttaaccag cacagccagt 1680
   taaaagatgc agcctcactg cttcaacgca gattttaatg tttacttaaa tataaacctg 1740
   gcactttaca aacaaataaa cattgttttg tactcacggc ggcgataata gcttgattta 1800
   tttggtttct acaccaaata cattctcctg accactaatg ggagccaatt cacaattcac 1860
   taagtgacta aagtaagtta aacttgtgta gactaagcat gtaattttta agttttattt 1920
   taatgaatta aaatatttgt taaccaactt taaagtcagt cctgtgtata cctagatatt 1980
   agtcagttgg tgccagatag aagacaggtt gtgtttttat cctgtggctt gtgtagtgtc 2040
   ctgggattct ctgccccctc tgagtagagt gttgtgggat aaaggaatct ctcagggcaa 2100
   ggagcttctt aagttaaatc actagaaatt taggggtgat ctgggccttc atatgtgtga 2160
   gaagccgttt cattttattt ctcactgtat tttcctcaac gtctggttga tgagaaaaaa 2220
   ttcttgaaga gttttcatat gtgggagcta aggtagtatt gtaaaatttc aagtcatcct 2280
   taaacaaaat gatccaccta agatcttgcc cctgttaagt ggtgaaatca actagaggtg 2340
   gttcctacaa gttgttcatt ctagttttgt ttggtgtaag taggttgtgt gagttaattc 2400
   atttatattt actatgtctg ttaaatcaga aattttttat tatctatgtt cttctagatt 2460
   ttacctgtag ttcataaaaa aaaaaaaaaa aaaaaaaaaa                      2500
<210> 422
   <211> 21
   <212> RNA
   <213> Artificial Sequence
<220>
   <223> Sense strand of siRNA oligonucleotide 544 against human ribonucleotide reductase R2 subunit mRNA
   <221> misc_feature
   <222> (20)...(21)
   <223> n at positions 20 and 21 are DNA (thymidine)
<400> 422
   ucccugaaac ccgaggagan n 21
<210> 423
   <211> 21
   <212> RNA
   <213> Artificial Sequence
<220>
   <223> Antisense strand of siRNA oligonucleotide 544 against human ribonucleotide reductase R2 subunit mRNA
   <221> misc_feature
   <222> (20)...(21)
   <223> n at positions 20 and 21 are DNA (thymidine)
<400> 423
   ucuccucggg uuucagggan n 21
<210> 424
   <211> 21
   <212> RNA
   <213> Artificial Sequence
<220>
   <223> Sense strand of siRNA oligonucleotide 624 against human ribonucleotide reductase R2 subunit mRNA
   <221> misc_feature
   <222> (20)...(21)
   <223> n at positions 20 and 21 are DNA (thymidine)
<400> 424
   cuugguggag cgauuuagcn n 21
<210> 425
   <211> 21
   <212> RNA
   <213> Artificial Sequence
<220>
   <223> Antisense strand of siRNA oligonucleotide 624 against human ribonucleotide reductase R2 subunit mRNA
   <221> misc_feature
   <222> (20)...(21)
   <223> n at positions 20 and 21 are DNA (thymidine)
<400> 425
   gcuaaaucgc uccaccaagn n 21
<210> 426
   <211> 22
   <212> RNA
   <213> Artificial Sequence
<220>
   <223> Sense strand of siRNA oligonucleotide 624+1 against human ribonucleotide reductase R2 subunit mRNA
   <221> misc_feature
   <222> (20) ... (21)
   <223> n at positions 21 and 22 are DNA (thymidine)
<400> 426
   cuugguggag cgauuuagcc nn 22
<210> 427
   <211> 22
   <212> DNA
   <213> Artificial Sequence
<220>
   <223> Antisense strand of siRNA oligonucleotide 624+1 against human ribonucleotide reductase R2 subunit mRNA
   <221> misc_feature
   <222> (20)...(21)
   <223> n at positions 21 and 22 are DNA (thymidine)
<400> 427
   ggcuaaaucg cuccaccaag nn 22
<210> 428
   <211> 21
   <212> RNA
   <213> Artificial Sequence
<220>
   <223> Sense strand of siRNA oligonucleotide 787 against human ribonucleotide reductase R2 subunit mRNA
<221> misc_feature
   <222> (20)...(21)
   <223> n at positions 20 and 21 are DNA (thymidine)
<400> 428
   acgaugccuu gugucaagan n 21
<210> 429
   <211> 21
   <212> RNA
   <213> Artificial Sequence
<220>
   <223> Antisense strand of siRNA oligonucleotide 787 against human ribonucleotide reductase R2 subunit mRNA
   <221> misc_feature
   <222> (20)...(21)
   <223> n at positions 20 and 21 are DNA (thymidine)
<400> 429
   ucuugacaca aggcaucgun n 21
<210> 430
   <211> 21
   <212> RNA
   <213> Artificial Sequence
<220>
   <223> Sense strand of siRNA oligonucleotide 844 against human ribonucleotide reductase R2 subunit mRNA
   <221> misc_feature
   <222> (20)... (1)
   <223> n at positions 20 and 21 are DNA (thymidine)
<400> 430
   gaggcuaccu auggugaacn n 21
<210> 431
   <211> 21
   <212> RNA
   <213> Artificial Sequence
<220>
   <223> Antisense strand of siRNA oligonucleotide 844 against human ribonucleotide reductase R2 subunit mRNA
   <221> misc_feature
   <222> (20)...(21)
   <223> n at positions 20 and 21 are DNA (thymidine)
<400> 431
   guucaccaua gguagccucn n 21
<210> 432
   <211> 21
   <212> RNA
   <213> Artificial Sequence
<220>
   <223> Sense strand of siRNA oligonucleotide 933 against human ribonucleotide reductase R2 subunit mRNA
   <221> misc_feature
   <222> (20)...(21)
   <223> n at positions 20 and 21 are DNA (thymidine)
<400> 432
   gaaacgagga cugaugccun n 21
<210> 433
   <211> 21
   <212> RNA
   <213> Artificial Sequence
<220>
   <223> Antisense strand of siRNA oligonucleotide 933 against human ribonucleotide reductase R2 subunit mRNA
   <221> misc_feature
   <222> (20)...(21)
   <223> n at positions 20 and 21 are DNA (thymidine)
<400> 433
   aggcaucagu ccucguuucn n 21
<210> 434
   <211> 21
   <212> DNA
   <213> Artificial Sequence
<220>
   <223> Sense strand of siRNA oligonucleotide 1284 against human ribonucleotide reductase R2 subunit mRNA
   <221> misc_feature
   <222> (20)...(21)
   <223> n at positions 20 and 21 are DNA (thymidine)
<400> 434
   gagaguaggc gaguaucagn n 21

<210> 435
   <211> 21
   <212> RNA
   <213> Artificial Sequence
<220>
   <223> Antisense strand of siRNA oligonucleotide 1284 against human ribonucleotide reductase R2 subunit mRNA
   <221> misc_feature
   <222> (20)...(21)
   <223> n at positions 20 and 21 are DNA (thymidine)
<400> 435
   cugauacucg ccuacucucn n 21
<210> 436
   <211> 10304
   <212> DNA
   <213> Homo sapiens
<220>
   <221>
   <222>
   <223> Genomic sequence for Homo sapiens ribonucleotide reductase M2 subunit (RRM2) gene including complete coding sequence
<400> 436
   ccccgagccg cggtttctcc accctaatgg tgaacagcct tttggaagtc gcgctaacct 60
   tggcctgaga cctgcaaact tgcccaggct ggggcgtgtg aaccggcgag cgcgcagcgg 120
   aaacggggcg gggcacctga ggctgggaat gcagaggagc cttccggggg gcggggcggg 180
   gcctcccgtg cataccaatg gtggggtaga ttcaaatgtc aattcgcgcg ctcaagtggc 240
   ttccgccagg aatcccgacc cttatggaag cggaaggaag atcgcttgat accaacctgg 300
   gctagctagc gagacctcgt ctgtttactt aaataaaacc aaaaaaacga gcaccgaggg 360
   aaaaaggagt gaatcccggg gctagcagca gcctgcggcg ggcgctctcc cgggagtggc 420
   tgcaccgccc gacctccccg gaggcggaac cgcccgcatt gccgcgtggc cctgggcgcc 480
   gccacctcct ccgcagcggg gcaaagttgc cggacctggg ggcaggaggg ccacgccgag 540
   atgactcagg tttagcgcgg gaggggagga tggcgacttc acccggcctt taacaacacg 600
   tacgcatctt tcggcgtctt ctacaatggc tatgttaatt acgtggccag gaactaaact 660
   atcaatgaag ccacctctga ctacttcagt tacagtgagt ttaacaggag caaaaaagca 720
   cgtggcgccc tagggcaacc gaaacgaggg ttttagacgc tgattatggg aaattgaaat 780
   ctgagttgag tatgagatga caccaataaa ttataatttt gttagataat agctttatca 840
   gccataaagt aatcaataaa aataccagtt tcctggagat ggatgcttta gtgtgtttgg 900
   ggtgaaaatg gcgatgaatg gcgagttgct ttaaacaaat catggtacac caaagtttta 960
   gttgtggctt tgtgtaagga atgtgatggg cacttattcc tgcaacacga gaatactatg 1020
   atttacaagt ccgtagtact tttaagaaat gagagaaaca gacctaggtg gggagggtac 1080
   ctgtcccacc ccaccctctt taaagtatct tatctagaaa aggctttgtg aaaaaaaaag 1140
   tcccgggtct ctctcaataa cagccctgag cgcagctgtt gaagctttct caggttaatg 1200
   atttctttct tggatcttaa agtttctttc tcttccttta tttttggcat tttgcccgtt 1260
   gcagggcctg gcaaatcaga aagccacata gaaaattaaa tgaaagctat tgctaagttc 1320
   cagtctctac accagtggag ttttcaaact cctcttcagc atatttgacg cccaatgagt 1380
   agtacattaa ttcctagtcc taaaatcatt ctgtgaactt tctcccagga atttttgctc 1440
   agtttgcaat taaaacaact ttttttcttc tctttttaat ggcagaggcg gggtctcact 1500
   atgttgccca ggatggtctc cagctcctgg cctcaaacaa tcctcctgcc ttggcctccc 1560
   aaagtgctgg gattacaggc gtgagccacc gcgcccagcc acagtttgca attcttaagg 1620
   caagggtgac aataggggtc aggggtctga caggagacag gatttctgtg gaaaactgca 1680
   ccaagggcct tctcgccatg tcccgtagtt tgaaggttta caaaggactg cacattttac 1740
   atgagtcatc tcaacgaacg ctctcctcac cgcattaaca gtccacgcgg ttacgagtcc 1800
   cattttactc acggggacac cgaatctgta agaagcctgg tcgcttgtcc cagcaaaacg 1860
   agccacgggg ctcagcggcc ctaactttta ggctgtaggg tcctcgccga ccaccccgcc 1920
   aaaatgtcag gcctcggggc ccttgcaccc ccaccgcagg gacacggatc gaaagggtcg 1980
   cagcaacgcc tcccccgcac ccaggagcgt tttccaggcc tttgcaccaa cctcgttggc 2040
   taagccccct gcccggcggc ggcccggctg ggaggaggtg ctttcgggag gcggggccgc 2100
   ggcccgggga tcctctcgcg cccgcgggct ccaatcgctg ctcctcacgc aatcctaaac 2160
   ggttcccggg cgaaccgggg cccgcgcgcg ccaaggccgc cgagaccctc aggggctgcg 2220
   gccctggtcc cgcgggacct gtgggggcct gggcggcggc gcccccgacc cagccagcgg 2280
   acgggccggg gggggaaccg ggaggtcccg gggggcgtcc acgggggtgt ccccgggggt 2340
   ctccggaagg cgccggcgga ggctcccgcg ctgcgcttga aaatcgcgcg cggccccgcg 2400
   gccagcctgg gtaggggcaa ggcgcagcca atgggaaggg tcggaggcat ggcacagcca 2460
   atgggaaggg ccggggcacc aaagccaatg ggaagggccg ggagcgcgcg gcgcgggaga 2520
   tttaaaggct gctggagtga ggggtcgccc gtgcaccctg tcccagccgt cctgtcctgg 2580
   ctgctcgctc tgcttcgctg cgccgccact atgctctccc tccgtgtccc gctcgcgccc 2640
   atcacggacc cgcagcagct gcagctctcg ccgctgaagg ggctcagctt ggtcgacaag 2700
   gagaacacgg tgagcccgcg gggagggcgc tgcgggcagg ggagggaggc agggaaagcg 2760
   aagccgctcc tcactcacac gcgtctcccc gcagccgccg gccctgagcg ggacccgcgt 2820
   cctggccagc aagaccgcga ggaggatctt ccaggagccc acggagccgg tgagtggcgg 2880
   gcgtggggca gaggggccag ggacggcctt gggcgtcttg gcgccaaagc cgcattgttt 2940
   cctcagctgt tcacactccc gccccggctc ctttcccgcc taggcggccc ctccccaggg 3000
   ctgcctcccg cgcccctcgg cccatttccc ggttcgggcg tgcgctcctc tgctgcgacc 3060
   cacggagtgc gacgggacag ccacgttttc acatcgggcc ccgtgaaatt gccgccaatg 3120
   gaaaggactt ggtccagaaa aacgttagtt tcatatggtt cgcccggtac ttaaatgttt 3180
   tattttctcc cccaacagaa aactaaagca gctgcccccg gcgtggagga tgagccgctg 3240
   ctgagagaaa acccccgccg ctttgtcatc ttccccatcg agtaccatga tatctggcag 3300
   atgtataaga aggcagaggc ttccttttgg accgccgagg aggtaatcgg aggaccccag 3360
   aagacccctg caggggtgac cgtcacgcct cagacataaa tgcacttgga ggttcccgtt 3420
   ggcaaggggg gctaactgtg gggcatagta agtggtgcca gcatacttaa agtttgagtg 3480
   ctcagtgtga gtcctgtagg ctttactctc ttccttttat gctaaaattg tgacttccga 3540
   acctcaggtg acctctccaa ggacattcag cactgggaat ccctgaaacc cgaggagaga 3600
   tattttatat cccatgttct ggctttcttt gcagcaagcg atggcatagt aaatgaaaac 3660
   ttggtgagtt tccaaaacat ctttcattca tttgacgttg acgatctgag gtcgaactag 3720
   ttcgctttcc tcgtcttgta cgtttttcca tgctgagtgc atctgtgtgt gtaagctggg 3780
   ttttatatta catggcattt cctgttttgt aacactttgc agttctttct tatggtattt 3840
   tcccgactct agagaagctg agacaatatt aagtggtagc aatgtgatga ctctttgtgg 3900
   ccaccacatc tgccccctct tttttttttt tttttgagac agagtctcac tctggcccag 3960
   gctggagtgc agtggcgtga tcttggctca ctgcaacctc cgcctcctgg gttcaagcga 4020
   ttccccaacc tcagcctcat gagtacctgg gattacagac gtgcgccacc atgcctagct 4080
   aatatctgta tttttagtag agacagggtt ttaccatgtt ggccaggctg gtctcgaact 4140
   gctgacctca ggtgatccac ccaccttggc ctcccaaagt gttgggatta caggcgtgag 4200
   ccaccacgcc cggctctgct ccctcctttt tgtggctttg ctgttttaat aataatttgg 4260
   ttgtatctct tattgcgaat ggatctttct tgacataaat taattaggaa atcgagcgct 4320
   cacaaatcct attttatatg tatctatttc ctgatatgta agttgagcat atgacataaa 4380
   atatcaaaga actgtgacaa attggatgaa atatatatag aaataaacct tataatggta 4440
   caaagagtgc gatgctgcca gtatccgttg acagttgctg ctgttggttt tttctcaagc 4500
   ttaactttga tgtgttttgc cactaggtgg agcgatttag ccaagaagtt cagattacag 4560
   aagcccgctg tttctatggc ttccaaattg ccatggaaaa catacattct gaaatgtata 4620
   gtcttcttat tgacacttac ataaaagatc ccaaagaaag gtgagtattc aagtggtatg 4680
   ccaagatttt taggactcac taattgttga tttattacac atttttagtt cacctaggga 4740
   taaaaatgac tccagaatga ctaagacagt cataggcatt cccagcaccc gtggtcatgt 4800
   ctgctcttag caaggggcct aaatgcactt tattattcac ttagagttgt gaaggtactc 4860
   cttttaaagt tggatgtcta ccaatgtaaa accttctttt gaaaaaattc ctagatgttg 4920
   ggtaagacaa actaaaacct atgtctgacc atctttgctc atttggtaaa gttgttgaga 4980
   agctagaatg tggggctgca gtgggatgga cggggaagga cttgcctcct aagaagcctg 5040
   cagtatagta taggcaaata agacttagta ggagttacat aaggcagagg cagcagtgaa 5100
   ccctgagact gatttaggca tgcaggagtt tggctgaata aaggtagctt aaggtctgtt 5160
   ttgttttgga gattggaggt ggggggatta gaaatgggct gctggagtag tctagataca 5220
   aaggtcagct ttagggtggc gcgcggtggt tctcgcctgt aatcccagca ctttgggagg 5280
   ctgaagcggg cggacaatga ggtcaggaga tcgagaccat cctggctaac acggtgaaac 5340
   cccgtctcta ctaaaaataa aaaaagtggt ggcgggcgcc tgtagtccca gctgcttggg 5400
   aggctgagac aggagaatgg cgtgaacccg ggaggcggag cttgcgctcc agcctgggtg 5460
   acagagcaag actccgtctc aaaaagcaaa acaaaacaaa aaaaacaaag gtcagctttg 5520
   gggaccagaa ccttgtatgg agtggaagtg gtgaagctgc aacctaaagt agccgttgta 5580
   gactttgaag tacatgaaga ggaaaagtgg taacttgaaa ggactgagga aacattggga 5640
   gtaaagagat ttgaacatgt ttataggtgg aaattgagaa aagaaggcaa agattagggg 5700
   tacgatcggg ggcaaatgcc cagaagggga acaggaaggt ctgctgggga agcctcaaaa 5760
   acaagggaga ggcagaccca ggtctcagag agagggacag tgagatggaa agaatgaacg 5820
   acagctgggc atggtagtct gagctagtag tcccagctac ttggcaggct gaggcagaag 5880
   gatggcttga gccctggagt ttggttttac cgtgagctgt gatcatctcg ctgcactcta 5940
   gcctgggcaa cagagtgaga ccctcatctc tttaaaaaaa aaaaaaaaaa aaaaaaaaaa 6000
   gggtctggtg cccttggctt cagaacacaa agtcatctgg gtaggaacag tctgggaaat 6060
   gagtagcctc tcaaggtggg caccagaata aagggaggca gaggagggtg gtaagggaga 6120
   tccagttaac tgtagtaccc atggatttgc tttcctgacc tgggatcgac agtgtagcac 6180
   agagtcacta gtaggaagca atcttagttt attggtttaa ttattttatg atatagatgt 6240
   ggcaactgag gccaaataat gcacctaatc atagtctgat aatagcacag cagttaggat 6300
   tttatggttc ttcaaattta aattctatga ttcttcaaat tgaacaatga tctggacttg 6360
   aaataatttt aaaggcaaca aatgtccctg ctgtactgga ctatgtttta ctgtctgtag 6420
   accctgaagc tcaatatgaa ctacagaata cccaaacttg tattaatgta aatcaagtgt 6480
   tgaggttttt aaaagaacac tggagggaaa aactgaccag taaaaataaa acatttcggt 6540
   gtgagttctt cctttaggaa gaggattggc aaatacttga atttggcctt tgtcccactt 6600
   atctagcagt tggtaatcgg aggtctttta ctgtaatgct tcaattgctg ataccgtatg 6660
   tgcctactag ggaatttctc ttcaatgcca ttgaaacgat gccttgtgtc aagaagaagg 6720
   cagactgggc cttgcgctgg attggggaca aagaggctac ctatggtaag gagacccttg 6780
   cccctactta aacctgagct tcatttccca agtaatgtta ctggattttt ggcccttgaa 6840
   taccaactca ctagaatcat gttggtgttt aactcctaaa taggtgaacg tgttgtagcc 6900
   tttgctgcag tggaaggcat tttcttttcc ggttcttttg cgtcgatatt ctggctcaag 6960
   aaacgaggac tgatgcctgg cctcacattt tctaatgaac ttattagcag agatgaggtg 7020
   agtctaagtc aaataatagg gtgacctaaa ccccaaacac aactcgggca tgctcttgtg 7080
   ttcactgacg gggacctgag atgctagatg gcatatatcc acatttaatg tgtgagttca 7140
   accatacaca tacttgacaa aagaaggaaa tactttcatt tactgaaact gttttacttg 7200
   cattctcaat atattgtaat acatttgtac atatgtattc ccctataggc tttgaatgca 7260
   taaaactaca agttctttgt tttttgaggt gacggaatct tgctctgtcg ttccaggctg 7320
   gagtgcaatg gcgtgatctt ggctcactgc aacctctgcc tcctaggttc aagtgattct 7380
   cccgcctcag cctcccaagt agctgggatt gtaggtgcct gccaccatgc ccagctaatt 7440
   ttcgtctttt tatacagacg gggtttcacc atgtttgcca gactggggtt gaactcctga 7500
   cctcaggtga tcaggtgatc cacccgcctc ggcctcccag agtgctggga ttataggcat 7560
   gagccaccat gcccagccaa aactacaagt tattgatggg attgggattt taagggatgt 7620
   tttattattt ttgcctggta ttaatatgtt atcccttttt ccgtaaaaat gttcatagta 7680
   gagccaggag cggtggctca tgcctgtaat cccagcactt tggtaggccg aggcgggtgg 7740
   atcatgaggt caggagattg agaccatcct ggctaacacg gtgaaacccc atctctacta 7800
   aaattacaac aaaattagcc gggtatggtg gacgtgccta ttgtcccagc tacttgagag 7860
   gctgacgtag gagaatcgcc tgaaacctgg aaagtggaag gtttgcagtg gagtccgaga 7920
   tcacaacaca ctgcactcca gcctgggtga cagtcccccc gaaaaagaat gttcatagta 7980
   gccattatgt ttctcctgtt tgatctagaa attgcccctc tacttcaata ttaataagca 8040
   tttcaatgaa atgagtatac attttggtct agtgtatgtc tttgattaag tcacatttga 8100
   aaagccagga gcatgaactc catcttactt ggagcccagt gggcaaatca aatatggtta 8160
   ccttgtagga gggcttcctt actggattgg gagataagct gtgaagcttg atgtttaatg 8220
   cagtaacttg caaacttgat ttacttgaaa ttgcatacaa tttcctgagc atctaaaaac 8280
   tagcttatta ctgagctttg ccttttcctg ctgggagtag tggcaaaatt agcactcatg 8340
   gctgtagaaa gatcactgag tgaagctctg actcctcctt tgccaaacac acagcagagc 8400
   aagaaataca ccttgcctgt cttcatctag gtggcaactt tgagggtctt gaatgggact 8460
   gagcttgcct tggtggtgac atcagcagag aagtcagtag ttgaagtcat cttccctttg 8520
   agagttcaag tgctctcagt atggctgagc atgttggata aggagaatgc agaaaaggac 8580
   aaagtaattt catattacca tgttaatgac agaagtcttc tggctttagt gatcttgaac 8640
   tttttttctt agggtttaca ctgtgatttt gcttgcctga tgttcaaaca cctggtacac 8700
   aaaccatcgg aggagagagt aagagaaata attatcaatg ctgttcggat agaacaggta 8760
   aagtgggtga tgaaatgggt cactcaagct tgctagaaaa tgcctgtgct ttagttgtat 8820
   tcagaagctg tattttggtt cctaggagtt cctcactgag gccttgcctg tgaagctcat 8880
   tgggatgaat tgcactctaa tgaagcaata cattgagttt gtggcagaca gacttatgct 8940
   ggaactgggt tttagcaagg taaagtattg tttacatagc cttttgcttg ttttgaagct 9000
   ggtgctctgt atttatatct tgatgtgaac ccttttcagg ttttcagagt agagaaccca 9060
   tttgacttta tggagaatat ttcactggaa ggaaagacta acttctttga gaagagagta 9120
   ggcgagtatc agaggatggg agtgatgtca agtccaacag agaattcttt taccttggat 9180
   gctgacttct aaatgaactg aagatgtgcc cttacttggc tgattttttt tttccatctc 9240
   ataagaaaaa tcagctgaag tgttaccaac tagccacacc atgaattgtc cgtaatgttc 9300
   attaacagca tctttaaaac tgtgtagcta cctcacaacc agtcctgtct gtttatagtg 9360
   ctggtagtat caccttttgc cagaaggcct ggctggctgt gacttaccat agcagtgaca 9420
   atggcagtct tggctttaaa gtgaggggtg accctttagt gagcttagca cagcgggatt 9480
   aaacagtcct ttaaccagca cagccagtta aaagatgcag cctcactgct tcaacgcaga 9540
   ttttaatgtt tacttaaata taaacctggc actttacaaa caaataaaca ttgttttgta 9600
   ctcacggcgg cgataatagc ttgatttatt tggtttctac accaaataca ttctcctgac 9660
   cactaatggg agccaattca caattcacta agtgactaaa gtaagttaaa cttgtgtaga 9720
   ctaagcatgt aatttttaag ttttatttta atgaattaaa atatttgtta accaacttta 9780
   aagtcagtcc tgtgtatacc tagatattag tcagttggtg ccagatagaa gacaggttgt 9840
   gtttttatcc tgtggcttgt gtagtgtcct gggattctct gccccctctg agtagagtgt 9900
   tgtgggataa aggaatctct cagggcaagg agcttcttaa gttaaatcac tagaaattta 9960
   ggggtgatct gggccttcat atgtgtgaga agccgtttca ttttatttct cactgtattt 10020
   tcctcaacgt ctggttgatg agaaaaaatt cttgaagagt tttcatatgt gggagctaag 10080
   gtagtattgt aaaatttcaa gtcatcctta aacaaaatga tccacctaag atcttgcccc 10140
   tgttaagtgg tgaaatcaac tagaggtggt tcctacaagt tgttcattct agttttgttt 10200
   ggtgtaagta ggttgtgtga gttaattcat ttatatttac tatgtctgtt aaatcagaaa 10260
   ttttttatta tctatgttct tctagatttt acctgtagtt catt                   10304

## Description

### FIELD OF THE INVENTION

The present invention pertains to the field of cancer therapeutics and in particular to siRNA molecules against ribonucleotide reductase for the treatment of cancer.

### BACKGROUND

A unique step leading to DNA synthesis is the conversion of ribonucleotides to their corresponding deoxyribonucleotides, a reaction that is catalyzed in a cell cycle specific manner by ribonucleotide reductase [Lewis et al., J. Cell Physiol. 94:287-2981978; Reichard, Science 60:1773-1777, 1993; Wright, Encyl. Pharmacol. Therapeut. 128:89-111, 1989; Wright et al., Biochem. Cell Biol. 68:1364-1371 1990; Stubbe, Ann. Rev. Biochem. 58:257-285, 1989]. The mammalian ribonucleotide reductase enzyme is composed of two dissimilar dimeric protein subunits often called R1 and R2, both of which are required for enzymatic activity, and which are encoded by two different genes located on different chromosomes [Bjorklund et al., Proc. Natl. Acad. Sci. USA 90:11322-11326, 1993; Tonin et al., Cytogenet Cell Genet. 45:102-108, 987].

The expression of ribonucleotide reductase, and in particular the R2 subunit, is elevated in transformed cells exposed to tumour promoters, or to transforming growth factors in growth factor mediated mechanisms of tumour progression [Amara et al., J. Biol. Chem. 271:20126-20131, 1996; Chen et al., EMBO J. 12:3977-3986, 1993; Amara et al., Nucleic Acids Res. 23:1461-1467, 1995]. Studies conducted in tumour cells obtained from rodent and human tissues [Weber, Cancer Res. 43:3466-3492, 1983; Wright et al., Encyl. Pharmacol. Therapeut. 128:89-111, 1989; Saeki, et al., Int. J. Oncol. 6:523-529, 1995; Jenson et al., Proc. Nat. Acad. Sci. USA 91:9257-9261, 1994] and in cultured cells selected for resistance to anti-tumour agents such as hydroxyurea [Lewis et al., J Cell Physiol. 97:87-97, 1978; Wright et al., Drug Resistance in Mammalian Cells, Boca Raton, FL; CRC Press, Inc; 15-27, 1989] suggested that interference with the expression of ribonucleotide reductase may be a useful approach to inhibit the proliferation of tumour cells.

In the last few years, advances in nucleic acid chemistry and gene transfer have inspired new approaches to engineer specific interference of gene expression. Antisense technology has been the most commonly described approach in protocols designed to achieve gene-specific interference and many antisense compounds have now entered clinical trials [see review in Holmlund, Ann N Y Acad Sci. 1002:244-51, 2003].

Antisense oligonucleotides specifically targeted against ribonucleotide reductase have been described, for example in, International Patent Application Nos. PCT/CA97/00454 (WO 98/05769) and PCT/CA00/00120 (WO 00/47733).

An alternative approach to engineer specific interference with gene expression has been enabled by the recent discovery of potent, sequence specific inactivation of gene function induced by small interfering RNA (siRNA). This mechanism of gene inactivation is termed RNA interference (RNAi), and has become a powerful and widely used tool for the analysis of gene function in invertebrates, vertebrates and plants (reviewed in Sharp, P. A. (2001) Genes Dev 15, 485-90). Introduction of a siRNA molecule comprising a RNA strand that corresponds to or is complementary to an endogenous RNA into the cells of these organisms leads to the sequence-specific destruction of the endogenous RNA and the consequent inhibition of the expression of the endogenous RNA. Endogenous RNA can thus be targeted for inhibition by designing siRNA molecules containing an RNA strand that is complementary to the sense strand of an endogenous RNA. Recent reports have indicated that siRNA may have greater *in vitro* and *in vivo* potency than antisense oligonucleotides [Bertrand, et al., Biochem. Biophys. Res. Commun. 296, 1000-1004, 2002; Aoki, et al., Clin. Exp. Pharmacol. Physiol. 30, 96-102,2003].

A number of patents and patent applications have described, in general terms, the use of siRNA molecules to inhibit gene expression, for example, U.S. Patent No. 6,506,559; U.S. Patent Application Nos. 20040053876, 20040102408, 26030055263, and International Application No. PCT/EP2003/007516 (WO 2004/007718). Potential siRNA molecules against ribonucleotide reductase R1 or R2 have been described generally in U.S. Patent Application No. 20040068763, which describes nucleic acid molecules from zebrafish, including ribonucleotide reductase R1 and R2, and the use of double-stranded RNA or antisense oligonucleotide comprising sequences of these nucleic acid molecules to inactivate a corresponding naturally-occurring nucleic acid sequence and in U.S. Patent Application Nos. 20040006035 and 20030175950, which mention the use of nucleic acid-based techniques including siRNA molecules to decrease the expression of cellular proteins required for HIV cell fusion and entry, two of which are ribonucleotide reductase subunits R1 and R2. However, no specific siRNA molecules against ribonucleotide reductase, or target sequences for such siRNA molecules, are described in any of these documents. International Application No. PCT/EP03/05513 (WO 03/099298) describes the general concept of siRNA molecules to inhibit the expression of genes found in regions where there is loss of heterozygosity, one of which is ribonucleotide reductase, and methods of using such siRNAs to treat cancer. Again, no specific siRNA molecules against ribonucleotide reductase or target sequences are described in this application.

In a recent publication investigating the role of the M2 (R2) subunit of ribonucleotide reductase (RRM2) in gemcitabine resistance in pancreatic adenocarcinoma, the use of a specific siRNA against RRM2 to reduce gemcitabine chemoresistance of pancreatic adenocarcinoma cells is described [Duxbury et al., (2004) Oncogene 23:1539-1548]. Three double-stranded siRNA molecules are described in this publication, however, only one of these molecules was able to suppress the expression of the R2 subunit *in vitro* and *in vivo*. This siRNA molecule was further evaluated to determine its effect on cellular proliferation and gemcitabine chemoresistance *in vitro,* in four different pancreatic ductal adenocarcinoma cell lines. The results demonstrated that, although the siRNA molecule decreased the gemcitabine IC₅₀ of all four cell lines, it was able to elicit only a minor reduction in cellular proliferation *in vitro* in the absence of gemcitabine, with the reduction being significant in only one of the four pancreatic ductal adenocarcinoma cell lines. The siRNA molecule decreased gemcitabine chemoresistance of pancreatic adenocarcinoma cells *in vivo*, demonstrating a synergistic effect when administered in combination with gemcitabine, but when administered alone resulted in only a small decrease in the rate of tumour growth. The authors conclude that RRM2 is a determinant of gemcitabine resistance in pancreatic adenocarcinoma and that siRNA against RRM2 may, therefore, be effective as a therapeutic adjunct to gemcitabine treatment *in vitro* and *in vivo*.

This background information is provided for the purpose of making known information believed by the applicant to be of possible relevance to the present invention. No admission is necessarily intended, nor should be construed, that any of the preceding information constitutes prior art against the present invention.

### SUMMARY OF THE INVENTION

An object of the present invention is to provide small interfering RNA molecules against ribonucleotide reductase and uses thereof. In accordance with one aspect of the present invention, there is provided an isolated siRNA molecule of between about 14 and about 200 nucleotides in length, wherein the term "about" refers to a +/-10% variation from the nominal value, comprising: an antisense nucleotide sequence complementary to a region of a human ribonucleotide reductase R2 mRNA; and a sense nucleotide sequence complementary to said antisense nucleotide sequence, wherein said isolated siRNA molecule inhibits expression of said ribonucleotide reductase R2 mRNA. and inhibits neoplastic cell proliferation, and wherein said region of the human ribonucleotide reductase R2 mRNA is within any one of exons 4, 6, 7 or 10.

In accordance with another aspect, there is provided a DNA sequence encoding an isolated siRNA molecule of the invention.

In accordance with another aspect, there is provided a vector comprising a DNA sequence of the invention.

In accordance with another aspect, there is provided a pharmaceutical composition comprising a siRNA molecule of the invention and a pharmaceutically acceptable carrier.

In accordance with another aspect, there is provided a pharmaceutical composition comprising a DNA sequence of the invention and a pharmaceutically acceptable carrier.

In accordance with another aspect, there is provided a pharmaceutical composition comprising a vector of the invention and a pharmaceutically acceptable carrier.

In accordance with another aspect of the invention, there is provided an isolated siRNA molecule of between about 14 and about 200 nucleotides in length, wherein the term "about" refers to a +/-10% variation from the nominal value, comprising: an antisense nucleotide sequence complementary to a region of a human ribonucleotide reductase R2 mRNA; and a sense nucleotide sequence complementary to said antisense nucleotide sequence, for use in inhibiting neoplastic cell proliferation in a mammal in need of such therapy, wherein said isolated siRNA molecule inhibits expression of said ribonucleotide reductase R2 mRNA and inhibits neoplastic cell proliferation, and wherein said region of the human ribonucleotide reductase R2 mRNA is within any one of exons 4, 6, 7 or 10.

In accordance with another aspect of the invention, there is provided an isolated siRNA molecule of between about 14 and about 200 nucleotides in length, wherein the term "about" refers to a +/-10% variation from the nominal value, comprising: an antisense nucleotide sequence complementary to a region of a human ribonucleotide reductase R2 mRNA; and a sense nucleotide sequence complementary to said antisense nucleotide sequence, for use in inhibiting tumour growth in a mammal in need of such therapy, wherein said isolated siRNA molecule inhibits expression of said ribonucleotide reductase R2 mRNA and inhibits neoplastic cell proliferation, and wherein said region of the human ribonucleotide reductase R2 mRNA is within any one of exons 4, 6, 7 or 10.

In accordance with another aspect, there is provided a vector of the invention for use in inhibiting neoplastic cell proliferation in a mammal in need of such therapy.

In accordance with another aspect, there is provided a vector of the invention for use in inhibiting tumour growth in a mammal in need of such therapy.

In accordance with another aspect, there is provided a use of an isolated siRNA molecule of the invention in the manufacture of a medicament.

In accordance with another aspect, there is provided a use of a vector of the invention in the manufacture of a medicament.

### BRIEF DESCRIPTION OF THE FIGURES

Figure 1 depicts the effect of siRNA molecules targeting ribonucleotide reductase R2 mRNA on the levels of this mRNA in A-498 human renal cancer cells (A and B) and MDA-MB-231 human breast cancer cells (C).
Figure 2 depicts the effect of siRNA molecules targeting ribonucleotide reductase R2 mRNA on the level of ribonucleotide reductase R2 protein in A-498 human renal cancer cells (A and B) and MDA-MB-231 human breast cancer cells (C).
Figure 3 depicts the effect of siRNA molecules 544 (A); 787 (B); 933 (C); 624, 624+1 (D); 844 (E); and 1284 (F) targeting ribonucleotide reductase R2 mRNA on the growth of A-498 human renal cancer cell-derived tumours in SCID mice.
Figure 4 depicts the effect of 3 weeks of treatment with a siRNA molecule targeting ribonucleotide reductase R2 mRNA on the growth of A-498 human renal cancer cell-derived tumours in SCID mice: Control (A); siRNA 1284 (B); and siRNA 1284-scrambled (C).
Figure 5 depicts the effect of 4 weeks of treatment with a siRNA molecule targeting ribonucleotide reductase R2 mRNA on the growth of A-498 human renal cancer cell-derived tumours in SCID mice: Control (A); siRNA 1284 (B); and siRNA 1284-scrambled (C).
Figure 6 depicts the effect of 5 weeks of treatment with a siRNA molecule targeting ribonucleotide reductase R2 mRNA on the tumour weight of A-498 human renal cancer cell-derived tumours in SCID mice.
Figure 7 depicts the effect of siRNA molecules targeting ribonucleotide reductase R2 mRNA on the level of ribonucleotide reductase R2 mRNA in A-498 human renal carcinoma tumours excised from SCID mice.
Figure 8 depicts the effect of siRNA molecules 1284 (A) and 933 (B) targeting ribonucleotide reductase R2 mRNA on the level of ribonucleotide reductase R2 protein in A-498 human renal cancer cell-derived tumours excised from SCID mice.
Figure 9 depicts the dose dependent effect of treatment with a siRNA molecule targeting ribonucleotide reductase R2 mRNA on the tumour weight of A-498 human renal cancer cell-derived tumours in SCID mice.
Figure 10 depicts the dose dependent effect of treatment with a siRNA molecule targeting ribonucleotide reductase R2 mRNA on the levels of ribonucleotide reductase R2 protein in A-498 human renal cancer cell-derived tumours in SCID mice.
Figure 11 depicts the effect of treatment with a siRNA molecule targeting ribonucleotide reductase R2 mRNA 0.025µM (A) and 0.0125 µM (B) on the cell proliferation/viability of A-498 human renal cancer cells *in vitro.*
Figure 12 depicts the effect of treatment with a siRNA molecule targeting ribonucleotide reductase R2 mRNA 0.025µM (A) and 0.0125 µM (B) on the cell proliferation/viability of A-498 human renal cancer cells *in vitro.*
Figure 13 depicts the effect of treatment with a siRNA molecule targeting ribonucleotide reductase R2 mRNA on the cell cycle distribution of A-498 human renal cancer cells *in vitro:* Control (-oligofectamine) (A); 1284 (0.025µM) (B); 1284S (0.025 µM) (C); Control (+ oligofectamine) (D); 1284 (0.0125µM) (E); and 1284S (0.0125µM) (F).
Figure 14 depicts the effect of treatment with a siRNA molecule targeting ribonucleotide reductase R2 mRNA on the cell cycle distribution of A-498 human renal cancer cells *in vitro:* Control (-oligofectamine) (A); 933 (B); Control (+ oligofectamine) (C); and Scrambled (D).
Figure 15 depicts the effect of 5 weeks of treatment with a siRNA molecule targeting ribonucleotide reductase R2 mRNA on the tumour weight of HT-29 human colon cancer cell-derived tumours in CD-1 nude mice.
Figure 16 depicts the effect of 5 weeks of treatment with a siRNA molecule targeting ribonucleotide reductase R2 mRNA on the tumour weight of A2058 human melanoma cancer cell-derived tumours in CD-1 nude mice.
Figure 17 depicts the mRNA sequence for the human ribonucleotide reductase R1 subunit [SEQ ID NO:420].
Figure 18 depicts the mRNA sequence for the human ribonucleotide reductase R2 subunit [SEQ ID NO:421].
Figure 19 depicts the nucleotide sequence for the human ribonucleotide reductase R2 subunit gene [SEQ ID NO:436].
Figure 20 depicts a representative example of an annealed double-stranded siRNA of the invention.
Figure 21 depicts the effect of treatment with a siRNA molecule targeting ribonucleotide reductase R2 mRNA on the cell proliferation of MDA-MB-231 human breast cancer cells *in vitro.*
Figure 22 depicts the effect of treatment with a siRNA molecule targeting ribonucleotide reductase R2 mRNA 0.2 µM (A) and 0.1 µM (B) on the cell proliferation/viability of MDA-MB-231 human breast cancer cells *in vitro*.
Figure 23 depicts the effect of treatment with a siRNA molecule targeting ribonucleotide reductase R2 RNA on the levels of ribonucleotide reductase R2 protein and mRNA in different human cancer cell lines.

### DETAILED DESCRIPTION OF THE INVENTION

The present invention provides for small interfering RNA (siRNA) molecules against ribonucleotide reductase. As is known in the art, the mammalian ribonucleotide reduce enzyme is composed of two protein subunits R1 and R2, both of which are required for enzymatic activity. The siRNA molecules of the present invention thus target a gene encoding the R2 subunit of a human
ribonucleotide reductase enzyme, and are capable of inhibiting the expression of their target gene. The siRNA molecules of the invention are also capable of attenuating neoplastic cell growth and/or proliferation *in vitro* and *in vivo* and, therefore, can be used to attenuate the growth and/or metastasis of various types of mammalian cancers.

One embodiment of the present invention thus provides for a siRNA molecule that targets ribonucleotide reductase R2 and is capable of inhibiting tumour cell proliferation both *in vitro* and *in vivo.* In a further embodiment, treatment of neoplastic cells *in vitro* with a ssRNA molecule of the invention results in a decrease of about 10% or more in the proliferation of the cells when compacted to untreated cells or to cells treated with an appropriate control siRNA, such as a scrambled control siRNA molecule.

Accordingly, the present invention provides for the use of a siRNA molecule against ribonucleotide reductase R2 in the treatment of cancer and for methods of treating a cancer in a mammal by administration of a siRNA molecule of the invention alone or in combination with one or more anti-cancer therapeutics.

### Definitions

Unless defined otherwise, all technical and scientific terms used herein have the same meaning as commonly understood by one of ordinary skill in the art to which this invention belongs.

As used herein, the term "about" refers to a +/-10% variation from the nominal value. It is to be understood that such a variation is always included in any given value provided herein, whether or not it is specifically referred to.

The term "gene," as used herein, refers to a segment of nucleic acid that encodes an individual protein or RNA and can include both exons and introns together with associated regulatory regions such as promoters, operators, terminators, 5' untranslated regions, 3' untranslated regions, and the like.

The term "RNA," as used herein, refers to a nucleic acid molecule of one or more nucleotides in length, wherein the nucleotide(s) are ribonucleotides. By "ribonucleotide" it is meant a naturally-occurring ribonucleotide comprising a nucleotide linked to a β-D-ribofuranose moiety having a hydroxyl group at the 2' position, as well modified versions thereof. The term "RNA" includes double-stranded RNA, single-stranded RNA, isolated RNA such as partially purified RNA, essentially pure RNA, synthetic RNA, recombinantly produced RNA, as well as modified RNA that differs from naturally-occurring RNA by the addition, deletion, substitution and/or alteration of one or more nucleotides as described herein.

The term "naturally-occurring," as used herein, as applied to an object, refers to the fact that the object can be found in nature. For example, a nucleotide or nucleotide sequence that is present in an organism (including viruses) that can be isolated from a source in nature and which has not been intentionally modified by man in the laboratory is said to be naturally-occurring.

The term "selectively hybridize," as used herein, refers to the ability of a particular nucleic acid sequence to bind detectably and specifically to a second nucleic acid sequence. Selective hybridization generally takes place under hybridization and wash conditions that minimize appreciable amounts of detectable binding to non-specific nucleic acids. High stringency conditions can be used to achieve selective hybridization conditions as known in the art and discussed herein. Typically, hybridization and washing conditions are performed at high stringency according to conventional hybridization procedures with washing conditions utilising a solution comprising 1-3 x SSC, 0.1-1% SDS at 50-70°C, with a change of wash solution after about 5-30 minutes.

The following terms are used herein to describe the sequence relationships between two or more polynucleotides: "reference sequence," "window of comparison," "sequence identity," "percent (%) sequence identity," and "substantial identity." A "reference sequence" is a defined sequence used as a basis for a sequence comparison; a reference sequence may be a subset of a larger sequence, for example, as a segment of a full-length cDNA or gene, or may comprise a complete cDNA or gene sequence. Generally, a reference polynucleotide sequence is at least 20 nucleotides in length, and often at least 50 nucleotides in length.

A "window of comparison", as used herein, refers to a conceptual segment of the reference sequence of at least 15 contiguous nucleotide positions over which a candidate sequence may be compared to the reference sequence and wherein the portion of the candidate sequence in the window of comparison may comprise additions or deletions (*i.e.* gaps) of 20 percent or less as compared to the reference sequence (which does not comprise additions or deletions) for optimal alignment of the two sequences. The present invention contemplates various lengths for the window of comparison, up to and including the full length of either the reference or candidate sequence. Optimal alignment of sequences for aligning a comparison window may be conducted using the local homology algorithm of Smith and Waterman (Adv. Appl. Math. (1981) 2:482), the homology alignment algorithm of Needleman and Wunsch (J. Mol. Biol. (1970) 48:443), the search for similarity method of Pearson and Lipman (Proc. Natl. Acad. Sci. (U.S.A.) (1988) 85:2444), using computerised implementations of these algorithms (such as GAP, BESTFIT, FASTA, and TFASTA in the Wisconsin Genetics Software Package Release 7.0, Genetics Computer Group, 573 Science Dr., Madison, WI), using publicly available computer software such as ALIGN or Megalign (DNASTAR), or by inspection. The best alignment (*i.e*. resulting in the highest percentage of identity over the comparison window) is then selected.

The term "sequence identity" means that two polynucleotide sequences are identical (*i.e*. on a nucleotide-by-nucleotide basis) over the window of comparison.

The term "percent (%) sequence identity," as used herein with respect to a reference sequence is defined as the percentage of nucleotides in a candidate sequence that are identical with the nucleotides in the reference polynucleotide sequence over the window of comparison after optimal alignment of the sequences and introducing gaps, if necessary, to achieve the maximum percent sequence identity.

The terms "substantial identity" or "substantially identical," as used herein, denote a characteristic of a polynucleotide sequence, wherein the polynucleotide comprises a sequence that has at least 50% sequence identity as compared to a reference sequence over the window of comparison allowing for gaps or mismatches of several bases, which may result from genetic mutation, polymorphism, evolutionary divergence or other phenomena. Polynucleotide sequences with at least 60% sequence identity, at least 70% sequence identity, at least 80% sequence identity, and at least 90% sequence identity as compared to a reference sequence over the window of comparison are also considered to have substantial identity with the reference sequence.

The terms "corresponding to" or "corresponds to" indicate that a polynucleotide sequence is identical to all or a portion of a reference polynucleotide sequence. In contradistinction, the term "complementary to" is used herein to indicate that the polynucleotide sequence is identical to all or a portion of the complementary strand of a reference polynucleotide sequence. For illustration, the nucleotide sequence "TATAC" corresponds to a reference sequence "TATAC" and is complementary to a reference sequence "GTATA."

The term "target gene," as used herein, refers to a gene the expression of which is to be modulated with a siRNA molecule of the invention. In the context of the present invention, the target gene is a mammalian ribonucleotide reductase gene.

The term "target mRNA, " as used herein refers to the mRNA transcribed from a target gene.

The term "antisense strand" refers to a nucleotide sequence that is complementary to a mRNA sequence. The term "sense strand" refers to a nucleotide sequence that corresponds to a mRNA sequence and thus is complementary to the antisense strand.

The term "specific antisense sequence" refers to a nucleotide sequence that is complementary to a portion of a target mRNA sequence.

The terms "target mRNA sequence" and "target sequence," as used herein, refer to a nucleotide sequence within a target mRNA that is complementary to the specific antisense sequence comprised by a siRNA molecule of the invention.

The terms "therapy," and "treatment," as used interchangeably herein, refer to an intervention performed with the intention of improving a recipient's status. The improvement can be subjective or objective and is related to the amelioration of the symptoms associated with, preventing the development of, or altering the pathology of a disease, disorder or condition being treated. Thus, the terms therapy and treatment are used in the broadest sense, and include the prevention (prophylaxis), moderation, reduction, and curing of a disease, disorder or condition at various stages. Prevention of deterioration of a recipient's status is also encompassed by the term. Those in need of therapy/treatment include those already having the disease, disorder or condition as well as those prone to, or at risk of developing, the disease, disorder or condition and those in whom the disease, disorder or condition is to be prevented.

The terms "ameliorate" or "amelioration" include the arrest, prevention, decrease, or improvement in one or more the symptoms, signs, and features of the disease being treated, both temporary and long-term.

The term "subject" or "patient," as used herein, refers to a mammal in need of treatment.

Administration of a siRNA molecule of the invention "in combination with" one or more anti-cancer therapeutics is intended to include simultaneous (concurrent) administration and consecutive administration. Consecutive administration is intended to encompass administration of the therapeutic(s) and the siRNA molecule(s) of the invention to the subject in various orders.

### SMALL INTERFERING DNA MOLECULES (siRNA) AGAINST RIBONUCLEOTIDE REDUCTASE

The siRNA molecules of the present invention are targeted to a human ribonucleotide reductase R2 gene and are capable of inhibiting the expression of their target gene. As is known in the art, mammalian ribonucleotide reductase comprises two subunits, the R1 and R2 subunits, which are encoded by two different genes. Accordingly, the siRNA molecules of the present invention target the gene encoding ribonucleotide reductase
R2. The siRNA molecules of the invention comprise a specific antisense sequence that is complementary to a portion of the mRNA transcribed from the target gene (*i.e*. the target mRNA). The siRNA molecules of the invention can be double stranded (*i.e.* composed of an antisense strand, comprising the specific antisense sequence, and a complementary sense strand) or single-stranded (*i.e.* composed of an antisense strand, comprising the specific antisense sequence, only) as described in more detail below.

In accordance with the present invention, a siRNA molecule refers to a short RNA molecule capable of inhibiting or downregulating expression of a target gene through an RNA interference (RNAi) mechanism (as described in, for example Bass, 2001, Nature, 411, 428-429; Elbashir et al., 2001, Nature, 411, 494-498; International PCT Publication No. WO 00/44895; International PCT Publication No. WO 01/36646; International PCT Publication No. WO 99/32619; International PCT Publication No. WO 00/01846; International PCT Publication No. WO 01/29058; International PCT Publication No. WO 99/07409; and International PCT Publication No. WO 00/44914; Allshire, 2002, Sciencce, 297, 1818-1819; Volpe et al., 2002, Science, 297, 1833-1837; Jenuwein, 2002, Science, 297, 2215-2218; Hall et al., 2002, Science, 297, 2232-2237; Hutvagner and Zamore, 2002, Science, 297, 2056-60; McManus et al., 2002, RNA, 8, 842-850; Reinhart et al., 2002, Gene & Dev., 16, 1616-1626; and Reinhart & Bartel, 2002, Science, 297, 1831). A siRNA molecule can achieve this effect by targeting one of a variety of RNA molecules corresponding to a target gene. Non-limiting examples of such RNAs include messenger RNA (mRNA), alternate RNA splice variants of the target gene, post-transcriptionally modified RNA of the target gene and pre-mRNA of the target gene. In the context of the present invention, the term "mRNA" includes all such RNA molecules corresponding to a target gene.

### Target mRNA Sequences

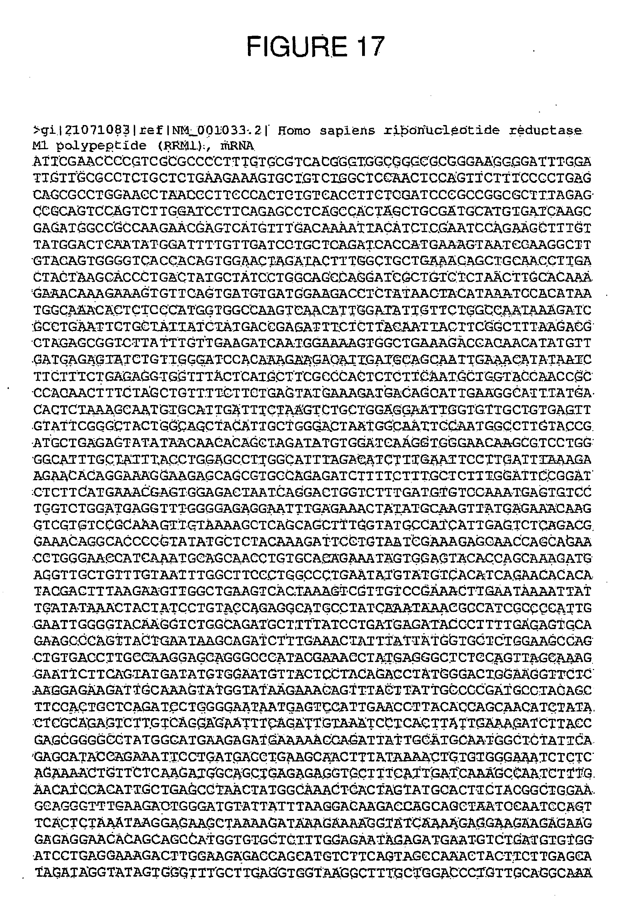

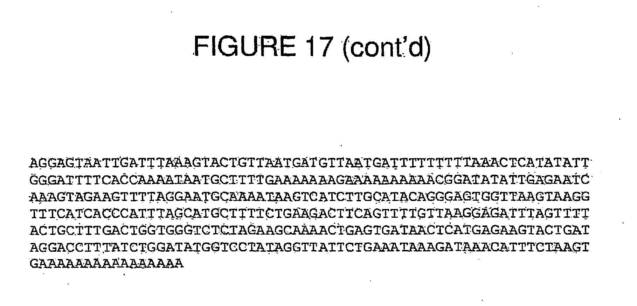

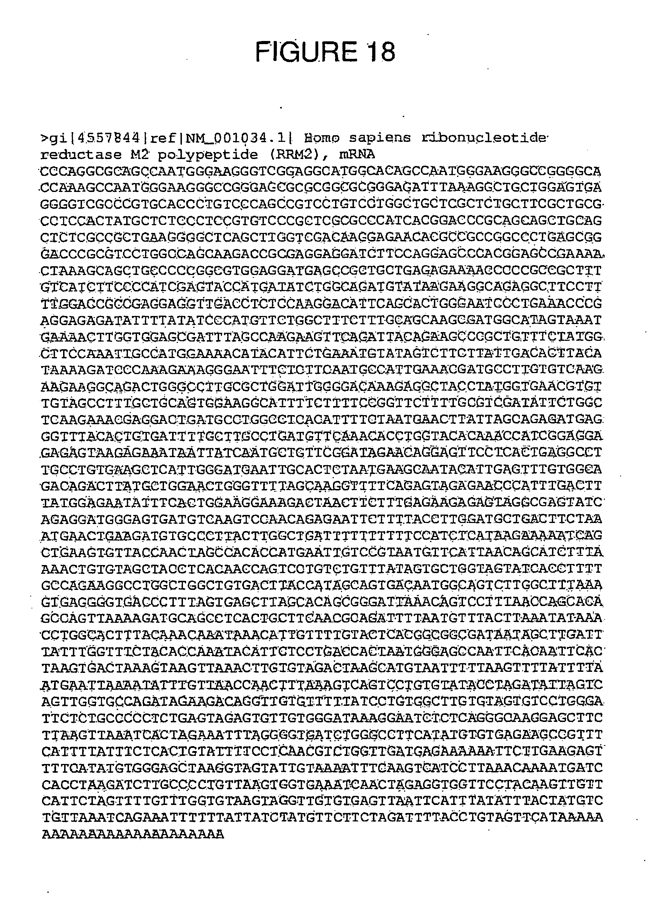

In order to design the siRNA molecules of the present invention, a target RNA is first chosen and then an appropriate target sequence within the target mRNA is selected. Once the target mRNA sequence has been selected, a siRNA molecule can be designed such that it comprises a nucleotide sequence complementary to all or a portion of this target sequence, *i.e*. a specific antisense sequence. In accordance with the present invention, as indicated above, the target mRNA is a mRNA transcribed from a mammalian ribonucleotide reductase R2 gene. The sequences of various mammalian ribonucleotide reductase mRNAs are known in the art. For example, the mRNA sequences for the human ribonucleotide reductase R1 subunit (GenBank^{™} Accession No. NM_001033) and R2 subunit (GenBank^{™} Accession No. NM_001034) are available from the GenBank^{™} database maintained by the NCBI and are provided herein as SEQ ID NO:420 and SEQ ID NO:421, and are also shown in Figures 17 and 18, respectively. The sequences of other mammalian ribonucleotide reductase mRNAs are also available from this database (for example, NM_009103 Mouse *(Mus musculus)* R1 subunit; NM_009104 Mouse *(Mus musculus)* R2 subunit, and X68127 Golden hamster *(Mesocricetus auratus)* R2 subunit). In one embodiment of the present invention, the target mRNA is a human ribonucleotide reductase R1 or R2 mRNA. In another embodiment, the target mRNA is a human ribonucleotide reductase R2 mRNA as set forth in SEQ ID NO: 421.

Suitable target sequences within the target mRNA are selected using one or more of several criteria known in the art (see for example, Elbashir, S. M., et al. (2001) Nature 411, 494-498; Elbashir, S. M., et al. (2002) Methods 26, 199-213; Elbashir, S. M., et al. (2001) Genes Dev. 15, 188-200; Elbashir, S. M., et al. (2001) EMBO J. 20, 6877-6888; and Zamore, P.D., et al. (2000) Cell 101, 25-33). Target mRNA sequences are typically between about 14 and about 50 nucleotides in length. The target mRNA sequence can be selected from various regions of the target mRNA, including the coding region, the 3' untranslated region and the 5' untranslated region. Typically, mammalian mRNA sequences comprise a series of exons spliced together.
Accordingly, the target mRNA sequence for the siRNA molecules of the present invention can be wholly within an exon or can span an exon-exon junction.

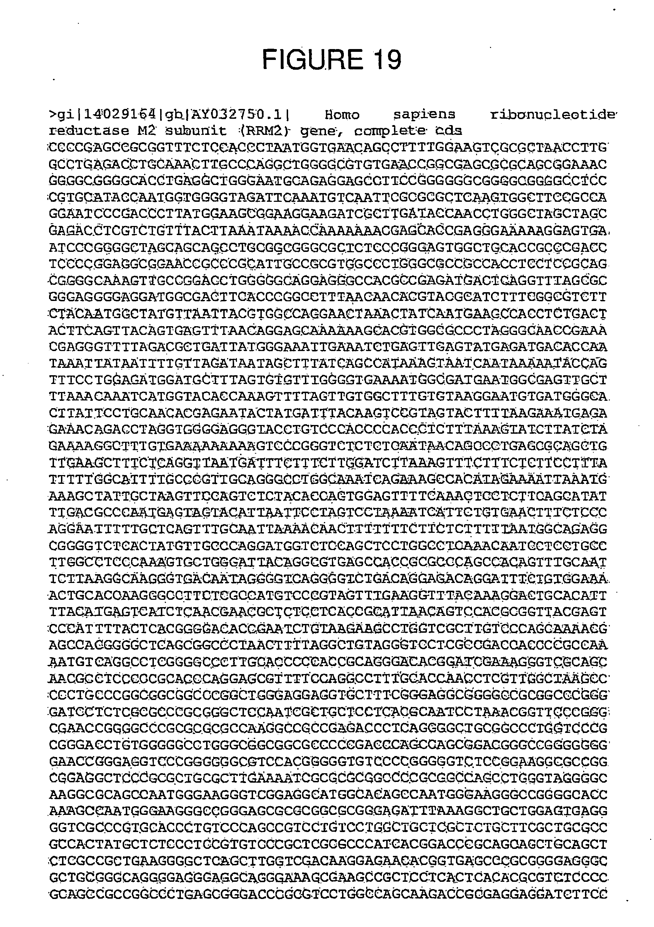

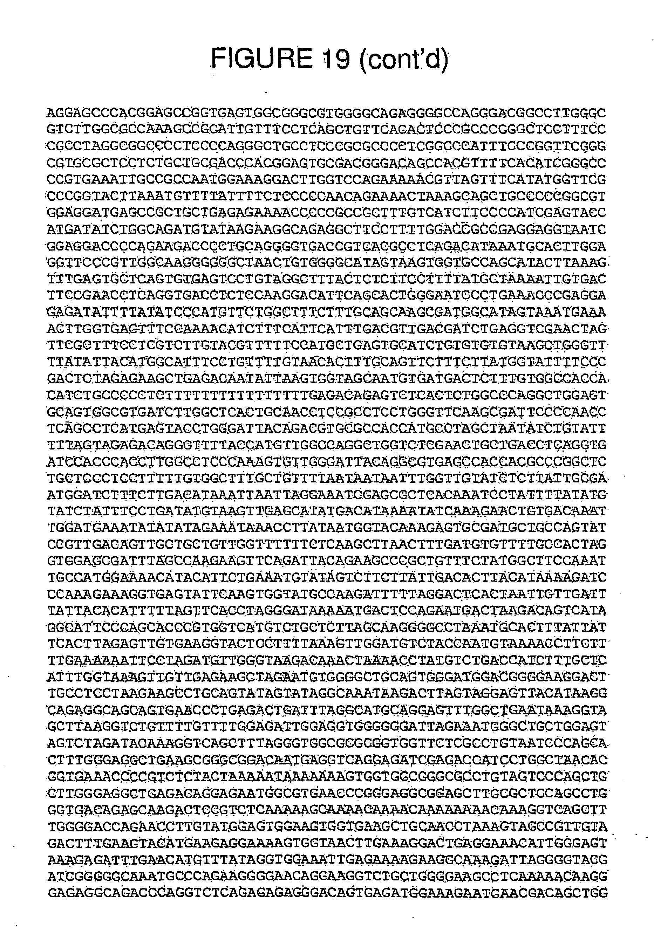

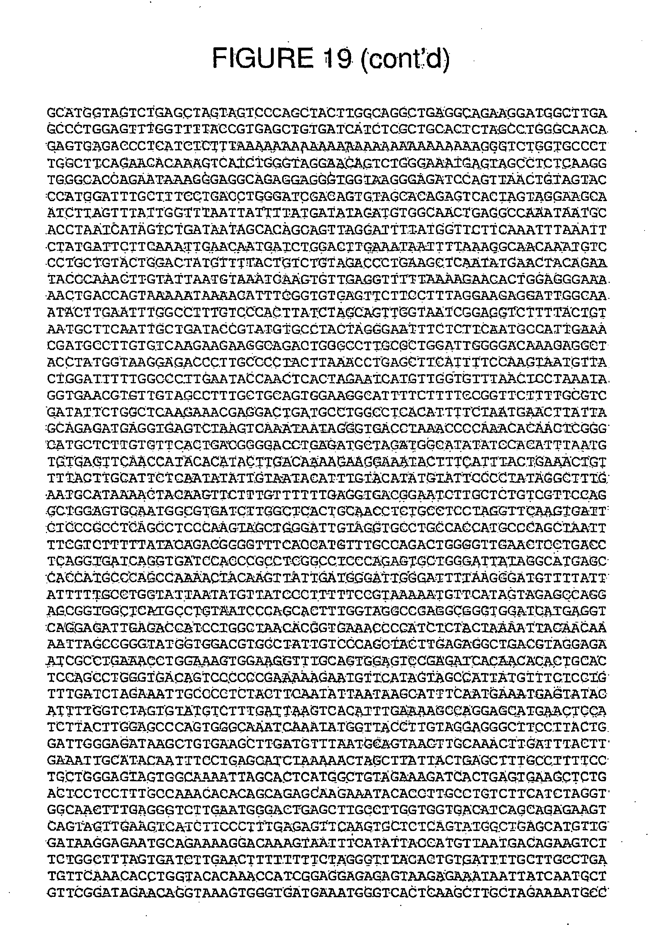

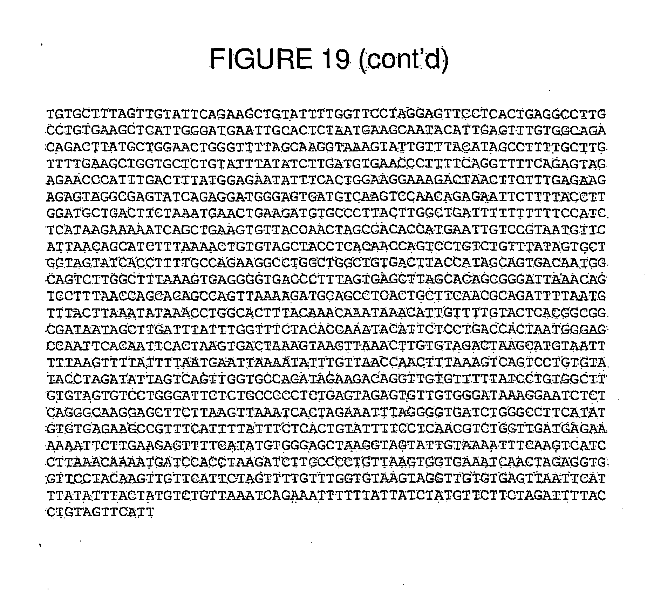

The sequences encompassed by the various regions of the target mRNA are known in the art. For example, the nucleotide sequence corresponding to the human ribonucleotide reductase R2 subunit gene is available from GenBank^{™} (Accession No. AY032750; also provided herein as SEQ ID NO:436 (see Figure 19)). As described in the Features section of Accession No. AY032750 under "mRNA", the R2 gene sequence comprises 10 exons. These exons correspond to the following nucleotide positions in the R2 gene sequence provided under Accession No. AY032750:
Exon 1 from nucleotides 2380 to 2709;
Exon 2 from nucleotides 2795 to 2869;
Exon 3 from nucleotides 3199 to 3344;
Exon 4 from nucleotides 3549 to 3663;
Exon 5 from nucleotides 4527 to 4660;
Exon 6 from nucleotides 6671 to 6765;
Exon 7 from nucleotides 6884 to 7017;
Exon 8 from nucleotides 8653 to 8757;
Exon 9 from nucleotides 8846 to 8959; and
Exon 10 from nucleotides 9040 to 10304.

The coding sequence (CDS) of the ribonucleotide reductase R2 gene is also described in the Features section of Accession No. AY032750 as the sequence provided when the following regions of the gene are joined: nucleotides 2611 to 2709, 2795 to 2869, 3199 to 3344, 3549 to 3663, 4527 to 4660, 6671 to 6765, 6884 to 7017, 8653 to 8757, 8846 to 8959, and 9040 to 9192. Similar information can readily be obtained for other R1 and R2 gene sequences.

In one embodiment of the present invention, the mRNA target sequence is within the coding region of the target mRNA. In another embodiment, the target sequence is selected from the region of the target mRNA beginning 50 to 100 nucleotides downstream of the start codon and ending at the stop codon. In a further embodiment, the target mRNA sequence is within an individual exon. In another embodiment, the target mRNA sequence is selected from a region of the target mRNA which spans an exon-exon junction.

In a specific embodiment of the present invention, the siRNA molecule is targeted to the ribonucleotide reductase R2 subunit and the mRNA target sequence is within the coding sequence (CDS) defined for the human ribonucleotide reductase R2 gene in GenBank^{™} Accession No. AY032750. In another embodiment of the present invention, the siRNA molecule is targeted to the ribonucleotide reductase R2 subunit and the mRNA target sequence is within any one of exons 4, 6, 7 or 10 described for the human ribonucleotide reductase R2 gene in the GenBank^{™} Accession No. AY032750 (and listed above). In a further embodiment, the ribonucleotide reductase R2 subunit mRNA target sequence is within Exon 4, 7 or 10. In another embodiment, the ribonucleotide reductase R2 subunit mRNA target sequence is within Exon 4 or 7. In another embodiment, the ribonucleotide reductase R2 subunit mRNA target sequence spans an exon-exon junction.

As indicated above, the target mRNA sequence is typically between about 14 to about 50 nucleotides in length. The target mRNA can, therefore, be scanned for regions between about 14 and about 50 nucleotides in length that meet one or more of the following criteria for a target sequence: an A+T/G+C ratio of between about 2:1 and about 1:2; an AA dinucleotide or a CA dinucleotide at the 5' end of the target sequence; a sequence of at least 10 consecutive nucleotides unique to the target mRNA (*i.e*. the sequence is not present other mRNA sequences from the same mammal); and no "runs" of more than three consecutive guanine (G) nucleotides or more than three consecutive cytosine (C) nucleotides. These criteria can be assessed using various techniques known in the art, for example, computer programs such as BLAST can be used to search publicly available databases to determine whether the selected target sequence is unique to the target mRNA. Alternatively, a target sequence can be selected (and a siRNA sequence designed) using computer software available commercially (*e.g*. OligoEngine^{™} (Seattle, Wash.); Dharmacon, Inc. (Lafayette, Colo.); Target Finder from Ambion Inc. (Austin, Tex.) and the siRNA Design Tool from QIAGEN, Inc. (Valencia, Calif.)).

In one embodiment of the present invention, target mRNA sequences are selected that are between about 14 and about 30 nucleotides in length that meet one or more of the above criteria. In another embodiment, target sequences are selected that are between about 16 and about 30 nucleotides in length that meet one or more of the above criteria. In a further embodiment, target sequences are selected that are between about 19 and about 30 nucleotides in length that meet one or more of the above criteria. In another embodiment, target sequences are selected that are between about 19 and about 25 nucleotides in length that meet one or more of the above criteria.

In a specific embodiment of the invention, a target mRNA sequence is selected that comprises the sequence 5'-AA(Nₓ)-3' or 5'-NA(Nₓ)-3', where N is any nucleotide and "x" is an integer between 10 and 50. In another embodiment, "x" is between 15 and 30. In yet another embodiment, "x" is between 19 and 23. In a further embodiment, "x" is 19 or 20.

In another embodiment, a target mRNA sequence is selected that comprises between about 30% and about 70% G/C content. In another embodiment, a target sequence is selected that comprises between about 30% and about 60% G/C content In another embodiment, a target sequence is selected that comprises between about 35% and about 55% G/C content.

Exemplary non-limiting target mRNA sequences for human ribonucleotide reductase R2 are provided in Table 2 below. In one embodiment of the present invention, one or more of the exemplary target sequences provided in Table 2 are used to design siRNA molecules against ribonucleotide reductase R2.

**Table 1: mRNA Target Sequences from Human Ribonucleotide Reductase R1 for the Design of siRNA Molecule**

| **SEQ ID NO** | **Position in R1 mRNA sequence** | **Sequence (5' to 3')** |
|---|---|---|
| 1 | 82 | AAGAAAGTGCTGTCTGGCTCC |
| 2 | 85 | AAAGTGCTGTCTGGCTCCAAC |
| 3 | 86 | AAGTGCTGTCTGGCTCCAACT |
| 4 | 135 | AACCTAACCCTTCCCACTCTG |
| 5 | 261 | AAGAACGAGTCATGTTTGACA |
| 6 | 295 | AATCCAGAAGCTTTGTTATGG |
| 7 | 356 | AAAGTAATCCAAGGCTTGTAC |
| 8 | 357 | AAGTAATCCAAGGCTTGTACA |
| 9 | 361 | AATCCAAGGCTTGTACAGTGG |
| 10 | 396 | AACTAGATACTTTGGCTGCTG |
| 11 | 417 | AAACAGCTGCAACCTTGACTA |
| 12 | 418 | AACAGCTGCAACCTTGACTAC |
| 13 | 427 | AACCTTGACTACTAAGCACCC |
| 14 | 440 | AAGCACCCTGACTATGCTATC |
| 15 | 503 | AGAAAGTGTTCAGTGATGTG |
| 16 | 506 | AAAGTGTTCAGTGATGTGATG |
| 17 | 507 | AAGTGTTCAGTGATGTGATGG |
| 18 | 548 | AATCCACATAATGGCAAACAC |
| 19 | 557 | AATGGCAAACACTCTCCCATG |
| 20 | 589 | AACATTGGATATTGTTCTGGC |
| 21 | 614 | AAAGATCGCCTGAATTCTGCT |
| 22 | 615 | AAGATCGCCTGAATTCTGCTA |
| 23 | 662 | AATTACTTCGGCTTTAAGACG |
| 24 | 677 | AAGACGCTAGAGCGGTCTTAT |
| 25 | 704 | AAGATCAATGGAAAAGTGGCT |
| 26 | 710 | AATGGAAAAGTGGCTGAAAGA |
| 27 | 715 | AAAAGTGGCTGAAAGACCACA |
| 28 | 716 | AAAGTGGCTGAAAGACCACAA |
| 29 | 717 | AAGTGGCTGAAAGACCACAAC |
| 30 | 770 | AAAGAAGACATTGATGCAGCA |
| 31 | 771 | AAGAAGACATTGATGCAGCAA |
| 32 | 774 | AAGACATTGATGCAGCAATTG |
| 33 | 803 | AATCTTCTTTCTGAGAGGTGG |
| 34 | 863 | AACCGCCCACAACTTTCTAGC |
| 35 | 902 | AAAGATGACAGCATTGAAGGC |
| 36 | 903 | AAGATGACAGCATTGAAGGCA |
| 37 | 959 | AAGTCTGCTGGAGGAATTGGT |
| 38 | 973 | AATTGGTGTTGCTGTGAGTTG |
| 39 | 1028 | AATGGCAATTCCAATGGCCTT |
| 40 | 1034 | AATTCCAATGGCCTTGTACCG |
| 41 | 1040 | AATGGCCTTGTACCGATGCTG |
| 42 | 1070 | AACAACACAGCTAGATATGTG |
| 43 | 1073 | AACACAGCTAGATATGTGGAT |
| 44 | 1174 | AAAGAAGAACACAGGAAAGGA |
| 45 | 1175 | AAGAAGAACACAGGAAAGGAA |
| 46 | 1178 | AAGAACACAGGAAAGGAAGAG |
| 47 | 1181 | AACACAGGAAAGGAAGAGCAG |
| 48 | 1250 | AAACGAGTGGAGACTAATCAG |
| 49 | 1251 | AACGAGTGGAGACTAATCAGG |
| 50 | 1265 | AATCAGGACTGGTCTTTGATG |
| 51 | 1291 | AAATGAGTGTCCTGGTCTGGA |
| 52 | 1292 | AATGAGTGTCCTGGTCTGGAT |
| 53 | 1348 | AAGTTATGAGAAACAAGGTCG |
| 54 | 1358 | AAACAAGGTCGTGTCCGCAAA |
| 55 | 1359 | AACAAGGTCGTGTCCGCAAAG |
| 56 | 1362 | AAGGTCGTGTCCGCAAAGTTG |
| 57 | 1376 | AAAGTTGTAAAAGCTCAGCAG |
| 58 | 1377 | AAGTTGTAAAAGCTCAGCAGC |
| 59 | 1385 | AAAGCTCAGCAGCTTTGGTAT |
| 60 | 1386 | AAGCTCAGCAGCTTTGGTATG |
| 61 | 1466 | AATCGAAAGAGCAACCAGCAG |
| 62 | 1471 | AAAGAGCAACCAGCAGAACCT |
| 63 | 1472 | AAGAGCAACCAGCAGAACCTG |
| 64 | 1487 | AACCTGGGAACCATCAAATGC |
| 65 | 1495 | AACCATCAAATGCAGCAACCT |
| 66 | 1502 | AAATGCAGCAACCTGTGCACA |
| 67 | 1503 | AATGCAGCAACCTGTGCACAG |
| 68 | 1511 | AACCTGTGCACAGAAATAGTG |
| 69 | 1524 | AAATAGTGGAGTACACCAGCA |
| 70 | 1525 | AATAGTGGAGTACACCAGCAA |
| 71 | 1544 | AAAGATGAGGTTGCTGTTTGT |
| 72 | 1545 | AAGATGAGGTTGCTGTTTGTA |
| 73 | 1622 | AAGAAGTTGGCTGAAGTCACT |
| 74 | 1625 | AAGTTGGCTGAAGTCACTAAA |
| 75 | 1635 | AAGTCACTAAAGTCGTTGTCC |
| 76 | 1643 | AAAGTCGTTGTCCGAAACTTG |
| 77 | 1644 | AAGTCGTTGTCCGAAACTTGA |
| 78 | 1681 | AAACTACTATCCTGTACCAGA |
| 79 | 1682 | AACTACTATCCTGTACCAGAG |
| 80 | 1749 | AAGGTCTGGCAGATGCTTTTA |
| 81 | 1800 | AAGCCCAGTTACTGAATAAGC |
| 82 | 1893 | AAACCTATGAGGGCTCTCCAG |
| 83 | 1894 | AACCTATGAGGGCTCTCCAGT |
| 84 | 1946 | AATGTTACTCCTACAGACCTA |
| 85 | 1976 | AAGGTTCTCAAGGAGAAGATT |
| 86 | 2072 | AATGAGTCCATTGAACCTTAC |
| 87 | 2085 | AACCTTACACCAGCAACATCT |
| 88 | 2099 | AACATCTATACTCGCAGAGTC |
| 89 | 2219 | AATGGCTCTATTCAGAGCATA |
| 90 | 2244 | AAATTCCTGATGACCTGAAGC |
| 91 | 2245 | AATTCCTGATGACCTGAAGCA |
| 92 | 2273 | AAAACTGTGTGGGAAATCTCT |
| 93 | 2274 | AAACTGTGTGGGAAATCTCTC |
| 94 | 2275 | AACTGTGTGGGAAATCTCTCA |
| 95 | 2297 | AAAACTGTTCTCAAGATGGCA |
| 96 | 2298 | AAACTGTTCTCAAGATGGCAG |
| 97 | 2299 | AACTGTTCTCAAGATGGCAGC |
| 98 | 2309 | AAGATGGCAGCTGAGAGAGGT |
| 99 | 2343 | AAAGCCAATCTTTGAACATCC |
| 100 | 2344 | AAGCCAATCTTTGAACATCCA |
| 101 | 2357 | AACATCCACATTGCTGAGCCT |
| 102 | 2378 | AACTATGGCAAACTCACTAGT |
| 103 | 2387 | AAACTCACTAGTATGCACTTC |
| 104 | 2388 | AACTCACTAGTATGCACTTCT |
| 105 | 2417 | AAGCAGGGTTTGAAGACTGGG |
| 106 | 2449 | AAGGACAAGACCAGCAGCTAA |
| 107 | 2455 | AAGACCAGCAGCTAATCCAAT |
| 108 | 2468 | AATCCAATCCAGTTCACTCTA |
| 109 | 2524 | AAAAGAGGAAGAAGAGAAGGA |
| 110 | 2525 | AAAGAGGAAGAAGAGAAGGAG |
| 111 | 2526 | AAGAGGAAGAAGAGAAGGAGA |
| 112 | 2532 | AAGAAGAGAAGGAGAGGAACA |
| 113 | 2535 | AAGAGAAGGAGAGGAACACAG |
| 114 | 2589 | AATGTCTGATGTGTGGATCCT |
| 115 | 2614 | AAAGACTTGGAAGAGACCAGC |
| 116 | 2615 | AAGACTTGGAAGAGACCAGCA |
| 117 | 2624 | AAGAGACCAGCATGTCTTCAG |
| 118 | 2699 | AAGGCTTTGCTGGACCCTGTT |
| 119 | 2880 | AAGTCATCTTGCATACAGGGA |
| 120 | 2907 | AAGTAAGGTTTCATCACCCAT |
| 121 | 2911 | AAGGTTTCATCACCCATTTAG |
| 122 | 3008 | AACTGAGTGATAACTCATGAG |
| 123 | 3019 | AACTCATGAGAAGTACTGATA |

**Table 2: Exemplary mRNA Target Sequences from Human Ribonucleotide Reductase R2 for the Design of siRNA Molecules**

| **SEQ ID NO** | **Position in R2 mRNA sequence** | **Sequence (5' to 3')** |
|---|---|---|
| 124 | 477 | AAGAAGGCAGAGGCTTCCTTT |
| 125 | 480 | AAGGCAGAGGCTTCCTTTTGG |
| 126 | 525 | AAGGACATTCAGCACTGGGAA |
| 127 | 544 | AATCCCTGAAACCCGAGGAGA |
| 128 | 552 | AAACCCGAGGAGAGATATTTT |
| 129 | 553 | AACCCGAGGAGAGATATTTTA |
| 130 | 602 | AAGCGATGGCATAGTAAATGA |
| 131 | 617 | AAATGAAAACTTGGTGGAGCG |
| 132 | 618 | AATGAAAACTTGGTGGAGCGA |
| 133 | 622 | AAAACTTGGTGGAGCGATTTA |
| 134 | 623 | AAACTTGGTGGAGCGATTTAG |
| 135 | 624 | AACTTGGTGGAGCGATTTAGC |
| 136 | 649 | AAGTTCAGATTACAGAAGCCC |
| 137 | 664 | AAGCCCGCTGTTTCTATGGCT |
| 138 | 746 | AAAAGATCCCAAAGAAAGGGA |
| 139 | 747 | AAAGATCCCAAAGAAAGGGAA |
| 140 | 748 | AAGATCCCAAAGAAAGGGAAT |
| 141 | 761 | AAGGGAATTTCTCTTCAATGC |
| 142 | 777 | AATGCCATTGAAACGATGCCT |
| 143 | 787 | AAACGATGCCTTGTGTCAAGA |
| 144 | 788 | AACGATGCCTTGTGTCAAGAA |
| 145 | 843 | AAAGAGGCTACCTATGGTGAA |
| 146 | 844 | AAGAGGCTACCTATGGTGAAC |
| 147 | 862 | AACGTGTTGTAGCCTTTGCTG |
| 148 | 889 | AAGGCATTTTCTTTTCCGGTT |
| 149 | 933 | AAGAAACGAGGACTGATGCCT |
| 150 | 973 | AACTTATTAGCAGAGATGAGG |
| 151 | 1026 | AAACACCTGGTACACAAACCA |
| 152 | 1027 | AACACCTGGTACACAAACCAT |
| 153 | 1041 | AAACCATCGGAGGAGAGAGTA |
| 154 | 1042 | AACCATCGGAGGAGAGAGTAA |
| 155 | 1077 | AATGCTGTTGGGATAGAACAG |
| 156 | 1093 | AACAGGAGTTCCTCACTGAGG |
| 157 | 1125 | AAGCTCATTGGGATGAATTGC |
| 158 | 1159 | AATACATTGAGTTTGTGGCAG |
| 159 | 1195 | AACTGGGTTTTAGCAAGGTTT |
| 160 | 1209 | AAGGTTTTCAGAGTAGAGAAC |
| 161 | 1227 | AACCCATTTGACTTTATGGAG |
| 162 | 1284 | AAGAGAGTAGGCGAGTATCAG |
| 163 | 1363 | AAATGAACTGAAGATGTGCCC |
| 164 | 1364 | AATGAACTGAAGATGTGCCCT |
| 165 | 1368 | AACTGAAGATGTGCCCTTACT |
| 166 | 1373 | AAGATGTGCCCTTACTTGGCT |
| 167 | 1419 | AAAATCAGCTGAAGTGTTACC |
| 168 | 1420 | AAATCAGCTGAAGTGTTACCA |
| 169 | 1421 | AATCAGCTGAAGTGTTACCAA |
| 170 | 1430 | AAGTGTTACCAACTAGCCACA |
| 171 | 1440 | AACTAGCCACACCATGAATTG |
| 172 | 1488 | AAAACTGTGTAGCTACCTCAC |
| 173 | 1489 | AAACTGTGTAGCTACCTCACA |
| 174 | 1490 | AACTGTGTAGCTACCTCACAA |
| 175 | 1509 | AACCAGTCCTGTCTGTTTATA |
| 176 | 1592 | AATGGCAGTCTTGGCTTTAAA |
| 177 | 1653 | AAACAGTCCTTTAACCAGCAC |
| 178 | 1654 | AACAGTCCTTTAACCAGCACA |
| 179 | 1665 | AACCAGCACAGCCAGTTAAAA |
| 180 | 1682 | AAAAGATGCAGCCTCACTGCT |
| 181 | 1683 | AAAGATGCAGCCTCACTGCTT |
| 182 | 1684 | AAGATGCAGCCTCACTGCTTC |
| 183 | 1734 | AAACCTGGCACTTTACAAACA |
| 184 | 1735 | AACCTGGCACTTTACAAACAA |
| 185 | 1759 | AACATTGTTTTGTACTCACGG |
| 186 | 1816 | AAATACATTCTCCTGACCACT |
| 187 | 1817 | AATACATTCTCCTGACCACTA |
| 188 | 1837 | AATGGGAGCCAATTCACAATT |
| 189 | 1881 | AACTTGTGTAGACTAAGCATG |
| 190 | 1942 | AACCAACTTTAAAGTCAGTCC |
| 191 | 1946 | AACTTTAAAGTCAGTCCTGTG |
| 192 | 1952 | AAAGTCAGTCCTGTGTATACC |
| 193 | 1953 | AAGTCAGTCCTGTGTATACCT |
| 194 | 2081 | AAAGGAATCTCTCAGGGCAAG |
| 195 | 2082 | AAGGAATCTCTCAGGGGAAGG |
| 196 | 2086 | AATCTCTCAGGGCAAGGAGCT |
| 197 | 2198 | AACGTCTGGTTGATGAGAAAA |
| 198 | 2227 | AAGAGTTTTCATATGTGGGAG |
| 199 | 2288 | AATGATCCACCTAAGATCTTG |
| 200 | 2317 | AAGTGGTGAAATCAACTAGAG |
| 201 | 2325 | AAATCAACTAGAGGTGGTTCC |
| 202 | 2326 | AATCAACTAGAGGTGGTTCCT |
| 203 | 2330 | AACTAGAGGTGGTTCCTACAA |

As is known in the art, target RNA sequences used to design antisense oligonucleotides that have been shown to be efficacious in decreasing the expression of a target gene can also be used for the design of siRNA molecules. Accordingly, in another embodiment of the present invention, siRNA molecules are designed based on the target sequence of known ribonucleotides reductase antisense oligonucleotides. The sequences provided in Table 5 correspond to the sequences of antisense oligonucleotides that have been demonstrated to be efficacious in inhibiting the expression of human ribonucleotide reductase R2. The complementary sequences of these antisense oligonucleotides, therefore, constitute suitable target mRNA sequences that may be used to design siRNA molecules of the present invention. Similarly all or a portion of these sequences can be employed as the specific antisense sequence comprised by the siRNA molecule of the present invention.

**Table 3: Sequences of Known Antisense Oligonucleotides Against Human Ribonucleotide Reductase R1**

| **SEQ ID No** | **Name** | **Sequence 5' - 3'** |
|---|---|---|
| 205 | AS-I-35-20 | GTT CCA GCC AGA CAG CAC TT |
| 206 | AS-I-37-20 | GAG TTC CAG CCA GAC AGC AC |
| 207 | AS-I-85-20 | CAG AGT GGG AAG GGT TAG GT |
| 208 | AS-I-91-20 | AGG TGA CAG AGT GGG AAG GG |
| 209 | AS-I-129-20 | GAC TGG ACT GCG GCT CTA AA |
| 210 | AS-I-203-20 | ATG ACT CGT TCT TGG CGG CC |
| 211 | AS-I-239-20 | CAA AGC TTC TGG ATT CGA GA |
| 212 | AS-I-287-20 | TTC ATG GTG ATC TGA GCA GG |
| 213 | AS-I-300-20 | GCC TTG GAT TAC TTT CAT GG |
| 214 | AS-I-348-20 | TTC AGC AGC CAA AGT ATC TA |
| 215 | AS-I-395-20 | GCC AGG ATA GCA TAG TCA GG |
| 216 | AS-I-439-20 | CTT TCT TTG TTT CTT TGT GC |
| 217 | AS-I-504-20 | GGG AGA GTG TTT GCC ATT AT |
| 218 | AS-I-520-20 | TTG ACT TGG CCA CCA TGG GA |
| 219 | AS-I-540-20 | GGC CAG AAC AAT ATC CAA TG |
| 220 | AS-I-556-20 | TCA GGC GAT CTT TAT TGG CC |
| 221 | AS-I-635-20 | TTC AAC AAA TAA GAC CGC TC |
| 222 | AS-I-653-20 | TTT CAG CCC CTT TTC CAT TG |
| 223 | AS-I-662-20 | GGT CTT TCA GCC ACT TTT CC |
| 224 | AS-I-782-20 | TTG AAG AGA GTG GGC GAA GC |
| 225 | AS-I-786-20 | AGC ATT GAA GAG AGT GGG CG |
| 226 | AS-I-809-20 | GAA AGT TGC GGG CGG TTG GT |
| 227 | AS-I-843-20 | GCT GTC ATC TTT CAT ACT CA |
| 228 | AS-I-908-20 | CCA ATT CCT CCA GCA GAC TT |
| 229 | AS-I-923-20 | CAA CTC ACA GCA ACA CCA AT |
| 230 | AS-I-932-20 | GCC CGA ATA CAA CTC ACA GC |
| 231 | AS-I-967-20 | AAT TGC CAT TAG TCC CAG CA |
| 232 | AS-I-1051-20 | ATG CCC CAG GAC GCT TGT TC |
| 233 | AS-I-1074-20 | CCA AGG CTC CAG GTA AAT AG |
| 234 | AS-I-1134-20 | ACG CTG CTC TTC CTT TCC TG |
| 235 | AS-I-1162-20 | TCC AAA GAG CAA AGA AAA GA |
| 236 | AS-I-1258-20 | CCT CTC CCC AAA CCT CAT CC |
| 237 | AS-I-1311-20 | AAC TTT GCG GAC ACG ACC TT |
| 238 | AS-I-1370-20 | GGG GTG CCT GTT TCC GTC TG |
| 239 | AS-I-1418-20 | TTC TGC TGG TTG CTC TTT CG |
| 240 | AS-I-1421-20 | AGG TTC TGC TGG TTG CTC TT |
| 241 | AS-I-1513-20 | GGG CCA GGG AAG CCA AAT TA |
| 242 | AS-I-1662-20 | GGG GCG ATG GCG TTT ATT TG |
| 243 | AS-I-1666-20 | CAA TGG GGC GAT GGC GTT TA |
| 244 | AS-I-1785-20 | TTC CAG AGC ACC ATA ATA AA |
| 245 | AS-I-1818-20 | TGG GCC CTG CTC CTT GGC AA |
| 246 | AS-I-1970-20 | GGC ATC GGG GCA ATA AGT AA |
| 247 | AS-I-1976-20 | GCT GTA GGC ATC GGG GCA AT |
| 248 | AS-I-2119-20 | CAT GCC ATA GGC CCC GCT CG |
| 249 | AS-I-2198-20 | AGT TGC TTC AGG TCA TCA GG |
| 250 | AS-I-2251-20 | CAG CTG CCA TCT TGA GAA CA |
| 251 | AS-I-2304-20 | CTC AGC AAT GTG GAT GTT CA |
| 252 | AS-I-2364-20 | AGT CTT CAA ACC CTG CTT CC |
| 253 | AS-I-2370-20 | CAT CCC AGT CTT CAA ACC CT |
| 254 | AS-I-2414-20 | GTG AAC TGG ATT GGA TTAGC |
| 255 | AS-I-2491-20 | TGG CTG CTG TGT TCC TCT CC |
| 256 | AS-I-2556-20 | CTT CCA AGT CTT TCC TCA GG |
| 257 | AS-I-2629-20 | TAC CAC CTC AAG CAA ACC CA |
| 258 | AS-I-2650-20 | CAA CAG GGT CCA GCA AAG CC |
| 259 | AS-I-2769-20 | TCC GTT TTT TTT TTC TTT TT |
| 260 | AS-I-2863-20 | TGC TAA ATG GGT GAT GAA AC |
| 261 | AS-I-2922-20 | CCC ACC AGT CAA AGC AGT AA |
| 262 | AS-I-2594-20 | CTC AAG AAG TAG TTT GGC TA |

**Table 4: Sequences of Known Antisense Oligonucleotides Against Human Ribonucleotide Reductase R1**

| **SEQ ID NO** | **Name** | **Sequence 5' - 3'** |
|---|---|---|
| 263 | AS-I-3-20 | AGG CGC AAC AAT CCA AAT CC |
| 264 | AS-I-19-20 | ACT TTC TTC AGA GCA GAG GC |
| 265 | AS-I-55-20 | GCT CAG GGG AAA GAA CTG GA |
| 266 | AS-I-73-20 | GGT TAG GTT CCA GGC GTT GC |
| 267 | AS-I-158-20 | GCT AGT GGC TGA GGC TCT GA |
| 268 | AS-I-329-20 | AGT TCC ACT GTG GTG ACC CC |
| 269 | AS-I-378-20 | AGG GTG CTT AGT AGT CAA GG |
| 270 | AS-I-420-20 | CAA GTT AGA GAC AGC GAT CC |
| 271 | AS-I-492-20 | GCC ATT ATG TGG ATT TAT GT |
| 272 | AS-I-578-20 | CGG TCA TAG ATA ATA GCA GA |
| 273 | AS-I-603-20 | GCC GAA GTA ATT GTA AGA GA |
| 274 | AS-I-618-20 | CTC TAG CGT CTT AAA GCC GA |
| 275 | AS-I-720-20 | TGC TGC ATC AAT GTC TTC TT |
| 276 | AS-I-758-20 | GTA AAC CAC CTC TCA GAA AG |
| 277 | AS-I-808-20 | AAA GTT GCG GGC GGT TGG TA |
| 278 | AS-I-863-20 | GTG TCA TAA ATG CCT TCA AT |
| 279 | AS-I-941-20 | CTG CCA GTA GCC CGA ATA CA |
| 280 | AS-I-996-20 | TAC TCT CAG CAT CGG TAC AA |
| 281 | AS-I-1057-20 | TAG CAA ATG CCC CAG GAC GC |
| 282 | AS-I-1083-20 | GTC TAA ATG CCA AGG CTC CA |
| 283 | AS-I-1135-20 | CAC GCT GCT CTT CCT TTC CT |
| 284 | AS-I-1235-20 | CCA GGA CAC TCA TTT GGA CA |
| 285 | AS-I-1298-20 | CGA CCT TGT TTC TCA TAA CT |
| 286 | AS-I-1319-20 | GCT TTT ACA ACT TTG CGG AC |
| 287 | AS-I-1351-20 | GAG ACT CAA TGA TGG CAT AC |
| 288 | AS-I-1441-20 | TGC TGC ATT TGA TGG TTC CC |
| 289 | AS-I-1483-20 | CCT CAT CTT TGC TGG TGT AC |
| 290 | AS-I-1570-20 | TGA CTT CAG CCA ACT TCT TA |
| 291 | AS-I-1599-20 | TTT ATT CAA GTT TCG GAC AA |
| 292 | AS-I-1636-20 | ATG CCT CTG GTA CAG GAT AG |
| 293 | AS-I-1661-20 | GGG CGA TGG CGT TTA TTT GA |
| 294 | AS-I-1685-20 | AGA CCT TGT ACC CCA ATT CC |
| 295 | AS-I-1704-20 | CAG GAT AAA AGC ATC TGC CA |
| 296 | AS-I-1721-20 | TCA AAA GGG TAT CTC ATC AG |
| 297 | AS-I-1839-20 | AGA GCC CTC ATA GGT TTC GT |
| 298 | AS-I-1840-20 | GAG AGC CCT CAT AGG TTT CG |
| 299 | AS-I-1900-20 | CCC ATA GGT CTG TAG GAG TA |
| 300 | AS-I-2004-20 | ATT ATT CCC CAG GAT CTG AG |
| 301 | AS-I-2034-20 | GAT GTT GCT GGT GTA AGG TT |
| 302 | AS-I-2060-20 | TCT CCT GAC AAG ACT CTG CG |
| 303 | AS-I-2220-20 | GAT TTC CCA CAC AGT TTT AT |
| 304 | AS-I-2324-20 | GTG AGT TTG CCA TAG TTA GG |
| 305 | AS-I-2358-20 | CAA ACC.CTG CTT CCA GCC GT |
| 306 | AS-I-2390-20 | GGT CTC GTC CTT AAA TAA TA |
| 307 | AS-I-2584-20 | AGT TTG GCT ACT GAA GAC AT |
| 308 | AS-I-2669-20 | CAA TTA CTC CTT TTG CCT GC |
| 309 | AS-I-2831-20 | TCC CTG TAT GCA AGA TGA CT |
| 310 | AS-I-2924-20 | CCC ACC AGT CAA AGC AGT AA |
| 311 | AS-I-2986-20 | CCA GAT AAA GGT CCT ATC AG |

**Table 5: Sequences of Known Antisense Oligonucleotides Against Human Ribonucleotide Reductase R2**

| **SEQ ID NO** | **Name** | **Sequence 5 - 3'** |
|---|---|---|
| 312 | AS-II-6-20 | ACCCTTCCCATTGGCTGCGC |
| 313 | AS-II-13-20 | GsCCsTCCGsACCsCTTCsCCsATTsG |
| 314 | AS-II-14-20 | TGCCTCCGACCCTTCCCATT |
| 315 | AS-II-16-18 | TGCCTCCGACCCTTCCCA |
| 316 | AS-II-75-20 | CsGCGsCGCsTCCsCGGsCCCsTTCsC |
| 317 | AS-II-75-20 | CGCGCGCTCCCGGCCCTTCC |
| 318 | AS-II-79-14 | CGCGCTCCCGGCCC |
| 319 | AS-II-109-20 | CsCCCsTCACsTCCsAGCsAGCsCTsT |
| 320 | AS-II-110-20 | ACCCCTCACTCCAGCAGCCT |
| 321 | AS-II-114-20 | GGCGACCCCTCACTCCAGCA |
| 322 | AS-II-127-12 | GCACGGGCGACC |
| 323 | AS-II-130-20 | TGGGACAGGGTGCACGGGCG |
| 324 | AS-II-134-20 | GACGGCTGGGACAGGGTGCA |
| 325 | AS-II-151-20 | GAGCAGCCAGGACAGGACGG |
| 326 | AS-II-163-20 | GsCGsAAGsCAGsAGCsGAGsCAGCsC |
| 327 | AS-II-166-20 | GCAGCGAAGCAGAGCGAGCA |
| 328 | AS-II-185-20 | GGGAGAGCATAGTGGAGGCG |
| 329 | AS-II-189-20 | CGGAGGGAGAGCATAGTGGA |
| 330 | AS-II-201-20 | GCGAGCGGGACACGGAGGGA |
| 331 | AS-II-217-20 | CGGGTCCGTGATGGGCGCGA |
| 332 | AS-II-225-20 | AGCTGCTGCGGGTCCGTGAT |
| 333 | AS-II-253-14 | CCCCTTCAGCGGCG |
| 334 | AS-II-280-20 | CGGCGGCGTGTTCTCCTTGT |
| 335 | AS-II-288-12 | CGGCGGCGTGTT |
| 336 | AS-II-323-20 | TCCTCGCGGTCTTGCTGGCC |
| 337 | AS-II-344-20 | CCGTGGGCTCCTGGAAGATC |
| 338 | AS-II-362-20 | CTGCTTTAGTTTTCGGCTCC |
| 339 | AS-II-391-17 | CGGCTCATCCTCCACGC |
| 340 | AS-II-404-20 | GGTTTTCTCTCAGCAGCGGC |
| 341 | AS-II-412-20 | GCGGCGGGGGTTTTCTCTCA |
| 342 | AS-II-414-20 | AAGCGGCGGGGGTTTTCTCT |
| 343 | AS-II-425-20 | GGAAGATGACAAAGCGGCGG |
| 344 | AS-II-439-20 | ATGGTACTCGATGGGGAAGA |
| 345 | AS-II-472-20 | AGCCTCTGCCTTCTTATACA |
| 346 | AS-II-494-20 | CCTCCTCGGCGGTCCAAAAG |
| 347 | AS-II-496-16 | TCCTCGGCGGTCCAAA |
| 348 | AS-II-549-20 | TATCTCTCCTCGGGTTTCAG |
| 349 | AS-II-579-20 | GCAAAGAAAGCCAGAACATG |
| 350 | AS-II-619-20 | TCGCTCCACCAAGTTTTCAT |
| 351 | AS-II-626-20 | GGCTAAATCGCTCCACCAAG |
| 352 | AS-II-634-20 | AACTTCTTGGCTAAATCGCT |
| 353 | AS-II-667-20 | GAAGCCATAGAAACAGCGGG |
| 354 | AS-II-784-20 | GACACAAGGCATCGTTTCAA |
| 355 | AS-II-798-20 | TCTGCCTTCTTCTTGACACA |
| 356 | AS-II-816-20 | ATCCAGCGCAAGGCCCAGTC |
| 357 | AS-II-861-20 | GCAAAGGCTACAACACGTTC |
| 358 | AS-II-890-20 | AACCGGAAAAGAAAATGCCT |
| 359 | AS-II-909-20 | CAGAATATCGACGCAAAAGA |
| 360 | AS-II-933-20 | GGCATCAGTCCTCGTTTCTT |
| 361 | AS-II-981-20 | TGTAAACCCTCATCTCTGCT |
| 362 | AS-II-1001-20 | TCAGGCAAGCAAAATCACAG |
| 363 | AS-II-1006-20 | GAACATCAGGCAAGCAAAAT |
| 364 | AS-II-1023-20 | TTGTGTACCAGGTGTTTGAA |
| 365 | AS-II-1040-20 | CTCTCTCCTCCGATGGTTTG |
| 366 | AS-II-1048-20 | TTCTCTTACTCTCTCCTCCG |
| 367 | AS-II-1144-20 | GTATTGCTTCATTAGAGTGC |
| 368 | AS-II-1182-20 | CCCAGTTCCAGCATAAGTCT |
| 369 | AS-II-1197-20 | AAAACCTTGCTAAAACCCAG |
| 370 | AS-II-1217-20 | CAAATGGGTTCTCTACTCTG |
| 371 | AS-II-1224-20 | ATAAAGTCAAATGGGTTCTC |
| 372 | AS-II-1254-20 | TTAGTCTTTCCTTCCAGTGA |
| 373 | AS-II-1278-20 | TCGCCTACTCTCTTCTCAAA |
| 374 | AS-II-1288-20 | CCTCTGATACTCGCCTACTC |
| 375 | AS-II-1302-20 | GACATCACTCCCATCCTCTG |
| 376 | AS-II-1335-20 | GCATCCAAGGTAAAAGAATT |
| 377 | AS-II-1338-20 | TCAGCATCCAAGGTAAAAGA |
| 378 | AS-II-1342-20 | GAAGTCAGCATCCAAGGTAA |
| 379 | AS-II-1345-20 | TTAGAAGTCAGCATCCAAGG |
| 380 | AS-II-1362-20 | GCACATCTTCAGTTCATTTA |
| 381 | AS-II-1364-20 | GGGCACATCTTCAGTTCATT |
| 382 | AS-II-1381-20 | AAAAATCAGCCAAGTAAGGG |
| 383 | AS-II-1390-20 | ATGGAAAAAAAAAATCAGCC |
| 384 | AS-II-1438-20 | TTCATGGTGTGGCTAGTTGG |
| 385 | AS-II-1499-20 | AGGACTGGTTGTGAGGTAGC |
| 386 | AS-II-1517-20 | CCAGCACTATAAACAGACAG |
| 387 | AS-II-1538-20 | TTCTGGCAAAAGGTGATACT |
| 388 | AS-II-1560-20 | GTAAGTCACAGCCAGCCAGG |
| 389 | AS-II-1581-20 | ACTGCCATTGTCACTGCTAT |
| 390 | AS-II-1659-20 | TGGCTGTGCTGGTTAAAGGA |
| 391 | AS-II-1666-20 | TTTTAACTGGCTGTGCTGGT |
| 392 | AS-II-1700-20 | ATTAAAATCTGCGTTGAAGC |
| 393 | AS-II-1768-20 | TATCGCCGCCGTGAGTACAA |
| 394 | AS-II-1773-20 | GCTATTATCGCCGCCGTGAG |
| 395 | AS-II-1775-12 | ATCGCCGCCGTG |
| 396 | AS-II-1790-20 | GAAACCAAATAATCAAGCT |
| 397 | AS-II-1819-20 | TTAGTGGTCAGGAGAATGTA |
| 398 | AS-II-1976-20 | TGGCACCAACTGACTAATAT |
| 399 | AS-II-1989-20 | CCTGTCTTCTATCTGGCACC |
| 400 | AS-II-2009-20 | GCCACAGGATAAAAACACAA |
| 401 | AS-II-2026-20 | CCCAGGACACTACACAAGCC |
| 402 | AS-II-2044-20 | TCAGAGGGGGCAGAGAATCC |
| 403 | AS-II-2067-20 | TCCTTTATCCCACAACACTC |
| 404 | AS-II-2083-20 | CCTTGCCCTGAGAGATTCCT |
| 405 | AS-II-2083-20 | CsCTsTGsCCsCTsGAsGAsGAsTTsCCsT |
| 406 | As-II-2128-20 | GGCCCAGATCACCCCTAAAT |
| 407 | AS-II-2151-20 | AAACGGCTTCTCACACATAT |
| 408 | AS-II-2164-20 | GAGATATAAAATGAAACGGC |
| 409 | AS-II-2182-20 | CGTTGAGGAAAATACAGTGA |
| 410 | AS-II-2229A-20 | GCTCCCACATATGAAAACTC |
| 411 | AS-II-2372-20 | CACACAACCTACTTACACCA |

| | | |
|---|---|---|
| Footnotes: Name includes the following: AS = antisense; II = R2; the first number indicates the first nucleotide position in the R2 mRNA sequence; the second number indicates the length of the sequence segment. Sequences were fully thioated unless partial thioation is indicated (s). | | |

In another embodiment, the ribonucleotide reductase R2 target mRNA sequence is selected from the group of SEQ ID NOs: 126 to 130, 142 to 144, 147 to 150 and 161 to 203 of SEQ ID NOs: 348, 349, 354 to 360 and 370 to 411.

### Design of siRNA Molecules

Following selection of an appropriate target mRNA sequence, siRNA molecules that comprise a nucleotide sequence complementary to all or a portion of the target sequence, *i.e*. an antisense sequence, can be designed and prepared. As indicated above, the siRNA molecule can be double stranded (i.e. a dsRNA molecule comprising an antisense strand and a complementary sense strand) or single-stranded (i.e. a ssRNA molecule comprising just an antisense strand). The siRNA molecules can comprise a duplex, asymmetric duplex, hairpin or asymmetric hairpin secondary structure, having self-complementary sense and antisense strands.

The siRNA molecule can be a double-stranded RNA (dsRNA) comprising two separate complementary RNA strands. The RNA strands of the dsRNA may be the same length in nucleotides, or may be different in length. In one embodiment, the siRNA is a dsRNA. In another embodiment, the siRNA is a dsRNA wherein both RNA strands are the same length.

The dsRNA molecules of the present invention also include siRNA molecules assembled from a single oligonucleotide in a stem-loop structure, wherein self-complementary sense and antisense regions of the siRNA molecule are linked by means of a nucleic acid based or non-nucleic acid-based linker(s), as well as circular single-stranded RNA having two or more loop structures and a stem comprising self-complementary sense and antisense strands, wherein the circular RNA can be processed either *in vivo* or *in vitro* to generate an active siRNA molecule capable of mediating RNAi.

Small hairpin RNA (shRNA) molecules thus are also contemplated by the present invention. These molecules comprise a specific antisense sequence in addition to the reverse complement (sense) sequence, typically separated by a spacer or loop sequence. Cleavage of the spacer or loop provides a single-stranded RNA molecule and its reverse complement, such that they may anneal to form a dsRNA molecule (optionally with additional processing steps that may result in addition or removal of one, two, three or more nucleotides from the 3' end and/or the 5' end of either or both strands). The spacer can be of a sufficient length to permit the antisense and sense sequences to anneal and form a double-stranded structure (or stem) prior to cleavage of the spacer (and, optionally, subsequent processing steps that may result in addition or removal of one, two, three, four, or more nucleotides from the 3' end and/or the 5' end of either or both strands). The spacer sequence is typically an unrelated nucleotide sequence that is situated between two complementary nucleotide sequence regions which, when annealed into a double-stranded nucleic acid, comprise a shRNA (see, for example, Brummelkamp et al., 2002 Science 296:550; Paddison et al., 2002 Genes Develop. 16:948; Paul et al., Nat. Biotechnol. 20:505-508 (2002); Grabarek et al., BioTechniques 34:734-44 (2003)). The spacer sequence generally comprises between about 3 and about 100 nucleotides.

The ssRNA molecules according to the present invention are generally single-stranded RNA molecules with little or no secondary structure.

The overall length of the siRNA molecules of the present invention can vary from about 14 to about 200 nucleotides depending on the type of siRNA molecule being designed. Between about 14 and about 50 of these nucleotides are complementary to the mRNA target sequence, i.e. constitute the specific antisense sequence of the siRNA molecule. For example, when the siRNA molecule is a dsRNA or ssRNA molecule, the length can vary from about 14 to about 50 nucleotides, whereas when the siRNA is a shRNA or circular molecule, the length can vary from about 40 nucleotides to about 200 nucleotides.

In one embodiment of the present invention, the siRNA molecule is a dsRNA or ssRNA molecule between about 15 and about 40 nucleotides in length. In another embodiment, the siRNA molecule is a dsRNA or ssRNA molecule between about 15 and about 35 nucleotides in length. In another embodiment, the siRNA molecule is a dsRNA or ssRNA molecule between about 17 and about 30 nucleotides in length. In another embodiment, the siRNA molecule is a dsRNA or ssRNA molecule between about 19 and about 25 nucleotides in length. In another embodiment, the siRNA molecule is a dsRNA or ssRNA molecule between about 21 to about 23 nucleotides in length.

In an alternative embodiment, the siRNA molecule is a shRNA molecule or circular siRNA molecule between about 50 and about 100 nucleotides in length. In a further embodiment, the siRNA molecule is a shRNA molecule between about 50 to about 60 nucleotide in length.

As indicated above, the siRNA molecule comprises an antisense strand that includes a specific antisense sequence complementary to all or a portion of a target mRNA sequence. One skilled in the art will appreciate that the entire length of the antisense stand comprised by the siRNA molecule does not need to be complementary to the target sequence. Thus, the antisense strand of the siRNA molecules may comprise a specific antisense sequence together with nucleotide sequences at the 5' and/or 3' termini that are not complementary to the target sequence. Such non-complementary nucleotides may provide additional functionality to the siRNA molecule. For example, they may provide a restriction enzyme recognition sequence or a "tag" that facilitates detection, isolation or purification. Alternatively, the additional nucleotides may provide a self-complementary sequence that allows the siRNA to adopt a hairpin configuration. Such configurations are useful when the siRNA molecule is a shRNA molecule, as described above.

Accordingly, within its overall length of about 14 to about 200 nucleotides, the siRNA molecules of the present invention comprise a specific antisense sequence of between about 14 to about 50 nucleotides in length that is complementary to all or a portion of a selected target mRNA sequence. In one embodiment, the length of the specific antisense sequence is from about 14 to about 40 nucleotides. In another embodiment, the length of the specific antisense sequence is from about 14 to about 35 nucleotides. In another embodiment, the length of the specific antisense sequence is from about 14 to about 30 nucleotides. In another embodiment, the length of the specific antisense sequence is from about 15 to about 30 nucleotides. In a further embodiment, the length of the specific antisense sequence is from about 17 to about 30 nucleotides. In other embodiments, the length of the specific antisense sequence is from about 19 to about 25, from about 19 to about 23, and from about 21 to about 23 nucleotides.

In a further embodiment of the present invention, the siRNA molecules comprise a specific antisense sequence that is complementary to at least 14 consecutive nucleotides of any one of the sequences as set forth in SEQ ID NOs: 126 to 130, 142 to 144, 147 to 150 and 161 to 203.

In n further embodiment, the specific antisense sequence of the siRNA molecules comprises at least 14 consecutive nucleotides of any one of the sequences as set forth in SEQ ID NOs: 348, 349, 354 to 360 and 370 to 411.

The specific antisense sequence comprised by the siRNA molecule can be identical or substantially identical to the complement of the target sequence. In one embodiment of the present invention, the specific antisense sequence comprised by the siRNA molecule is at least about 75% identical to the complement of the target mRNA sequence. In another embodiment, the specific antisense sequence comprised by the siRNA molecule is at least about 90% identical to the complement of the target mRNA sequence. In a further embodiment, the specific antisense sequence comprised by the siRNA molecule is at least about 95% identical to the complement of the target mRNA sequence. In another embodiment, the specific antisense sequence comprised by the siRNA molecule is at least about 98% identical to the complement of the target mRNA sequence. Methods of determining sequence identity are known in the art and can be determined, for example, by using the BLASTN program of the University of Wisconsin Computer Group (GCG) software or provided on the NCBI website.

The specific antisense sequence of the siRNA molecules of the present invention may exhibit variability by differing (e.g. by nucleotide substitution, including transition or transversion) at one, two, three, four or more nucleotides from the sequence of the target mRNA. When such nucleotide substitutions are present in the antisense strand of a dsRNA molecule, the complementary nucleotide in the sense strand with which the substitute nucleotide would typically form hydrogen bond base-pairing may or may not be correspondingly substituted. dsRNA molecules in which one or more nucleotide substitution occurs in the sense sequence, but not in the antisense strand, are also contemplated by the present invention. When the antisense sequence of an siRNA molecule comprises one or more mismatches between the nucleotide sequence of the siRNA and the target nucleotide sequence, as described above, the mismatches may be found at the 3' terminus, the 5' terminus or in the central portion of the antisense sequence.

In another embodiment of the invention, the siRNA molecules comprise a specific antisense sequence that is capable of selectively hybridizing under stringent conditions to a portion of a naturally occurring target gene or target mRNA. Suitable stringent conditions include, for example, hybridization according to conventional hybridization procedures and washing conditions of 1-3 x SSC, 0.1-1% SDS, 50-70°C with a change of wash solution after about 5-30 minutes. As known to those of ordinary skill in the art, variations in stringency of hybridization conditions may be achieved by altering the time, temperature, and/or concentration of the solutions used for the hybridization and wash steps. Suitable conditions can also depend in part on the particular nucleotide sequences used, for example the sequence of the target mRNA or gene.

According to the present invention, siRNA molecules having a duplex or double-stranded structure, for example dsRNA or shRNA, can have blunt ends, or can have 3' and/or 5' overhangs. As used herein, "overhang" refers to the unpaired nucleotide or nucleotides that protrude from a duplex structure when a 3'-terminus of one RNA strand extends beyond the 5'-terminus of the other strand (3' overhang), or vice versa (5' overhang). The nucleotides comprising the overhang can be ribonucleotides, deoxyribonucleotides or modified versions thereof. In one embodiment, at least one strand of the siRNA molecule has a 3' overhang from about 1 to about 6 nucleotides in length. In other embodiments, the 3' overhang is from about 1 to about 5 nucleotides, from about 1 to about 3 nucleotides and from about 2 to about 4 nucleotides in length.

When the siRNA molecule comprises a 3' overhang at one end of the molecule, the other end can be blunt-ended or have also an overhang (5' or 3'). When the siRNA molecule comprises an overhang at both ends of the molecule, the length of the overhangs may be the same or different. In one embodiment, the siRNA molecule of the present invention comprises 3' overhangs of about 1 to about 3 nucleotides on both ends of the molecule. In a further embodiment, the siRNA molecule is a dsRNA having a 3' overhang of 2 nucleotides at both ends of the molecule. In yet another embodiment, the nucleotides comprising the overhang of the siRNA are TT dinucleotides or UU dinucleotides.

When determining the percentage identity of the siRNA molecule comprising one or more overhangs to the target mRNA sequence, the overhang(s) may or may not be taken into account. For example, the nucleotides from a 3' overhang and up to 2 nucleotides from the 5'- and/or 3'-terminus of the double strand may be modified without significant loss of activity of the siRNA molecule.

In the context of this invention, the term "RNA" refers to a nucleic acid molecule of one or more ribonucleotides in length, wherein the ribonucleotide(s) are naturally-occurring ribonucleotides or modified ribonucleotides. In general RNA refers to an oligomer or polymer of ribonucleotides. The siRNA molecules of the present invention, therefore, can be composed of naturally-occurring nucleobases, sugars and covalent internucleoside (backbone) linkages or can comprise one or more non-naturally-occurring nucleotides or linkages, which function similarly. Such modified siRNA molecules are often preferred over native forms because of desirable properties such as, for example, enhanced cellular uptake and/or increased bioavailability, enhanced affinity for nucleic acid target, and increased stability in the presence of nucleases. For example, certain chemical modifications can improve the bioavailability of the siRNA molecules by targeting particular cells or tissues and/or improving cellular uptake of the molecule. Typically, siRNA molecules comprising modified nucleotides and/or, linkages retain substantially the same activity as the unmodified molecule. However, it is also contemplated that the activity of a modified siRNA molecule may be reduced as compared to an unmodified siRNA molecule, but the overall activity of the modified siRNA molecule can be greater than that of the unmodified siRNA molecule due to improved stability and/or delivery of the molecule. Modified siRNA molecules can also minimise the possibility of activating interferon activity.

A modified siRNA molecule of the invention can comprise one or more modified nucleotides, for example, a siRNA molecule comprising modified ribonucleotide(s) can comprise about 5% to about 100% modified nucleotides (for example, 5%, 10%, 15%, 20%, 25%, 30%, 35%, 40%, 45%, 50%, 55%, 60%, 65%, 70%, 75%, 80%, 85%, 90%, 95% or 100% modified nucleotides). The actual percentage of modified nucleotides present in a given siRNA molecule will depend on the total number of nucleotides present in the siRNA. If the siRNA molecule is a ssRNA molecule, the percent modification will be based upon the total number of nucleotides present in the ssRNA molecule. When the siRNA molecule is a dsRNA molecule, the percent modification can be based upon the total number of nucleotides present in the sense strand, antisense strand, or both the sense and antisense strands of the molecule. In accordance with the present invention, a siRNA molecule that comprises one or more modified nucleotides or linkages maintains the ability to mediate RNAi and to inhibit expression of the target gene.

As is known in the art, a nucleoside is a base-sugar combination and a nucleotide is a nucleoside that further includes a phosphate group covalently linked to the sugar portion of the nucleoside. In forming RNA molecules, the phosphate groups covalently link adjacent nucleosides to one another to form a linear polymeric compound, with the normal linkage or backbone of RNA being a 3' to 5' phosphodiester linkage. Specific examples of siRNA molecules useful in this invention include siRNA molecules containing modified backbones or non-natural internucleoside linkages. As defined in this specification, siRNA molecules having modified backbones include both those that retain a phosphorus atom in the backbone and those that lack a phosphorus atom in the backbone.

Exemplary siRNA molecules having modified backbones include, for example, those with one or more modified internucleotide linkages that are phosphorothioates, chiral phosphorothioates, phosphorodithioates, phosphotriesters, aminoalkylphosphotriesters, methyl and other alkyl phosphonates including 3'-alkylene phosphonates and chiral phosphonates, phosphinates, phosphoramidates including 3'amino phosphoramidate and aminoalkylphosphoramidates, thionophosphoramidates, thionoalkylphosphonates, thionoalkylphosphotriesters, and boranophosphates having normal 3'-5' linkages, 2'-5' linked analogs of these, and those having inverted polarity wherein the adjacent pairs of nucleoside units are linked 3'-5' to 5'-3' or 2'-5' to 5'-2'. Various salts, mixed salts and free acid forms are also included. The modified linkages can link one or more of the nucleotides comprised by the siRNA, for example, the modified linkages can link the four, five or six 3'-terminal nucleotides of the siRNA molecule. Alternatively, In a further embodiment, the modified linkages can link all the nucleotides of the siRNA molecule.

Exemplary modified RNA backbones that do not include a phosphorus atom are formed by short chain alkyl or cycloalkyl internucleoside linkages, mixed heteroatom and alkyl or cycloalkyl internucleoside linkages, or one or more short chain heteroatomic or heterocyclic internucleoside linkages. Such backbones include morpholino linkages (formed in part from the sugar portion of a nucleoside); siloxane backbones; sulfide, sulfoxide and sulphone backbones; formacetyl and thioformacetyl backbones; methylene formacetyl and thioformacetyl backbones; alkene containing backbones; sulphamate backbones; methyleneimino and methylenehydrazino backbones; sulphonate and sulfonamide backbones; amide backbones; and others having mixed N, O, S and CH₂ component parts.

The siRNA molecules can also comprise one or more universal base. The term "universal base," as used herein, refers to a nucleotide analogue that forms base pairs with each of the natural DNA/RNA bases with little discrimination between them.

Non-limiting examples of universal bases include inosine, azole carboxamides, and nitroazole derivatives such as 3-nitropyrrole, 4-nitroindole, 5-nitroindole, and 6-nitroindole (see, for example, Loakes, 2001, Nucleic Acids Research, 29, 2437-2447).

The siRNA molecules can comprise one or more 5' and/or 3'-cap structure. The siRNA molecule can comprise a cap structure at the 3'-end of the sense strand, the antisense strand, or both the sense and antisense strands; or at the 5'-end of the sense strand, the antisense strand, or both the sense and antisense strands of the siRNA molecule. Alternatively, the siRNA molecule can comprise a cap structure at both the 3'-end and 5'-end of the siRNA molecule. The term "cap structure" refers to a chemical modification incorporated at either terminus of an oligonucleotide (see, for example, U.S. Patent No. 5,998,203), which protects the molecule from exonuclease degradation, and may also facilitate delivery and/or localisation within a cell.

Examples of suitable 5'-cap structures include, but are not limited to, glyceryl, inverted deoxy abasic residue; 4',5'-methylene nucleotide; 1-(beta-D-erythrofuranosyl) nucleotide; 4'-thio nucleotide; carbocyclic nucleotide; 1,5-anhydrohexitol nucleotide; L-nucleotides; alpha-nucleotides; modified base nucleotide; phosphorodithioate linkage; threo-pentofuranosyl nucleotide; acyclic 3',4'-seco nucleotide; acyclic 3,4-dihydroxybutyl nucleotide; acyclic 3,5-dihydroxypentyl nucleotide, 3'-3'-inverted nucleotide moiety; 3'-3'-inverted abasic moiety; 3'-2'-inverted nucleotide moiety; 3'-2'-inverted abasic moiety; 1,4-butanediol phosphate; 3'-phosphoramidate; hexylphosphate; aminohexyl phosphate; 3'-phosphate; 3'-phosphorothioate; phosphorodithioate; and bridging or non-bridging methylphosphonate moieties.

Examples of suitable 3'-cap structures include, but are not limited to, glyceryl, inverted deoxy abasic residue; 4',5'-methylene nucleotide; 1-(beta-D-erythrofuranosyl) nucleotide; 4'-thio nucleotide, carbocyclic nucleotide; 5'-amino-alkyl phosphate; 1,3-diamino-2-propyl phosphate; 3-aminopropyl phosphate; 6-aminohexyl phosphate; 1,2-aminododecyl phosphate; hydroxypropyl phosphate; 1,5-anhydrohexitol nucleotide; L-nucleotide; alpha-nucleotide; modified base nucleotide; phosphorodithioate; threo-pentofuranosyl nucleotide; acyclic 3',4'-seco nucleotide; 3,4-dihydroxybutyl nucleotide; 3,5-dihydroxypentyl nucleotide, 5'-5'-inverted nucleotide moiety; 5'-5'-inverted abasic moiety; 5'-phosphoramidate; 5'-phosphorothioate; 1,4-butanediol phosphate; 5'-amino; bridging and/or non-bridging 5'-phosphoramidate, phosphorothioate and/or phosphorodithioate, bridging or non bridging methylphosphonate and 5'-mercapto moieties (see, for example, Beaucage and Iyer, 1993, Tetrahedron 49, 1925).

The term "abasic residue" refers to a nucleotide comprising a sugar moiety lacking a base or having another chemical group in place of a base at the 1' position (see, for example, U.S. Patent No. 5,998,203).

The present invention also contemplates ribonucleotide mimetics in which both the sugar and the internucleoside linkage of the nucleotide units are replaced with novel groups. The base units are maintained for hybridisation with an appropriate nucleic acid target compound. An example of such a mimetic, which has been shown to have excellent hybridisation properties, is a peptide nucleic acid (PNA) [Nielsen et al., Science, 254:1497-1500 (1991)]. In PNA compounds, the sugar-backbone of an oligonucleotide is replaced with an amide containing backbone, in particular an aminoethylglycine backbone. The nucleobases are retained and are bound directly or indirectly to aza-nitrogen atoms of the amide portion of the backbone.

The present invention also contemplates siRNA molecules comprising "locket nucleic acids" (LNAs), which are novel conformationally restricted nucleic acid analogues containing a methylene bridge that connects the 2'-O of ribose with the 4'-C (see, Singh et al., Chem. Commun., 1998, 4:455-456). LNA and LNA analogues display very high duplex thermal stabilities with complementary DNA and RNA, stability towards 3'-exonuclease degradation, and good solubility properties. Synthesis of the LNA analogues of adenine, cytosine, guanine, 5-methylcytosine, thymine and uracil, their oligomerization, and nucleic acid recognition properties have been described (see Koshkin et al., Tetrahedron, 1998, 54:3607-3630). Studies of mismatched sequences show that LNA obey the Watson-Crick base-pairing rules with generally improved selectivity compared to the corresponding unmodified reference strands.

LNAs form duplexes with complementary DNA or RNA or with complementary LNA, with high thermal affinities. The universality of LNA-mediated hybridization has been emphasized by the formation of exceedingly stable LNA:LNA duplexes (Koshkin et al., J. Am. Chem. Soc., 1998, 120:13252-13253). LNA:LNA hybridization was shown to be the most thermally stable nucleic acid type duplex system, and the RNA-mimicking character of LNA was established at the duplex level. Introduction of three LNA monomers (T or A) resulted in significantly increased melting points toward DNA complements.

Synthesis of 2'-amino-LNA (Singh et al., J. Org. Chem., 1998, 63, 10035-10039) and 2'-methylamino-LNA has been described and thermal stability of their duplexes with complementary RNA and DNA strands reported. Preparation of phosphorothioate-LNA and 2'-thio-LNA have also been described (Kumar et al., Bioorg. Med. Chem. Lett., 1998, 8:2219-2222).

Modified siRNA molecules may also contain one or more substituted sugar moieties. For example, siRNA molecules may comprise sugars with one of the following substituents at the 2' position: OH; F; O-, S-, or N-alkyl; O-, S-, or N-alkenyl; O-, S- or N-alkynyl; or O-alkyl-O-alkyl, wherein the alkyl, alkenyl and alkynyl may be substituted or unsubstituted C₁ to C₁₀ alkyl or C₂ to C₁₀ alkenyl and alkynyl. Examples of such groups are: O[(C)₂)ₙO]ₘCH₃, O(CH₂)ₙOCH₃, O(CH₂)ₙNH₂, O(CH₂)ₙCH₃, O(CH₂)ₙ ONH₂, and O(CH₂)ₙON[(CH₂)ₙCH₃)]₂, where n and m are from 1 to about 10. Alternatively, the siRNA molecules may comprise one of the following substituents at the 2' position: C₁ to C₁₀ lower alkyl, substituted lower alkyl, alkaryl, aralkyl, O-alkaryl or O-aralkyl, SH, SCH₃, OCN, Cl, Br, CN, CF₃, OCF₃, SOCH₃, SO₂ CH₃, ONO₂, NO₂, N₃, NH₂, heterocycloalkyl, heterocycloalkaryl, aminoalkylamino, polyalkylamino, substituted silyl, an RNA cleaving group, a reporter group, an intercalator, a group for improving the pharmacokinetic properties of a siRNA molecule, or a group for improving the pharmacodynamic properties of a siRNA molecule, and other substituents having similar properties. Specific examples include 2'-methoxyethoxy (2'-O-CH₂ CH₂ OCH₃, also known as 2'-O-(2-methoxyethyl) or 2'-MOE) [Martin et al., Helv. Chim. Acta, 78:486-504(1995)], 2'-dimethylaminooxyethoxy (O(CH₂)₂ ON(CH₃)₂ group, also known as 2'-DMAOE), 2'-methoxy (2'-O--CH₃), 2'-aminopiopoxy (2'-OCH₂ CH₂ CH₂ NH₂) and 2'-fluoro (2'-F).

Similar modifications may also be made at other positions on the siRNA molecule, particularly the 3' position of the sugar on the 3' terminal nucleotide or in 2'-5' linked siRNA molecules and the 5' position of 5' terminal nucleotide. siRNA molecules may also comprise sugar mimetics such as cyclobutyl moieties in place of the pentofuranosyl sugar.

siRNA molecules may also include modifications or substitutions to the nucleobase. As used herein, "unmodified" or "natural" nucleobases include the purine bases adenine (A) and guanine (G), and the pyrimidine bases thymine (T), cytosine (C) and uracil (U). Modified nucleobases include other synthetic and natural nucleobases such as 5-methylcytosine (5-me-C), 5- hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 8-azaguanine and 8-azaadenine, 7-deazaguanine and 7-deazaadenine and 3-deazaguanine and 3-deazaadenine. Further nucleobases include those described in U.S. Patent No. 3,687,808; The Concise Encyclopedia Of Polymer Science And Engineering, (1990) pp 858-859, Kroschwitz, J. I., ed. John Wiley & Sons; Englisch et al., Angewandte Chemie, Int. Ed., 30:613 (1991); and Sanghvi, Y. S., (1993) Antisense Research and Applications, pp 289-302, Crooke, S. T. and Lebleu, B., ed., CRC Press. Certain of these nucleobases are particularly useful for increasing the binding affinity of oligonucleotides. These include 5-substituted pyrimidines, 6-azapyrimidines and N-2, N-6 and O-6 substituted purines, including 2-aminopropyladenine, 5-propynyluracil and 5-propynylcytosine. 5-methylcytosine substitutions have been shown to increase nucleic acid duplex stability by 0.6-1.2°C [Sanghvi, Y. S., (1993) Antisense Research and Applications, pp 276-278, Crooke, S. T. and Lebleu, B., ed., CRC Press, Boca Raton].

Another modification applicable to the siRNA molecules of the present invention is the chemical linkage to the siRNA molecule of one or more moieties or conjugates which enhance the activity, cellular distribution, cellular uptake, bioavailability, pharmacokinetic properties and/or stability of the siRNA molecule. Such moieties include, but are not limited to, lipid moieties such as a cholesterol moiety [Letsinger et al., Proc. Natl. Acad. Sci. USA, 86:6553-6556 (1989)], cholic acid [Manoharan et al., Bioorg. Med. Chem. Let., 4:1053-1060 (1994)], a thioether, e.g. hexyl-S-tritylthiol [Manoharan et al., Ann. N.Y. Acad. Sci., 660:306-309 (1992); Manoharan et al., Bioorg. Med. Chem. Lett., 3:2765-2770 (1993)], a thiocholesterol [Oberhauser et al., Nucl. Acids Res., 20:533-538 (1992)], an aliphatic chain, e.g. dodecandiol or undecyl residues [Saison-Behmoaras et al., EMBO J., 10:1111-1118 (1991); Kabanov et al., FEBS Lett., 259:327-330 (1990); Svinarchuk et al., Biochimie, 75:49-54 (1993)], a phospholipid, e.g. di-hexadecyl-rac-glycerol or triethylammonium 1,2-di-O-hexadecyl-rac-glycero-3-H-phosphonate [Manoharan et al., Tetrahedron Lett., 36:3651-3654 (1995); Shea et al., Nucl. Acids Res., 18:3777-3783 (1990)], a polyamine or a polyethylene glycol chain [Manoharan et al., Nucleosides & Nucleotides, 14:969-973 (1995)], or adamantane acetic acid [Manoharan et al., Tetrahedron Lett., 36:3651-3654 (1995)], a palmityl moiety [Mishra et al., Biochim. Biophys. Acta, 1264:229-237 (1995)], or an octadecylamine or hexylamino-carbonyl-oxycholesterol moiety [Crooke et al., J. Pharmacol. Exp. Ther., 277:923-937 (1996)]. The conjugate molecule can be linked to the siRNA molecule by way of a linker, for example, via a biodegradable linker. The conjugate molecule can be attached at the 3'-end of the sense strand, the antisense strand, or both the sense and antisense strands of the siRNA molecule. Alternatively, the conjugate molecule can be attached at the 5'-end of the sense strand, the antisense strand, or both the sense and antisense strands of the siRNA molecule. It is also contemplated that a conjugate molecule can be attached at both the 3'-end and 5'-end of the siRNA molecule. When more than one conjugate molecule is attached to the siRNA molecule, the conjugate molecules can be the same or different.

One skilled in the art will recognise that it is not necessary for all positions in a given siRNA molecule to be uniformly modified. The present invention, therefore, contemplates the incorporation of more than one of the aforementioned modifications into a single siRNA molecule, or even at a single nucleoside within the siRNA molecule.

In the context of the present invention, a siRNA molecule is "nuclease resistant" when it has either been modified such that it is not susceptible to degradation by nucleases or alternatively has been placed in a delivery vehicle which in itself protects the siRNA molecule from nucleases. Nuclease resistant siRNA molecules include, for example, methyl phosphonates, phosphorothioates, phosphorodithioates, phosphotriesters, and morpholino oligomers. Suitable delivery vehicles for conferring nuclease resistance include, for example, liposomes. In one embodiment of the present invention, the siRNA molecules are nuclease resistant.

### PREPARATION OF siRNA MOLECULES

The siRNA molecules of the present invention can be prepared using several methods known in the art, such as chemical synthesis, *in vitro* transcription and the use siRNA expression vectors.

Chemical synthesis of the siRNA molecules can be carried out by conventional techniques well-known to those skilled in the art. In general, RNA synthetic chemistry is based on standard solid-phase synthesis technology using commercially available equipment and the selective incorporation of various protecting groups into the RNA molecule at pre-determined points in the synthetic pathway. General methods of RNA synthesis and use of appropriate protecting groups is well known in the art (see, for example, Scaringe, S. A., et al., J. Am. Chem. Soc., 1998, 120, 11820-11821; Matteucci, M. D. and Caruthers, M. H. J. Am. Chem. Soc., 1981, 103, 3185-3191; Beaucage, S. L. and Caruthers, M. H. Tetrahedron Lett., 1981, 22, 1859-1862: Dahl, B. J., et al., Acta Chem. Scand,. 1990, 44, 639-641; Reddy, M. P., et al., Tetrahedron Lett., 1994, 25, 4311-4314; Wincott, F. et al., Nucleic Acids Res., 1995, 23, 2677-2684; Griffin, B. E., et al., Tetrahedron, 1967, 23, 2301-2313; Griffin, B. E., et al., Tetrahedron, 1967, 23, 2315-2331). As is also well known in the art, modified siRNA molecules, such as phosphorothioated and alkylated derivatives, can also be readily prepared by similar methods.

RNA molecules are typically synthesized as single-stranded RNA oligonucleotides. Once synthesized, complementary RNA oligonucleotides can be annealed by methods known in the art to form double-stranded siRNA molecules, if desired. For example, dsRNA molecules can be formed by combining appropriate amounts of two complementary RNA oligonucleotides in a suitable annealing buffer, heating the solution, for example to about 90°C, then allowing the solution to cool gradually to between 37°C and room temperature.

The siRNA molecules can also be synthesized by standard *in vitro* transcription methods by placing a DNA sequence encoding the siRNA molecule downstream of a promoter sequence of a DNA-dependent RNA polymerase, for example, T3, T7 or SP6 RNA polymerase. U.S. Patent No. 5,795,715, for example, teaches a process for the simultaneous transcription of the two complementary strands of a DNA sequence, carried out under pre-determined conditions and in the same reaction compartment. The two resulting transcripts hybridize immediately after transcription giving rise to a dsRNA molecule. In addition, kits providing a rapid and efficient means of constructing siRNA molecules by *in vitro* transcription are commercially available, for example, from Ambion (Austin, TX) and New England Biolabs (Beverly, MA) and are suitable for constructing the siRNA molecules of the present invention.

Standard recombinant techniques in which a sequence encoding the siRNA molecule is inserted into a recombinant expression vector can also be used to prepare the siRNA molecules of the invention. Suitable expression vectors for expressing a siRNA molecule of the invention include chromosomal, nonchromosomal and synthetic DNA sequences (for example, derivatives of SV40); bacterial plasmids; phage DNA; baculovirus; yeast plasmids; vectors derived from combinations of plasmids and phage DNA; and viral vectors, such as vaccinia virus, adenovirus, fowl pox virus, and pseudorabies virus vectors. Retroviral plasmid vectors are also suitable for use as expression vectors, including for example, those derived from Moloney Murine Leukemia Virus, spleen necrosis virus, Rous Sarcoma Virus, Harvey Sarcoma virus, avian leukosis virus, gibbon ape leukemia virus, human immunodeficiency virus, adenovirus, Myeloproliferative Sarcoma Virus, and mammary tumour virus.

The appropriate DNA sequence(s) encoding the siRNA molecule can be inserted into the vector by a variety of procedures known in the art. Standard techniques for cloning, DNA isolation, amplification and purification, for enzymatic reactions involving DNA ligase, DNA polymerase, restriction endonucleases and the like, and various separation techniques can be employed, such as those described, for example, in Ausubel et al. (Current Protocols in Molecular Biology, 1993 & updates, John Wiley & Sons, Inc., Boston, Mass.) and Sambrook et al. (Molecular Cloning, Third Ed., 2001, Cold Spring Harbor Laboratory, Plainview, N.Y.).

The DNA sequence in the expression vector is operatively linked to at least one appropriate expression control sequence, such as a promoter or a regulated promoter, to direct RNA synthesis. Representative examples of such expression control sequences include bacterial promoters such as the lac, lacZ, T3, T7, gpt, lambda P_{R}, lambda P_{L} and trp; eukaryotic promoters including the human U6 snRNA promoter (see, for example, Miyagishi et al, Nat. Biotechnol. 20:497-500 (2002); Lee et al., Nat. Biotechnol. 20:500-505 (2002); Paul et al., Nat. Biotechnol. 20:505-508 (2002); Grabarek et al., BioTechniques 34:73544 (2003); Sui et al., Proc, Natl. Acad Sci. USA 99:5515-20 (2002)), the H1 RNA promoter (see, for example, Brummelkamp et al., Science 296:550-53 (2002)), human cytomegalovirus (CMV) immediate early (Miller, et al., Biotechniques 7:980-990 (1989)), HSV thymidine kinase, early and late SV40, LTRs from retrovirus, mouse metallothionein-I and eukaryotic cellular promoters including, but not limited to, the histone, pol III, and β-actin promoters. Other regulatory regions that may be included in the expression vector as necessary include, but are not limited to, enhancers, ribosome binding sites, polyadenylation sites, splice donor and/or acceptor sites, transcriptional termination sequences, and 5' flanking nontranscribed sequences. Selection of the appropriate vector and regulatory regions is well within the level of ordinary skill in the art.

Transcription of one or more copies of the encoded siRNA molecule can be achieved by an endogenous RNA polymerase of the cell transformed with the expression vector or by a cloned RNA polymerase (for example, T3, T7 or SP6 RNA polymerase), which may be encoded by the same or a different expression vector. When the encoded siRNA molecule is a dsRNA molecule, each strand of the dsRNA can be transcribed separately, each under the direction of a separate promoter, and then can hybridize within the cell to form the dsRNA duplex or each strand can be transcribed from a separate vector (see Lee *et al., supra*). Alternatively, when the siRNA molecule is a shRNA, then the sense and antisense sequences can be transcribed as a single sequence under the control of a single promoter such that the transcribed RNA molecule forms a hairpin (Paul *et al., supra*). DNA vectors useful for insertion of sequences for transcription of an siRNA polynucleotide include pSUPER vector (see, for example, Brummelkamp *et al., supra*); pAV vectors derived from pCWRSVN (see, for example, Paul *et al., supra*); and pIND (see, for example, Lee *et al., supra*), or the like.

The expression vector can be introduced into a suitable host cell for propagation of the vector and/or expression of the encoded siRNA molecule by one of a number of standard techniques. In general the host cell is transduced, transformed or transfected by electroporation, the use of liposomes including cationic liposomes, calcium phosphate precipitation, DEAE-dextran mediated transfection, other suitable technique. Suitable host cells include prokaryotic cells, such as a bacterial cells; lower eukaryotic cells, such as a yeast cells or higher eukaryotic cells, such as a mammalian cells, plant cells or insect cells.

Suitable prokaryotic host cells for transformation include *E. coli, Bacillus subtilis, Salmonella typhimurium* and various species within the genera *Pseudomonas, Streptomyces* and *Staphylococcus.* Suitable lower eukaryotic host cells include the yeasts, *Saccharomyces* and *Pichia.* Representative examples of appropriate higher eukaryotic host cells include insect cells, such as Drosophila S2 and Spodoptera Sf9; mammalian cells, such as CHO, COS, 293, C127, 3T3, HeLa, HEK, and BHK cell lines; and plant cells.

When a retroviral expression vector is employed, the vector can be used to transduce a packaging cell line to form a producer cell line. Examples of packaging cells which may be transfected include, but are not limited to, the PE501, PA317, psi-2, psi-AM, PA12, T19-14X, VT-19-17-H2, psi CRE, psi CRIP, GP+E-86, GP+envAm12, and DAN cell lines (see, for example, Miller, Human Gene Therapy, 1:5-14 (1990)). Transduction can be achieved through various techniques known in the art, including those listed above. The producer cell line generates infectious retroviral vector particles that include the nucleic acid sequence(s) encoding the siRNA molecule of the invention. Such retroviral vector particles can subsequently be employed to transduce eukaryotic cells, either *in vitro* or *in vivo.* Eukaryotic cells which may be transduced include, but are not limited to, non-human embryonic stem cells, embryonic carcinoma cells, as well as hematopoietic stem cells, hepatocytes, fibroblasts, myoblasts, keratinocytes, endothelial cells, bronchial epithelial cells and various other culture-adapted cell lines.

The engineered host cells into which the expression vector has been introduced can be cultured in conventional nutrient media modified as appropriate for activating promoters, selecting transformants, amplifying particular sequences, etc. The culture conditions for particular host cells selected for expression, such as temperature, pH and the like, will be readily apparent to the ordinarily skilled artisan. Cells are typically harvested by centrifugation, disrupted by physical or chemical means, and the resulting crude extract retained for further purification. Microbial cells employed in expression of proteins can be disrupted by any convenient method, including freeze-thaw cycling, sonication, mechanical disruption, or use of cell lysing agents. Such methods are well known to those skilled in the art.

The siRNAs according to the present invention may also be prepared using the Drosophila *in vitro* system described in U.S. Patent Application No. 20030108923. Use of this Drosophila *in vitro* system entails combining dsRNA with a soluble extract derived from Drosophila embryo, thereby producing a combination. The combination is maintained under conditions in which the dsRNA is processed to RNA of about 21 to about 23 nucleotides.

The siRNAs may be derived from dsRNAs longer than 50 nucleotides, or from microRNAs (miRNAs) according to methods known in the art, including the Drosophila *in vitro* system described above.

The siRNA molecules of the present invention can be provided in crude mixtures or as purified or partially purified molecules. For example, recombinant siRNA molecules can be provided in the form of intact host cells, intact organelles (such as cell membranes, intracellular vesicles and the like), disrupted cell preparations (such as cell homogenates, cell lysates, microsomes, uni- and multilamellar membrane vesicles and other preparations). Alternatively, recombinant siRNA molecules can be recovered and purified from host cell cultures by standard purification techniques, including ammonium sulphate precipitation, ethanol precipitation, acid extraction, and various chromatographic techniques (including anion exchange, cation exchange, phosphocellulose, hydrophobic interaction, affinity, hydroxylapatite and lectin chromatography). High performance liquid chromatography (HPLC) is also suitable for final purification steps. Similarly, when synthesized chemically or by *in vitro* transcription, the siRNA molecules can be purified by standard techniques, such as extraction with a solvent or resin, precipitation, electrophoresis, chromatography, or a combination thereof. Alternatively, chemically synthesized and *in vitro* transcribed siRNA molecules can be used without purification or with a minimum of purification steps in order to minimise losses due to sample processing. The siRNA molecules can be dried for storage or dissolved in an aqueous solution, which may contain buffers or salts to promote annealing, and/or stabilization of duplex strands.

In addition, a number of commercial packages and services are available for the preparation of siRNA molecules that are suitable for use in the preparation of the siRNA molecules of the present invention. These include the *in vitro* transcription kits available from Ambion (Austin, TX) and New England Biolabs (Beverly, MA) as described above; viral siRNA construction kits commercially available from Invitrogen (Carlsbad, CA) and Ambion (Austin, TX), and custom siRNA construction services provided by Ambion (Austin, TX), Qiagen (Valencia, CA), Dharmacon (Lafayette, CO) and Sequitur, Inc (Natick, MA).

### EFFICACY OF THE siRNA MOLECULES

In accordance with the present invention, the siRNA molecules are able to inhibit the expression of their target mammalian ribonucleotide reductase gene. The ability of the siRNA molecules to inhibit the expression of
ribonucleotide reductase R2 mRNA or protein can be tested by one or more of a number of standard *in vitro or in vivo* techniques. The siRNA molecules can also be tested to determine their ability to attenuate the proliferation or growth and/or metastasis of neoplastic cells *in vitro* and/or *in vivo* using standard techniques. Exemplary, non-limiting tests are provided below and in the Examples.

### In vitro Testing

The ability of the siRNA molecules to inhibit the expression of a mammalian ribonucleotide reductase gene can be determined by culturing cells ofa selected cell line in a suitable medium. After an appropriate incubation time, the cells are transfected with the siRNA molecule, for example in the presence of a commercial lipid carrier such as lipofectamine, and are incubated, for a further period of time. The expression of the ribonucleotide reductase R2 gene can
be measured by determining the amount of mRNA transcribed from the gene and/or by determining the amount of protein expressed according to standard methods known in the art. For example, mRNA levels can be measured using Northern blot analysis or quantitative RT-PCR procedures and protein levels can be measured using Western blot analysis. The levels of mRNA and protein corresponding to ribonucleotide reductase R2 subunits can be compared to an appropriate control. Suitable controls include, for example, untreated cells and/or cells treated with a compound known to inhibit ribonucleotide reductase expression.

In accordance with the present invention, the siRNA molecules inhibit expression of their target ribonucleotide reductase gene in a test cell population by at least 10% as compared to an untreated control cell population. In one embodiment, the siRNA molecules inhibit expression of their target ribonucleotide reductase gene in a test cell population by at least 33%. In another embodiment, the siRNA molecules inhibit expression of their target ribonucleotide reductase gene in a test cell population by at least 50%. In other embodiments, the siRNA molecules inhibit expression of their target ribonucleotide reductase gene in a test cell population by at least 75%, 80%, 90%, 95% and 98%. Alternatively, the extent of inhibition by the siRNA molecules can be expressed in terms of the number of cells within a population in which inhibition of expression of the target ribonucleotide reductase gene is observed. This could be assessed, for example, by FACS analysis. As would be apparent to a worker skilled in the art, lower doses of administered siRNA molecules and longer times after administration of siRNA molecules may result in inhibition being observed in only a small fraction of cells. Thus, the siRNA molecules demonstrate inhibition of expression of the target ribonucleotide reductase gene in at least 10% of targeted cells. In various embodiments, the siRNA molecules demonstrate inhibition of expression of the target ribonucleotide reductase gene in at least 20%, 50%, 75%, 90%, and 95% of targeted cells. Quantitation of expression in a cell or cell population may show similar amounts of inhibition at the level of accumulation of target mRNA and translation of target protein, or the levels of inhibition may be different.

The ability of the siRNA molecules to attenuate the growth or proliferation of neoplastic cells can be tested by a number of standard techniques. For example, the cytotoxicity of the siRNA molecule can be assayed *in vitro* using a suitable cancer cell line. In general, cells of the selected test cell line are grown to an appropriate density and the candidate siRNA molecule is added. After an appropriate incubation time (for example, about 48 to 72 hours), cell survival is assessed. Methods of determining cell survival are well known in the art and include, but are not limited to, the resazurin reduction test (see Fields & Lancaster (1993) Am. Biotechnol. Lab. 11:48-50; O'Brien et al., (2000) Eur. J. Biochem. 267:5421-5426 and U.S. Patent No. 5,501,959), the sulforhodamine assay (Rubinstein et al., (1990) J. Natl. Cancer Inst. 82:113-118), the neutral red dye test (Kitano et al., (1991) Euro. J. Clin. Investg. 21:53-58; West et al., (1992) J. Investigative Derm. 99:95-100) or the XTT assay. Cytotoxicity is determined by comparison of cell survival in the treated culture with cell survival in one or more control cultures, for example, untreated cultures and/or cultures pre-treated with a control compound (typically a known therapeutic).

Alternatively, the ability of the siRNA molecules to inhibit proliferation of neoplastic cells can be assessed by culturing cells of a cancer cell line of interest in a suitable medium. After an appropriate incubation time, the cells can be transfected with the siRNA molecule, as described above, and incubated for a further period of time. Cells are then counted and compared to an appropriate control, as described above.

In accordance with one embodiment, the siRNA molecules of the invention are capable of producing a decrease of about 10% or more in the proliferation of neoplastic cells when compared to untreated cells or to cells treated with an appropriate control siRNA, such as a scrambled control siRNA molecule. In another embodiment, the siRNA molecules of the invention are capable of producing a decrease of about 15% or more in the proliferation of neoplastic cells when compared to untreated cells or to cells treated with an appropriate control siRNA. In another embodiment, the siRNA molecules of the invention are capable of producing a decrease of about 20% or more in the proliferation of neoplastic cells when compared to untreated cells or to cells treated with an appropriate control siRNA. In a further embodiment, the siRNA molecules of the invention are capable of producing a decrease of about 25% or more in the proliferation of neoplastic cells when compared to untreated cells or to cells treated with an appropriate control siRNA.

The siRNA molecules can also be tested *in vitro* by determining their ability to inhibit anchorage-independent growth of tumour cells. Anchorage-independent growth is known in the art to be a good indicator of tumourigenicity. In general, anchorage-independent growth is assessed by plating cells from a selected cancer cell-line onto soft agar and determining the number of colonies formed after an appropriate incubation period. Growth of cells treated with the siRNA molecule can then be compared with that of control cells (as described above).

A variety of cancer cell-lines suitable for testing the candidate siRNA molecules are known in the art and many are commercially available (for example, from the American Type Culture Collection, Manassas, VA). In one embodiment of the present invention, *in vitro* testing of the siRNA molecules is conducted in a human cancer cell-line. Examples of suitable cancer cell-lines for *in vitro* testing include, but are not limited to, breast cancer cell-lines MDA-MB-231 and MCF-7, renal carcinoma cell-line A-498, mesothelial cell lines MSTO-211H, NCI-H2052 and NCI-H28, ovarian cancer cell-lines OV90 and SK-OV-3, , colon cancer cell-lines CaCo, HCT116 and HT29, cervical cancer cell-line HeLa, non-small cell lung carcinoma cell-lines A549 and H1299, pancreatic cancer cell-lines MIA-PaCa-2 and AsPC-1, prostatic cancer-cell line PC-3, bladder cancer cell-line T24, liver cancer cell-lineHepG2, brain cancer cell-line U-87 MG, melanoma cell-line A2058, lung cancer cell-line NCI-H460. Other examples of suitable cell-lines are known in the art.

If desired, the siRNA molecules can be tested to determine whether they activate the interferon pathway. Methods of determining the ability of the siRNA molecules to activate the interferon response pathway are known in the art, and are described in, for example, Sledz, et al. (2003) Nature Cell Biology 5:834-839, and Bridge et al., (2003) Nature Genetics 34:263-264.

In one embodiment of the present invention, the siRNA molecules are used in combination with one or more standard chemotherapeutics. The efficacy of the combinations of siRNA molecules and one or more chemotherapeutic can be tested using standard techniques including those outlined above for the siRNA molecules. Additional controls may be included in such assays, such as cells treated with the siRNA molecule alone and/or the chemotherapeutic(s) alone in order to determine whether the effect of the combination is greater than the effect of the siRNA molecule and/or the chemotherapeutic(s) alone.

Therapeutic efficacy of siRNA molecules can be determined by standard pharmaceutical procedures in cell cultures or experimental animals, for example, by determining the LD50 (the dose lethal to 50% of the population) and the ED50 (the dose therapeutically effective in 50% of the population). The dose ratio between toxic and therapeutic effects is the therapeutic index, which can be expressed as the ratio LD50/ED50.

The toxicity of the siRNA molecules can also be assessed *in vitro,* if necessary, using standard techniques. For example, human primary fibroblasts can be transfected *in vitro* with the siRNA molecule and then tested at different time points following treatment for their viability using a standard viability assay, such as those described above. Cells can also be assayed for their ability to synthesize DNA, for example, using a thymidine incorporation assay, and for changes in cell cycle dynamics, for example, using a standard cell sorting assay in conjunction with a fluorocytometer cell sorter (FACS).

### In vivo Testing

The ability of the siRNA molecules to inhibit tumour growth or proliferation *in vivo* can be determined in an appropriate animal model using standard techniques known in the art (see, for example, Enna, et al., Current Protocols in Pharmacology, J. Wiley & Sons, Inc., New York, NY).

In general, current animal models for screening anti-tumour compounds are xenograft models, in which a human tumour has been implanted into an animal. Examples of xenograft models of human cancer include, but are not limited to, human solid tumour xenografts, implanted by sub-cutaneous injection or implantation and used in tumour growth assays; human solid tumour isografts, implanted by fat pad injection and used in tumour growth assays; human solid tumour orthotopic xenografts, implanted directly into the relevant tissue and used in tumour growth assays; experimental models of lymphoma and leukaemia in mice, used in survival assays, and experimental models of lung metastasis in mice.

For example, the siRNA molecules can be tested *in vivo* on solid tumours using mice that are subcutaneously grafted bilaterally with 30 to 60 mg of a tumour fragment, or implanted with an appropriate number of cancer cells, on day 0. The animals bearing tumours are mixed before being subjected to the various treatments and controls. In the case of treatment of advanced tumours, tumours are allowed to develop to the desired size, animals having insufficiently developed tumours being eliminated. The selected animals are distributed at random to undergo the treatments and controls. Animals not bearing tumours may also be subjected to the same treatments as the tumour-bearing animals in order to be able to dissociate any toxic effect of the test siRNA molecule(s) from the specific effect on the tumour. Chemotherapy generally begins from 3 to 22 days after grafting, depending on the type of tumour, and the animals are observed every day. The siRNA molecules of the present invention can be administered to the animals, for example, by i.p. injection or bolus infusion. The different animal groups are weighed about 3 or 4 times a week until the maximum weight loss is attained, after which the groups are weighed at least once a week until the end of the trial.

The tumours are measured after a pre-determined time period, or they can be monitored continuously by measuring about 2 or 3 times a week until the tumour reaches a pre-determined size and/or weight, or until the animal dies if this occurs before the tumour reaches the pre-determined size/weight. The animals are then sacrificed and the tissue histology, size and/or proliferation of the tumour assessed.

For the study of the effect of the siRNA molecules on leukaemias, the animals are grafted with a particular number of cells, and the anti-tumour activity is determined by the increase in the survival time of the treated mice relative to the controls.

To study the effect of the siRNA molecules of the present invention on tumour metastasis, tumour cells are typically treated with the siRNA molecule(s) *ex vivo* and then injected into a suitable test animal. The spread of the tumour cells from the site of injection is then monitored over a suitable period of time.

*In vivo* toxic effects of the siRNA molecules can be evaluated by measuring their effect on animal body weight during treatment and by performing haematological profiles and liver enzyme analysis after the animal has been sacrificed.

**Table 6: Examples of Xenograft Models of Human Cancer**

| **Cancer Model** | **Cell Type** |
|---|---|
| Tumour Growth Assay | Prostate (PC-3, DU145) |
| Human solid tumour xenografts in mice (sub-cutaneous injection) | Breast (MDA-MB-231, MVB-9) |
| | Colon (HT-29) |
| | Lung (NCI-H460, NCI-H209) |
| | Pancreatic (ASPC-1, SU86.86) |
| | Pancreatic: drug resistant (BxPC-3) |
| | Skin (A2058, C8161) |
| | Cervical (SIHA, HeLa-S3) |
| | Cervical: drug resistant (HeLa S3-HU-resistance) |
| | Liver (HepG2) |
| | Brain (U87-MG) |
| | Renal (Caki-1, A498) |
| | Ovary (SK-OV-3) |
| Tumour Growth Assay | Breast: drug resistant (MDA-CDDP-S4, MDA-MB435-To.1) |
| Human solid tumour isografts in mice (fat pad injection) | |
| Survival Assay | Human: Burkitts lymphoma (Non-Hodgkin's) (raji) |
| Experimental model of lymphoma and leukaemia in mice | Murine: erythroleukemia (CB7 Friend retrovirus-induced) |
| Experimental model of lung metastasis in mice | Human: melanoma (C8161) |
| | Murine: fibrosarcoma (R3) |

### PHARMACEUTICAL COMPOSITIONS

The present invention provides for pharmaceutical compositions comprising one or more siRNA molecules in admixture with a conventional non-toxic pharmaceutically acceptable carrier, adjuvant or vehicle. The pharmaceutical compositions can be formulated for oral, topical, parenteral or rectal administration or for administration by inhalation or spray. The term parenteral as used herein includes subcutaneous injections, intravenous, intramuscular, intrasternal injection or infusion techniques. The siRNA molecules can be present in the compositions in association with one or more other active ingredients, if desired.

Compositions containing only "naked" dsRNA and a physiologically acceptable solvent or carrier have been shown to be taken up by cells *in vivo.* The pharmaceutical compositions of the present invention, therefore, may comprise one or more siRNA molecule in aqueous suspension. Suitable solvents and carriers for such compositions include those described below. Alternatively, the siRNA molecules may be formulated for administration by associating the siRNA molecule with a biodegradable polymer or may be encapsulated to protect the siRNA against rapid elimination from the body, for example, in the form of a controlled release formulation, such as an implant or microencapsulated delivery system. Biodegradable, biocompatible polymers that can be used for such formulations include, for example, polypeptides, ethylene vinyl acetate, polyanhydrides, polyglycolic acid, collagen, polyorthoesters, polylactic acid, poly(d, 1-lactic-co-glycolic acid) (PLGA), polylysin, polylysin conjugates, polylysine-graft-imidazole acetic acid and poly(beta-amino ester). Microparticles, such as microspheres, nanoparticles or nanospheres can also be employed. Alternatively, the siRNA molecules may be covalently coupled to the polymer or microparticle, wherein the covalent coupling particularly is effected via the 3'-terminus of the siRNA. Liposomal suspensions can also be used as pharmaceutically acceptable carriers. These can be prepared according to methods known to those skilled in the art, for example, as described in U.S. Pat. No. 4,522,811; PCT publication WO 91/06309; and European patent publication EP-A-43075. Similarly, the pharmaceutical compositions of the present invention can comprise one or more siRNA associated with or encapsulated by liposomes or other artificial membrane vesicles known in the art [for example, see "Liposomes as Drug Carriers" G. Gregoriadis, Wiley & Sons, New-York (1988); Gregoriadis, G., "Liposome preparation and related techniques," in: G. Gregoriadis (Ed.) "Liposome Technology" Vol. 1, 2nd Edition, CRC Press, Baton Rouge, FL, (1993), pp.1-63].

The pharmaceutical compositions may be in a form suitable for oral use, for example, as tablets, troches, lozenges, aqueous or oily suspensions, dispersible powders or granules, emulsion hard or soft capsules, or syrups or elixirs. Compositions intended for oral use may be prepared according to methods known to the art for the manufacture of pharmaceutical compositions and may contain one or more agents selected from the group of sweetening agents, flavouring agents, colouring agents and preserving agents in order to provide pharmaceutically elegant and palatable preparations. Tablets contain the active ingredient in admixture with suitable non-toxic pharmaceutically acceptable excipients including, for example, inert diluents, such as calcium carbonate, sodium carbonate, lactose, calcium phosphate or sodium phosphate; granulating and disintegrating agents, such as corn starch, or alginic acid; binding agents, such as starch, gelatine or acacia, and lubricating agents, such as magnesium stearate, stearic acid or talc. The tablets can be uncoated, or they may be coated by known techniques in order to delay disintegration and absorption in the gastrointestinal tract and thereby provide a sustained action over a longer period. For example, a time delay material such as glyceryl monosterate or glyceryl distearate may be employed.

Pharmaceutical compositions for oral use may also be presented as hard gelatine capsules wherein the active ingredient is mixed with an inert solid diluent, for example, calcium carbonate, calcium phosphate or kaolin, or as soft gelatine capsules wherein the active ingredient is mixed with water or an oil medium such as peanut oil, liquid paraffin or olive oil.

Aqueous suspensions contain the active siRNA in admixture with suitable excipients including, for example, suspending agents, such as sodium carboxymethylcellulose, methyl cellulose, hydropropylmethylcellulose, sodium alginate, polyvinylpyrrolidone, gum tragacanth and gum acacia; dispersing or wetting agents such as a naturally-occurring phosphatide, for example, lecithin, or condensation products of an alkylene oxide with fatty acids, for example, polyoxyethyene stearate, or condensation products of ethylene oxide with long chain aliphatic alcohols, for example, hepta-decaethyleneoxycetanol, or condensation products of ethylene oxide with partial esters derived from fatty acids and a hexitol for example, polyoxyethylene sorbitol monooleate, or condensation products of ethylene oxide with partial esters derived from fatty acids and hexitol anhydrides, for example, polyethylene sorbitan monooleate. The aqueous suspensions may also contain one or more preservatives, for example ethyl, or *n*-propyl *p*-hydroxy-benzoate, one or more colouring agents, one or more flavouring agents or one or more sweetening agents, such as sucrose or saccharin.

Oily suspensions may be formulated by suspending the active ingredients in a vegetable oil, for example, arachis oil, olive oil, sesame oil or coconut oil, or in a mineral oil such as liquid paraffin. The oily suspensions may contain a thickening agent, for example, beeswax, hard paraffin or cetyl alcohol. Sweetening agents such as those set forth above, and/or flavouring agents may be added to provide palatable oral preparations. These compositions can be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavouring and colouring agents, may also be present.

Pharmaceutical compositions of the invention may also be in the form of oil-in-water emulsions. The oil phase may be a vegetable oil, for example, olive oil or arachis oil, or a mineral oil, for example, liquid paraffin, or it may be a mixtures of these oils. Suitable emulsifying agents may be naturally-occurring gums, for example, gum acacia or gum tragacanth; naturally-occurring phosphatides, for example, soy bean, lecithin; or esters or partial esters derived from fatty acids and hexitol, anhydrides, for example, sorbitan monoleate, and condensation products of the said partial esters with ethylene oxide, for example, polyoxyethylene sorbitan monoleate. The emulsions may also contain sweetening and flavouring agents.

Syrups and elixirs may be formulated with sweetening agents, for example glycerol, propylene glycol, sorbitol or sucrose. Such formulations may also contain a demulcent, a preservative and flavouring and colouring agents.

The pharmaceutical compositions may be in the form of a sterile injectable aqueous or oleaginous suspension. This suspension may be formulated according to known art using suitable dispersing or wetting agents and suspending agents such as those mentioned above. The sterile injectable preparation may also be sterile injectable solution or suspension in a non-toxic parentally acceptable diluent or solvent, for example, as a solution in 1,3-butanediol. Acceptable vehicles and solvents that may be employed include, but are not limited to, water, Ringer's solution, lactated Ringer's solution and isotonic sodium chloride solution. Other examples are, sterile, fixed oils which are conventionally employed as a solvent or suspending medium, and a variety of bland fixed oils including, for example, synthetic mono- or diglycerides. In addition, fatty acids such as oleic acid find use in the preparation of injectables.

Other pharmaceutical compositions and methods of preparing pharmaceutical compositions are known in the art and are described, for example, in *"*Remington: The Science and Practice of Pharmacy" (formerly "Remingtons Pharmaceutical Sciences"); Gennaro, A., Lippincott, Williams & Wilkins, Philidelphia, PA (2000).

### USE OF THE siRNA MOLECULES

The siRNA molecules of the present invention attenuate the growth and/or proliferation of neoplastic cells. The present invention, therefore, provides for the use of the siRNA molecules in the treatment, stabilization or prevention of various cancers. In this context, the siRNA molecules may exert either a cytotoxic or cytostatic effect resulting in a reduction in the size of a tumour, the slowing or prevention of an increase in the size of a tumour, an increase in the disease-free survival time between the disappearance or removal of a tumour and its reappearance, prevention of an initial or subsequent occurrence of a tumour (*e.g*. metastasis), an increase in the time to progression, reduction of one or more adverse symptom associated with a tumour, or an increase in the overall survival time of a subject having cancer.

Examples of cancers which may be treated or stabilized in accordance with the present invention include, but are not limited to haematologic neoplasms, including leukaemias and lymphomas; carcinomas, including adenocarcinomas; melanomas and sarcomas. Carcinomas, adenocarcinoma and sarcomas are also frequently referred to as "solid tumours," examples of commonly occurring solid tumours include, but are not limited to, cancer of the brain, breast, cervix, colon, head and neck, kidney, lung, ovary, pancreas, prostate, stomach and uterus, non-small cell lung cancer and colorectal cancer. Various forms of lymphoma also may result in the formation of a solid tumour and, therefore, are often considered to be solid tumours. In one embodiment of the present invention, the siRNA molecule is used to treat or stabilize a solid tumour. In another embodiment, the siRNA molecule is used to treat or stabilize a solid tumour other than a pancreatic adenocarcinoma.

The term "leukaemia" refers broadly to progressive, malignant diseases of the blood-forming organs. Leukaemia is typically characterized by a distorted proliferation and development of leukocytes and their precursors in the blood and bone marrow but can also refer to malignant diseases of other blood cells such as erythroleukaemia, which affects immature red blood cells. Leukaemia is generally clinically classified on the basis of (1) the duration and character of the disease - acute or chronic; (2) the type of cell involved - myeloid (myelogenous), lymphoid (lymphogenous) or monocytic, and (3) the increase or non-increase in the number of abnormal cells in the blood-leukaemic or aleukaemic (subleukaemic). Leukaemia includes, for example, acute nonlymphocytic leukaemia, chronic lymphocytic leukaemia, acute granulocytic leukaemia, chronic granulocytic leukaemia, acute promyelocytic leukaemia, adult T-cell leukaemia, aleukaemic leukaemia, aleukocythemic leukaemia, basophylic leukaemia, blast cell leukaemia, bovine leukaemia, chronic myelocytic leukaemia, leukaemia cutis, embryonal leukaemia, eosinophilic leukaemia, Gross' leukaemia, hairy-cell leukaemia, hemoblastic leukaemia, hemocytoblastic leukaemia, histiocytic leukaemia, stem cell leukaemia, acute monocytic leukaemia, leukopenic leukaemia, lymphatic leukaemia, lymphoblastic leukaemia, lymphocytic leukaemia, lymphogenous leukaemia, lymphoid leukaemia, lymphosarcoma cell leukaemia, mast cell leukaemia, megakaryocytic leukaemia, micromyeloblastic leukaemia, monocytic leukaemia, myeloblastic leukaemia, myelocytic leukaemia, myeloid granulocytic leukaemia, myelomonocytic leukaemia, Naegeli leukaemia, plasma cell leukaemia, plasmacytic leukaemia, promyelocytic leukaemia, Rieder cell leukaemia, Schilling's leukaemia, stem cell leukaemia, subleukaemic leukaemia, and undifferentiated cell leukaemia.

The term "lymphoma" generally refers to a malignant neoplasm of the lymphatic system, including cancer of the lymphatic system. The two main types of lymphoma are Hodgkin's disease (HD or HL) and non-Hodgkin's lymphoma (NHL). Abnormal cells appear as congregations which enlarge the lymph nodes, form solid tumours in the body, or more rarely, like leukemia, circulate in the blood. Hodgkin's disease lymphomas, include nodular lymphocyte predominance Hodgkin's lymphoma; classical Hodgkin's lymphoma; nodular sclerosis Hodgkin's lymphoma; lymphocyte-rich classical Hodgkin's lymphoma; mixed cellularity Hodgkin's lymphoma; lymphocyte depletion Hodgkin's lymphoma. Non-Hodgkin's lymphomas include small lymphocytic NHL, follicular NHL; mantle cell NHL; mucosa-associated lymphoid tissue (MALT) NHL; diffuse large cell B-cell NHL; mediastinal large B-cell NHL; precursor T lymphoblastic NHL; cutaneous T-cell NHL; T-cell and natural killer cell NHL; mature (peripheral) T-cell NHL; Burkitt's lymphoma; mycosis fungoides; Sézary Syndrome; precursor B-lymophoblastic lymphoma; B-cell small lymphocytic lymphoma; lymphoplasmacytic lymphoma; spenic marginal zome B-cell lymphoma; nodal marginal zome lymphoma; plasma cell myeloma/plasmacytoma; intravascular large B-cell NHL; primary effusion lymphoma; blastic natural killer cell lymphoma; enteropathy-type T-cell lymphoma; hepatosplenic gamma-delta T-cell lymphoma; subcutaneous panniculitis-like T-cell lymphoma; angioimmunoblastic T-cell lymphoma; and primary systemic anaplastic large T/null cell lymphoma.

The term "sarcoma" generally refers to a tumour which originates in connective tissue, such as muscle, bone, cartilage or fat, and is made up of a substance like embryonic connective tissue and is generally composed of closely packed cells embedded in a fibrillar or homogeneous substance. Sarcomas include soft tissue sarcomas, chondrosarcoma, fibrosarcoma, lymphosarcoma, melanosarcoma, myxosarcoma, osteosarcoma, Abemethy's sarcoma, adipose sarcoma, liposarcoma, alveolar soft part sarcoma, ameloblastic sarcoma, botryoid sarcoma, chloroma sarcoma, chorio carcinoma, embryonal sarcoma, Wilms' tumour sarcoma, endometrial sarcoma, stromal sarcoma, Ewing's sarcoma, fascial sarcoma, fibroblastic sarcoma, giant cell sarcoma, granulocytic sarcoma, Hodgkin's sarcoma, idiopathic multiple pigmented haemorrhagic sarcoma, immunoblastic sarcoma ofB cells, lymphoma, immunoblastic sarcoma of T-cells, Jensen's sarcoma, Kaposi's sarcoma, Kupffer cell sarcoma, angiosarcoma, leukosarcoma, malignant mesenchymoma sarcoma, parosteal sarcoma, reticulocytic sarcoma, Rous sarcoma, serocystic sarcoma, synovial sarcoma, and telangiectaltic sarcoma. The term "melanoma" is taken to mean a tumour arising from the melanocytic system of the skin and other organs. Melanomas include, for example, acral-lentiginous melanoma, amelanotic melanoma, benign juvenile melanoma, Cloudman's melanoma, S91 melanoma, Harding-Passey melanoma, juvenile melanoma, lentigo maligna melanoma, malignant melanoma, nodular melanoma, subungal melanoma, and superficial spreading melanoma.

The term "carcinoma" refers to a malignant new growth made up of epithelial cells tending to infiltrate the surrounding tissues and give rise to metastases. Exemplary carcinomas include, for example, acinar carcinoma, acinous carcinoma, adenocystic carcinoma, adenoid cystic carcinoma, carcinoma adenomatosum, carcinoma of adrenal cortex, alveolar carcinoma, alveolar cell carcinoma, basal cell carcinoma, carcinoma basocellulare, basaloid carcinoma, basosquamous cell carcinoma, bronchioalveolar carcinoma, bronchiolar carcinoma, bronchogenic carcinoma, cerebriform carcinoma, cholangiocellular carcinoma, chorionic carcinoma, colorectal carcinoma, colloid carcinoma, comedo carcinoma, corpus carcinoma, cribriform carcinoma, carcinoma en cuirasse, carcinoma cutaneum, cylindrical carcinoma, cylindrical cell carcinoma, duct carcinoma, carcinoma durum, embryonal carcinoma, encephaloid carcinoma, epiermoid carcinoma, carcinoma epitheliale adenoides, exophytic carcinoma, carcinoma ex ulcere, carcinoma fibrosum, gelatiniform carcinoma, gelatinous carcinoma, giant cell carcinoma, carcinoma gigantocellulare, glandular carcinoma, granulosa cell carcinoma, hair-matrix carcinoma, haematoid carcinoma, hepatocellular carcinoma, Hurthle cell carcinoma, hyaline carcinoma, hypemephroid carcinoma, infantile embryonal carcinoma, carcinoma in situ, intraepidermal carcinoma, intraepithelial carcinoma, Krompecher's carcinoma, Kulchitzky-cell carcinoma, large-cell carcinoma, lenticular carcinoma, carcinoma lenticulare, lipomatous carcinoma, lymphoepithelial carcinoma, carcinoma medullare, medullary carcinoma, melanotic carcinoma, carcinoma molle, mucinous carcinoma, carcinoma muciparum, carcinoma mucocellulare, mucoepidermoid carcinoma, carcinoma mucosum, mucous carcinoma, carcinoma myxomatodes, naspharyngeal carcinoma, oat cell carcinoma, non-small cell carcinoma, carcinoma ossificans, osteoid carcinoma, papillary carcinoma, periportal carcinoma, preinvasive carcinoma, prickle cell carcinoma, pultaceous carcinoma, renal cell carcinoma of kidney, reserve cell carcinoma, carcinoma sarcomatodes, schneiderian carcinoma, scirrhous carcinoma, carcinoma scroti, signet-ring cell carcinoma, carcinoma simplex, small-cell carcinoma, solanoid carcinoma, spheroidal cell carcinoma, spindle cell carcinoma, carcinoma spongiosum, squamous carcinoma, squamous cell carcinoma, string carcinoma, carcinoma telangiectaticum, carcinoma telangiectodes, transitional cell carcinoma, carcinoma tuberosum, tuberous carcinoma, verrucous carcinoma, and carcinoma villosum.

The term "carcinoma" also encompasses adenocarcinomas. Adenocarcinomas are carcinomas that originate in cells that make organs which have glandular (secretory) properties or that originate in cells that line hollow viscera, such as the gastrointestinal tract or bronchial epithelia. Examples include, but are not limited to, adenocarcinomas of the breast, lung, pancreas and prostate.

Additional cancers encompassed by the present invention include, for example, multiple myeloma, neuroblastoma, rhabdomyosarcoma, primary thrombocytosis, primary macroglobulinemia, small-cell lung tumours, primary brain tumours, malignant pancreatic insulanoma, malignant carcinoid, urinary bladder cancer, premalignant skin lesions, gliomas, testicular cancer, thyroid cancer, esophageal cancer, genitourinary tract cancer, malignant hypercalcemia, endometrial cancer, adrenal cortical cancer, mesothelioma and medulloblastoma.

The cancer to be treated may be indolent or it may be aggressive. The present invention contemplates the use of the siRNA molecules in the treatment of refractory cancers, advanced cancers, recurrent cancers and metastatic cancers. One skilled in the art will appreciate that many of these categories may overlap, for example, aggressive cancers are typically also metastatic.

"Aggressive cancer," as used herein, refers to a rapidly growing cancer. One skilled in the art will appreciate that for some cancers, such as breast cancer or prostate cancer the term "aggressive cancer" will refer to an advanced cancer that has relapsed within approximately the earlier two-thirds of the spectrum of relapse times for a given cancer, whereas for other types of cancer, such as small cell lung carcinoma (SCLC) nearly all cases present rapidly growing cancers which are considered to be aggressive. The term can thus cover a subsection of a certain cancer type or it may encompass all of other cancer types. A "refractory" cancer or tumour refers to a cancer or tumour that has not responded to treatment. "Advanced cancer," refers to overt disease in a patient, wherein such overt disease is not amenable to cure by local modalities of treatment, such as surgery or radiotherapy. Advanced disease may refer to a locally advanced cancer or it may refer to metastatic cancer. The term "metastatic cancer" refers to cancer that has spread from one part of the body to another.
Advanced cancers may also be unresectable, that is, they have spread to surrounding tissue and cannot be surgically removed.

The siRNA molecules may also be used to treat drug resistant cancers, including multidrug resistant tumours. As is known in the art, the resistance of cancer cells to chemotherapy is one of the central problems in the management of cancer.

Certain cancers, such as prostate and breast cancer, can be treated by hormone therapy, *i*.*e*. with hormones or anti-hormone drugs that slow or stop the growth of certain cancers by blocking the body's natural hormones. Such cancers may develop resistance, or be intrinsically resistant, to hormone therapy. The present invention further contemplates the use of the siRNA molecules in the treatment of such "hormone-resistant " or "hormone-refractory" cancers.

### Administration of the siRNA Molecules

Typically in the treatment of cancer, therapeutic compounds are administered systemically to patients. Thus, the siRNA molecules of the present invention can be administered to a subject, for example, orally, by bolus injection or by infusion into the subject's bloodstream. The siRNA molecules can also be administered using a hydrodynamic protocol, such as that described in International patent application PCT/US02/22869 (WO 03/10180). Alternatively, the siRNA molecules can be administered through the use of viral vectors or liposome formulations as is known in the art, or by microparticle bombardment (for example, through use of a "gene gun"; Biolistic, Dupont).

The siRNA molecules of the present invention may be used as part of a neo-adjuvant therapy (to primary therapy), or as part of an adjuvant therapy regimen. As indicated above, the present invention contemplates the use of the siRNA molecules at various stages in tumour development and progression, including in the treatment of advanced and/or aggressive neoplasias (*i.e*. overt disease in a subject that is not amenable to cure by local modalities of treatment, such as surgery or radiotherapy), metastatic disease, locally advanced disease and/or refractory tumours (*i.e*. a cancer or tumour that has not responded to treatment).

"Primary therapy" refers to a first line of treatment upon the initial diagnosis of cancer in a subject. Exemplary primary therapies may involve surgery, a wide range of chemotherapies and radiotherapy. "Adjuvant therapy" refers to a therapy that follows a primary therapy and that is administered to subjects at risk of relapsing. Adjuvant systemic therapy is begun soon after primary therapy to delay recurrence, prolong survival or cure a subject.

It is contemplated that the siRNA molecules of the invention can be used alone or in combination with one or more anti-cancer therapeutucs, such as chemotherapeutic agents or immunotherapeutic agents, as part of an adjuvant therapy. Combinations of the siRNA molecules and standard chemotherapeutics may act to improve the efficacy of the chemotherapeutic and, therefore, can be used to improve standard cancer therapies. Inclusion of one or more immunotherapeutic in the combiantion therapy regimen can increase the efficacy of the Antisense oligonucleotide and/or chemotherapeutic or reduce the side effects associated with either agent. This application is particularly important in the treatment of drug-resistant cancers which are not responsive to standard treatment. Drug-resistant cancers can arise, for example, from heterogeneity of tumour cell populations, alterations in response to chemotherapy and increased malignant potential. Such changes are often more pronounced at advanced stages of disease.

When employed in combination therapy, the present invention contemplates that the siRNA molecules may be used as "sensitizing agents" or "chemosensitizers." In this case, the siRNA molecule alone does not have a cytotoxic effect on the cancer cells, but provides a means of weakening the cells, thereby facilitating the benefit from conventional anti-cancer therapeutics.

When used in conjunction with one or more known chemotherapeutic and/or immunotherapeutic agents, the compounds can be administered prior to, or after, administration of the other agent(s), or they can be administered concurrently. The one or more chemotherapeutic and/or immunotherapeutic may be administered systemically, for example, by bolus injection or continuous infusion, or it may be administered orally.

The dosage to be administered is not subject to defined limits, but it will usually be an effective amount. It will usually be the equivalent, on a molar basis of the pharmacologically active free form produced from a dosage formulation upon the metabolic release of the active free drug to achieve its desired pharmacological and physiological effects. The compositions may be formulated in a unit dosage form. The term "unit dosage form" refers to physically discrete units suitable as unitary dosages for human subjects and other mammals, each unit containing a predetermined quantity of active material calculated to produce the desired therapeutic effect, in association with a suitable pharmaceutical excipient.

Daily dosages of the siRNA molecules of the present invention will typically fall within the range of about 0.01 to about 100 mg/kg of body weight, in a single dose, a divided dose, or by administration over a pre-determined time period. However, it will be understood that the actual amount of the siRNA molecule(s) to be administered will be determined by a physician, in the light of the relevant circumstances, including the condition to be treated, the chosen route of administration, the actual siRNA molecule administered, the age, weight, and response of the individual patient, and the severity of the patient's symptoms. The above dosage range is given by way of example only and is not intended to limit the scope of the invention in any way. In some instances dosage levels below the lower limit of the aforesaid range may be more than adequate, while in other cases still larger doses may be employed without causing harmful side effects, for example, by first dividing the larger dose into several smaller doses for administration throughout the day.

### Chemotherapeutic Agents

When the siRNA molecules of the present invention are used in combination with one or more chemotherapeutic agents, the chemotherapeutic agent can be selected from a wide range of cancer chemotherapeutic agents known in the art. Known chemotherapeutic agents include those that are specific for the treatment of a particular type of cancer as well as those that are applicable to a range of cancers, such as doxorubicin, docetaxel, 5-fluorouracil, capecitabine, mitoxantrone, irinotecan (CPT-11), cisplatin and gemcitabine. Etoposide is generally applicable in the treatment of leukaemias (including acute lymphocytic leukaemia and acute myeloid leukaemia), germ cell tumours, Hodgkin's disease and various sarcomas. Cytarabine (Ara-C) is also applicable in the treatment of various leukaemias, including acute myeloid leukaemia, meningeal leukaemia, acute lymphocytic leukaemia, chronic myeloid leukaemia, erythroleukaemia, as well as non-Hodgkin's lymphoma.

The present invention contemplates the use of both cancer-specific and broad-spectrum chemotherapeutic agent in conjunction with the siRNA molecules. In one embodiment of the present invention, the siRNA molecules are used in combination with a broad-spectrum chemotherapeutic. In another embodiment, the siRNA molecules are used in combination with a chemotherapeutic other than gemcitabine. Exemplary chemotherapeutics that can be used alone or in various combinations for the treatment specific cancers are provided in Table 7 and are suitable for use in combination with the siRNA molecules of the present invention. One skilled in the art will appreciate that many other chemotherapeutics are available and that the following list is representative only.

**Table 7: Exemplary Chemotherapeutics used in the Treatment of Some Common Cancers**

| **CANCER** | **CHEMOTHERAPEUTIC** | |
|---|---|---|
| Acute lymphocytic | Pegaspargase (*e.g*. Oncaspar®) | L-asparaginase |
| leukaemia (ALL) | Cytarabine | |
| Acute myeloid leukaemia (AML) | Cytarabine | Idarubicin |
| Brain cancer | Procarbazine (*e.g*. Matulane®) | Nitrosoureas |
| | Platinum analogues | Temozolomide |
| Breast cancer | Capecitabine (*e.g*. Xeloda®) | Cyclophosphamide |
| | 5-fluorouracil (5-FU) | Carboplatin |
| | Paclitaxel (*e.g*. Taxol®) | Cisplatin |
| | Docetaxel (*e.g*. Taxotere®) | Ifosfamide |
| | Epi-doxorubicin (epirubicin) | Tamoxifen |
| | Doxorubicin (*e.g*. Adriamycin®) | |
| Chronic myeloid leukaemia (CML) | Cytarabine | |
| Colon cancer | Edatrexate (10-ethyl-10-deaza-aminopterin) | |
| | Methyl-chloroethyl-cyclohexyl-nitrosourea | |
| | 5-fluorouracil (5-FU) | Levamisole |
| | Fluorodeoxyuridine (FUdR) | Vincristine |
| | Capecitabine (*e.g*. Xeloda®) | Oxaliplatin |
| Colorectal cancer | Irinotecan (CPT-11, *e.g*. Camptosar®) | |
| | Loperamide (*e.g*. Imodium®) | 5-fluorouracil (5-FU) |
| | Topotecan (*e.g*. Hycamtin®) | Methotrexate |
| | Capecitabine (*e.g*. Xeloda®) | Oxaliplatin |
| Gall bladder | 5-fluorouracil (5-FU) | |
| Genitourinary cancer | Docetaxel (*e.g*. Taxotere®) | |
| Head and neck cancer | Docetaxel (*e.g*. Taxotere®) | Cisplatin |
| Non-Hodgkin's Lymphoma | Procarbazine (*e.g*. Matulane®) | Cytarabine |
| | Etoposide | |
| Non-small-cell lung (NSCL) | Vinorelbine Tartrate (*e.g*. Navelbine®) | |
| cancer | Irinotecan (CAT-11, *e.g*. Camptosar®) | |
| | Docetaxel (*e.g*. Taxotere®) | Paclitaxel (*e.g*. Taxol®) |
| | Gemcitabine (*e.g*. Gemzar®) | Topotecan |
| Oesophageal cancer | Porfimer Sodium (*e.g*. Photofrin®) | |
| | Cisplatin | |
| Ovarian cancer | Irinotecan (CAT-11, *e.g*. Camptosar®) | |
| | Topotecan (*e.g*. Hycamtin®) | |
| | Docetaxel (*e.g*. Taxotere®) | Paclitaxel (*e.g*. Taxol®) |
| | Gemcitabine (*e.g*. Gemzar®) | Amifostine (*e.g*. Ethyol®) |
| Pancreatic cancer | Irinotecan (CPT-11, *e.g*. Camptosar®) | |
| | Gemcitabine (*e.g*. Gemzar®) | 5-fluorouracil (5-FU) |
| Promyelocytic leukaemia | Tretinoin (*e.g*. Vesanoid®) | |
| Prostate cancer | Goserelin Acetate (*e.g*. Zoladex®) | |
| | Mitoxantrone (*e.g*. Novantrone®) | |
| | Prednisone (*e.g*. Deltasone®) | Liarozole |
| | Nilutamide (*e.g*. Nilandron®) | Flutamide (*e.g*. Eulexin®) |
| | Finasteride (*e.g*. Proscar®) | Terazosin (*e.g*. Hytrin®) |
| | Doxazosin (*e.g*. Cardura®) | Cyclophosphamide |
| | Docetaxel (*e.g*. Taxotere®) | Estramustine |
| | Luteinizing hormone releasing hormone agonist | |
| Renal cancer | Capecitabine (e.g. Xeloda®) | Gemcitabine (*e.g*. Gemzar®) |
| Small cell lung cancer | Cyclophosphamide | Vincristine |
| | Doxorubicin | Etoposide |
| Solid tumours | Gemicitabine (*e.g*. Gemzar®) | Cyclophosphamide |
| | Capecitabine (*e.g*. Xeloda®) | Ifosfamide |
| | Paclitaxel (*e.g*. Taxol®) | Cisplatin |
| | Docetaxel (*e.g*. Taxotere®) | Carboplatin |
| | Epi-doxorubicin (epirubicin) | 5-fluorouracil (5-FU) |
| | Doxorubicin (*e.g*. Adriamycin®) | |

As indicated above, combinations of chemotherapeutics may be employed. Combination therapies using standard cancer chemotherapeutics are well known in the art and such combinations also can be used in conjunction with the siRNA molecules of the invention.

Exemplary combination therapies include for the treatment of breast cancers the combination of epirubicin with paclitaxel or docetaxel, or the combination of doxorubicin or epirubicin with cyclophosphamide. Polychemotherapeutic regimens are also useful and may consist, for example, of doxorubicin/cyclophosphamide/5-fluorouracil or cyclophosphamide/epirubicin/5-fluorouracil. Many of the above combinations are useful in the treatment of a variety of other solid tumours.

Combinations of etoposide with either cisplatin or carboplatin are used in the treatment of small cell lung cancer. In the treatment of stomach or oesophageal cancer, combinations of doxorubicin or epirubicin with cisplatin and 5-fluorouracil are useful. For colorectal cancer, CPT-11 in combination with 5-fluorouracil-based drugs, or oxaliplatin in combination with 5-fluorouracil-based drugs can be used. Oxaliplatin may also be used in combination with capecitabine.

Other examples include the combination of cyclophosphamide, doxorubicin, vincristine and prednisone in the treatment of non-Hodgkin's lymphoma; the combination of doxorubicin, bleomycin, vinblastine and dacarbazine (DTIC) in the treatment of Hodgkin's disease and the combination of cisplatin or carboplatin with any one, or a combination, of gemcitabine, paclitaxel, docetaxel, vinorelbine or etoposide in the treatment of non-small cell lung cancer.

Various sarcomas are treated by combination therapy, for example, for osteosarcoma combinations of doxorubicin and cisplatin or methotrexate with leucovorin are used; for advanced sarcomas etoposide can be used in combination with ifosfamide; for soft tissue sarcoma doxorubicin or dacarbazine can be used alone or, for advanced sarcomas, doxorubicin can be used in combination with ifosfamide or dacarbazine, or etoposide in combination with ifosfamide.

Ewing's sarcoma/peripheral neuroectodermal tumour (PNET) or rhabdomyosarcoma can be treated using etoposide and ifosfamide, or a combination of vincristine, doxorubicin and cyclophosphamide.

The alkylating agents cyclophosphamide, cisplatin and melphalan are also often used in combination therapies with other chemotherapeutics in the treatment of various cancers.

### Immunotherapeutic Agents

Immunotherapeutic agents suitable for use in combination with the siRNA molecules of the invention, with or without one or more chemotherapeutic agent, include, but are not limited to, cytokines, cancer vaccines, monoclonal antibodies and non-cytokine adjuvants. "Immunotherapeutic agents" in general refers to a compound, composition or treatment that indirectly or directly enhances, stimulates or augments the body's immune response against cancer cells and/or that lessens the side effects of other anticancer therapies.

Immunotherapeutic agents can be non-specific, *i.e*. boost the immune system generally so that it becomes more effective in fighting the growth and/or spread of cancer cells, or they can be specific, *i.e*. targeted to the cancer cells themselves. Immunotherapy regimens may combine the use of non-specific and specific immunotherapeutic agents. The present invention contemplates the use of the siRNA molecules with either non-specific or specific immunotherapeutic agents, or with combinations thereof. In one embodiment, the siRNA molecules are used in combination therapies with one or more non-specific immunotherapeutic agents.

Non-specific immunotherapeutic agents are substances that stimulate or indirectly augment the immune system. Some of these agents can be used alone as the main therapy for the treatment of cancer. Alternatively, non-specific immunotherapeutic agents may be given in addition to a main therapy and thus function as an adjuvant to enhance the effectiveness of other therapies (*e.g*. cancer vaccines) or reduce the side effects of other therapies, for example, bone marrow suppression induced by certain chemotherapeutic agents. Non-specific immunotherapeutic agents can act on key immune system cells and cause secondary responses, such as increased production of cytokines and immunoglobulins. Alternatively, the agents can themselves comprise cytokines. Non-specific immunotherapeutic agents are generally classified as cytokines or non-cytokine adjuvants.

A number of cytokines have found application in the treatment of cancer either as general non-specific immunotherapies designed to boost the immune system, or as adjuvants provided with other therapies. Suitable cytokines for use in the combination therapies of the present invention include interferons, interleukins and colony-stimulating factors.

Interferons (IFNs) contemplated by the present invention for use in combination with the siRNA molecules include the common types of IFNs, IFN-alpha (IFN-α), IFN-beta (IFN-β) and IFN-gamma (IFN-γ). IFNs can act directly on cancer cells, for example, by slowing their growth, promoting their development into cells with more normal behaviour and/or increasing their production of antigens thus making the cancer cells easier for the immune system to recognise and destroy. IFNs can also act indirectly on cancer cells, for example, by slowing down angiogenesis, boosting the immune system and/or stimulating natural killer (NK) cells, T cells and macrophages.

Recombinant IFN-α is available commercially as Roferon (Roche Pharmaceuticals) and Intron A (Schering Corporation). The use of IFN-α, alone or in combination with other immunotherapeutics or with chemotherapeutics, has shown efficacy in the treatment of various cancers including melanoma (including metastatic melanoma), renal cancer (including metastatic renal cancer), breast cancer, prostate cancer, cervical cancer (including metastatic cervical cancer), Kaposi's sarcoma, hairy cell leukemia, chronic myeloid leukemia (CML), multiple myeloma, follicular non-Hodgkin's lymphoma and cutaneous T cell lymphoma.

Interleukins contemplated by the present invention for use in combination with the siRNA molecules include IL-2 (or aldesleukin), IL-4, IL-11 and IL-12 (or oprelvekin). Examples of commercially available recombinant interleukins include Proleukin (IL-2; Chiron Corporation) and Neumega (IL-12; Wyeth Pharmaceuticals). Zymogenetics, Inc. (Seattle, WA) is currently testing a recombinant form of IL-21, which is also contemplated for use in the combinations of the present invention. Interleukins, alone or in combination with other immunotherapeutics or with chemotherapeutics, have shown efficacy in the treatment of various cancers including renal cancer (including metastatic renal cancer), melanoma (including metastatic melanoma), ovarian cancer (including recurrent ovarian cancer), cervical cancer (including metastatic cervical cancer), breast cancer, colorectal cancer, lung cancer, brain cancer, prostate cancer, leukemias and lymphomas.

Interleukins have also shown good activity in combination with IFN-α in the treatment of various cancers and the present invention contemplates the use of one or more interleukins and IFN-α in combination therapies with one or more siRNA molecules. An interleukin-immunotoxin conjugate known as denileukin diftitox (or Ontak; Seragen, Inc), which comprises IL-2 conjugated to diptheria toxin, has been approved by the FDA for the treatment of cutaneous T cell lymphoma.

Colony-stimulating factors (CSFs) contemplated by the present invention for use in combination with the siRNA molecules include granulocyte colony stimulating factor (G-CSF or filgrastim), granulocyte-macrophage colony stimulating factor (GM-CSF or sargramostim) and erythropoietin (epoetin alfa, darbepoietin). Treatment with one or more growth factors can help to stimulate the generation of new blood cells in patients undergoing traditional chemotherapy. Accordingly, treatment with CSFs can be helpful in decreasing the side effects associated with chemotherapy and can allow for higher doses of chemotherapeutic agents to be used. One embodiment of the present invention provides for the use of higher than standard doses of a chemotherapeutic agent in combination therapies with a siRNA molecule and one or more CSFs.

Various recombinant colony stimulating factors are available commercially, for example, Neupogen® (G-CSF; Amgen), Neulasta (pelfilgrastim; Amgen), Leukine (GM-CSF; Berlex), Procrit (erythropoietin; Ortho Biotech), Epogen (erythropoietin; Amgen), Arnesp (erythropoietin). Colony stimulating factors have shown efficacy in the treatment of cancer, including melanoma, colorectal cancer (including metastatic colorectal cancer), lung cancer and leukemia.

Non-cytokine adjuvants suitable for use in the combinations of the present invention include, but are not limited to, levamisole, alum hydroxide (alum), bacillus Calmette-Guerin (BCG), incomplete Freund's Adjuvant (IFA), QS-21, DETOX, Keyhole limpet hemocyanin (KLH) and dinitrophenyl (DNP). Non-cytokine adjuvants in combination with other immuno- and/or chemotherapeutics have demonstrated efficacy against various cancers including, for example, colon cancer and colorectal cancer (Levimasole); melanoma (BCG and QS-21); renal cancer and bladder cancer (BCG).

In addition to having specific or non-specific targets, immunotherapeutic agents can be active, *i.e*. stimulate the body's own immune response, or they can be passive, *i.e*. comprise immune system components that were generated external to the body. Both types of immunotherapeutic agents are suitable for use with the siRNA molecules in the combination therapies of the present invention. In one embodiment, the siRNA molecules are used in combination therapies with one or more active immunotherapeutic agents.

Passive immunotherapy typically involves the use of one or more monoclonal antibodies that are specific for a particular antigen found on the surface of a cancer cell or that are specific for a particular cell growth factor. Monoclonal antibodies may be used in the treatment of cancer in a number of ways, for example, to enhance a subject's immune response to a specific type of cancer, to interfere with the growth of cancer cells by targeting specific cell growth factors, such as those involved in angiogenesis, or by enhancing the delivery of other anticancer agents to cancer cells when linked to such agents.

The present invention contemplates the use of one or more monoclonal antibody in combination with a siRNA molecule for the treatment of cancer. Monoclonal antibodies currently used as cancer immunotherapeutic agents that are suitable for inclusion in the combinations of the present invention include, but are not limited to, rituximab (Rituxan®), trastuzumab (Herceptin®), ibritumomab tiuxetan (Zevalin®), tositumomab (Bexxar®), cetuximab (C-225, Erbitux®), bevacizumab (Avastin®), gemtuzumab ozogamicin (Mylotarg), alemtuzumab (Campath) and ibritumomab tiuxetan (Zevalin).

Monoclonal antibodies are used in the treatment of a wide range of cancers including lymphomas (such as non-Hodgkin's lymphoma, B cell chronic lymphocytic leukemia (B-CLL)), myelomas (such as multiple myeloma), leukemias (such as B cell leukemia), breast cancer (including advanced metastatic breast cancer), colorectal cancer (including advanced and/or metastatic colorectal cancer), ovarian cancer, lung cancer, prostate cancer, cervical cancer, melanoma and brain tumours. Monoclonal antibodies can be used alone or in combination with other immunotherapeutic agents or chemotherapeutic agents.

Active specific immunotherapy typically involves the use of cancer vaccines. Cancer vaccines have been developed that comprise whole cancer cells, parts of cancer cells or one or more antigens derived from cancer cells. Cancer vaccines, alone or in combination with one or more immuno- or chemotherapeutic agents are being investigated in the treatment of several types of cancer including melanoma, renal cancer, ovarian cancer, breast cancer, colorectal cancer, lung cancer and leukemia. Non-specific immunotherapeutics are useful in combination with cancer vaccines in order to enhance the body's immune response. The present invention encompasses combination therapies comprising a cancer vaccine in combination with a siRNA molecule. The combination may further comprise one or more non-specific immunotherapeutic agents.

### CLINICAL TRIALS IN CANCER PATIENTS

One skilled in the art will appreciate that, following the demonstrated effectiveness of a siRNA molecule *in vitro* and in animal models, it should be be submitted to standard GLP animal toxicology and pharmacokinetic studies and then be entered into Clinical Trials in order to further evaluate its efficacy in the treatment of cancer and to obtain regulatory approval for therapeutic use. As is known in the art, clinical trials progress through phases of testing, which are identified as Phases I, II, III, and IV.

Initially, the selected siRNA molecule will be evaluated in a Phase I trial, which is usually an open-label trial. Typically Phase I trials are used to determine the best mode of administration (for example, by pill or by injection), the frequency of administration, and the toxicity for the siRNA molecule. Phase I studies frequently include laboratory tests, such as blood tests and biopsies, to evaluate the effects of the siRNA molecule in the body of the patient. For a Phase I trial, a small group of cancer patients are treated with a specific dose of the siRNA molecule. During the trial, the dose is typically increased group by group in order to determine the maximum tolerated dose (MTD) and the dose-limiting toxicities (DLT) associated with the siRNA molecule. This process determines an appropriate dose to use in a subsequent Phase II trial.

A Phase II trial can be conducted to further evaluate the effectiveness and safety of the siRNA molecule. Phase II trials are usually open-label, but may also be blinded. In Phase II trials, the siRNA molecule is administered to groups of patients with either one specific type of cancer or with related cancers, using the dosage found to be effective in Phase I trials.

Phase III trials focus on determining how the selected siRNA molecule compares to the standard, or most widely accepted, treatment. Phase III trials are generally blinded. In Phase III trials, patients are randomly assigned to one of two or more "arms". In a trial with two arms, for example, one arm will receive the standard treatment (control group) and the other arm will receive siRNA treatment (investigational group).

Phase IV trials are used to further evaluate the long-term safety and effectiveness of the siRNA molecule. Phase IV trials are less common than Phase I, II and III trials and will take place after the siRNA molecule has been approved for standard use.

### Eligibility of Patients for Clinical Trials

Participant eligibility criteria can range from general (for example, age, sex, type of cancer) to specific (for example, type and number of prior treatments, tumour characteristics, blood cell counts, organ function). Eligibility criteria may also vary with trial phase. For example, in Phase I and II trials, the criteria often exclude patients who may be at risk from the investigational treatment because of abnormal organ function or other factors. In Phase II and III trials additional criteria are often included regarding disease type and stage, and number and type of prior treatments.

Phase I cancer trials usually comprise 15 to 30 participants for whom other treatment options have not been effective. Phase II trials typically comprise up to 100 participants who have already received chemotherapy, surgery, or radiation treatment, but for whom the treatment has not been effective. Participation in Phase II trials is often restricted based on the previous treatment received. Phase III trials usually comprise hundreds to thousands of participants. This large number of participants is necessary in order to determine whether there are true differences between the effectiveness of the siRNA molecule and the standard treatment. Phase III may comprise patients ranging from those newly diagnosed with cancer to those with extensive disease in order to cover the disease continuum.

One skilled in the art will appreciate that clinical trials should be designed to be as inclusive as possible without making the study population too diverse to determine whether the treatment might be as effective on a more narrowly defmed population. The more diverse the population included in the trial, the more applicable the results could be to the general population, particularly in Phase III trials. Selection of appropriate participants in each phase of clinical trial is considered to be within the ordinary skills of a worker in the art.

### Assessment of patients prior to treatment

Prior to commencement of the study, several measures known in the art can be used to first classify the patients. Patients can first be assessed, for example, using the Eastern Cooperative Oncology Group (ECOG) Performance Status (PS) scale. ECOG PS is a widely accepted standard for the assessment of the progression of a patient's disease as measured by functional impairment in the patient, with ECOG PS 0 indicating no functional impairment, ECOG PS 1 and 2 indicating that the patients have progressively greater functional impairment but are still ambulatory and ECOG PS 3 and 4 indicating progressive disablement and lack of mobility.

Patients' overall quality of life can be assessed, for example, using the McGill Quality of Life Questionnaire (MQOL) (Cohen et al (1995) Palliative Medicine 9: 207-219). The MQOL measures physical symptoms; physical, psychological and existential well-being; support; and overall quality of life. To assess symptoms such as nausea, mood, appetite, insomnia, mobility and fatigue the Symptom Distress Scale (SDS) developed by McCorkle and Young ((1978) Cancer Nursing 1: 373-378) can be used.

Patients can also be classified according to the type and/or stage of their disease and/or by tumour size.

### Administration of the siRNA Molecule in Clinical Trials

The selected siRNA molecule is typically administered to the trial participants parenterally, for example, by intravenous infusion. Methods of administering drugs by intravenous infusion are known in the art. Usually intravenous infusion takes place over a certain time period, for example, over the course of 60 minutes to several days. A range of doses of the siRNA molecule can be tested.

### Pharmacokinetic monitoring

To fulfil Phase I criteria, distribution of the siRNA molecule is monitored, for example, by chemical analysis of samples, such as blood or urine, collected at regular intervals. For example, samples can be taken at regular intervals up until about 72 hours after the start of infusion. In one embodiment, samples are taken at 0, 0.33, 0.67, 1, 1.25, 1.5, 2, 4, 6, 8, 12, 24, 48 and 72 hours after the start of each infusion of the siRNA molecule.

If analysis is not conducted immediately, the samples can be placed on dry ice after collection and subsequently transported to a freezer to be stored at -70 °C until analysis can be conducted. Samples can be prepared for analysis using standard techniques known in the art and the amount of the siRNA molecule present can be determined, for example, by high-performance liquid chromatography (HPLC).

Pharmacokinetic data can be generated and analyzed in collaboration with an expert clinical pharmacologist and used to determine, for example, clearance, half-life and maximum plasma concentration.

### Monitoring of Patient Outcome

The endpoint of a clinical trial is a measurable outcome that indicates the effectiveness of the siRNA molecule under evaluation. The endpoint is established prior to the commencement of the trial and will vary depending on the type and phase of the clinical trial. Examples of endpoints include, for example, tumour response rate - the proportion of trial participants whose tumour was reduced in size by a specific amount, usually described as a percentage; disease-free survival - the amount of time a participant survives without cancer occurring or recurring, usually measured in months; overall survival - the amount of time a participant lives, typically measured from the beginning of the clinical trial until the time of death. For advanced and/or metastatic cancers, disease stabilization - the proportion of trial participants whose disease has stabilized, for example, whose tumour(s) has ceased to grow and/or metastasize, can be used as an endpoint. Other endpoints include toxicity and quality of life.

Tumour response rate is a typical endpoint in Phase II trials. However, even if a treatment reduces the size of a participant's tumour and lengthens the period of disease-free survival, it may not lengthen overall survival. In such a case, side effects and failure to extend overall survival might outweigh the benefit of longer disease-free survival. Alternatively, the participant's improved quality of life during the tumour-free interval might outweigh other factors. Thus, because tumour response rates are often temporary and may not translate into long-term survival benefits for the participant, response rate is a reasonable measure of a treatment's effectiveness in a Phase II trial, whereas participant survival and quality of life are typically used as endpoints in a Phase III trial.

### PHARMACEUTICAL KITS

The present invention additionally provides for therapeutic kits containing one or more siRNA molecules or a pharmaceutical composition comprising the siRNA molecule(s) for use in the treatment of cancer. Individual components of the kit would be packaged in separate containers and, associated with such containers, can be a notice in the form prescribed by a governmental agency regulating the manufacture, use or sale of pharmaceuticals or biological products, which notice reflects approval by the agency of manufacture, use or sale for human administration.

When the components of the kit are provided in one or more liquid solutions, the liquid solution can be an aqueous solution, for example a sterile aqueous solution. In this case the container means may itself be an inhalant, syringe, pipette, eye dropper, or other such like apparatus, from which the composition may be administered to a patient or applied to and mixed with the other components of the kit.

The components of the kit may also be provided in dried or lyophilised form and the kit can additionally contain a suitable solvent for reconstitution of the lyophilised components. Irrespective of the number or type of containers, the kits of the invention also may comprise an instrument for assisting with the administration of the composition to a patient. Such an instrument may be an inhalant, syringe, pipette, forceps, measured spoon, eye dropper or any such medically approved delivery vehicle.

The kit may further comprise appropriate reagents for formulating the siRNA molecule(s) for delivery, for example, for encapsulation of the siRNA molecule, association with a biodegradable polymer or for the preparation of a liposomal solution.

The kit may additionally comprise one or more other chemotherapeutic and/or immunotherapeutic agents for administration in conjunction with the siRNA molecule(s).

To gain a better understanding of the invention described herein, the following examples are set forth. It should be understood that these examples are for illustrative purposes only. Therefore, they should not limit the scope of this invention in any way.

### EXAMPLES

### EXAMPLE 1: IDENTIFICATION OF SUITABLE TARGET SITES IN THE HUMAN RIBONUCLEOTIDE REDUCTASE R1 AND R2 mRNA

Ambion's siRNA Target Finder® and Qiagen's siRNA design tool were employed to find potential siRNA target sequences within the mRNA sequences encoding the R1 and R2 subunits of human ribonucleotide reductase. Results from these searches were compared and combined to generate a list of potential siRNA target sequences to the R1 subunit (see Table 1) and the R2 subunit (see Table 2). To reduce the size of this list, so as, to find potentially the best siRNA targets, sequences that had a G/C-content close to 50% (ranging from 42.5% to 52.5%) were selected. The list was further reduced by performing a BLAST-search of the NCBI database to rule out sequences with homology to other regions of the human genome. siRNA target sequences were eliminated from the list when they contained 10 or more nucleotides of homology in a row to other human sequences or when they had a total of 20 or 21 nucleotides of homology to other human sequences, *i.e*. fewer than 2 mismatches. In addition, sequences within the 5' and 3' UTRs were not chosen at this stage.

The above search allowed for the establishment of a more concise and definite list of siRNA target sequences for the R1 subunit (see Table 8) and the R2 subunit (see Table 9).

**Table 8: Target Sites for siRNA within the mRNA encoding the R1 Subunit**

| **Position in mRNA sequence** | **Sequence (5' to 3')** | **SEQ ID NO** |
|---|---|---|
| 361 | AATCCAAGGCTTGTACAGTGG | 9 |
| 396 | AACTAGATACTTTGGCTGCTG | 10 |
| 427 | AACCTTGACTACTAAGCACCC | 13 |
| 614 | AAAGATCGCCTGAATTCTGCT | 21 |
| 615 | AAGATCGCCTGAATTCTGCTA | 22 |
| 677 | AAGACGCTAGAGCGGTCTTAT | 24 |
| 973 | AATTGGTGTTGCTGTGAGTTG | 38 |
| 1040 | AATGGCCTTGTACCGATGCTG | 41 |
| 1181 | AACACAGGAAAGGAAGAGCAG | 47 |
| 1250 | AAACGAGTGGAGACTAATCAG | 48 |
| 1251 | AACGAGTGGAGACTAATCAGG | 49 |
| 1358 | AAACAAGGTCGTGTCCGCAAA | 54 |
| 1359 | AACAAGGTCGTGTCCGCAAAG | 55 |
| 1362 | AAGGTCGTGTCCGCAAAGTTG | 56 |
| 1487 | AACCTGGGAACCATCAAATGC | 64 |
| 1524 | AAATAGTGGAGTACACCAGCA | 69 |
| 1525 | AATAGTGGAGTACACCAGCAA | 70 |
| 1635 | AAGTCACTAAAGTCGTTGTCC | 75 |
| 1643 | AAAGTCGTTGTCCGAAACTTG | 76 |
| 1644 | AAGTCGTTGTCCGAAACTTGA | 77 |
| 1682 | AACTACTATCCTGTACCAGAG | 79 |
| 1800 | AAGCCCAGTTACTGAATAAGC | 81 |
| 1893 | AAACCTATGAGGGCTCTCCAG | 82 |
| 1894 | AACCTATGAGGGCTCTCCAGT | 83 |
| 2099 | AACATCTATACTCGCAGAGTC | 88 |
| 2417 | AAGCAGGGTTTGAAGACTGGG | 105 |

### EXAMPLE 2: SELECTION OF RIBONUCLEOTIDE REDUCTASE R2 MRNA TARGET SEQUENCES FOR DESIGN OF siRNAs

siRNAs were designed to target the R2 subunit of ribonucleotide reductase. The criteria used for selection of mRNA target sequences were as described above. Additional criteria included, selection of siRNA target sites containing the sequence motif AA (N19), where N represents any nucleotide, with sense and antisense strands being designed such that the N19 region would form a duplex with 2 T's at each end producing 3' overhangs. Sequences containing more than 3 G's in a row were avoided. In addition, siRNA sequences targeting R2 mRNA in the region 100 bases downstream of the start codon AUG were avoided.

Table 9 provides a list of 7 R2 subunit mRNA target sequences selected for the design of siRNAs. Scrambled sequences of each target sequence were also used to design control siRNAs for use in both *in vitro* and *in vivo* experiments, as described in following sections. All siRNAs were purchased from Qiagen. The siRNA sequences described in Table 9 include sequences (624 and 624+1) complementary to a R2 antisense sequence known to be effective in the treatment of cancer (see Lee et al., Cancer Res. 63:2802-2811 (2003)). Table 10 provides the sequences for the sense and antisense strands of the siRNA molecules designed to the target sequences listed in Table 9, and Table 10 provides the sequences for the sense and antisense strands of the scrambled control sequences. The sequences provided in Table 10 target mRNA sequences within the coding region of the ribonucleotide reductase mRNA that are either within or between exons 4, 5, 6, 7 and 10.

**Table 9A: siRNA Target Sequences Identifed within the mRNA Encoding the R2 Subunit**

| **Position of Target Sequence in R2 mRNA** | **R2 Target Sequences (5' to 3')** | **SEQ ID NO** |
|---|---|---|
| 544 | AATCCCTGAAACCCGAGGAGA | 127 |
| 624 | AACTTGGTGGAGCGATTTAGC | 135 |
| 624+1 | AACTTGGTGGAGCGATTTAGCC | 412 |
| 787 | AAACGATGCCTTGTGTCAAGA | 143 |
| 844 | AAGAGGCTACCTATGGTGAAC | 146 |
| 933 | AAGAAACGAGGACTGATGCCT | 149 |
| 1284 | AAGAGAGTAGGCGAGTATCAG | 162 |

**Table 9B: Scrambled Control Sequences Corresponding to siRNA Target Sequences Identifed within the mRNA Encoding the R2 Subunit**

| **Position of Target Sequence in R2 mRNA** | **Scrambled Sequences (5' to 3')** | **SEQ ID NO** |
|---|---|---|
| 544 | AACTAGGTACCACACGAGAGC | 413 |
| 624 | AATGCTAGTGTCGTAGTCAGC | 414 |
| 624+1 | AATGCTAGTGTCGTAGTGAGCC | 415 |
| 787 | AATCGAGTACTGAGACTACTG | 416 |
| 844 | AATGCAGGACTGAGACTACTG | 417 |
| 933 | AACTAGCGTACAGATGAGAGC | 418 |
| 1284 | AACTAGGGTAGACGATGAGAG | 419 |

**Table 10: Sequences of Sense and Antisense Strands of siRNA Molecules Against Ribonucleotide Reductase**

| **siRNA** | **siRNA Sequences (5' to 3')*** | | **SEQ ID NO** |
|---|---|---|---|
| 544 | **Sense**: | r(UCCCUGAAACCCGAGGAGA)d(TT) | 422 |
| | **Antisense:** | r(UCUCCUCGGGUUUCAGGGA)d(TT) | 423 |
| 624 | **Sense:** | r(CUUGGUGGAGCGAUUUAGC)d(TT) | 424 |
| | **Antisense:** | r(GCUAAAUCGCUCCACCAAG)d(TT) | 425 |
| 624+1 | **Sense:** | r(CUUGGUGGAGCGAUUUAGCC)d(TT) | 426 |
| | **Antisense:** | r(GGCUAAAUCGCUCCACCAAG)d(TT) | 427 |
| 787 | **Sense:** | r(ACGAUGCCUUGUGUCAAGA)d(TT) | 428 |
| | **Antisense:** | r(UCUUGACACAAGGCAUCGU)d(TT) | 429 |
| 844 | **Sense:** | r(GAGGCUACCUAUGGUGAAC)d(TT) | 430 |
| | **Antisense:** | r(GUUCACCAUAGGUAGCCUC)d(TT) | 431 |
| 933 | **Sense:** | r(GAAACGAGGACUGAUGCCU)d(TT) | 432 |
| | **Antisense:** | r(AGGCAUCAGUCCUCGUUUC)d(TT) | 433 |
| 1284 | **Sense:** | r(GAGAGUAGGCGAGUAUCAG)d(TT) | 434 |
| | **Antisense:** | rCUGAUACUCGCCUACUCUC)d(TT) | 435 |

| | | | |
|---|---|---|---|
| *The siRNA molecules contain ribonucleotides in the regions that anneal to form the dsRNA and deoxyribonucleotides in the overhangs. This distinction is indicated in the table, wherein 'r' indicates ribonucleotides and 'd' indicates deoxyribonucleotides, with all nucleotides the designation applies to being in parentheses. | | | |

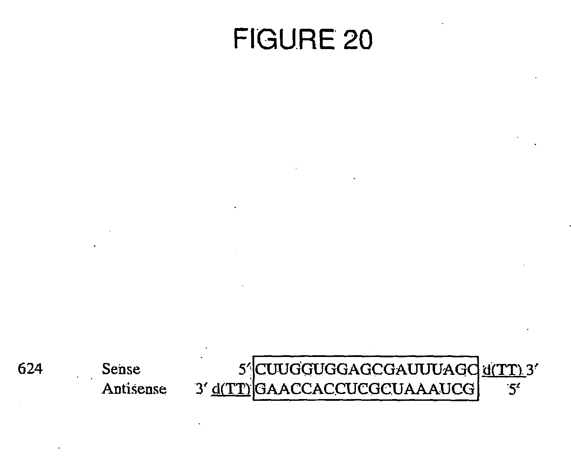

A representative example of an annealed siRNA duplex is shown in Figure 20. In this Figure, the boxed in ribonucleotides represent the RNA duplex and the d(TT) overhangs at each of the 3' ends are deoxyribonucleotides (which help to stabilize the siRNAs).

### EXAMPLE 3: EFFECT OF siRNAS ON LEVELS OF RIBONUCLEOTIDE REDUCTASE R2 mRNA IN RENAL AND BREAST CANCER CELLS IN VITRO

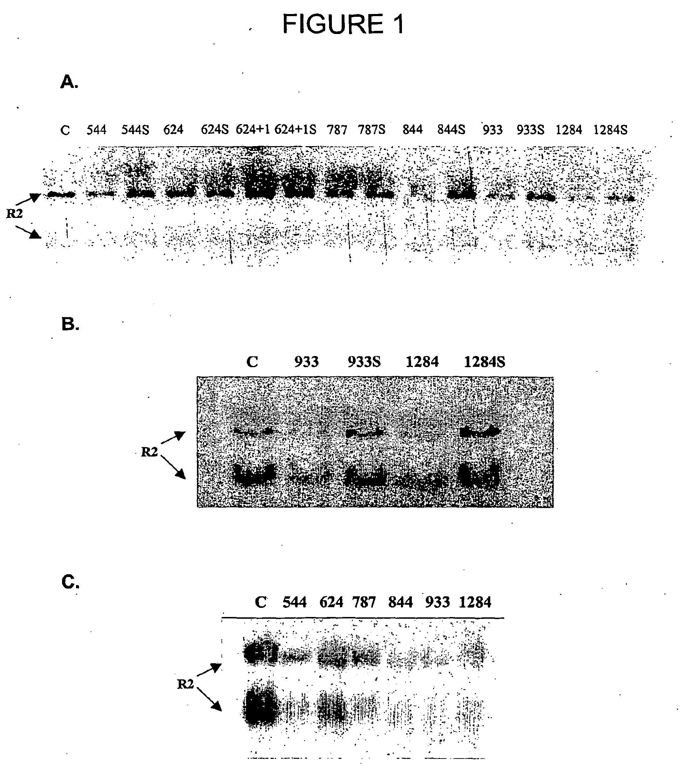

The effects of siRNA treatment on R2 mRNA levels in renal carcinoma cells (A-498) and breast adenocarcinoma cells (MDA-MB-231) were investigated as follows. A-498 cells and MDA-MB-231 cells were grown to subconfluency (30-50%) and then transfected with 0.2 µM of siRNAs 544, 624, 624+1, 787, 844, 933, 1284 and the corresponding scrambled control sequence of each (544S, 624S, 624+1S, 787S, 844S, 933S, 1284S) as indicated in Figure 1. Transfection proceeded for 28 hours in the presence of Oligofectamine Reagent (Invitrogen) prior to the isolation ofRNA from cells using TRIzol reagent (GIBCO BRL) and Northern blot analysis. RNA was subjected to electrophoresis through 1% agarose gels followed by transfer to nylon membranes and hybridization to a fragment of R2 cDNA labelled with α³²P-dCTP.

The results of these experiments are shown in Figure 1. Northern blot analysis indicated that transfection of A-498 cells and MDA-MB-231 cells with siRNAs 844, 933, 1284 and to a lesser extent 544 and 787, specifically reduces R2 mRNA levels, in comparison to cells treated with Oligofectamine alone (control - C) or those cells treated with scrambled sequences (S).

### EXAMPLE 4: EFFECT OF siRNAS ON LEVELS OF RIBONUCLEOTIDE REDUCTASE R2 PROTEIN IN RENAL AND BREAST CANCER CELLS IN VITRO

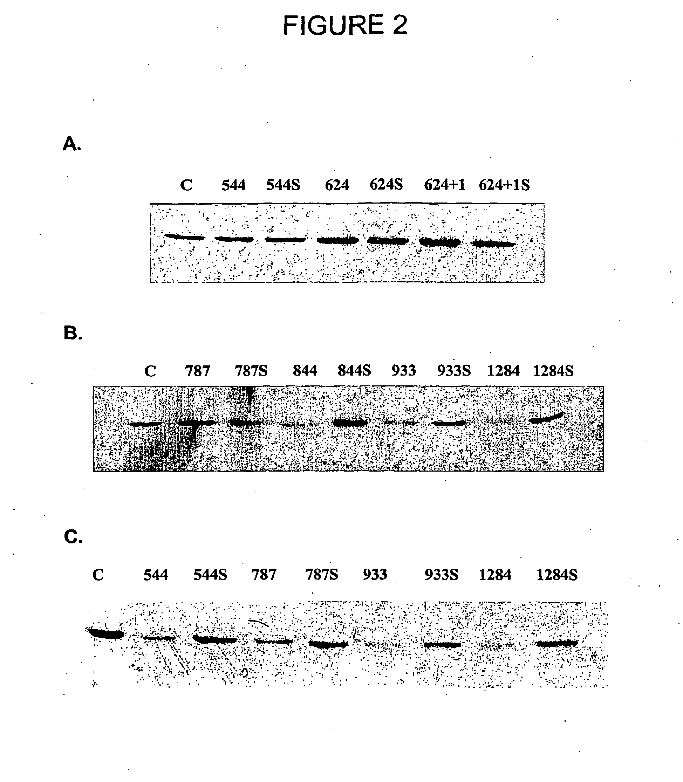

The effects of siRNA treatment on R2 protein levels in renal carcinoma cells (A-498) and breast adenocarcinoma cells (MDA-MB-231) were investigated as follows. A-498 cells and MDA-MB-231 cells were grown to subconfluency (30-50%) and then transfected with 0.2 µM of siRNAs 544, 624, 624+1, 787, 844, 933, 1284 and the corresponding scrambled control sequence of each (544S, 624S, 624+1S, 787S, 844S, 933S, 1284S) as indicated in Figure 2. Transfection proceeded for 28 hours in the presence of Oligofectamine Reagent (Invitrogen) prior cell lysis and western blot analysis. Protein samples were subjected to electrophoresis through 10% SDS-polyacrylamide gels followed by transfer to nitrocellulose membranes. Immunodetection was performed by incubating the membrane with anti-R2 primary antibody, anti-goat IgG horseradish peroxidase linked secondary antibody, and ECL detection reagents.

The results of this experiment are depicted in Figure 2. Western blot analysis indicated that transfection of A-498 cells and MDA-MB-231 cells with siRNAs 844, 933, and 1284, and to a lesser extent siRNAs 544 and 787, specifically reduces R2 protein levels, in comparison to cells treated with Oligofectamine alone (control - C) or those cells treated with scrambled sequences (S).

### EXAMPLE 5: EFFECT OF siRNAS ON THE GROWTH OF RENAL CARCINOMA CELLS IN SCID MICE IN VIVO

The ability of each of the siRNAs designed from sequences listed in Table 9 to inhibit the growth of human renal carcinoma (A-498) in SCID mice was examined as follows. A-498 human renal cancer cells were subcutaneously injected into the right flank of SCID mice. After the size of tumours reached an approximate volume of 80 to 90 mm³, 17 days post-tumour cell injection, R2 target siRNA sequences and scrambled siRNA sequences were administered via the tail vein three times per week at 250 µg/kg. Control animals received saline alone, also three times per week. Treatment was administered for approximately 5 weeks. Anti-tumour activity or potential was determined by the inhibition of tumour growth, based on mean tumour volume, which was measured with a caliper once per week and based on mean tumour weight of excised tumours which was determined at the end of the experiment. The mean tumour volume and mean tumour weight was calculated from 8 animals per experimental group.

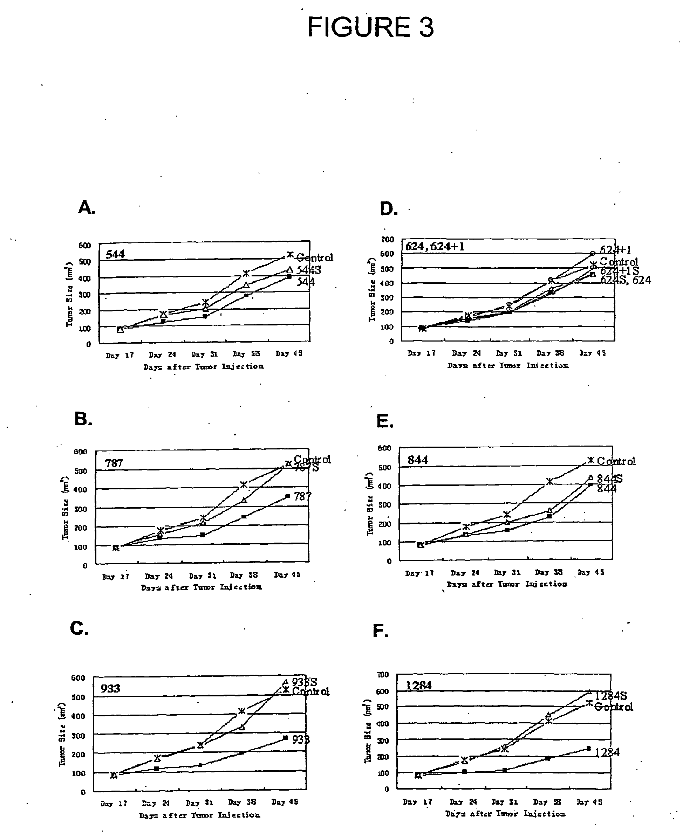

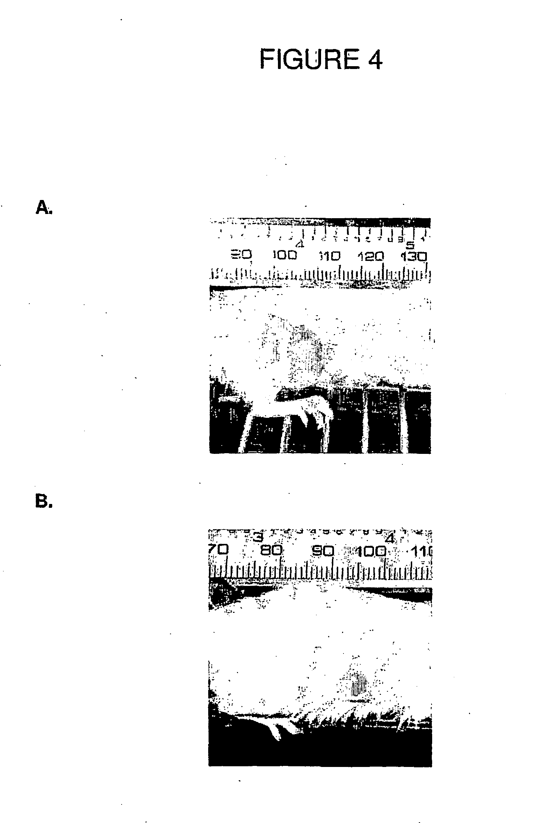

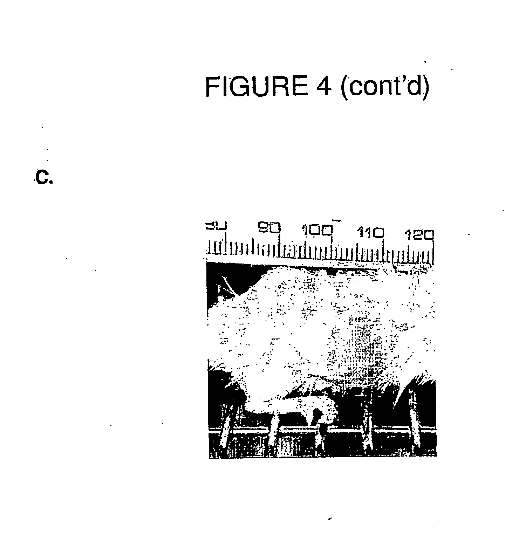

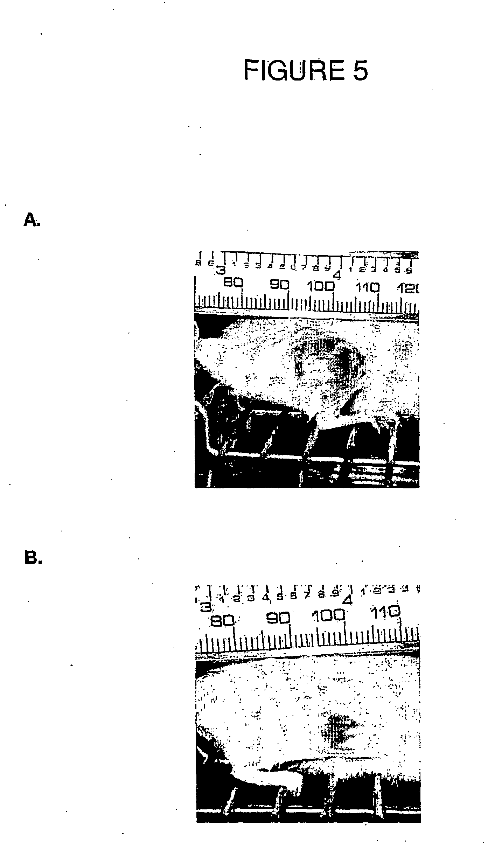

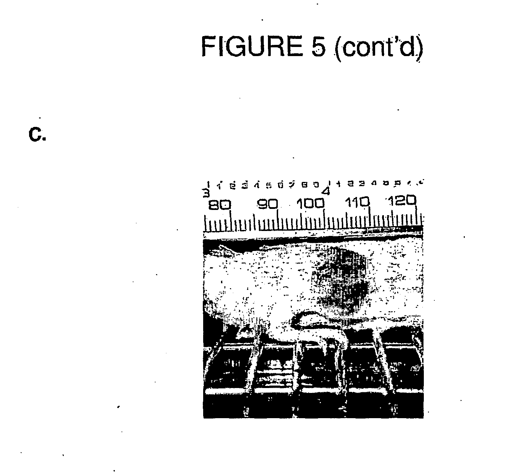

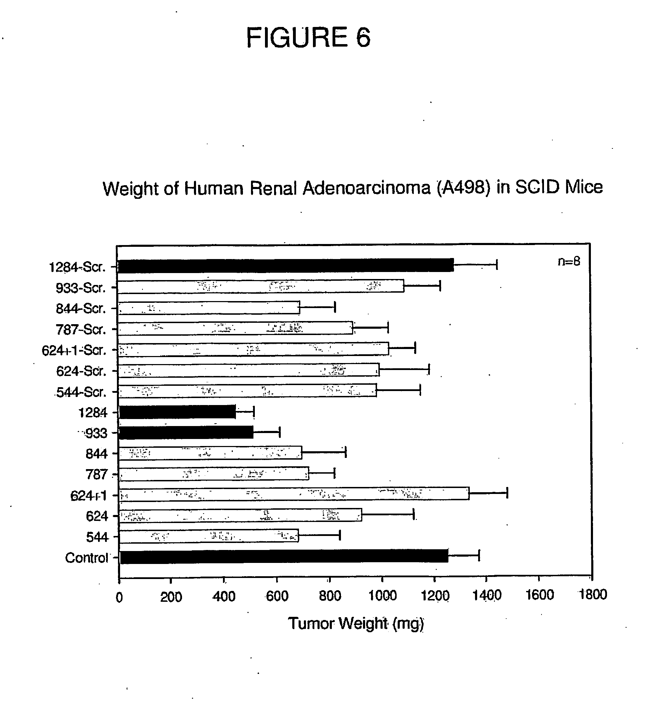

The results of this experiment are shown in Figures 3-6. Mean tumour volume (mm³) for each group *i.e*. 544, 624, 624+1, 787, 844, 933, 1284, 544S, 624S, 624+1S, 787S, 844S, 933S, 1284S and control, was determined each week for five weeks. As shown in Figure 3, siRNAs 933 and 1284 significantly inhibited tumour growth in this treatment period as compared to their corresponding scrambled siRNA sequences and saline control. Intermediate effects in inhibition of tumour growth were observed with siRNA 787 and 544 and 844. Photographs of the tumours, after three and four weeks of treatment, are provided in Figures 4 and 5 for 1284, 1284S and control groups. Tumour weight for each group at the end of approximately 5 weeks of treatment is shown in Figure 6.

Treatment with the siRNAs can be continued to six weeks, or longer if desired. As is known in the art the length of treatment can influence the efficacy of a therapeutic compound, therefore, siRNAs that showed only minimal or no effects after 4 weeks of treatment may be shown to be effective after an additional time period. At the end of the additional period of treatment, tumours can be excised from the animals in different treatment groups and tumour weight can be determined.

### EXAMPLE 6: EFFECT OF siRNAS ON LEVELS OF RIBONUCLEOTIDE REDUCTASE R2 mRNA IN RENAL CARCINOMA TUMOURS FROM SCID MICE

The effects of siRNA treatment on R2 mRNA levels in renal carcinoma (A-498) tumours excised from SCID mice, from Example 5, were investigated. Briefly, tumour samples were excised from animals in the groups treated with siRNAs 544, 544S, 933, 933S, 1284, 1284S and control described in Example 5, for Northern blot analysis. To extract RNA from the tumours, samples were homogenized in the presence of TRIzof^{™} reagent (GIBCO BRL), followed by chloroform extraction and ethanol precipitation. RNA was subjected to electrophoresis through 1% agarose gel containing formaldehyde followed by transfer to a nylon membrane and hybridization to a fragment of R2 labelled with α³²P-dCTP. The 23 kD highly basic protein was used as a loading control.

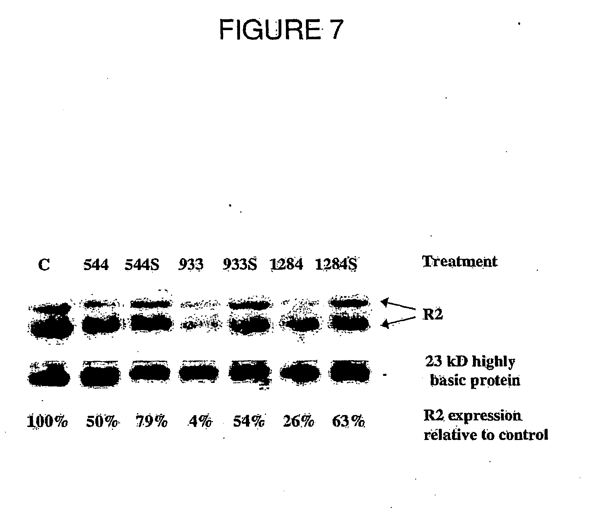

The results of this experiment are shown in Figure 7. Northern blot analysis indicated that treatment of SCID mice, carrying human renal carcinoma (A-498) tumours, with 933 and 1284 and to a lesser extent, with 544, specifically reduces R2 mRNA levels, in comparison to mice treated with saline alone (control - C) or those mice treated with scrambled sequences (S) (Figure 7).

### EXAMPLE 7: EFFECT OF siRNAS ON LEVELS OF RIBONUCLEOTIDE REDUCTASE R2 PROTEIN IN RENAL CARCINOMA TUMOURS FROM SCID MICE

The effects of siRNA treatment on R2 protein levels in renal carcinoma (A-498) tumours excised from SCID mice, from Example 5, were investigated as follows. Tumour samples were excised from animals in the groups treated with siRNAs 933, 933S, 1284, 1284S and control described in Example 5, for Western blot analysis. To extract protein from the tumours, samples were homogenized in the presence of protein extraction buffer. Protein samples were subjected to electrophoresis through 10% SDS-polyacrylamide gels followed by transfer to nitrocellulose membranes. Immunodetection, was performed by incubating the membrane with anti-R2 primary antibody, anti-goat IgG horseradish peroxidase linked secondary antibody and ECL detection reagents. GAPDH was used as a loading control.

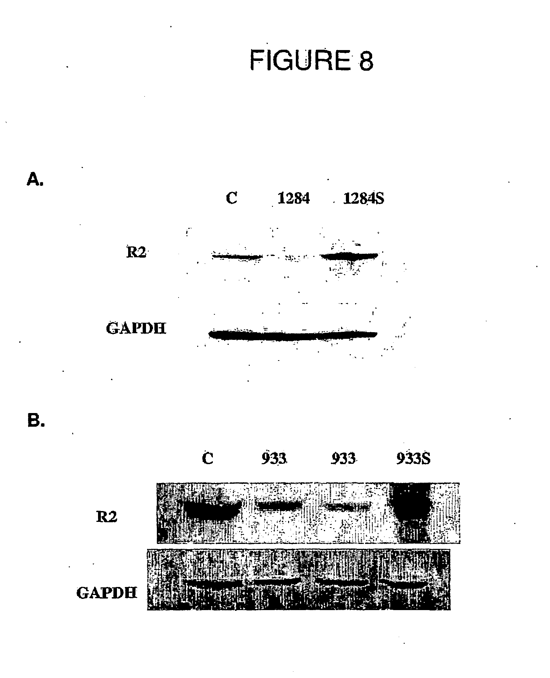

The results of this study are shown in Figure 8. Western blot analysis indicated that treatment of SCID mice carrying human renal carcinoma (A-498) tumours with siRNAs 933 and 1284 specifically reduces R2 protein levels, in comparison to mice treated with saline alone (control - C) or those mice treated with scrambled sequences (S). One sample for 1284 and two samples for 933 are shown in Figure 8.

### EXAMPLE 8: DOSE DEPENDENT EFFECT OF siRNAS ON THE GROWTH OF RENAL CARCINOMA CELLS IN SCID MICE IN VIVO

A dose response experiment was performed to examine the inhibition of growth of human renal carcinoma (A-498) in SCID mice when treated with three different concentrations of siRNA 1284 as follows. A-498 human renal cancer cells were subcutaneously injected into the right flank of SCID mice. After the size of tumours reached an approximate volume of 80 to 90 mm³, 17 days post-tumour cell injection, siRNA 1284 (targeting R2) was administered via the tail vein three times per week at 125 µg/kg, 250 µg/kg or 500 µg/kg. The scrambled sequence of 1284 was administered three times per week (500 µg/kg) and control animals received saline alone, also three times per week. Tumour volume was monitored each week over 5 weeks and at the end of the experiment, tumours were excised from the animals in different treatment groups and tumour weight was determined for each group. The mean tumour weight was calculated from 10 animals per experimental group.

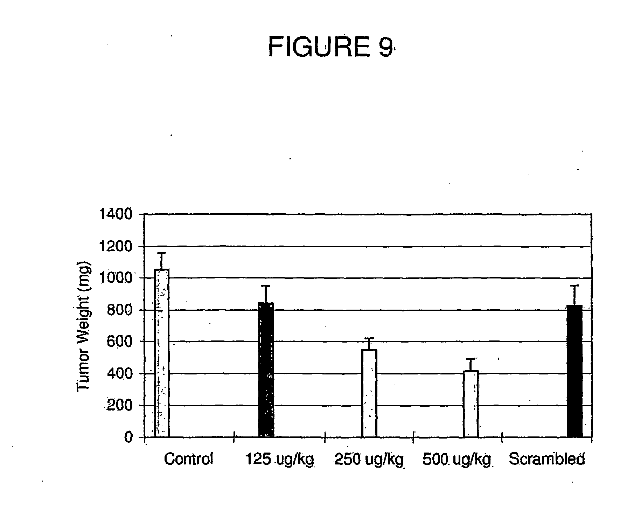

Mean tumour weight for all groups is presented in Figure 9. A dose dependent response to treatment with siRNA 1284 occurred as siRNA treatment with 1284 - 500 µg/kg produced the greatest inhibition of tumour growth, followed by 250 µg/kg, and then by 125 µg/kg. The inhibition of growth of tumours treated with 1284 (500 µg/kg and 250 µg/kg) was statistically significant as compared to the corresponding scrambled siRNA sequences and saline control treatment groups.

### EXAMPLE 9: DOSE DEPENDENT EFFECT OF siRNAS ON LEVELS OF RIBONUCLEOTIDE REDUCTASE R2 PROTEIN IN RENAL CARCINOMA TUMOURS FROM SCID MICE

The effects of siRNA treatment on R2 protein levels in renal carcinoma (A-498) tumours excised from SCID mice were investigated as follows. At the end of the *in vivo* study (described in Example 8), tumour samples were excised from all treatment groups for Western blot analysis. To extract protein from the tumours, samples were homogenized in the presence of protein extraction buffer. Protein samples were subjected to electrophoresis through 10% SDS-polyacrylamide gels followed by transfer to nitrocellulose membranes. Immunodetection was performed by incubating the membrane with anti-R2 primary antibody, anti-goat IgG horseradish peroxidase linked secondary antibody and ECL detection reagents. GAPDH was used as a loading control.

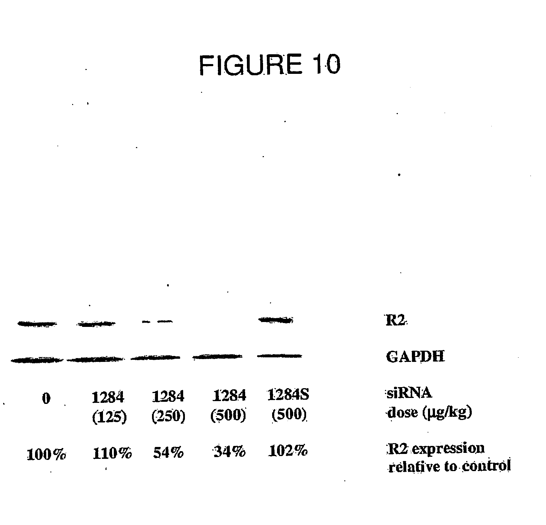

The results of this study are shown in Figure 10. The data indicate that inhibition of R2 protein expression is dose dependent.siRNA treatment with 1284 at 500 µg/kg produced the greatest inhibition of R2 protein expression, followed by 250 µg/kg, with 125 µg/kg producing no inhibition of R2 protein expression in comparison to scrambled and saline control groups (Figure 10).

### EXAMPLE 10: EFFECT OF siRNAS ON THE GROWTH OF RENAL CARCINOMA CELLS IN VITRO

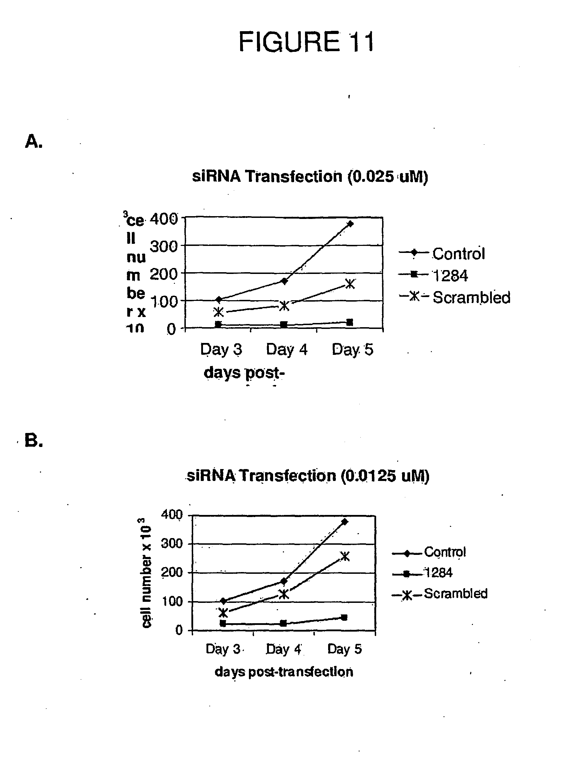

The effect of siRNA treatment on cellular proliferation of human renal cancer cell line (A-498) was investigated as follows. A-498 cells were grown to subconfluency (30-50%) and then transfected with 0.025 or 0.0125 µM of siRNA 1284 or the corresponding scrambled control sequence (1284S). Control samples (without siRNA) were also prepared. Transfection proceeded for 16 hours in the presence of Oligofectamine Reagent (Invitrogen) at which point siRNA sequences were removed and replaced with α-MEM complete media. At 3 days, 4 days and 5 days post-transfection cells were counted and cell numbers graphed as indicated in Figure 11.

Treatment with 0.025 µM and 0.0125 µM siRNA 1284, inhibited cell proliferation compared to scrambled or control treatment as shown in Figure 11A (0.025 µM) Figure 11B (0.0125 µM).

Protein samples were prepared from A-498 cells transfected with 0.025 and 0.0125 µM siRNA 1284 and analyzed by Western blot. Both concentrations of siRNA 1284 tested significantly dowri-regulated R2 protein.

### EXAMPLE 11: EFFECT OF siRNAS ON THE GROWTH OF RENAL CANCER CELLS IN VITRO

The effect of siRNA treatment on cellular proliferation of human renal tumour cell line (A-498) was investigated using a XTT assay as follows. A-498 cells were grown to subconfluency (30-50%) in 96-well plates and then transfected with 0.025 or 0.0125 µM of siRNA 1284 or the corresponding scrambled control sequence (1284S). Control samples (without siRNA) were also prepared. Transfection proceeded for 16 hours in the presence of Oligofectamine Reagent (Invitrogen) at which point siRNA sequences were removed and replaced with 100 µL α-MEM complete media for 48 hours. The XTT assay was then performed by adding 50 µL of XTT reagents to each well. Absorbance at 490 to 650 nm was monitored over time (2 to 4 hours). The XTT assay is a colorimetric assay that measures the ability of viable cells to metabolize tetrazolium salts to a formazan dye. The absorbance measured at 490 to 650 nm was determined for each treatment. Control samples (without siRNA) represented 100% viability and numbers produced from reading the absorbance for cells transfected with 1284 and scrambled siRNAs were then calculated as a percentage of control.

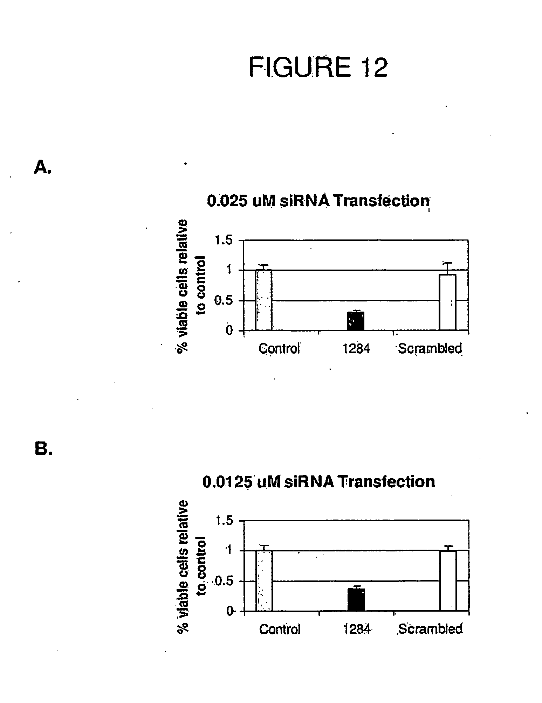

Treatment with 0.025 µM and 0.0125 µM 1284 reduced the metabolic activity of these cells to approximately 40% that of control or scrambled treated cells as shown in Figure 12A (0.025 µM) and Figure 12B (0.0125 µM). This difference in metabolic activity indicates that treatment with siRNA 1284 affected cell viability and/or cell proliferation.

### EXAMPLE 12: EFFECT of siRNAS ON CELL CYCLE PROGRESSION OF RENAL CARCINOMA CELLS IN VITRO

The effect of siRNA treatment on cell cycle progression of A-498 cells was investigated as follows. A-498 cells were grown to subconfluency (30-50%) and then transfected with 0.025 or 0.0125 µM of siRNA 1284 or with 0.2 µM siRNA 933 and their corresponding scrambled control sequences (1284S and 933S, respectively), in the presence of oligofectamine. Control samples (without siRNA and plus or minus oligofectamine) were also prepared. Transfection proceeded for 6 to 10 hours at which point siRNA sequences were removed and replaced with α-MEM complete media. At approximately 35 hours post-transfection cells were collected, fixed and then stained with propidium iodide for cell cycle analysis using the FACSCALIBUR cell analyzer.

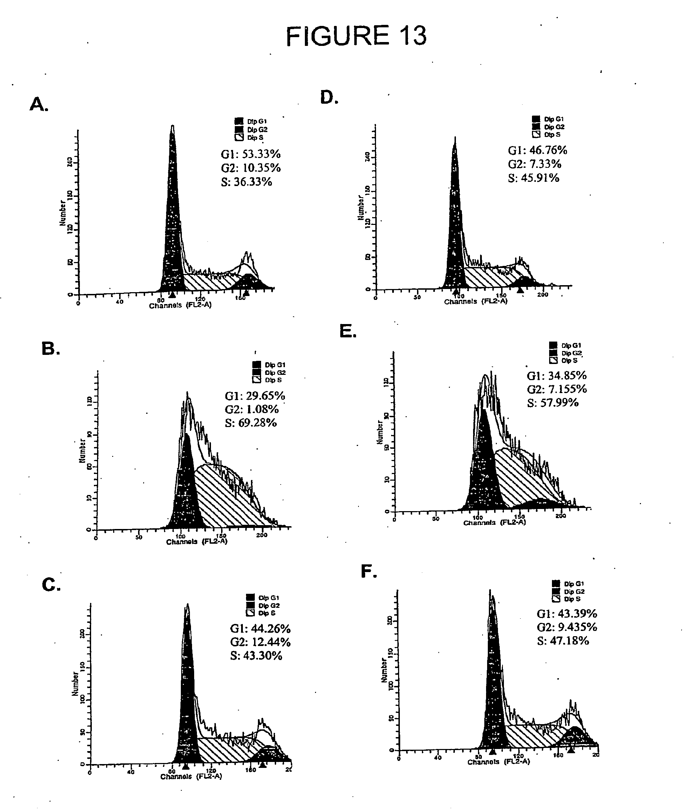

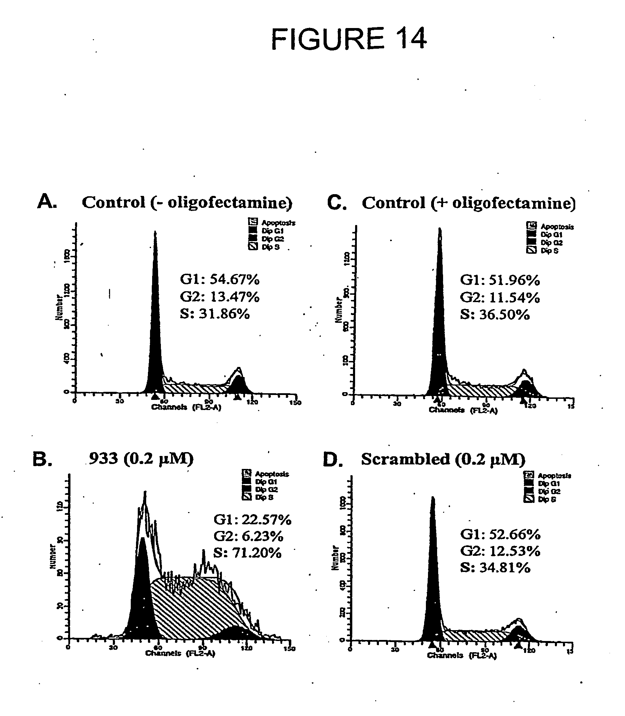

The results of this study are shown in Figures 13 and 14. Cell cycle analysis indicates that treatment of A-498 cells with 0.025 µM and 0.0125 µM of 1284 (Figure 13) produces a block in early S-phase. The percentage of cells in S-phase was found to increase to 69.28%and 57.99%when cells were treated with 0.025 µM and 0.0125 µM, respectively, of siRNA 1284, a significant increase from 43.3% and 47.18% occurring when cells were treated with 0.025 µM and 0.0125 µM, respectively, of the control 1284S (Figure 13). Cells transfected with siRNA 933 (and not the scrambled control 933S) were also blocked in S-phase (Figure 14).

### EXAMPLE 13: EFFECT OF siRNAS ON THE GROWTH OF COLON CANCER CELLS IN CD-1 NUDE MICE IN VIVO

The ability of siRNA 933 to inhibit the growth of human colon adenocarcinoma (HT-29) tumours in CD-1 nude mice was examined as follows. HT-29 cells were subcutaneously injected into the right flank of CD-1 nude mice. After the size of tumours reached an approximate volume of 60 to 70 mm³, approximately 5 days post-tumour cell injection, R2 siRNA 933 and the corresponding scrambled siRNA sequence (933S) was administered via the tail vein three times per week at 250 µg/kg. Control animals received saline alone, also three times per week. Treatment was administered for approximately five weeks and mean tumour volume was monitored each week. At the end of the experiment tumours were excised from the animals and mean tumour weight was determined for each group. The mean tumour weight was calculated from 10 animals per experimental group.

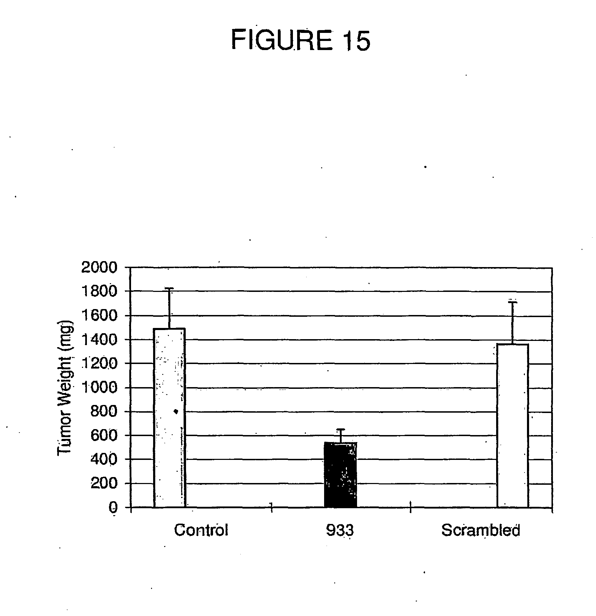

As shown in Figure 15, treatment with siRNA 933 inhibited HT-29 tumour growth in CD-1 nude mice. Inhibition of tumour growth was significant in comparison to control and scrambled treatment groups.

### EXAMPLE 14: EFFECT OF siRNAS ON THE GROWTH OF MELANOMA CANCER CELLS IN CD-1 NUDE MICE IN VIVO

The ability of siRNA 1284 to inhibit the growth of human melanoma (A2058) tumours in CD-1 nude mice was examined as follows. A2058 cells were subcutaneously injected into the right flank of CD-1 nude mice. After the size of tumours reached an approximate volume of 60 to 70 mm³, approximately 5 days post-tumour cell injection, R2 siRNA 1284 and the corresponding scrambled siRNA sequence (1284S) was administered via the tail vein three times per week at 250 µg/kg. Control animals received saline alone, also three times per week. Treatment was administered for approximately five weeks and mean tumour volume was monitored each week. At the end of the experiment tumours were excised from the animals and mean tumour weight was determined for each group. The mean tumour weight was calculated from 10 animals per experimental group.

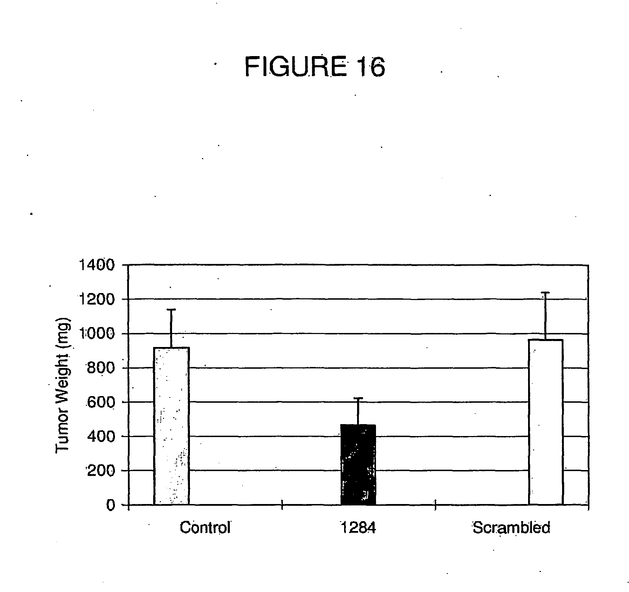

As shown in Figure 16, treatment with siRNA 1284 inhibited A2058 melanoma tumour growth in CD-1 nude mice. Inhibition of tumour growth was significant in comparison to control and scrambled treatment groups.

### EXAMPLE 15: EFFECT OF siRNAS ON THE GROWTH OF BREAST ADENOCARCINOMA CELLS IN VITRO

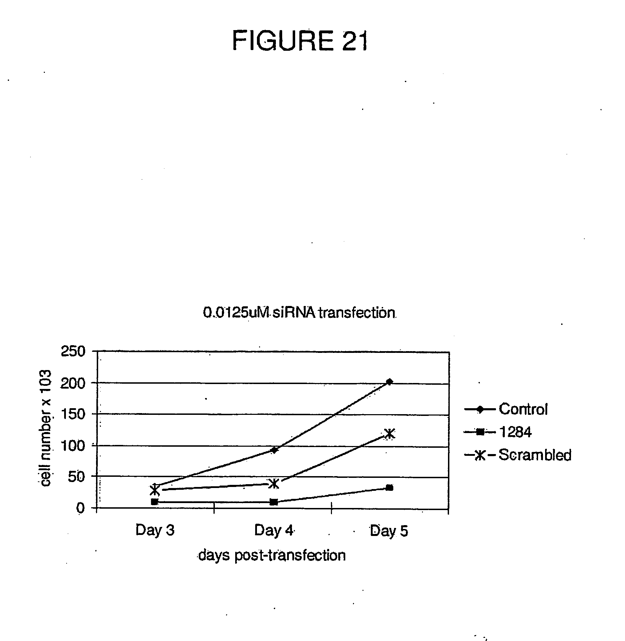

The effect of siRNA treatment on cellular proliferation of human breast cancer cell line (MDA-MB-231) was investigated as follows. MDA-MB-231 cells were grown to subconfluency (30-50%) and then transfected with 0.0125 µM of siRNA 1284 and the corresponding scrambled control sequence (1284S). Control samples (without siRNA) were also prepared. Transfection proceeded for 6 hours in the presence of Oligofectamine Reagent (Invitrogen^{™}) at which point siRNA sequences were removed and replaced with α-MEM complete media. At 3 days, 4 days and 5 days post-transfection cells were counted and cell numbers graphed as indicated in Figure 21.

Cell count results shown in Figure 21 indicate that at 0.0125 µM siRNA, a difference in cell numbers is observed between siRNA 1284 and 1284S indicating that treatment with siRNA 1284 inhibits cell proliferation. Protein samples were prepared for MDA-MB-231 cells transfected with 0.0125 µM siRNA 1284 and analyzed by Western blot. The concentration of siRNA 1284 tested was found to significantly down-regulate ribonucleotide reductase R2 protein levels.

### EXAMPLE 16: EFFECT OF siRNAS ON THE GROWTH OF BREAST ADENOCARCINOMA CELLS IN VITRO

The effect of siRNA treatment on cellular proliferation of human breast cancer cell line (MDA-MB-231) was investigated as follows. MDA-MB-231 cells were grown to subconfluency (30-50%) in 96-well plates and then transfected with 0.2 or 0.1 µM of siRNA 1284 and the corresponding scrambled control sequence (1284S). Control samples (without siRNA) were also prepared. Transfection proceeded for 6 hours in the presence of Oligofectamine Reagent (Invitrogen^{™}) at which point siRNA sequences were removed and replaced with 100 µl α-MEM complete media for 48 hours. The XTT assay was then performed by adding 50 µl of XTT reagents to each well. Absorbance at 490 to 650 nm was monitored over time (2 to 4 hours).

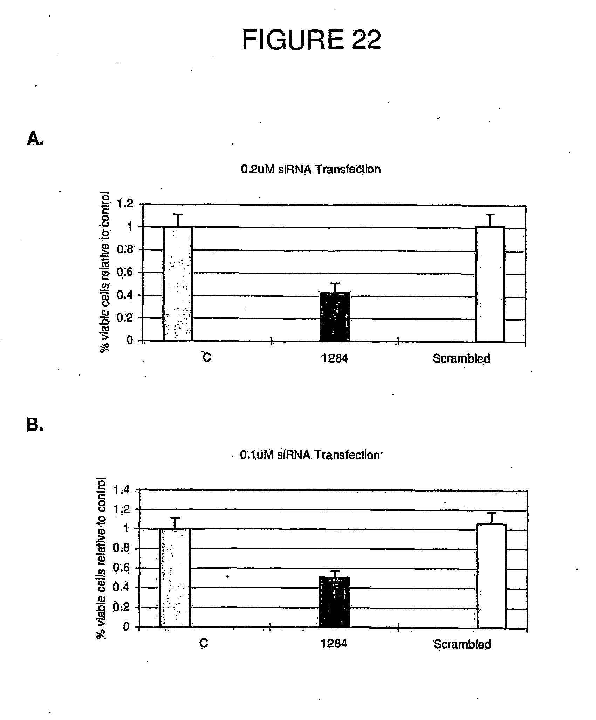

As described above, the XTT assay is a colorimetric assay that measures the ability of viable cells to metabolize tetrazolium salts to a formazan dye. The absorbance measured at 490 to 650 nm was determined for each treatment. Control samples (without siRNA) represented 100% viability and numbers produced from reading the absorbance for cells transfected with siRNA 1284 and scrambled siRNA (1284S) were then calculated as a percentage of control. Results of an XTT assay as shown in Figure 22 indicates that treatment with siRNA 1284 0.2 µM (Figure 22A) and 0.1 µM (Figure 22B) reduces the metabolic activity of these cells to approximately 45% that of control or scrambled treated cells. This difference in metabolic activity indicates that treatment with siRNA 1284 affects MDA-MB-231 cell viability and/or cell proliferation.

### EXAMPLE 17: EFFECT OF siRNAS ON LEVELS OF RIBONUCLEOTIDE REDUCTASE R2 mRNA AND PROTEIN IN CANCER CELLS

The effects of siRNA treatment on ribonucleotide reductase R2 mRNA and protein levels in human renal carcinoma cell line (A-498), human breast adenocarcinoma cell line (MDA-MB-231), human colon adenocarcinoma cell line (HT-29) and human melanoma cell line (A-2058) were investigated as follows. A-498 cells, MDA-MB-231, HT-29 and A-2058 cells were grown to subconfluency (30-50%) and then transfected with 0.2 µM of siRNAs 1284 and scrambled control 1284S. Transfection proceeded for 28 hours in the presence of Oligofectamine Reagent (invitrogen^{™}) prior to the isolation of RNA from cells using TRIzol^{™} reagent (GIBCO BRL) and Northern blot analysis. RNA was subjected to electrophoresis through 1% agarose gels followed by transfer to nylon membranes and hybridization to a fragment of ribonucleotide reductase R2 labelled with α³²P-dCTP. For Western blot analysis, protein samples were subjected to electrophoresis through 10% SDS-polyacrylamide gels followed by transfer to nitrocellulose membranes. Immunodetection was performed by incubating the membrane with anti-R2 primary antibody, anti-goat IgG horseradish peroxidase linked secondary antibody and ECL detection reagents.

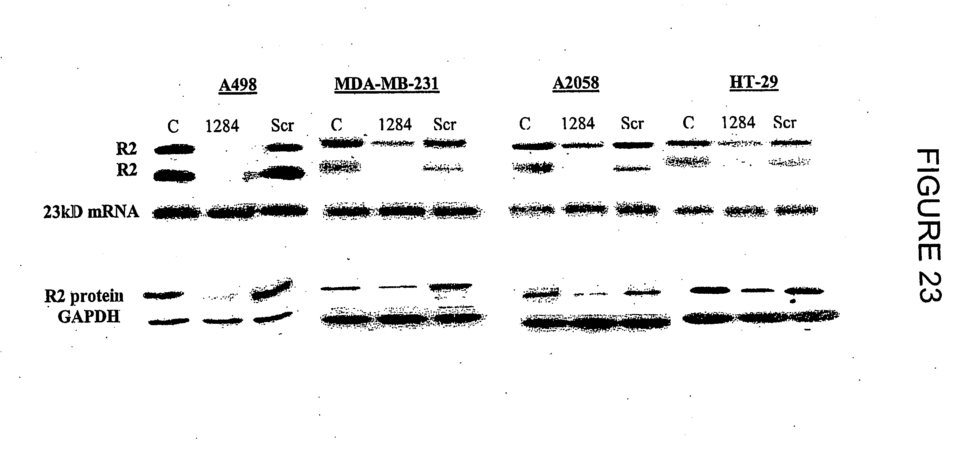

Northern and Western blot analysis indicated that transfection of A-498, MDA-MB-231, HT-29 and A2058 cells with siRNA 1284 specifically reduces ribonucleotide reductase R2 mRNA and protein levels as shown in Figure 23, in comparison to cells treated with Oligofectamine alone (control - C) or those cells treated with scrambled sequences (S). 23kD and GAPDH serve as loading controls for Northern and Western blots-respectively.

## Claims

1. An isolated siRNA molecule of between about 14 and about 200 nucleotides in length, wherein the term "about" refers to a +/-10% variation from the nominal value, comprising: an antisense nucleotide sequence complementary to a region of a human ribonucleotide reductase R2 mRNA; and a sense nucleotide sequence complementary to said antisense nucleotide sequence, wherein said isolated siRNA molecule inhibits expression of said ribonucleotide reductase R2 mRNA and inhibits neoplastic cell proliferation, and wherein said region of the human ribonucleotide reductase R2 mRNA is within any one of exons 4, 6, 7 or 10.

2. The isolated siRNA molecule according to claim 1, wherein said isolated siRNA molecule is between about 14 and about 50 nucleotides in length.

3. The isolated siRNA molecule according to claim 1, wherein said isolated siRNA molecule is between about 40 and about 200 nucleotides in length.

4. The isolated siRNA molecule according to claim 1 or 2, wherein said isolated siRNA molecule is a double-stranded RNA molecule comprising said antisense nucleotide sequence and said sense nucleotide sequence as separate oligonucleotides.

5. The isolated siRNA molecule according to any one of claims 1, 2 or 3, wherein said antisense nucleotide sequence and said sense nucleotide sequence are comprised by a single oligonucleotide.

6. The isolated siRNA molecule according to any one of claims 1, 2, 3 or 5, wherein said siRNA molecule is a shRNA molecule or a circular siRNA molecule.

7. The isolated siRNA molecule according to any one of claims 1, 2, 3, 4, 5 or 6, wherein said antisense nucleotide sequence comprises at least 14 consecutive nucleotides of any one of the sequences as set forth in SEQ ID NOs: 348, 349, 354 to 360 and 370 to 411 or complementary to any one of the sequences as set forth in SEQ ID NOs: 126 to 130, 142 to 144, 147 to 150 and 161 to 203.

8. The isolated siRNA molecule according to any one of claims 1, 2, 3, 4, 5 or 6, wherein said antisense nucleotide sequence comprises at least 14 consecutive nucleotides complementary to any one of the sequences as set forth in SEQ ID NOs: 126 to 130, 142 to 144, 147 to 150 and 161 to 203.

9. The isolated siRNA molecule according to any one of claims 1, 2, 3, 4, 5 or 6, wherein said antisense nucleotide sequence comprises at least 14 consecutive nucleotides complementary to any one of the sequences as set forth in SEQ ID NOs: 127, 143, 149 and 162.

10. The isolated siRNA molecule according to any one af claims 1, 2, 3, 4, 5 or 6, wherein said antisense nucleotide sequence and said sense nucleotide sequence are:
(a) a sequence comprising SEQ ID NO: 423 and a sequence comprising SEQ ID NO: 422;
(b) a sequence comprising SEQ ID NO: 429 and a sequence comprising SEQ ID NO: 428;
(c) a sequence comprising SEQ ID NO: 433 and a sequence comprising SEQ ID NO: 432, or
(d) a sequence comprising SEQ ID NO: 435 and a sequence comprising SEQ ID NO: 434.

11. The isolated siRNA molecule according to any one of claims 1, 2, 3, 4, 5, 6, 7, 8, 9 or 10, wherein said isolated siRNA molecule comprises one or more modified ribonucleotides.

12. A DNA sequence encoding the isolated siRNA molecule according to any one of claims 1, 2, 3, 4, 5, 6, 7, 8, 9 or 10, operatively linked to one or more regulatory control regions.

13. A vector comprising the DNA sequence according to claim 12.

14. A pharmaceutical composition comprising the siRNA molecule according to any one of claims 1, 2, 3, 4, 5, 6, 7, 8, 9, 10 or 11 and a pharmaceutically acceptable carrier.

15. A pharmaceutical composition comprising the DNA sequence according to claim 12 and a pharmaceutically acceptable carrier.

16. A pharmaceutical composition comprising the vector according to claim 13 and a pharmaceutically acceptable carrier.

17. An isolated siRNA molecule of between about 14 and about 200 nucleotides in length, wherein the term "about" refers to a +/-10% variation from the nominal value, comprising: an antisense nucleotide sequence complementary to a region of a human ribonucleotide reductase R2 mRNA; and a sense nucleotide sequence complementary to said antisense nucleotide sequence, for use in inhibiting neoplastic cell proliferation in a mammal in need of such therapy, wherein said isolated siRNA molecule inhibits expression of said ribonucleotide reductase R2 mRNA and inhibits neoplastic cell proliferation, and wherein said region of the human ribonucleotide reductase R2 mRNA is within any one of exons 4, 6, 7 or 10.

18. An isolated siRNA molecule of between about 14 and about 200 nucleotides in length, wherein the term "about" refers to a +/-10% variation from the nominal value, comprising: an antisense nucleotide sequence complementary to a region of a human ribonucleotide reductase R2 mRNA; and a sense nucleotide sequence complementary to said antisense nucleotide sequence, for use in inhibiting tumour growth in a mammal in need of such therapy, wherein said isolated siRNA molecule inhibits expression of said ribonucleotide reductase R2 mRNA and inhibits neoplastic cell proliferation, and wherein said region of the human ribonucleotide reductase R2 mRNA is within any one of exons 4, 6, 7 or 10.

19. The isolated siRNA molecule according to claim 18, wherein said tumour is a solid tumour.

20. The isolated siRNA molecule according to any one of claims 17, 18 or 19,
wherein said isolated siRNA molecule is between about 14 and about 50 nucleotides in length.

21. The isolated siRNA molecule according to any one of claims 17, 18 or 19,
wherein said isolated siRNA molecule is between about 40 and about 200 nucleotides in length.

22. The isolated siRNA molecule according to any one of claims 17, 18, 19 or 20,
wherein said isolated siRNA molecule is a double-stranded RNA molecule comprising said antisense nucleotide sequence and said sense nucleotide sequence as separate oligonucleotides.

23. The isolated siRNA molecule according to any one of claims 17, 18, 19, 20 or 21,
wherein said antisense nucleotide sequence and said sense nucleotide sequence are comprised by a single oligonucleotide.

24. The isolated siRNA molecule according to any one of claims 17, 18, 19, 20, 21 or 23, wherein said siRNA molecule is a shRNA molecule or a circular siRNA molecule.

25. The isolated siRNA molecule according to any one of claims 17, 18, 19, 20, 21, 22, 23 or 24, wherein said antisense nucleotide sequence comprises at least 14 consecutive nucleotides of any one of the sequences as set forth in SEQ ID NOs: 348, 349, 354 to 360 and 370 to 411 or complementary to any one of the sequences as set forth in SEQ ID NOs: 126 to 130, 142 to 144, 147 to 150 and 161 to 203.

26. The isolated siRNA molecule according to any one of claims 17, 18, 19, 20, 21, 22, 23 or 24, wherein said antisense nucleotide sequence comprises at least 14 consecutive nucleotides complementary to any one of the sequences as set forth in SEQ ID NOs: 126 to 130, 142 to 144, 147 to 150 and 161 to 20.3.

27. The isolated siRNA molecule according to any one of claims 17, 18, 19, 20, 21, 22, 23 or 24, wherein said antisense nucleotide sequence comprises at least 14 consecutive nucleotides complementary to any one of the sequences as set forth in SEQ ID NOs: 127, 143, 149 and 162.

28. The isolated siRNA molecule according to any one of claims 17, 18, 19, 20, 21, 22, 23 or 24, wherein said antisense nucleotide sequence and said sense nucleotide sequence are:
(a) a sequence comprising SEQ ID NO: 423 and a sequence comprising SEQ ID NO: 422;
(b) a sequence comprising SEQ ID NO: 429 and a sequence comprising SEQ ID NO: 428;
(c) a sequence comprising SEQ ID NO: 433 and a sequence comprising SEQ ID NO: 432, or
(d) a sequence comprising SEQ ID NO: 435 and a sequence comprising SEQ ID NO: 434.

29. The isolated siRNA molecule according to any one of claims 17, 18, 19, 20, 21, 22, 23, 24, 25, 26, 27 or 28, wherein said isolated siRNA molecule comprises one or more modified ribonucleotides.

30. The isolated siRNA molecule according to any one of claims 17, 18, 19, 20, 21, 22, 23, 24, 25, 26, 27, 28 or 29, wherein said use is in combination with one or more anti-cancer therapeutics.

31. The vector according to claim 13, for use in inhibiting neoplastic cell proliferation in a mammal in need of such therapy.

32. The vector according to claim 13, for use in inhibiting tumour growth in a mammal in need of such therapy.

33. The vector according to claim 31 or 32, wherein said use is in combination with one or more anti-cancer therapeutics.

34. Use of the isolated siRNA molecule according to any one of claims 1, 2, 3, 4, 5, 6, 7, 8, 9, 10 or 11 in the manufacture of a medicament.

35. The use according to claim 34, wherein said medicament is for inhibiting neoplastic cell proliferation.

36. The use according to claim 34, wherein said medicament is for inhibiting tumour growth.

37. Use of the vector according to claim 13, in the manufacture of a medicament.

38. The use according to claim 37, wherein said medicament is for inhibiting neoplastic cell proliferation.

39. The use according to claim 37, wherein said medicament is for inhibiting tumour growth.

## Patentansprüche

1. Isoliertes siRNA-Molekül mit einer Länge von zwischen etwa 14 und etwa 200 Nukleotiden, wobei der Begriff "etwa" sich auf eine +/-10 % Abweichung vom Nennwert bezieht, umfassend: eine Antisense-Nukleotidsequenz, die komplementär zu einer Region der menschlichen Ribonukleotidreduktase R2-mRNA ist; und eine Sense-Nukleotidsequenz, die zu der Antisense-Nukleotidsequenz komplementär ist, wobei das isolierte siRNA-Molekül die Expression der Ribonukleotidreduktase R2 mRNA hemmt und neoplastische Zellproliferation hemmt, und wobei die Region der menschlichen Ribonukleotidreduktase R2 mRNA innerhalb eines der Exons 4, 6, 7 oder 10 liegt.

2. Isoliertes siRNA-Molekül nach Anspruch 1, wobei das isolierte siRNA-Molekül eine Länge von zwischen etwa 14 und etwa 50 Nukleotiden aufweist.

3. Isoliertes siRNA-Molekül nach Anspruch 1, wobei das isolierte siRNA-Molekül eine Länge von zwischen etwa 40 und etwa 200 Nukleotiden aufweist.

4. Isoliertes siRNA-Molekül nach Anspruch 1 oder 2, wobei das isolierte siRNA-Molekül ein doppelsträngiges RNA-Molekül ist, das die Antisense-Nukleotidsequenz und die Sense-Nukleotidsequenz als separate Oligonukleotide umfasst.

5. Isoliertes siRNA-Molekül nach einem der Ansprüche 1, 2 oder 3, wobei die Antisense-Nukleotidsequenz und die Sense-Nukleotidsequenz in einem einzigen Oligonukleotid enthalten sind.

6. Isoliertes siRNA-Molekül nach einem der Ansprüche 1, 2, 3 oder 5, wobei das siRNA-Molekül ein shRNA-Molekül oder ein ringförmiges siRNA-Molekül ist.

7. Isoliertes siRNA-Molekül nach einem der Ansprüche 1, 2, 3, 4, 5 oder 6, wobei die Antisense-Nukleotidsequenz mindestens 14 aufeinanderfolgende Nukleotide mit einer der Sequenzen von SEQ ID NRN 348, 349, 354 bis 360 und 370 bis 411 oder die komplementär zu einer der Sequenzen von SEQ ID NRN 126 bis 130, 142 bis 144, 147 bis 150 und 161 bis 203 sind, umfasst.

8. Isoliertes siRNA-Molekül nach einem der Ansprüche 1, 2, 3, 4, 5 oder 6, wobei die Antisense-Nukleotidsequenz mindestens 14 aufeinanderfolgende Nukleotide, die komplementär zu einer der Sequenzen von SEQ ID NRN 126 bis 130, 142 bis 144, 147 bis 150 und 161 bis 203 sind, umfasst.

9. Isoliertes siRNA-Molekül nach einem der Ansprüche 1, 2, 3, 4, 5 oder 6, wobei die Antisense-Nukleotidsequenz mindestens 14 aufeinanderfolgende Nukleotide, die komplementär zu einer der Sequenzen von SEQ ID NRN 127, 143, 149 und 162 sind, umfasst.

10. Isoliertes siRNA-Molekül nach einem der Ansprüche 1, 2, 3, 4, 5 oder 6, wobei die Antisense-Nukleotidsequenz und die Sense-Nukleotidsequenz:
(a) eine Sequenz, die SEQ ID NR 423 umfasst, und eine Sequenz, die SEQ ID NR 422 umfasst;
(b) eine Sequenz, die SEQ ID NR 429 umfasst, und eine Sequenz, die SEQ ID NO 428 umfasst;
(c) eine Sequenz, die SEQ ID NR 433 umfasst, und eine Sequenz, die SEQ ID NO 432 umfasst, oder
(d) eine Sequenz, die SEQ ID NR 435 umfasst, und eine Sequenz, die SEQ ID NO 434 umfasst,
sind.

11. Isoliertes siRNA-Molekül nach einem der Ansprüche 1, 2, 3, 4, 5, 6, 7, 8, 9 oder 10, wobei das isolierte siRNA-Molekül ein oder mehrere modifizierte Ribonukleotide umfasst.

12. DNA-Sequenz, die für das isolierte siRNA-Molekül nach einem der Ansprüche 1, 2, 3, 4, 5, 6, 7, 8, 9 oder 10 kodiert, und die operativ mit einer oder mehreren Regulationsregionen verbunden ist.

13. Vektor, der die DNA-Sequenz nach Anspruch 12 umfasst.

14. Pharmazeutische Zusammensetzung, die das siRNA-Molekül nach einem der Ansprüche 1, 2, 3, 4, 5, 6, 7, 8, 9, 10 oder 11 und einen pharmazeutisch annehmbaren Träger umfasst.

15. Pharmazeutische Zusammensetzung, die die DNA-Sequenz nach Anspruch 12 und einen pharmazeutisch annehmbaren Träger umfasst.

16. Pharmazeutische Zusammensetzung, die den Vektor nach Anspruch 13 und einem pharmazeutisch annehmbaren Träger umfasst.

17. Isoliertes siRNA-Molekül mit einer Länge von zwischen etwa 14 und etwa 200 Nukleotiden, wobei der Begriff "etwa" sich auf eine +/-10 % Abweichung vom Nennwert bezieht, umfassend: eine Antisense-Nukleotidsequenz, die komplementär zu einer Region der menschlichen Ribonukleotidreduktase R2-mRNA ist; und eine Sense-Nukleotidsequenz, die zu der Antisense-Nukleotidsequenz komplementär ist, zur Verwendung zum Hemmen neoplastischer Zellproliferation bei einem Säuger, der einer solchen Therapie bedarf, wobei das isolierte siRNA-Molekül die Expression der Ribonukleotidreduktase R2 mRNA hemmt und neoplastische Zellproliferation hemmt, und wobei die Region der menschlichen Ribonukleotidreduktase R2 mRNA innerhalb eines der Exons 4, 6, 7 oder 10 liegt.

18. Isoliertes siRNA-Molekül mit einer Länge von zwischen etwa 14 und etwa 200 Nukleotiden, wobei der Begriff "etwa" sich auf eine +/-10 Abweichung vom Nennwert bezieht, umfassend: eine Antisense-Nukleotidsequenz, die komplementär zu einer Region der menschlichen Ribonukleotidreduktase R2-mRNA ist; und eine Sense-Nukleotidsequenz, die zu der Antisense-Nukleotidsequenz komplementär ist, zur Verwendung zum Hemmen von Tumorwachstum bei einem Säuger, der einer solchen Therapie bedarf, wobei das isolierte siRNA-Molekül die Expression der Ribonukleotidreduktase R2 mRNA hemmt und neoplastische Zellproliferation hemmt, und wobei die Region der menschlichen Ribonukleotidreduktase R2 mRNA innerhalb eines der Exons 4, 6, 7 oder 10 liegt.

19. Isoliertes siRNA-Molekül nach Anspruch 18, wobei der Tumor ein solider Tumor ist.

20. Isoliertes siRNA-Molekül nach einem der Ansprüche 17, 18 oder 19, wobei das isolierte siRNA-Molekül eine Länge von zwischen etwa 14 und etwa 50 Nukleotiden aufweist.

21. Isoliertes siRNA-Molekül nach einem der Ansprüche 17, 18 oder 19, wobei das isolierte siRNA-Molekül eine Länge von zwischen etwa 40 und etwa 200 Nukleotiden aufweist.

22. Isoliertes siRNA-Molekül nach Anspruch 17, 18, 19 oder 20, wobei das isolierte siRNA-Molekül ein Doppelstrang-RNA-Molekül ist, das die Antisense-Nukleotidsequenz und die Sense-Nukleotidsequenz als separate Oligonukleotide umfasst.

23. Isoliertes siRNA-Molekül nach einem der Ansprüche 17, 18, 19, 20 oder 21, wobei die Antisense-Nukleotidsequenz und die Sense-Nukleotidsequenz in einem einzigen Oligonukleotid enthalten sind.

24. Isoliertes siRNA-Molekül nach einem der Ansprüche 17, 18, 19, 20, 21 oder 23, wobei das siRNA-Molekül ein shRNA-Molekül oder ein ringförmiges siRNA-Molekül ist.

25. Isoliertes siRNA-Molekül nach einem der Ansprüche 17, 18, 19, 20, 21, 22, 23 oder 24, wobei die Antisense-Nukleotidsequenz mindestens 14 aufeinanderfolgende Nukleotide von einer der Sequenzen von SEQ ID NRN 348, 349, 354 bis 360 und 370 bis 411 oder die komplementär zu einer der Sequenzen von SEQ ID NRN 126 bis 130, 142 bis 144, 147 bis 150 und 161 bis 203 sind, umfasst.

26. Isoliertes siRNA-Molekül nach einem der Ansprüche 17, 18, 19, 20, 21, 22, 23 oder 24, wobei die Antisense-Nukleotidsequenz mindestens 14 aufeinanderfolgende Nukleotide, die komplementär zu einer der Sequenzen von SEQ ID NRN 126 bis 130, 142 bis 144, 147 bis 150 und 161 bis 203 sind, umfasst.

27. Isoliertes siRNA-Molekül nach einem der Ansprüche 17, 18, 19, 20, 21, 22, 23 oder 24, wobei die Antisense-Nukleotidsequenz mindestens 14 aufeinanderfolgende Nukleotide, die komplementär zu einer der Sequenzen von SEQ ID NRN 127, 143, 149 und 162 sind, umfasst.

28. Isoliertes siRNA-Molekül nach einem der Ansprüche 17, 18, 19, 20, 21, 22, 23 oder 24, wobei die Antisense-Nukleotidsequenz und die Sense-Nukleotidsequenz:
(a) eine Sequenz, die SEQ ID NR 423 umfasst, und eine Sequenz, die SEQ ID NR 422 umfasst;
(b) eine Sequenz, die SEQ ID NR 429 umfasst, und eine Sequenz, die SEQ ID NO 428 umfasst;
(c) eine Sequenz, die SEQ ID NR 433 umfasst, und eine Sequenz, die SEQ ID NO 432 umfasst, oder
(d) eine Sequenz, die SEQ ID NR 435 umfasst, und eine Sequenz die SEQ ID NO 434 umfasst,
sind.

29. Isoliertes siRNA-Molekül nach einem der Ansprüche 17, 18, 19, 20, 21, 22, 23, 24, 25, 26, 27 oder 28, wobei das isolierte siRNA-Molekül ein oder mehrere modifizierte Ribonukleotide umfasst.

30. Isoliertes siRNA-Molekül nach einem der Ansprüche 17, 18, 19, 20, 21, 22, 23, 24, 25, 26, 27, 28 oder 29, wobei die Verwendung in Kombination mit einem oder mehreren Anti-Krebs-Therapeutika erfolgt.

31. Vektor nach Anspruch 13 zur Verwendung beim Hemmen neoplastischer Zellproliferation bei einem Säuger, der einer solchen Therapie bedarf.

32. Vektor nach Anspruch 13 zur Verwendung beim Hemmen des Tumorwachstums bei einem Säuger, der einer solchen Therapie bedarf.

33. Vektor nach Anspruch 31 oder 32, wobei die Verwendung in Kombination mit einem oder mehreren Anti-Krebs-Therapeutika erfolgt.

34. Verwendung des isolierten siRNA-Moleküls nach einem der Ansprüche 1, 2, 3, 4, 5, 6, 7, 8, 9, 10 oder 11 in der Herstellung eines Medikaments.

35. Verwendung nach Anspruch 34, wobei das Medikament zum Hemmen der neoplastischen Zellproliferation dient.

36. Verwendung nach Anspruch 34, wobei das Medikament zum Hemmen des Tumorwachstums dient.

37. Verwenden des Vektors nach Anspruch 13 bei der Herstellung eines Medikaments.

38. Verwendung nach Anspruch 37, wobei das Medikament zum Hemmen neoplastischer Zellproliferation dient.

39. Verwendung nach Anspruch 37, wobei das Medikament zum Hemmen des Tumorwachstums dient.

## Revendications

1. Molécule d'ARNsi isolée ayant une longueur entre environ 14 et environ 200 nucléotides, dans lequel le terme « environ » se rapporte à une variation de +/-10% de la valeur nominale, comprenant : une séquence nucléotidique antisens complémentaire à une région de l'ARNm de la ribonucléotide réductase R2 humaine ; et une séquence nucléotidique sens complémentaire à ladite séquence nucléotidique antisens, dans lequel ladite molécule d'ARNsi isolée inhibe l'expression dudit ARNm de la ribonucléotide réductase R2 et inhibe la prolifération cellulaire néoplasique, et dans lequel ladite région de l'ARNm de la ribonucléotide réductase R2 humaine se trouve dans l'un des exons 4, 6, 7 ou 10.

2. Molécule d'ARNsi isolée selon la revendication 1, dans laquelle ladite molécule d'ARNsi isolée a une longueur entre environ 14 à environ 50 nucléotides.

3. Molécule d'ARNsi isolée selon la revendication 1, dans laquelle ladite molécule d'ARNsi isolée a une longueur entre environ 40 à environ 200 nucléotides.

4. Molécule d'ARNsi isolée selon les revendications 1 et 2, dans laquelle ladite molécule d'ARNsi isolée est une molécule d'ARN double brin comprenant ladite séquence nucléotidique antisens et ladite séquence nucléotidique sens sous forme d'oligonucléotides distincts.

5. Molécule d'ARNsi isolée selon l'une quelconque des revendications 1, 2 ou 3, dans laquelle ladite séquence nucléotidique antisens et ladite séquence nucléotidique sens sont composées d'un oligonucléotide unique.

6. Molécule d'ARNsi isolée selon l'une quelconque des revendications 1, 2, 3 ou 5, dans laquelle ladite molécule d'ARNsi est une molécule d'ARNsh ou une molécule d'ARNsi circulaire.

7. Molécule d'ARNsi isolée selon l'une quelconque des revendications 1, 2, 3, 4, 5 ou 6, dans laquelle ladite séquence nucléotidique antisens comprend au moins 14 nucléotides consécutifs de l'au moins une des séquences décrite dans SEQ ID Nos : 348, 349, 354 à 360 et 370 à 411 ou complémentaire à l'une quelconque des séquences décrites dans SEQ ID Nos : 126 à 130, 142 à 144, 147 à 150 et 161 à 203.

8. Molécule d'ARNsi isolée selon l'une quelconque des revendications 1, 2, 3, 4, 5 ou 6, dans laquelle ladite séquence nucléotidique antisens comprend au moins 14 nucléotides consécutifs complémentaires à l'une quelconque des séquences décrites dans SEQ ID Nos : 126 à 130, 142 à 144, 147 à 150 et 161 à 203.

9. Molécule d'ARNsi isolée selon l'une quelconque des revendications 1, 2, 3, 4, 5 ou 6, dans laquelle ladite séquence nucléotidique antisens comprend au moins 14 nucléotides consécutifs complémentaires à l'une quelconque des séquences décrites dans SEQ ID Nos : 127, 143, 149 et 162.

10. Molécule d'ARNsi isolée selon l'une quelconque des revendications 1, 2, 3, 4, 5 ou 6, dans laquelle ladite séquence nucléotidique antisens et ladite séquence nucléotidique sens sont :
a) une séquence comprenant SEQ ID NO : 423 et une séquence comprenant SEQ ID NO : 422 ;
b) une séquence comprenant SEQ ID NO : 429 et une séquence comprenant SEQ ID NO : 428 ;
c) une séquence comprenant SEQ ID NO : 433 et une séquence comprenant SEQ ID NO : 432, ou
d) une séquence comprenant SEQ ID NO : 435 et une séquence comprenant SEQ ID NO : 434.

11. Molécule d'ARNsi isolée selon l'une quelconque des revendications 1, 2, 3, 4, 5, 6, 7, 8, 9 ou 10, dans laquelle ladite molécule d'ARNsi isolée comprend un ou plusieurs ribonucléotides modifiés.

12. Séquence d'ADN codant pour la molécule d'ARNsi isolée selon l'une quelconque des revendications 1, 2, 3, 4, 5, 6, 7, 8, 9 ou 10, liée de façon fonctionnelle à une ou plusieurs régions de contrôle régulatrices.

13. Vecteur contenant la séquence d'ADN selon la revendication 12.

14. Composition pharmaceutique contenant la molécule d'ARNsi isolée selon l'une quelconque des revendications 1, 2, 3, 4, 5, 6, 7, 8, 9, 10 ou 11 et un support phannaceutiquement acceptable.

15. Composition pharmaceutique contenant la séquence d'ADN selon la revendication 12 et un support pharmaceutiquement acceptable.

16. Composition pharmaceutique contenant le vecteur selon la revendication 13 et un support pharmaceutiquement acceptable.

17. Molécule d'ARNsi isolée ayant une longueur entre environ 14 et environ 200 nucléotides, dans lequel le terme « environ » se rapporte à une variation de +/-10% de la valeur nominale, comprenant : une séquence nucléotidique antisens complémentaire à une région de l'ARNm de la ribonucléotide réductase R2 humaine ; et une séquence nucléotidique sens complémentaire à ladite séquence nucléotidique antisens, devant être utilisée dans l'inhibition de la prolifération cellulaire néoplasique chez un mammifère ayant besoin d'une telle thérapie, dans laquelle ladite molécule d'ARNsi isolée inhibe l'expression dudit ARNm de la ribonucléotide réductase R2 et inhibe la prolifération cellulaire néoplasique, et dans laquelle ladite région de l'ARNm de la ribonucléotide réductase R2 humaine se trouve dans l'un quelconque des exons 4, 6, 7 ou 10.

18. Molécule d'ARNsi isolée ayant une longueur entre environ 14 et environ 200 nucléotides, dans lequel le terme « environ » se rapporte à une variation de +/-10% de la valeur nominale, comprenant : une séquence nucléotidique antisens complémentaire à une région de l'ARNm de la ribonucléotide réductase R2 humaine ; et une séquence nucléotidique sens complémentaire à ladite séquence nucléotidique antisens, devant être utilisé dans l'inhibition de la croissance tumorale chez un mammifère ayant besoin d'une telle thérapie, dans laquelle ladite molécule d'ARNsi isolée inhibe l'expression dudit ARNm de la ribonucléotide réductase R2 et inhibe la prolifération cellulaire néoplasique, et dans laquelle ladite région de l'ARNm de la ribonucléotide réductase R2 humaine se trouve dans l'un quelconque des exons 4, 6, 7 ou 10.

19. Molécule d'ARNsi isolée selon la revendication 18, dans laquelle ladite tumeur est une tumeur solide.

20. Molécule d'ARNsi isolée selon l'une quelconque des revendications 17, 18 ou 19, dans laquelle ladite molécule d'ARNsi isolée a une longueur entre environ 14 à environ 50 nucléotides.

21. Molécule d'ARNsi isolée selon l'une quelconque des revendications 17, 18 ou 19, dans laquelle ladite molécule d'ARNsi isolée a une longueur entre environ 40 à environ 200 nucléotides.

22. Molécule d'ARNsi isolée selon les revendications 17, 18, 19 ou 20, dans laquelle ladite molécule d'ARNsi isolée est une molécule d'ARN double brin comprenant ladite séquence nucléotidique antisens et ladite séquence nucléotidique sens sous forme d'oligonucléotides distincts.

23. Molécule d'ARNsi isolée selon l'une quelconque des revendications 17, 18, 19, 20 ou 21, dans laquelle ladite séquence nucléotidique antisens et ladite séquence nucléotidique sens sont composées d'un oligonucléotide unique.

24. Molécule d'ARNsi isolée selon l'une quelconque des revendications 17, 18, 19, 20, 21 ou 23, dans laquelle ladite molécule d'ARNsi est une molécule d'ARNsh ou une molécule d'ARNsi circulaire.

25. Molécule d'ARNsi isolée selon l'une quelconque des revendications 17, 18, 19, 20, 21, 22, 23 ou 24, dans laquelle ladite séquence nucléotidique antisens comprend au moins 14 nucléotides consécutifs de l'une quelconque des séquences décrites dans SEQ ID Nos : 348, 349, 354 à 360 et 370 à 411 ou complémentaire à l'une quelconque des séquences décrites dans SEQ ID Nos : 126 à 130, 142 à 144, 147 à 150 et 161 à 203.

26. Molécule d'ARNsi isolée selon l'une quelconque des revendications 17, 18, 19, 20, 21, 22, 23 ou 24, dans laquelle ladite séquence nucléotidique antisens comprend au moins 14 nucléotides consécutifs complémentaires à l'une quelconque des séquences décrites dans SEQ ID Nos : 126 à 130, 142 à 144, 147 à 150 et 161 à 203.

27. Molécule d'ARNsi isolée selon l'une quelconque des revendications 17, 18, 19, 20, 21, 22, 23 ou 24, dans laquelle ladite séquence nucléotidique antisens comprend au moins 14 nucléotides consécutifs complémentaires de l'une quelconque des séquences décrites dans SEQ ID Nos : 127, 143, 149 et 162.

28. Molécule d'ARNsi isolée selon l'une quelconque des revendications 17, 18, 19, 20, 21, 22, 23 ou 24, dans laquelle ladite séquence nucléotidique antisens et ladite séquence nucléotidique sens sont :
a. une séquence comprenant SEQ ID NO : 423 et une séquence comprenant SEQ ID NO : 422 ;
b. une séquence comprenant SEQ ID NO : 429 et une séquence comprenant SEQ ID NO : 428 ;
c. une séquence comprenant SEQ ID NO : 433 et une séquence comprenant SEQ ID NO : 432, ou
d. une séquence comprenant SEQ ID NO : 435 et une séquence comprenant SEQ ID NO : 434

29. Molécule d'ARNsi isolée selon l'une quelconque des revendications 17, 18, 19, 20, 21, 22, 23, 24, 25, 26, 27 ou 28, dans laquelle la molécule d'ARNsi isolée comprend un ou plusieurs ribonucléotides modifiés.

30. Molécule d'ARNsi isolée selon l'une quelconque des revendications 17, 18, 19, 20, 21, 22, 23, 24, 25, 26, 27, 28 ou 29, dans laquelle ladite utilisation est en association avec un ou plusieurs agents thérapeutiques anti-cancéreux.

31. Vecteur selon la revendication 13, devant être utilisé pour l'inhibition de la prolifération cellulaire néoplasique chez un mammifère ayant besoin d'une telle thérapie.

32. Vecteur selon la revendication 13, devant être utilisé pour l'inhibition la croissance tumorale chez un mammifère ayant besoin d'une telle thérapie.

33. Vecteur selon la revendication 31 ou 32, dans lequel ladite utilisation est en association avec un ou plusieurs agents thérapeutiques anti-cancéreux.

34. Utilisation de la molécule d'ARNsi isolée selon l'une quelconque des revendications 1, 2, 3, 4, 5, 5, 6, 7, 8, 9, 10 ou 11 pour la préparation d'un médicament.

35. Utilisation selon la revendication 34, dans laquelle le dit médicament est destiné à l'inhibition de la prolifération cellulaire néoplasique.

36. Utilisation selon la revendication 34, dans laquelle ledit médicament est destiné à l'inhibition de la croissance tumorale.

37. Utilisation du vecteur selon la revendication 13, pour la préparation d'un médicament.

38. Utilisation selon la revendication 37, dans laquelle ledit médicament est destiné à l'inhibition de la prolifération cellulaire néoplasique.

39. Utilisation selon la revendication 37, dans laquelle ledit médicament est destiné à l'inhibition de la croissance tumorale.
